# EUROPEAN PATENT SPECIFICATION

(11) **EP 2 552 893 B1**
(45) Date of publication and mention of the grant of the patent: **29.10.2014**
(21) Application number: 11720867.8
(22) Date of filing: 30.03.2011
(51) Int. Cl.: C07D 217/22, C07D 401/04, C07D 405/04, C07D 409/04, C07D 471/04, A61K 31/472, A61K 31/4725, A61P 31/04

(54) **ANTIBACTERIAL ISOQUINOLIN-3-YLUREA DERIVATIVES**
ANTIBAKTERIELLE ISOCHINOLIN-3-YLUREA-DERIVATE
DÉRIVÉS D'ISOQUINOLÉIN-3-YLURÉE ANTIBACTÉRIENS

(30) Priority: 07.01.2011 WO PCT/IB2011/050065; 31.03.2010 WO PCT/IB2010/051406
(43) Date of publication of application: 06.02.2013
(73) Proprietor: Actelion Pharmaceuticals Ltd., 4123 Allschwil (CH)
(72) Inventor: BUR, Daniel, CH-4123 Allschwil (CH); GUDE, Markus, CH-4123 Allschwil (CH); HUBSCHWERLEN, Christian, CH-4123 Allschwil (CH); PANCHAUD, Philippe, CH-4123 Allschwil (CH)
(74) Representative: Ruhlmann, Eric
(86) International application number: PCT/IB2011/051362
(87) International publication number: WO 2011/121555

(56) References cited:
- WO-A1-2009/074812

## Description

The present invention concerns novel isoquinolin-3-ylurea derivatives, a pharmaceutical antibacterial composition containing them and the use of these compounds in the manufacture of a medicament for the treatment of infections (e.g. bacterial infections). These compounds are useful antimicrobial agents effective against a variety of human and veterinary pathogens including among others Gram-positive and Gram-negative aerobic and anaerobic bacteria and mycobacteria.

The intensive use of antibiotics has exerted a selective evolutionary pressure on microorganisms to produce genetically based resistance mechanisms. Modem medicine and socio-economic behaviour exacerbates the problem of resistance development by creating slow growth situations for pathogenic microbes, e.g. in artificial joints, and by supporting long-term host reservoirs, e.g. in immuno-compromised patients.

An increasing number of strains of *Staphylococcus aureus, Streptococcus pneumoniae, Enterococcus spp.,* and *Pseudomonas aeruginosa,* major sources of infections, are becoming multi-drug resistant and therefore difficult if not impossible to treat:
- *S. aureus* is resistant to ß-lactams, quinolones and now even to vancomycin;
- *S. pneumoniae* is becoming resistant to penicillin or quinolone antibiotics and even to new macrolides;
- *Enteroccocci* are quinolone and vancomycin resistant and ß-lactam antibiotics are inefficacious against these strains;
- *Enterobacteriacea* are cephalosporin and quinolone resistant;
- *P. aeruginosa* are ß-lactam and quinolone resistant.

There is also an increasing number of cases of resistance in upper respiratory tract infections caused by fastidious Gram negative pathogens such as *H. influenzae* and *M. catarrhalis.* Further resistant strains of *S. aureus* have spread out of the clinical settings into the community.

Therefore, there is a high medical need for new antibacterial agents harbouring a novel mechanism of action and/or containing new pharmacophoric groups and covering these pathogenic strains.

In addition, microorganisms that are causing persistent infections are increasingly being recognized as causative agents or cofactors of severe chronic diseases like peptic ulcers or heart diseases.

WO 02/060879, WO 2003/105846, WO 2005/089763, WO 2006/038116, WO 2007/056330, WO 2007/148093, WO 2009/074810, WO 2009/074812, WO 2009/147431, WO 2009/156966 and US 2010/0063069 disclose antibacterial benzimidazole and benzothiazole derivatives and their corresponding azaisosteres wherein the alkyl urea is attached to the 5-membered heteroaromatic ring.

WO 2009/027732 and WO 2009/027733 disclose antibacterial benzimidazole derivatives and their corresponding azaisosteres wherein the alkyl urea is attached to the 6-membered heteroaromatic ring.

WO 2008/068470, WO 2009/106885, WO 2009/147433. WO 2009/147440, WO 2010/136817, WO 2010/142978 and WO 2011/024004 disclose pyridine, pyrimidine and thiazole urea derivatives as antibacterial compounds.

1-(isoquinolin-3-yl)-3-(aryl)urea or 1-(isoquinolin-3-yl)-3-(heteroaryl)urea derivatives have been disclosed for example in WO 01/07411, WO 02/062763, WO 2004/078747, WO 2006/049941, US 2006/0025415 or WO 2007/004749.

Moreover, 1-(isoquinolin-3-yl)-3-(alkyl)urea derivatives have been disclosed generically (among many other types of compounds) in US 2004/009931, WO 2007/051408, WO 2007/125405, WO 2008/082487 or WO 2009/155121. Nevertheless, there is no concrete example of any 1-(isoquinolin-3-yl)-3-(alkyl)urea in these documents.

The Applicants have now found particular isoquinolin-3-ylurea antibiotic derivatives corresponding to the formula I described hereafter.

Various embodiments of the invention are presented hereafter:
1) The invention firstly relates to compounds of formula I 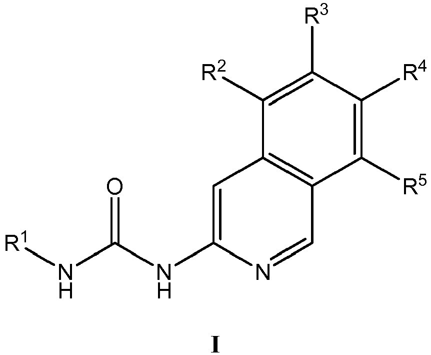 wherein
   R¹ represents (C₁-C₃)alkyl, (C₂-C₃)haloalkyl or cyclopropyl;
   R⁴ represents H;
   R³ represents H, R⁵ represents H and R² represents phenyl or 4-carboxyphenyl; or
   R² represents H, R⁵ represents H and R³ represents benzoylamino; or
   R² represents H, R³ represents H and R⁵ represents -NH-CO-R⁶, -CH₂-O-R⁷, -NH-R⁸, -CH₂-NH-R⁹, -CH₂-R¹⁰, (pyridin-3-ylmethyl)amino, pyridin-3-yloxy or 4-carboxyphenyl; or
   R³ represents H, R⁵ represents methyl and R² represents halogen, (C₁-C₃)alkylamino, (C₁-C₃)alkylaminomethyl, 2-(aminocarbonyl)-ethyl, pyrimidin-4-yl, pyridazin-3-yl, pyridazin-4-yl, 1*H*-imidazol-4-yl, 1-methyl-1*H*-imidazol-4-yl, 1*H*-pyrazol-4-yl, 1-methyl-1*H-*pyrazol-4-yl, imidazo[1,2-*a*]pyridin-6-yl, 4-methyl-thiophen-3-yl, thiazol-4-yl, isoquinolin-5-yl or 1*H-*indol-4-yl or a group having the formula (A1), (A2) or (A3) shown hereafter 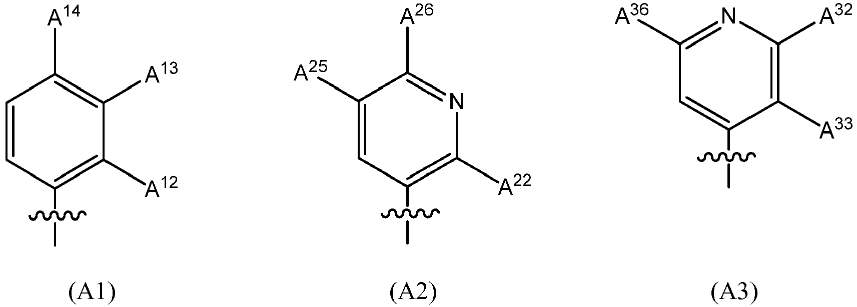
   wherein
   each of A¹³ and A¹⁴ represents H and A¹² represents H or OH, or
   each of A¹² and A¹⁴ represents H and A¹³ represents OH, acetylamino, aminomethyl, sulfamoyl or hydroxymethyl, or
   each of A¹² and A¹³ represents H and A¹⁴ represents OH, methylaminocarbonyl, dimethylaminocarbonyl, methoxycarbonylamino, sulfamoyl or hydroxymethyl;
   each of A²⁵ and A²⁶ represents H and A²² represents H or halogen, or
   each of A²² and A²⁶ represents H and A²⁵ represents methyl or halogen, or
   each of A²² and A²⁵ represents H and A²⁶ represents hydroxymethyl;
   each of A³³ and A³⁶ represents H and A³² represents H, halogen, amino, methyl or methoxy, or
   A³³ represents H and each of A³² and A³⁶ represents methyl; or
   R² represents H, R⁵ represents methyl and R³ represents amino, vinyl, (C₁-C₄)alkylamino, cyclopropylmethylamino, (2-(benzyloxy)ethyl)amino, methoxymethylcarbonylamino, (thiazol-5-yl)carbonylamino, pyridin-3-ylcarbonylamino, pyridin-4-ylcarbonylamino, naphthalen-2-yl, furan-2-yl, furan-3-yl, 1*H*-pyrazol-4-yl, thiophen-3-yl, 4-methyl-thiophen-3-yl, quinolin-3-yl, isoquinolin-4-yl, isoquinolin-5-yl, imidazo[1,2-*a*]pyridin-6-yl, 1*H-*indol-4-yl or a group having the formula (B1), (B2), (B3), (B4), (B5), (B6), (B7), (B8), (B9), (B10), (B11) or (B12) shown hereafter 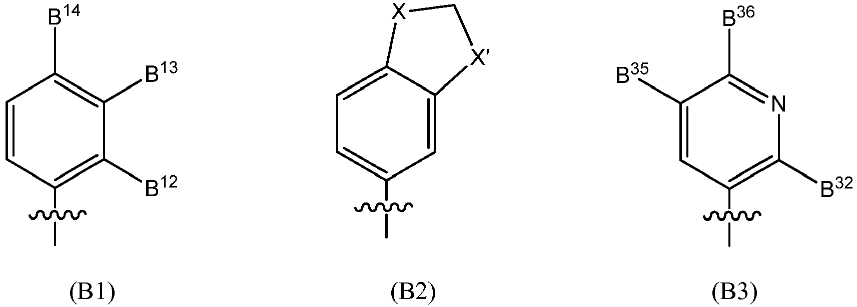 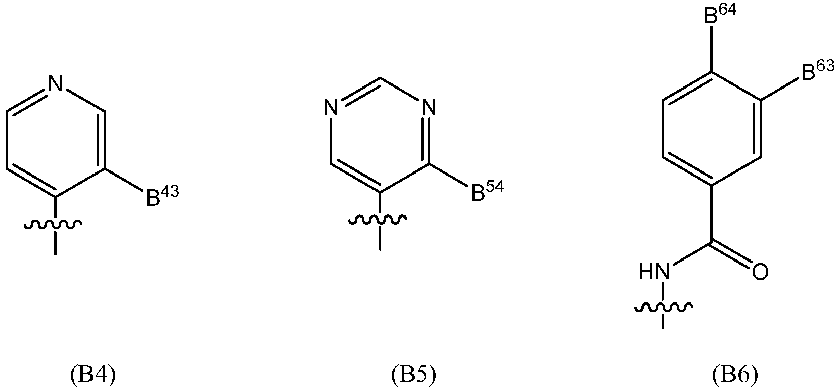 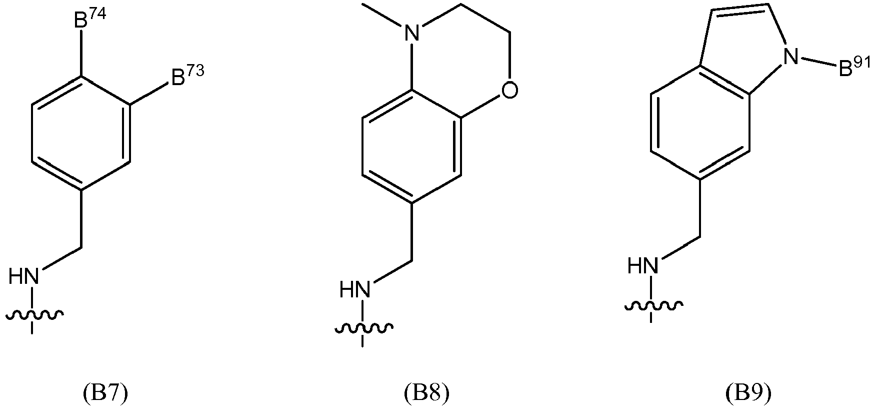 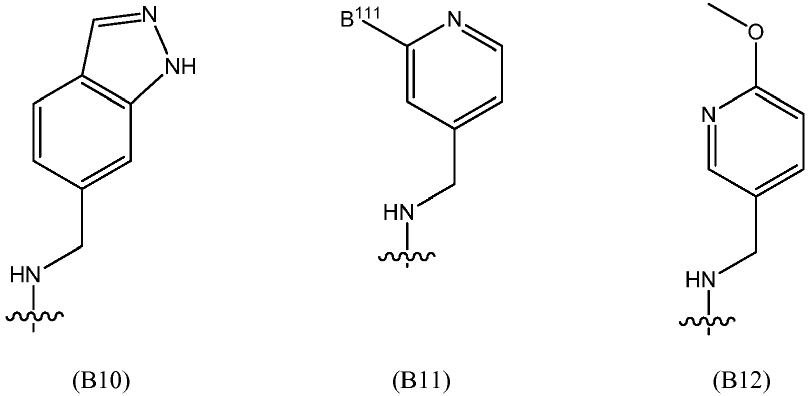
   wherein
   each of B¹³ and B¹⁴ represents H and B¹² represents OH, methyl, acetylamino, (C₁-C₂)alkoxycarbonyl or dimethylaminocarbonyl, or
   each of B¹² and B¹⁴ represents H and B¹³ represents OH, halogen, acetyl, acetylamino, acetylaminomethyl, aminocarbonyl, sulfamoyl, (C₁-C₂)alkoxy, (C₁-C₂)alkoxycarbonyl, cyano, amino-(C₁-C₂)alkyl or hydroxy-(C₁-C₂)alkyl, or
   each of B¹² and B¹³ represents H and B¹⁴ represents acetyl, acetylamino, acetylaminomethyl, aminocarbonyl, dimethylaminocarbonyl, sulfamoyl, (C₁-C₂)alkylsulfonyl, cyanomethyl or hydroxymethyl;
   X and X' each represent -O- or one of X and X' represents -O- and the other represents -CH₂-;
   each of B³⁵ and B³⁶ represents H and B³² represents H or halogen, or
   each of B³² and B³⁶ represents H and B³⁵ represents halogen, methyl, methylsulfanyl, methanesulfonyl or methoxycarbonyl, or
   each of B³² and B³⁵ represents H and B³⁶ represents halogen, methoxy or hydroxymethyl; B⁴³ represents H or halogen;
   B⁵⁴ represents H or (C₁-C₃)alkyl;
   B⁶⁴ represents H and B⁶³ represents H, methoxycarbonyl or dimethylamino, or
   B⁶³ represents H and B⁶⁴ represents methoxy, methoxycarbonyl, dimethylamino or methanesulfonyl;
   B⁷⁴ represents H and B⁷³ represents H, methoxy, methoxycarbonyl, carboxy, 2-hydroxyethoxy, 1*H*-1,2,4-triazol-1-yl or 1*H-*1,2,4-triazol-1-ylmethyl, or
   B⁷³ represents H and B⁷⁴ represents carboxy, acetylamino, ((aminocarbonyl)methyl)oxy, *N*-methylsulfamoyl or 3-methyl-1*H*-1,2,4-triazol-1-yl;
   B⁹¹ represents H or methyl;
   B¹¹¹ represents H or morpholino; or
   R² represents H, R⁵ represents pyridin-3-ylamino and R³ represents propylamino, 3-acetylamino-phenyl, benzylamino, 5-methylpyridin-3-yl or 3-(*N*,*N*-dimethylamino)phenylcarbonylamino; or
   R² represents fluorine, R³ represents H and R⁵ represents bromine, benzyl, benzoylamino, 4-carboxyphenyl, benzylamino, pyridin-3-yl or (pyridin-3-ylmethyl)amino; or R² represents bromine, R³ represents H and R⁵ represents methoxy; or
   R² represents fluorine, R⁵ represents methyl and R³ represents quinolin-3-yl, benzylamino, a phenyl group optionally substituted once by acetylamino or sulfamoyl, pyridin-2-yl, or a pyridin-3-yl group optionally substituted once by methyl; or
   R² represents fluorine, R³ represents pyridin-3-yl and R⁵ represents pyridin-3-yl or 4-carboxyphenyl; or
   R² represents fluorine, R³ represents benzylamino and R⁵ represents chlorine or 2-methoxy-pyrimidin-5-yl; or
   R² represents fluorine, R³ represents quinolin-3-yl and R⁵ represents quinolin-3-yl; or
   R² represents fluorine, R³ represents 3-(acetylamino)phenyl and R⁵ represents pyridin-3-yl, pyridin-3-ylamino or 2-methoxypyrimidin-5-yl; or
   R² represents fluorine, R³ represents 4-sulfamoyl-phenyl and R⁵ represents chlorine; or
   R² represents fluorine, R⁵ represents pyridin-3-ylamino and R³ represents benzylamino or 5-methylpyridin-3-yl; or
   R³ represents H, R² represents (C₁-C₃)alkylaminomethyl, *N*,*N*-dimethylaminomethyl, pyridin-3-yl, pyridin-4-yl, 2-methylpyridin-4-yl, 2-methoxypyridin-4-yl, 2-aminopyridin-4-yl, 2,6-dimethylpyridin-4-yl, pyrimidin-4-yl, 1*H*-imidazol-4-yl, 1*H*-pyrazol-4-yl, thiazol-4-yl, pyridin-3-ylamino, 3-(aminomethyl)phenyl, 4-hydroxyphenyl, 4-(hydroxymethyl)phenyl, 4-sulfamoyl-phenyl, morpholino, morpholinomethyl, 4-methylpiperazin-1-yl or 4-methyl-piperazin-1-ylmethyl and R⁵ represents chlorine, vinyl, methoxy, (3-carboxyphenyl)amino, (3-(methoxycarbonyl)phenyl)amino, (3-(dimethylcarbamoyl)phenyl)amino, (3-(methylcarbamoyl)phenyl)amino, pyridin-4-yl, (6-methyl-pyridin-2-yl)amino, (6-methoxy-pyridin-2-yl)amino, pyridin-3-ylamino, (6-methyl-pyridin-3-yl)amino or pyridin-3-yloxy;
   R⁶ represents phenyl, 1*H*-pyrrol-2-yl or 1*H*-1,2,3-triazol-5-yl;
   R⁷ represents pyridin-2-yl, 5-methyl-pyridin-2-yl, 2-methyl-pyridin-3-yl, 6-methyl-pyridin-3-yl, pyridin-4-yl, 3-methoxycarbonyl-pyridin-4-yl, 2-(methoxycarbonyl)phenyl, 3-hydroxy-phenyl or 3-amino-phenyl;
   R⁸ represents pyridin-2-yl, 6-methoxy-pyridin-2-yl, 5-methoxy-pyridin-2-yl, 4-methoxy-pyridin-2-yl, 3-methyl-pyridin-2-yl, 4-methyl-pyridin-2-yl, 5-methyl-pyridin-2-yl, 6-methyl-pyridin-2-yl, 5-aminocarbonyl-pyridin-2-yl, 4-fluoro-pyridin-2-yl, 5-methoxycarbonyl-pyridin-2-yl, 6-methoxycarbonyl-pyridin-2-yl, pyridin-3-yl, 5-fluoro-pyridin-3-yl, 2-methoxypyridin-3-yl, 5-methoxy-pyridin-3-yl, 6-methoxy-pyridin-3-yl, 6-acetylamino-pyridin-3-yl, 6-methyl-pyridin-3-yl, pyridin-4-yl, 2-methoxy-pyridin-4-yl, 3-fluoro-pyridin-4-yl, pyrazin-2-yl, pyrimidin-2-yl, pyrimidin-4-yl, pyrimidin-5-yl, phenyl, 3-hydroxyphenyl, 3-methoxyphenyl, 3-carboxyphenyl, 3-carbamoylphenyl, 3-acetylamino-phenyl, 3-sulfamoylphenyl, 3-methoxycarbonyl-phenyl, 4-methyl-3-methoxycarbonyl-phenyl, 4-methyl-3-carboxyphenyl, 3-(carboxymethyl)phenyl, 3-(methylcarbamoyl)phenyl, 3-(dimethylcarbamoyl)phenyl, 3-(benzylcarbamoyl)phenyl, 3-(2-amino-2-oxoethyl)phenyl, 3-(2-(dimethylamino)-2-oxoethyl)phenyl, 3-(2-(benzylamino)-2-oxoethyl)phenyl, 4-methoxycarbonyl-phenyl, 3-methyl-4-methoxycarbonyl-phenyl, 4-carboxyphenyl, 3-methyl-4-carboxyphenyl, 3-carbamoyl-4-methoxyphenyl, 4-methoxy-3-(methylcarbamoyl)phenyl, 3-(dimethylcarbamoyl)-4-methoxyphenyl, 4-hydroxyphenyl, 4-(carboxymethyl)phenyl, 4-cyanophenyl, 4-acetylamino-phenyl, 4-carbamoylphenyl, 4-(methylcarbamoyl)phenyl, 4-(dimethylcarbamoyl)phenyl, 4-(2-amino-2-oxoethyl)phenyl, 4-(2-(dimethylamino)-2-oxoethyl)phenyl, 4-(2-(methylamino)-2-oxoethyl)phenyl, 4-(2-(benzylamino)-2-oxoethyl)phenyl, 5-methyl-1,3,4-oxadiazol-2-yl or imidazo[1,2-*a*]pyridin-7-yl;
   R⁹ represents pyridin-2-yl, pyridin-3-yl, 6-methoxypyridin-3-yl, pyrimidin-2-yl, 1,3-dimethyl-1*H*-pyrazol-5-yl, 1-methyl-1*H*-pyrazol-5-yl, 5-methylisoxazol-3-yl or 3-methylisothiazol-5-yl;
   R¹⁰ represents 1*H*-imidazol-1-yl, 4-(hydroxymethyl)-1*H*-imidazol-1-yl, 5-(hydroxymethyl)-1*H*-imidazol-1-yl, 2-carboxy-1*H*-imidazol-1-yl, 2,4-dimethyl-1*H*-imidazol-1-yl, 2,5-dimethyl-1*H*-imidazol-1-yl, 2-methyl-1*H*-imidazol-1-yl, 1*H*-1,2,3-triazol-1-yl, 1*H*-pyrazol-1-yl, 4-methyl-1*H*-pyrazol-1-yl or 4-(hydroxymethyl)-1*H-*pyrazol-1-yl;
   and to salts (in particular pharmaceutically acceptable salts) of compounds of formula I.
   The following paragraphs provide definitions of the various chemical moieties for the compounds according to the invention and are intended to apply uniformly throughout the specification and claims, unless an otherwise expressly set out definition provides a broader or narrower definition:
   ❖ The term "alkyl", used alone or in combination, refers to a saturated straight or branched chain alkyl group containing from one to four carbon atoms. Representative examples of alkyl groups include methyl, ethyl, propyl, *iso*-propyl, *n*-butyl, *iso*-butyl, *sec*-butyl and *tert*-butyl. The term "(Cₓ-C_{y})alkyl" (x and y each being an integer) refers to an alkyl group as defined before containing x to y carbon atoms.
   ❖ The term "alkoxy", used alone or in combination, refers to a straight or branched chain alkoxy group containing from one to four carbon atoms. The term "(Cₓ-C_{y})alkoxy" (x and y each being an integer) refers to an alkoxy group as defined before containing x to y carbon atoms. For example, a (C₁-C₃)alkoxy group contains from one to three carbon atoms. Representative examples of alkoxy groups include methoxy, ethoxy, n-propoxy and *iso*-propoxy. Preferred are methoxy and ethoxy.
   ❖ The term "halogen" refers to fluorine, chlorine, bromine or iodine, preferably to fluorine or chlorine.
   ❖ The term "haloalkyl", used alone or in combination, refers to a saturated straight or branched chain alkyl group containing from one to four carbon atoms wherein at least one hydrogen atom has been replaced by a halogen atom. Representative examples of haloalkyl groups include trifluoromethyl and 2-fluoro-ethyl. The term "(Cₓ-C_{y})haloalkyl" (x and y each being an integer) refers to a haloalkyl group as defined before containing x to y carbon atoms.
   ❖ In this patent application, a bond interrupted by a wavy line shows the point of attachment of the radical drawn. For example, the radical drawn below 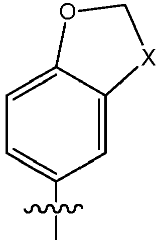
   wherein X represents -CH₂- is the 2,3-dihydrobenzofuran-5-yl group.
   The term "pharmaceutically acceptable salts" refers to non-toxic, inorganic or organic acid and/or base addition salts. Reference can be made to "Salt selection for basic drugs", Int. J. Pharm. (1986), 33, 201-217.
   Besides, the term "room temperature" as used herein refers to a temperature of 20 to 30°C, and preferably 25°C.
   Unless used regarding temperatures, the term "about" placed before a numerical value "X" refers in the current application to an interval extending from X minus 10% of X to X plus 10% of X, and preferably to an interval extending from X minus 5% of X to X plus 5% of X. In the particular case of temperatures, the term "about" placed before a temperature "Y" refers in the current application to an interval extending from the temperature Y minus 10°C to Y plus 10°C, and preferably to an interval extending from Y minus 5°C to Y plus 5°C.
2) The invention in particular relates to compounds of formula I according to embodiment 1) that are also compounds of formula I_{P2} 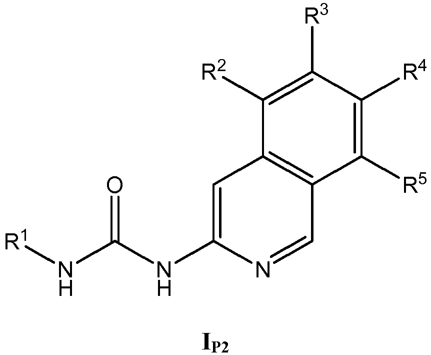 wherein
   R¹ represents (C₁-C₃)alkyl, (C₂-C₃)haloalkyl or cyclopropyl;
   R⁴ represents H;
   R³ represents H, R⁵ represents H and R² represents phenyl or 4-carboxyphenyl; or
   R² represents H, R⁵ represents H and R³ represents benzoylamino; or
   R² represents H, R³ represents H and R⁵ represents -NH-CO-R⁶, -CH₂-O-R⁷, -NH-R⁸, -CH₂-NH-R⁹, (pyridin-3-ylmethyl)amino, pyridin-3-yloxy or 4-carboxyphenyl; or
   R³ represents H, R⁵ represents methyl and R² represents halogen, (C₁-C₃)alkylamino, 2-(aminocarbonyl)-ethyl, imidazo[1,2-*a*]pyridin-6-yl, 4-methyl-thiophen-3-yl, isoquinolin-5-yl or 1*H*-indol-4-yl or a group having the formula (A1), (A2) or (A3) shown hereafter 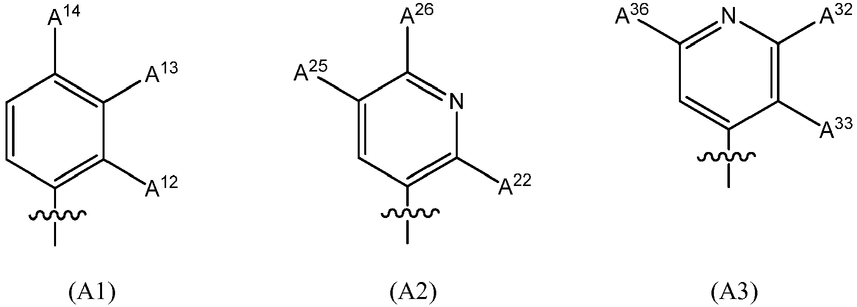
   wherein
   each of A¹³ and A¹⁴ represents H and A¹² represents H or OH, or
   each of A¹² and A¹⁴ represents H and A¹³ represents OH, acetylamino, aminomethyl, sulfamoyl or hydroxymethyl, or
   each of A¹² and A¹³ represents H and A¹⁴ represents OH, methylaminocarbonyl, dimethylaminocarbonyl, methoxycarbonylamino, sulfamoyl or hydroxymethyl;
   each of A²⁵ and A²⁶ represents H and A²² represents H or halogen, or
   each of A²² and A²⁶ represents H and A²⁵ represents methyl or halogen, or
   each of A²² and A²⁵ represents H and A²⁶ represents hydroxymethyl;
   each of A³³ and A³⁶ represents H and A³² represents H, halogen, amino, methyl or methoxy, or
   A³³ represents H and each of A³² and A³⁶ represents methyl; or
   R² represents H, R⁵ represents methyl and R³ represents amino, vinyl, (C₁-C₄)alkylamino, cyclopropylmethylamino, (2-(benzyloxy)ethyl)amino, methoxymethylcarbonylamino, (thiazol-5-yl)carbonylamino, pyridin-3-ylcarbonylamino, pyridin-4-ylcarbonylamino, naphthalen-2-yl, furan-2-yl, furan-3-yl, 1*H*-pyrazol-4-yl, thiophen-3-yl, 4-methyl-thiophen-3-yl, quinolin-3-yl, isoquinolin-4-yl, isoquinolin-5-yl, imidazo[1,2-*a*]pyridin-6-yl, 1*H*-indol-4-yl or a group having the formula (B1), (B2), (B3), (B4), (B5), (B6), (B7), (B8), (B9), (B10), (B11) or (B12) shown hereafter 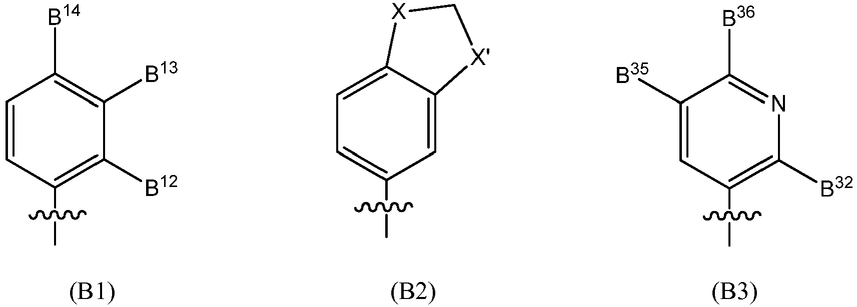 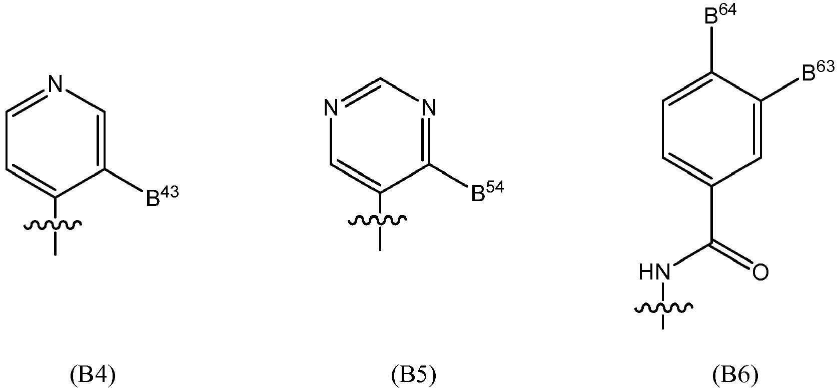 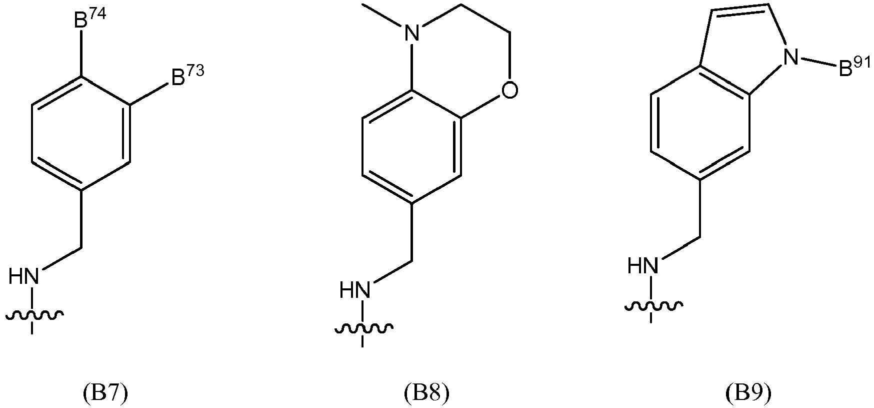 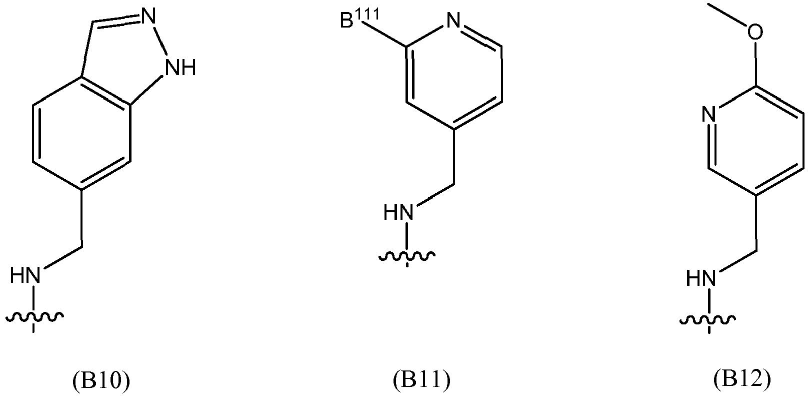
   wherein
   each of B¹³ and B¹⁴ represents H and B¹² represents OH, methyl, acetylamino, (C₁-C₂)alkoxycarbonyl or dimethylaminocarbonyl, or
   each of B¹² and B¹⁴ represents H and B¹³ represents OH, halogen, acetyl, acetylamino, acetylaminomethyl, aminocarbonyl, sulfamoyl, (C₁-C₂)alkoxy, (C₁-C₂)alkoxycarbonyl, cyano, amino-(C₁-C₂)alkyl or hydroxy-(C₁-C₂)alkyl, or
   each of B¹² and B¹³ represents H and B¹⁴ represents acetyl, acetylamino, acetylaminomethyl, aminocarbonyl, dimethylaminocarbonyl, sulfamoyl, (C₁-C₂)alkylsulfonyl, cyanomethyl or hydroxymethyl;
   X and X' each represent -O- or one of X and X' represents -O- and the other represents -CH₂-;
   each of B³⁵ and B³⁶ represents H and B³² represents H or halogen, or
   each of B³² and B³⁶ represents H and B³⁵ represents halogen, methyl, methylsulfanyl, methanesulfonyl or methoxycarbonyl, or
   each of B³² and B³⁵ represents H and B³⁶ represents halogen, methoxy or hydroxymethyl;
   B⁴³ represents H or halogen;
   B⁵⁴ represents H or (C₁-C₃)alkyl;
   B⁶⁴ represents H and B⁶³ represents H, methoxycarbonyl or dimethylamino, or
   B⁶³ represents H and B⁶⁴ represents methoxy, methoxycarbonyl, dimethylamino or methanesulfonyl;
   B⁷⁴ represents H and B⁷³ represents H, methoxy, methoxycarbonyl, carboxy, 2-hydroxyethoxy, 1*H*-1,2,4-triazol-1-yl or 1*H*-1,2,4-triazol-1-ylmethyl, or
   B⁷³ represents H and B⁷⁴ represents carboxy, acetylamino, ((aminocarbonyl)methyl)oxy, *N*-methylsulfamoyl or 3-methyl-1*H*-1,2,4-triazol-1-yl;
   B⁹¹ represents H or methyl;
   B¹¹¹ represents H or morpholino; or
   R² represents H, R⁵ represents pyridin-3-ylamino and R³ represents propylamino, 3-acetylamino-phenyl, benzylamino, 5-methylpyridin-3-yl or 3-(*N*,*N*-dimethylamino)phenylcarbonylamino; or
   R² represents fluorine, R³ represents H and R⁵ represents bromine, benzyl, benzoylamino, 4-carboxyphenyl, benzylamino, pyridin-3-yl or (pyridin-3-ylmethyl)amino; or
   R² represents bromine, R³ represents H and R⁵ represents methoxy; or
   R² represents fluorine, R⁵ represents methyl and R³ represents quinolin-3-yl, benzylamino, a phenyl group optionally substituted once by acetylamino or sulfamoyl, pyridin-2-yl, or a pyridin-3-yl group optionally substituted once by methyl; or
   R² represents fluorine, R³ represents pyridin-3-yl and R⁵ represents pyridin-3-yl or 4-carboxyphenyl; or
   R² represents fluorine, R³ represents benzylamino and R⁵ represents chlorine or 2-methoxy-pyrimidin-5-yl; or
   R² represents fluorine, R³ represents quinolin-3-yl and R⁵ represents quinolin-3-yl; or
   R² represents fluorine, R³ represents 3-(acetylamino)phenyl and R⁵ represents pyridin-3-yl, pyridin-3-ylamino or 2-methoxypyrimidin-5-yl; or
   R² represents fluorine, R³ represents 4-sulfamoyl-phenyl and R⁵ represents chlorine; or
   R² represents fluorine, R⁵ represents pyridin-3-ylamino and R³ represents benzylamino or 5-methylpyridin-3-yl; or
   R³ represents H, R² represents pyridin-3-yl, pyridin-4-yl, 2-methylpyridin-4-yl, 2-methoxypyridin-4-yl, 2-aminopyridin-4-yl, 2,6-dimethylpyridin-4-yl, 3-(aminomethyl)phenyl, 4-hydroxyphenyl, 4-(hydroxymethyl)phenyl or 4-sulfamoyl-phenyl and R⁵ represents vinyl, methoxy, (3-carboxyphenyl)amino, (3-(methoxycarbonyl)phenyl)amino, (3-(dimethylcarbamoyl)phenyl)amino, (3-(methylcarbamoyl)phenyl)amino, pyridin-4-yl, (6-methyl-pyridin-2-yl)amino, (6-methoxy-pyridin-2-yl)amino, pyridin-3-ylamino, (6-methyl-pyridin-3-yl)amino or pyridin-3-yloxy;
   R⁶ represents phenyl, 1*H*-pyrrol-2-yl or 1*H*-1,2,3-triazol-5-yl;
   R⁷ represents pyridin-2-yl, 5-methyl-pyridin-2-yl, 2-methyl-pyridin-3-yl, 6-methyl-pyridin-3-yl, pyridin-4-yl, 3-methoxycarbonyl-pyridin-4-yl, 2-(methoxycarbonyl)phenyl, 3-hydroxy-phenyl or 3-amino-phenyl;
   R⁸ represents pyridin-2-yl, 6-methoxy-pyridin-2-yl, 5-methoxy-pyridin-2-yl, 4-methoxy-pyridin-2-yl, 3-methyl-pyridin-2-yl, 4-methyl-pyridin-2-yl, 5-methyl-pyridin-2-yl, 6-methyl-pyridin-2-yl, 5-aminocarbonyl-pyridin-2-yl, 4-fluoro-pyridin-2-yl, 5-methoxycarbonyl-pyridin-2-yl, 6-methoxycarbonyl-pyridin-2-yl, pyridin-3-yl, 5-fluoro-pyridin-3-yl, 5-methoxy-pyridin-3-yl, 6-methoxy-pyridin-3-yl, 6-acetylamino-pyridin-3-yl, 6-methyl-pyridin-3-yl, pyridin-4-yl, 2-methoxy-pyridin-4-yl, 3-fluoro-pyridin-4-yl, pyrazin-2-yl, pyrimidin-2-yl, pyrimidin-4-yl, pyrimidin-5-yl, phenyl, 3-hydroxyphenyl, 3-methoxyphenyl, 3-carboxyphenyl, 3-carbamoylphenyl, 3-acetylamino-phenyl, 3-sulfamoylphenyl, 3-methoxycarbonyl-phenyl, 3-(carboxymethyl)phenyl, 3-(methylcarbamoyl)phenyl, 3-(dimethylcarbamoyl)phenyl, 3-(benzylcarbamoyl)phenyl, 3-(2-amino-2-oxoethyl)phenyl, 3-(2-(dimethylamino)-2-oxoethyl)phenyl, 3-(2-(benzylamino)-2-oxoethyl)phenyl, 4-methoxycarbonyl-phenyl, 4-carboxyphenyl, 3-carbamoyl-4-methoxyphenyl, 4-methoxy-3-(methylcarbamoyl)phenyl, 3-(dimethylcarbamoyl)-4-methoxyphenyl, 4-hydroxyphenyl, 4-(carboxymethyl)phenyl, 4-cyanophenyl, 4-acetylamino-phenyl, 4-carbamoylphenyl, 4-(methylcarbamoyl)phenyl, 4-(dimethylcarbamoyl)phenyl, 4-(2-amino-2-oxoethyl)phenyl, 4-(2-(dimethylamino)-2-oxoethyl)phenyl, 4-(2-(methylamino)-2-oxoethyl)phenyl, 4-(2-(benzylamino)-2-oxoethyl)phenyl, 5-methyl-1,3,4-oxadiazol-2-yl or imidazo[1,2-*a*]pyridin-7-yl;
   R⁹ represents pyridin-2-yl, pyridin-3-yl, 1,3-dimethyl-1*H-*pyrazol-5-yl or 3-methylisothiazol-5-yl;
   and to salts (in particular pharmaceutically acceptable salts) of compounds of formula I_{P2}.
3) The invention furthermore relates to compounds of formula I according to embodiment 1) that are also compounds of formula I_{P1} 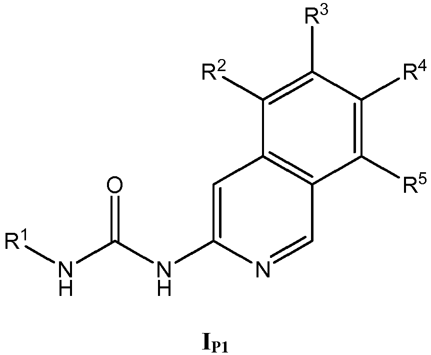 wherein
   R¹ represents (C₁-C₃)alkyl, (C₂-C₃)haloalkyl or cyclopropyl;
   R⁴ represents H;
   R³ represents H, R⁵ represents H and R² represents phenyl or 4-carboxyphenyl; or
   R² represents H, R⁵ represents H and R³ represents benzoylamino; or
   R² represents H, R³ represents H and R⁵ represents benzoylamino, pyridin-3-ylmethylamino, pyridin-2-ylamino, pyridin-3-ylamino, pyridin-3-yloxy or 4-carboxyphenyl; or
   R³ represents H, R⁵ represents methyl and R² represents halogen, (C₁-C₃)alkylamino, 2-(aminocarbonyl)-ethyl, imidazo[1,2-*a*]pyridin-6-yl, 4-methyl-thiophen-3-yl, isoquinolin-5-yl or 1*H*-indol-4-yl or a group having the formula (A1), (A2) or (A3) shown hereafter 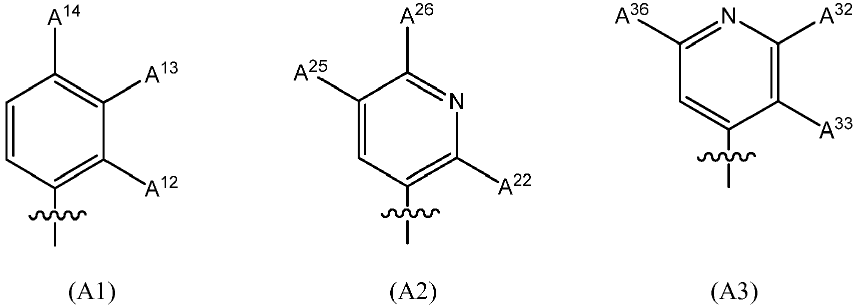
   wherein
   each of A¹³ and A¹⁴ represents H and A¹² represents OH, or
   each of A¹² and A¹⁴ represents H and A¹³ represents OH, acetylamino, aminomethyl, aminosulfonyl or hydroxymethyl, or
   each of A¹² and A¹³ represents H and A¹⁴ represents OH, methylaminocarbonyl, dimethylaminocarbonyl, methoxycarbonylamino, aminosulfonyl or hydroxymethyl;
   each of A²⁵ and A²⁶ represents H and A²² represents H or halogen, or
   each of A²² and A²⁶ represents H and A²⁵ represents methyl or halogen, or
   each of A²² and A²⁵ represents H and A²⁶ represents hydroxymethyl;
   each of A³³ and A³⁶ represents H and A³² represents H, halogen, amino, methyl or methoxy, or
   A³³ represents H and each of A³² and A³⁶ represents methyl; or
   R² represents H, R⁵ represents methyl and R³ represents amino, (C₁-C₄)alkylamino, cyclopropylmethylamino, 2-benzyloxy-ethylamino, (thiazol-5-yl)carbonylamino, pyridin-3-ylcarbonylamino, pyridin-4-ylcarbonylamino, pyridin-4-ylmethylamino, naphthalen-2-yl, furan-2-yl, furan-3-yl, 1*H*-pyrazol-4-yl, thiophen-3-yl, 4-methyl-thiophen-3-yl, quinolin-3-yl, isoquinolin-4-yl, isoquinolin-5-yl, imidazo[1,2-*a*]pyridin-6-yl, 1*H*-indol-4-yl or a group having the formula (B1), (B2), (B3), (B4), (B5), (B6), (B7), (B8) or (B9) shown hereafter 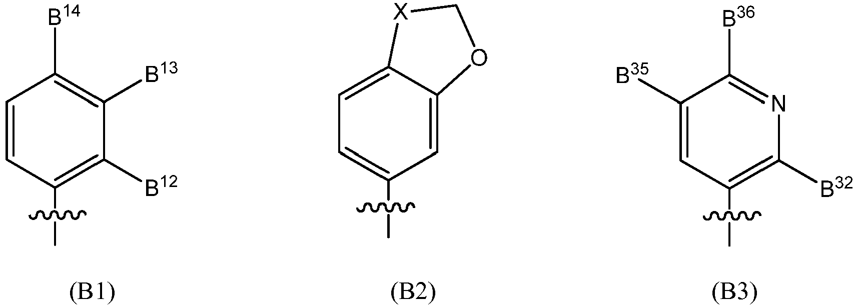 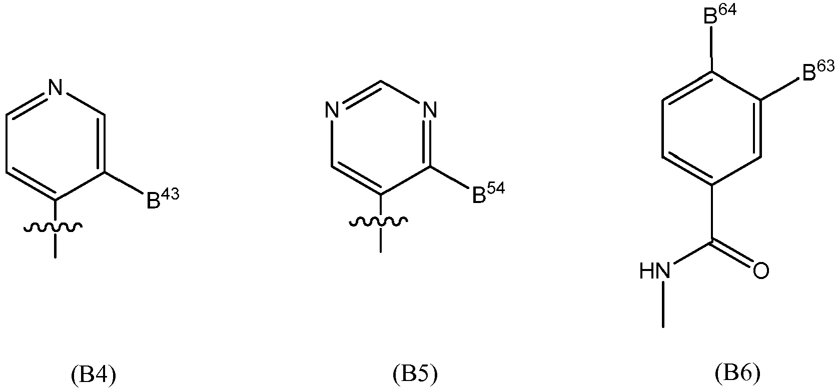 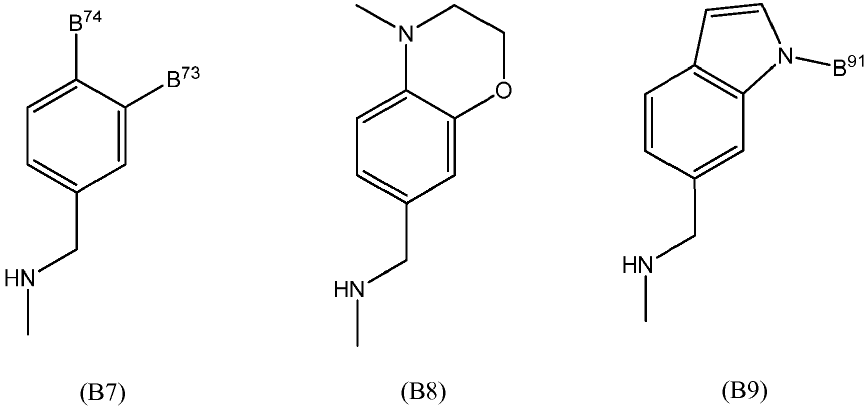
   wherein
   each of B¹³ and B¹⁴ represents H and B¹² represents OH, methyl, acetylamino, ethoxycarbonyl or dimethylaminocarbonyl, or
   each of B¹² and B¹⁴ represents H and B¹³ represents OH, halogen, acetyl, acetylamino, acetylaminomethyl, aminocarbonyl, aminosulfonyl, (C₁-C₂)alkoxy, (C₁-C₂)alkoxycarbonyl, cyano, amino-(C₁-C₂)alkyl or hydroxy-(C₁-C₂)alkyl, or
   each of B¹² and B¹³ represents H and B¹⁴ represents acetyl, acetylamino, acetylaminomethyl, aminocarbonyl, dimethylaminocarbonyl, aminosulfonyl, (C₁-C₂)alkylsulfonyl, cyanomethyl or hydroxymethyl;
   X represents -O- or -CH₂-;
   each of B³⁵ and B³⁶ represents H and B³² represents H or halogen, or
   each of B³² and B³⁶ represents H and B³⁵ represents halogen, methyl, methylsulfanyl, methanesulfonyl or methoxycarbonyl, or
   each of B³² and B³⁵ represents H and B³⁶ represents halogen, methoxy or hydroxymethyl;
   B⁴³ represents H or halogen;
   B⁵⁴ represents H or (C₁-C₃)alkyl;
   B⁶⁴ represents H and B⁶³ represents H or dimethylamino, or
   B⁶³ represents H and B⁶⁴ represents methoxy, dimethylamino or methylsulfonyl;
   B⁷⁴ represents H and B⁷³ represents H, methoxycarbonyl, carboxy or 2-hydroxyethoxy, or
   B⁷³ represents H and B⁷⁴ represents carboxy, acetylamino or 3-methyl-[1,2,4]triazol-1-yl;
   B⁹¹ represents H or methyl;
   R² represents fluorine, R³ represents H and R⁵ represents bromine, benzyl, benzoylamino, 4-carboxyphenyl, benzylamino or (pyridin-3-yl)methylamino; or
   R² represents bromine, R³ represents H and R⁵ represents methyl or methoxy; or
   R² represents fluorine, R⁵ represents methyl and R³ represents H, quinolin-3-yl, benzylamino, a phenyl group optionally substituted once by acetylamino or aminosulfonyl, pyridin-2-yl, or a pyridin-3-yl group optionally substituted once by methyl; or
   R² represents fluorine, R³ represents pyridin-3-yl and R⁵ represents pyridin-3-yl or 4-carboxyphenyl; or
   R² represents fluorine, R³ represents benzylamino and R⁵ represents chlorine or 2-methoxy-pyrimidin-5-yl; or
   R² represents fluorine, R³ represents quinolin-3-yl and R⁵ represents quinolin-3-yl; or
   R² represents fluorine, R³ represents 3-acetylaminophenyl and R⁵ represents pyridin-3-yl or 2-methoxypyrimidin-5-yl; or
   R² represents fluorine, R³ represents 4-aminosulfonylphenyl and R⁵ represents chlorine; or
   R² represents pyridin-4-yl, R³ represents H and R⁵ represents vinyl, methoxy or pyridin-4-yl;
   and to salts (in particular pharmaceutically acceptable salts) of compounds of formula I_{P1}.
4) According to one embodiment of the invention, the compounds of formula I as defined in one of embodiments 1) to 3) will be such that R¹ represents (C₁-C₃)alkyl.
5) Preferably, the compounds of formula I as defined in embodiment 4) will be such that R¹ represents ethyl.
6) According to another embodiment of the invention, the compounds of formula I as defined in one of embodiments 1) to 3) will be such that R¹ represents (C₁-C₃)haloalkyl (notably (C₁-C₂)haloalkyl and in particular 2-fluoroethyl).
7) According to yet another embodiment of the invention, the compounds of formula I as defined in one of embodiments 1) to 3) will be such that R¹ represents cyclopropyl.
8) According to a main variant of the invention, the compounds of formula I as defined in one of embodiments 1) to 7) will be such that R² represents H.
9) According to a sub-variant of embodiment 8), the compounds of formula I as defined in embodiment 8) will be such that R⁵ represents H.
10) According to yet another sub-variant of embodiment 8), the compounds of formula I as defined in embodiment 8) will be such that R⁵ represents benzoylamino, pyridin-2-ylamino, pyridin-3-ylamino, pyridin-3-yloxy or pyridin-3-ylmethylamino.
11) According to yet another sub-variant of embodiment 8), the compounds of formula I as defined in embodiment 8) will be such that R⁵ represents 4-carboxyphenyl.
12) According to a further sub-variant of embodiment 8), the compounds of formula I as defined in embodiment 8) will be such that R⁵ represents methyl.
13) A particular category of the compounds of formula I as defined in embodiment 12) relates to the compounds of formula I as defined in embodiment 12) wherein R³ represents a group (B1) (and preferably such that R³ represents a group (B1) and R¹ represents ethyl, and notably such that R³ represents a group (B1) as defined in embodiment 3) and R¹ represents ethyl).
14) Another particular category of the compounds of formula I as defined in embodiment 12) relates to the compounds of formula I as defined in embodiment 12) wherein R³ represents a group (B2) (and preferably such that R³ represents a group (B2) and R¹ represents ethyl, and notably such that R³ represents a group (B2) as defined in embodiment 3) and R¹ represents ethyl).
15) Yet another particular category of the compounds of formula I as defined in embodiment 12) relates to the compounds of formula I as defined in embodiment 12) wherein R³ represents a group (B3) or (B4) (and preferably such that R³ represents a group (B3) or (B4) and R¹ represents ethyl, and notably such that R³ represents a group (B3) or (B4) as defined in embodiment 3) and R¹ represents ethyl).
16) A further particular category of the compounds of formula I as defined in embodiment 12) relates to the compounds of formula I as defined in embodiment 12) wherein R³ represents a group (B6) (and preferably such that R³ represents a group (B6) and R¹ represents ethyl, and notably such that R³ represents a group (B6) as defined in embodiment 3) and R¹ represents ethyl).
17) A further particular category of the compounds of formula I as defined in embodiment 12) relates to the compounds of formula I as defined in embodiment 12) wherein R³ represents quinolin-3-yl or imidazo[1,2-*a*]pyridin-6-yl (and preferably such that R³ represents quinolin-3-yl or imidazo[1,2-*a*]pyridin-6-yl and R¹ represents ethyl).
18) Yet a further particular category of the compounds of formula I as defined in embodiment 12) relates to the compounds of formula I as defined in embodiment 12) wherein R³ represents (thiazol-5-yl)carbonylamino, pyridin-3-ylcarbonylamino or pyridin-4-ylcarbonylamino (and preferably such that R³ represents (thiazol-5-yl)carbonylamino, pyridin-3-ylcarbonylamino or pyridin-4-ylcarbonylamino and R¹ represents ethyl).
19) Yet a further category of the compounds of formula I as defined in embodiment 12) relates to the compounds of formula I as defined in embodiment 12) wherein R³ represents a group (B7) (and preferably such that R³ represents a group (B7) and R¹ represents ethyl).
20) Yet a further category of the compounds of formula I as defined in embodiment 12) relates to the compounds of formula I as defined in embodiment 12) wherein R³ represents a group (B8) (and preferably such that R³ represents a group (B8) and R¹ represents ethyl).
21) Yet a further category of the compounds of formula I as defined in embodiment 12) relates to the compounds of formula I as defined in embodiment 12) wherein R³ represents a group (B9) (and preferably such that R³ represents a group (B9) and R¹ represents ethyl).
22) Yet a further category of the compounds of formula I as defined in embodiment 12) relates to the compounds of formula I as defined in embodiment 12) wherein R³ represents a group (B10) (and preferably such that R³ represents a group (B10) and R¹ represents ethyl).
23) Yet a further category of the compounds of formula I as defined in embodiment 12) relates to the compounds of formula I as defined in embodiment 12) wherein R³ represents a group (B11) (and preferably such that R³ represents a group (B11) and R¹ represents ethyl).
24) Yet a further category of the compounds of formula I as defined in embodiment 12) relates to the compounds of formula I as defined in embodiment 12) wherein R³ represents a group (B12) (and preferably such that R³ represents a group (B12) and R¹ represents ethyl).
25) The compounds of formula I as defined in embodiment 12) can notably be such that:
   - R³ represents a group (B1) wherein either each of B¹² and B¹⁴ represents H and B¹³ represents OH, halogen, acetyl, acetylamino, acetylaminomethyl, aminocarbonyl, dimethylaminocarbonyl, aminosulfonyl, (C₁-C₂)alkylsulfonyl, (C₁-C₂)alkoxy, (C₁-C₂)alkoxycarbonyl, cyano, amino-(C₁-C₂)alkyl or hydroxy-(C₁-C₂)alkyl, or each of B¹² and B¹³ represents H and B¹⁴ represents acetyl, acetylamino, acetylaminomethyl, aminocarbonyl, dimethylaminocarbonyl, aminosulfonyl, (C₁-C₂)alkylsulfonyl, cyanomethyl or hydroxymethyl; or
   - R³ represents a group (B2) wherein X represents CH₂; or
   - R³ represents a group (B3); or
   - R³ represents a group (B4); or
   - R³ represents a group (B6); or
   - R³ represents quinolin-3-yl, imidazo[1,2-*a*]pyridin-6-yl, (thiazol-5-yl)carbonylamino, pyridin-3-ylcarbonylamino or pyridin-4-ylcarbonylamino;
   whereby the groups (B2), (B3), (B4) and (B6) will notably be defined as in embodiment 3).
26) The compounds of formula I as defined in embodiment 12) can furthermore be such that R¹ represents ethyl and:
   - R³ represents a group (B1) wherein either each of B¹² and B¹⁴ represents H and B¹³ represents OH, aminosulfonyl or ethoxycarbonyl, or each of B¹² and B¹³ represents H and B¹⁴ represents acetyl, acetylamino, acetylaminomethyl, dimethylaminocarbonyl, aminosulfonyl, methylsulfonyl, cyanomethyl or hydroxymethyl; or
   - R³ represents a group (B2) wherein X represents CH₂; or
   - R³ represents a group (B3) wherein either each of B³² and B³⁶ represents H and B³⁵ represents methanesulfonyl, or each of B³² and B³⁵ represents H and B³⁶ represents methoxy; or
   - R³ represents a group (B4) wherein B⁴³ represents H; or
   - R³ represents a group (B6) wherein each of B⁶³ and B⁶⁴ represents H, or B⁶⁴ represents H and B⁶³ represents dimethylamino, or also B⁶³ represents H and B⁶⁴ represents methoxy, dimethylamino or methylsulfonyl; or
   - R³ represents quinolin-3-yl, imidazo[1,2-*a*]pyridin-6-yl, (thiazol-5-yl)carbonylamino, pyridin-3-ylcarbonylamino or pyridin-4-ylcarbonylamino.
27) According to a sub-variant of embodiment 8), the compounds of formula I as defined in embodiment 8) will be such that R³ represents H.
28) A particular category of the compounds of formula I as defined in embodiment 27) relates to the compounds of formula I as defined in embodiment 27) wherein R⁵ represents NH-CO-R⁶ (whereby R¹ will preferably in addition represent ethyl).
29) Yet a further particular category of the compounds of formula I as defined in embodiment 27) relates to the compounds of formula I as defined in embodiment 27) wherein R⁵ represents -CH₂-O-R⁷ (whereby R¹ will preferably in addition represent ethyl).
30) A further particular category of the compounds of formula I as defined in embodiment 27) relates to the compounds of formula I as defined in embodiment 27) wherein R⁵ represents -NH-R⁸ (whereby R¹ will preferably in addition represent ethyl).
31) A particular sub-category of the compounds of formula I as defined in embodiment 30) relates to the compounds of formula I as defined in embodiment 30) wherein R⁸ represents pyridin-2-yl, 6-methoxy-pyridin-2-yl, 5-methoxy-pyridin-2-yl, 4-methoxy-pyridin-2-yl, 3-methyl-pyridin-2-yl, 4-methyl-pyridin-2-yl, 5-methyl-pyridin-2-yl, 6-methyl-pyridin-2-yl, 5-aminocarbonyl-pyridin-2-yl, 4-fluoro-pyridin-2-yl, 5-methoxycarbonyl-pyridin-2-yl or 6-methoxycarbonyl-pyridin-2-yl (whereby R¹ will preferably in addition represent ethyl).
32) Another particular sub-category of the compounds of formula I as defined in embodiment 30) relates to the compounds of formula I as defined in embodiment 30) wherein R⁸ represents pyridin-3-yl, 5-fluoro-pyridin-3-yl, 2-methoxypyridin-3-yl, 5-methoxy-pyridin-3-yl, 6-methoxy-pyridin-3-yl, 6-acetylamino-pyridin-3-yl or 6-methyl-pyridin-3-yl, and notably to the compounds of formula I as defined in embodiment 30) wherein R⁸ represents pyridin-3-yl, 5-fluoro-pyridin-3-yl, 5-methoxy-pyridin-3-yl, 6-methoxy-pyridin-3-yl, 6-acetylamino-pyridin-3-yl or 6-methyl-pyridin-3-yl (whereby R¹ will preferably in addition represent ethyl).
33) A further particular sub-category of the compounds of formula I as defined in embodiment 30) relates to the compounds of formula I as defined in embodiment 30) wherein R⁸ represents pyridin-4-yl, 2-methoxy-pyridin-4-yl or 3-fluoro-pyridin-4-yl (whereby R¹ will preferably in addition represent ethyl).
34) Yet a further particular sub-category of the compounds of formula I as defined in embodiment 30) relates to the compounds of formula I as defined in embodiment 30) wherein R⁸ is as defined in one of embodiments 31) to 33) (whereby R¹ will preferably in addition represent ethyl).
35) Yet a further particular sub-category of the compounds of formula I as defined in embodiment 30) relates to the compounds of formula I as defined in embodiment 30) wherein R⁸ represents pyrazin-2-yl, pyrimidin-2-yl, pyrimidin-4-yl or pyrimidin-5-yl (whereby R¹ will preferably in addition represent ethyl).
36) Yet a further particular sub-category of the compounds of formula I as defined in embodiment 30) relates to the compounds of formula I as defined in embodiment 30) wherein R⁸ represents phenyl, 3-hydroxy-phenyl, 3-methoxy-phenyl, 3-carboxy-phenyl, 3-carbamoylphenyl, 3-acetylamino-phenyl, 3-sulfamoyl-phenyl, 3-methoxycarbonyl-phenyl, 4-methyl-3-methoxycarbonyl-phenyl, 4-methyl-3-carboxyphenyl, 3-(carboxymethyl)phenyl, 3-(methylcarbamoyl)phenyl, 3-(dimethylcarbamoyl)phenyl, 3-(benzylcarbamoyl)phenyl, 3-(2-amino-2-oxoethyl)phenyl, 3-(2-(dimethylamino)-2-oxoethyl)phenyl, 3-(2-(benzylamino)-2-oxoethyl)phenyl, 4-methoxycarbonyl-phenyl, 4-carboxy-phenyl, 3-carbamoyl-4-methoxyphenyl, 4-methoxy-3-(methylcarbamoyl)phenyl, 3-(dimethylcarbamoyl)-4-methoxyphenyl, 4-hydroxy-phenyl, 4-(carboxymethyl)phenyl, 4-cyano-phenyl, 4-acetylamino-phenyl, 4-carbamoylphenyl, 4-(methylcarbamoyl)phenyl, 4-(dimethylcarbamoyl)phenyl, 4-(2-amino-2-oxoethyl)phenyl, 4-(2-(dimethylamino)-2-oxoethyl)phenyl, 4-(2-(methylamino)-2-oxoethyl)phenyl or 4-(2-(benzylamino)-2-oxoethyl)phenyl, and notably to the compounds of formula I as defined in embodiment 30) wherein R⁸ represents phenyl, 3-hydroxy-phenyl, 3-methoxy-phenyl, 3-carboxy-phenyl, 3-carbamoylphenyl, 3-acetylamino-phenyl, 3-sulfamoyl-phenyl, 3-methoxycarbonyl-phenyl, 3-(carboxymethyl)phenyl, 3-(methylcarbamoyl)phenyl, 3-(dimethylcarbamoyl)phenyl, 3-(benzylcarbamoyl)phenyl, 3-(2-amino-2-oxoethyl)phenyl, 3-(2-(dimethylamino)-2-oxoethyl)phenyl, 3-(2-(benzylamino)-2-oxoethyl)phenyl, 4-methoxycarbonyl-phenyl, 4-carboxy-phenyl, 3-carbamoyl-4-methoxyphenyl, 4-methoxy-3-(methylcarbamoyl)phenyl, 3-(dimethylcarbamoyl)-4-methoxyphenyl, 4-hydroxy-phenyl, 4-(carboxymethyl)phenyl, 4-cyano-phenyl, 4-acetylamino-phenyl, 4-carbamoylphenyl, 4-(methylcarbamoyl)phenyl, 4-(dimethylcarbamoyl)phenyl, 4-(2-amino-2-oxoethyl)phenyl, 4-(2-(dimethylamino)-2-oxoethyl)phenyl, 4-(2-(methylamino)-2-oxoethyl)phenyl or 4-(2-(benzylamino)-2-oxoethyl)phenyl (whereby R¹ will preferably in addition represent ethyl).
37) Yet a further particular sub-category of the compounds of formula I as defined in embodiment 30) relates to the compounds of formula I as defined in embodiment 30) wherein R⁸ represents 5-methyl-1,3,4-oxadiazol-2-yl or imidazo[1,2-*a*]pyridin-7-yl (whereby R¹ will preferably in addition represent ethyl).
38) Yet a further particular category of the compounds of formula I as defined in embodiment 27) relates to the compounds of formula I as defined in embodiment 27) wherein R⁵ represents -CH₂-NH-R⁹ (and preferably such that R³ represents -CH₂-NH-R⁹ and R¹ represents ethyl).
39) A particular sub-category of the compounds of formula I as defined in embodiment 38) relates to the compounds of formula I as defined in embodiment 38) wherein R⁹ represents pyridin-2-yl or pyridin-3-yl (whereby R¹ will preferably in addition represent ethyl).
40) Another particular sub-category of the compounds of formula I as defined in embodiment 38) relates to the compounds of formula I as defined in embodiment 38) wherein R⁹ represents 1,3-dimethyl-1*H*-pyrazol-5-yl or 3-methylisothiazol-5-yl (whereby R¹ will preferably in addition represent ethyl).
41) Another particular category of the compounds of formula I as defined in embodiment 27) relates to the compounds of formula I as defined in embodiment 27) wherein R⁵ represents -CH₂-R¹⁰ (whereby R¹ will preferably in addition represent ethyl).
42) Yet another particular category of the compounds of formula I as defined in embodiment 27) relates to the compounds of formula I as defined in embodiment 27) wherein R⁵ represents (pyridin-3-ylmethyl)amino (whereby R¹ will preferably in addition represent ethyl).
43) According to another main variant of the invention, the compounds of formula I as defined in one of embodiments 1) to 7) will be such that R² represents fluorine.
44) According to a sub-variant of embodiment 43), the compounds of formula I as defined in embodiment 43) will be such that R⁵ represents methyl.
45) In particular, the compounds of formula I as defined in embodiment 44) will be such that R³ represents quinolin-3-yl, benzylamino, 3-acetylamino-phenyl, 4-aminosulfonylphenyl or 5-methyl-pyridin-3-yl (whereby R¹ will preferably in addition represent ethyl).
46) According to another sub-variant of embodiment 43), the compounds of formula I as defined in embodiment 43) will be such that R⁵ represents pyridin-3-yl.
47) According to yet another sub-variant of embodiment 43), the compounds of formula I as defined in embodiment 43) will be such that R⁵ represents 4-carboxyphenyl.
48) According to a further sub-variant of embodiment 43), the compounds of formula I as defined in embodiment 43) will be such that R⁵ represents chlorine.
49) According to yet a further sub-variant of embodiment 43), the compounds of formula I as defined in embodiment 43) will be such that R⁵ represents 2-methoxy-pyrimidin-5-yl.
50) According to yet another main variant of the invention, the compounds of formula I as defined in one of embodiments 1) to 7) will be such that R² represents bromine.
51) According to a further main variant of the invention, the compounds of formula I as defined in one of embodiments 1) to 7) will be such that R² represents pyridin-4-yl.
52) According to another main variant of the invention, the compounds of formula I as defined in one of embodiments 1) to 7) will be such that R³ represents H.
53) According to a sub-variant of embodiment 52), the compounds of formula I as defined in embodiment 52) will be such that R⁵ represents H.
54) According to another sub-variant of embodiment 52), the compounds of formula I as defined in embodiment 52) will be such that R⁵ represents methyl.
55) A particular category of the compounds of formula I as defined in embodiment 54) relates to the compounds of formula I as defined in embodiment 54) wherein R² represents halogen (and preferably such that R² represents halogen (notably bromine or fluorine) and R¹ represents ethyl).
56) Another particular category of the compounds of formula I as defined in embodiment 54) relates to the compounds of formula I as defined in embodiment 54) wherein R² represents a group (A1) (and preferably such that R² represents a group (A1) and R¹ represents ethyl).
57) Yet another particular category of the compounds of formula I as defined in embodiment 54) relates to the compounds of formula I as defined in embodiment 54) wherein R² represents a group (A2) (and preferably such that R² represents a group (A2) and R¹ represents ethyl).
58) A further particular category of the compounds of formula I as defined in embodiment 54) relates to the compounds of formula I as defined in embodiment 54) wherein R² represents a group (A3) (and preferably such that R² represents a group (A3) and R¹ represents ethyl).
59) Yet a further particular category of the compounds of formula I as defined in embodiment 54) relates to the compounds of formula I as defined in embodiment 54) wherein R² represents pyrimidin-4-yl, pyridazin-4-yl, 1*H*-imidazol-4-yl, 1*H*-pyrazol-4-yl or thiazol-4-yl (whereby R¹ will preferably in addition represent ethyl).
60) The compounds of formula I as defined in embodiment 54) can in particular be such that:
   - R² represents halogen (preferably bromine or fluorine); or
   - R² represents a group (A1); or
   - R² represents a group (A3).
   whereby the groups (A1) and (A3) will notably be defined as in embodiment 3).
61) The compounds of formula I as defined in embodiment 54) can more particularly be such that R¹ represents ethyl and:
   - R² represents a group (A1) wherein each of A¹² and A¹³ represents H and A¹⁴ represents hydroxymethyl; or
   - R² represents a group (A3) wherein each of A²², A²⁵ and A²⁶ represents H.
62) According to yet another sub-variant of embodiment 52), the compounds of formula I as defined in embodiment 52) will be such that R⁵ represents methoxy.
63) According to a further sub-variant of embodiment 52), the compounds of formula I as defined in embodiment 52) will be such that R⁵ represents pyridin-4-yl.
64) According to yet a further sub-variant of embodiment 52), the compounds of formula I as defined in embodiment 52) will be such that R² represents (C₁-C₃)alkylaminomethyl or *N*,*N*-dimethylaminomethyl.
65) According to yet a further sub-variant of embodiment 52), the compounds of formula I as defined in embodiment 52) will be such that R² represents pyridin-3-yl.
66) According to yet a further sub-variant of embodiment 52), the compounds of formula I as defined in embodiment 52) will be such that R² represents pyridin-4-yl, 2-methylpyridin-4-yl, 2-methoxypyridin-4-yl, 2-aminopyridin-4-yl or 2,6-dimethylpyridin-4-yl.
67) According to another sub-variant of embodiment 52), the compounds of formula I as defined in embodiment 52) will be such that R² represents pyrimidin-4-yl.
68) According to another sub-variant of embodiment 52), the compounds of formula I as defined in embodiment 52) will be such that R² represents thiazol-4-yl.
69) In particular, the compounds of formula I as defined in one of embodiments 66) to 68), and notably as defined in embodiment 66), will be such that R⁵ represents (3-carboxyphenyl)amino, (3-(dimethylcarbamoyl)phenyl)amino, (3-(methylcarbamoyl)phenyl)amino, (6-methyl-pyridin-2-yl)amino, (6-methoxy-pyridin-2-yl)amino, pyridin-3-ylamino, (6-methyl-pyridin-3-yl)amino.
70) The compounds of formula I as defined in embodiment 66) will notably be such that R² represents pyridin-4-yl or 2-methylpyridin-4-yl.
71) In particular, the compounds of formula I as defined in embodiment 70) will be such that R⁵ represents (3-carboxyphenyl)amino or (6-methyl-pyridin-3-yl)amino.
72) According to yet a further sub-variant of embodiment 52), the compounds of formula I as defined in embodiment 52) will be such that R² represents 3-(aminomethyl)phenyl.
73) According to yet a further sub-variant of embodiment 52), the compounds of formula I as defined in embodiment 52) will be such that R² represents 4-hydroxyphenyl, 4-(hydroxymethyl)phenyl or 4-sulfamoyl-phenyl.
74) In particular, the compounds of formula I as defined in embodiment 73) will be such that R⁵ represents (3-carboxyphenyl)amino, (3-(dimethylcarbamoyl)phenyl)amino, (3-(methylcarbamoyl)phenyl)amino or pyridin-3-ylamino.
75) The compounds of formula I as defined in embodiment 73) will notably be such that R² represents 4-(hydroxymethyl)phenyl or 4-sulfamoyl-phenyl.
76) In particular, the compounds of formula I as defined in embodiment 75) will be such that R⁵ represents (3-carboxyphenyl)amino or pyridin-3-ylamino.
77) According to yet another main variant of the invention, the compounds of formula I as defined in one of embodiments 1) to 7) will be such that R⁵ represents H.
78) According to a sub-variant of embodiment 77), the compounds of formula I as defined in embodiment 77) will be such that R² represents H.
79) According to another sub-variant of embodiment 77), the compounds of formula I as defined in embodiment 77) will be such that R³ represents H.
80) Another embodiment of this invention relates to compounds of formula I as defined in one of embodiments 1) to 79) as well as to isotopically labelled, especially ²H (deuterium) labelled compounds of formula I as defined in one of embodiments 1) to 79), which compounds are identical to the compounds of formula I as defined in one of embodiments 1) to 79) except that one or more atoms has or have each been replaced by an atom having the same atomic number but an atomic mass different from the atomic mass usually found in nature. Isotopically labelled, especially ²H (deuterium) labelled compounds of formula I and salts (in particular pharmaceutically acceptable salts) thereof are thus within the scope of the present invention. Substitution of hydrogen with the heavier isotope ²H (deuterium) may lead to greater metabolic stability, resulting e.g. in increased *in-vivo* half-life or reduced dosage requirements, or may lead to reduced inhibition of cytochrome P450 enzymes, resulting e.g. in an improved safety profile. In one variant of the invention, the compounds of formula I are not isotopically labelled, or they are labelled only with one or more deuterium atoms. Isotopically labelled compounds of formula I may be prepared in analogy to the methods described hereinafter, but using the appropriate isotopic variation of suitable reagents or starting materials.
81) Particularly preferred are the following compounds of formula I as defined in one of embodiments 1) to 3):
   - 1-(5-bromo-8-methyl-isoquinolin-3-yl)-3-ethyl-urea;
   - 1-ethyl-3-[5-(4-hydroxy-phenyl)-8-methyl-isoquinolin-3-yl]-urea;
   - 1-ethyl-3-[5-(2-hydroxy-phenyl)-8-methyl-isoquinolin-3-yl]-urea;
   - *N*-{3-[3-(3-ethyl-ureido)-8-methyl-isoquinolin-5-yl]-phenyl}-acetamide;
   - 4-[3-(3-ethyl-ureido)-8-methyl-isoquinolin-5-yl]-*N*,*N*-dimethyl-benzamide;
   - 4-[3-(3-ethyl-ureido)-8-methyl-isoquinolin-5-yl]-*N*-methyl-benzamide;
   - 1-ethyl-3-(5-imidazo[1,2-*a*]pyridin-6-yl-8-methyl-isoquinolin-3-yl)-urea;
   - 1-ethyl-3-[5-(6-hydroxymethyl-pyridin-3-yl)-8-methyl-isoquinolin-3-yl]-urea;
   - 1-ethyl-3-[5-(5-fluoro-pyridin-3-yl)-8-methyl-isoquinolin-3-yl]-urea;
   - 1-ethyl-3-[8-methyl-5-(5-methyl-pyridin-3-yl)-isoquinolin-3-yl]-urea;
   - 1-[5-(3-(aminomethyl)-phenyl)-8-methyl-isoquinolin-3-yl]-3-ethyl-urea;
   - 1-ethyl-3-[5-(3-hydroxymethyl-phenyl)-8-methyl-isoquinolin-3-yl]-urea;
   - 3-[3-(3-ethyl-ureido)-8-methyl-isoquinolin-5-yl]-benzenesulfonamide;
   - 1-ethyl-3-(6-furan-3-yl-8-methyl-isoquinolin-3-yl)-urea;
   - 1-ethyl-3-[6-(4-hydroxymethyl-phenyl)-8-methyl-isoquinolin-3-yl]-urea;
   - 1-ethyl-3-(8-methyl-6-pyridin-3-yl-isoquinolin-3-yl)-urea;
   - *N*-{3-[3-(3-ethyl-ureido)-8-methyl-isoquinolin-6-yl]-benzyl}-acetamide;
   - *N*-{4-[3-(3-ethyl-ureido)-8-methyl-isoquinolin-6-yl]-phenyl}-acetamide;
   - 1-ethyl-3-[6-(3-hydroxymethyl-phenyl)-8-methyl-isoquinolin-3-yl]-urea;
   - 1-ethyl-3-[6-(3-hydroxy-phenyl)-8-methyl-isoquinolin-3-yl]-urea;
   - 1-ethyl-3-(8-methyl-6-pyridin-4-yl-isoquinolin-3-yl)-urea;
   - 3-[3-(3-ethyl-ureido)-8-methyl-isoquinolin-6-yl]-benzenesulfonamide;
   - 2-[3-(3-ethyl-ureido)-8-methyl-isoquinolin-6-yl]-*N*,*N*-dimethyl-benzamide;
   - 4-[3-(3-ethyl-ureido)-8-methyl-isoquinolin-6-yl]-*N*,*N*-dimethyl-benzamide;
   - 1-ethyl-3-(6-imidazo[1,2-*a*]pyridin-6-yl-8-methyl-isoquinolin-3-yl)-urea;
   - 1-[6-(3-(aminomethyl)-phenyl)-8-methyl-isoquinolin-3-yl]-3-ethyl-urea;
   - 1-[5-(2-chloro-pyridin-4-yl)-8-methyl-isoquinolin-3-yl]-3-ethyl-urea;
   - 1-ethyl-3-[8-methyl-6-(1*H*-pyrazol-4-yl)-isoquinolin-3-yl]-urea;
   - 1-[6-(3-acetyl-phenyl)-8-methyl-isoquinolin-3-yl]-3-ethyl-urea;
   - 4-[3-(3-ethyl-ureido)-8-methyl-isoquinolin-6-yl]-benzamide;
   - 3-[3-(3-ethyl-ureido)-8-methyl-isoquinolin-6-yl]-benzamide;
   - 1-(8-bromo-5-fluoro-isoquinolin-3-yl)-3-ethyl-urea;
   - 4-[3-(3-ethyl-ureido)-5-fluoro-6-pyridin-3-yl-isoquinolin-8-yl]-benzoic acid;
   - 1-ethyl-3-[5-(4-hydroxymethyl-phenyl)-8-methyl-isoquinolin-3-yl]-urea;
   - 4-[3-(3-ethyl-ureido)-8-methyl-isoquinolin-5-yl]-benzenesulfonamide;
   - 1-ethyl-3-(8-methyl-6-quinolin-3-yl-isoquinolin-3-yl)-urea;
   - *N*-{3-[3-(3-ethyl-ureido)-8-methyl-isoquinolin-6-yl]-phenyl}-acetamide;
   - 4-[3-(3-ethyl-ureido)-8-methyl-isoquinolin-6-yl]-benzenesulfonamide;
   - 1-ethyl-3-[8-methyl-6-(5-methyl-pyridin-3-yl)-isoquinolin-3-yl]-urea;
   - 1-ethyl-3-[8-methyl-5-(2-methyl-pyridin-4-yl)-isoquinolin-3-yl]-urea;
   - 1-ethyl-3-[5-(2-fluoro-pyridin-4-yl)-8-methyl-isoquinolin-3-yl]-urea;
   - 1-ethyl-3-[5-(2-methoxy-pyridin-4-yl)-8-methyl-isoquinolin-3-yl]-urea;
   - 1-ethyl-3-(5-phenyl-isoquinolin-3-yl)-urea;
   - 1-[5-(2,6-dimethyl-pyridin-4-yl)-8-methyl-isoquinolin-3-yl]-3-ethyl-urea;
   - 1-[5-(2-amino-pyridin-4-yl)-8-methyl-isoquinolin-3-yl]-3-ethyl-urea;
   - 1-(8-benzyl-5-fluoro-isoquinolin-3-yl)-3-ethyl-urea;
   - 1-ethyl-3-(5-fluoro-8-methyl-6-pyridin-3-yl-isoquinolin-3-yl)-urea;
   - 1-ethyl-3-(5-fluoro-8-methyl-6-pyridin-2-yl-isoquinolin-3-yl)-urea;
   - 1-(6-benzylamino-8-chloro-5-fluoro-isoquinolin-3-yl)-3-ethyl-urea;
   - 1-ethyl-3-(8-methyl-5-pyridin-3-yl-isoquinolin-3-yl)-urea;
   - *N*-[3-(3-ethyl-ureido)-5-fluoro-isoquinolin-8-yl]-benzamide;
   - *N*-[3-(3-ethyl-ureido)-isoquinolin-6-yl]-benzamide;
   - 1-ethyl-3-(5-fluoro-6,8-di-pyridin-3-yl-isoquinolin-3-yl)-urea;
   - 1-(6-amino-8-methyl-isoquinolin-3-yl)-3-ethyl-urea;
   - 1-ethyl-3-{5-fluoro-8-[(pyridin-3-ylmethyl)-amino]-isoquinolin-3-yl}-urea;
   - 1-(8-benzylamino-5-fluoro-isoquinolin-3-yl)-3-ethyl-urea;
   - 1-(6-benzylamino-5-fluoro-8-methyl-isoquinolin-3-yl)-3-ethyl-urea;
   - 1-(6-benzylamino-8-methyl-isoquinolin-3-yl)-3-ethyl-urea;
   - 3-[3-(3-ethyl-ureido)-8-methyl-isoquinolin-5-yl]-propionamide;
   - 1-ethyl-3-(5-fluoro-8-methyl-isoquinolin-3-yl)-urea;
   - 1-ethyl-3-(8-methyl-5-pyridin-4-yl-isoquinolin-3-yl)-urea;
   - thiazole-5-carboxylic acid [3-(3-ethyl-ureido)-8-methyl-isoquinolin-6-yl]-amide;
   - 1-ethyl-3-(5-ethylamino-8-methyl-isoquinolin-3-yl)-urea;
   - 1-ethyl-3-[8-methyl-5-(4-methyl-thiophen-3-yl)-isoquinolin-3-yl]-urea;
   - {4-[3-(3-ethyl-ureido)-8-methyl-isoquinolin-5-yl]-phenyl}-carbamic acid methyl ester;
   - 1-ethyl-3-[5-(2-fluoro-pyridin-3-yl)-8-methyl-isoquinolin-3-yl]-urea;
   - 1-ethyl-3-(8-methyl-[5,5']biisoquinolinyl-3-yl)-urea;
   - 1-ethyl-3-[5-(3-hydroxy-phenyl)-8-methyl-isoquinolin-3-yl]-urea;
   - 1-ethyl-3-[5-(1*H*-indol-4-yl)-8-methyl-isoquinolin-3-yl]-urea;
   - *N*-[3-(3-ethyl-ureido)-isoquinolin-8-yl]-benzamide;
   - 1-ethyl-3-{8-[(pyridin-3-ylmethyl)-amino]-isoquinolin-3-yl}-urea;
   - 1-(5,8-di-pyridin-4-yl-isoquinolin-3-yl)-3-ethyl-urea;
   - 1-(5-bromo-8-methoxy-isoquinolin-3-yl)-3-ethyl-urea;
   - 4-[3-(3-ethyl-ureido)-5-fluoro-isoquinolin-8-yl]-benzoic acid;
   - 1-ethyl-3-(8-methyl-6-pyrimidin-5-yl-isoquinolin-3-yl)-urea;
   - 1-ethyl-3-[6-(3-methoxy-phenyl)-8-methyl-isoquinolin-3-yl]-urea;
   - 1-(6-(benzo[1,3]dioxol-5-yl)-8-methyl-isoquinolin-3-yl)-3-ethyl-urea;
   - 1-ethyl-3-(6-furan-2-yl-8-methyl-isoquinolin-3-yl)-urea;
   - 1-ethyl-3-(8-methyl-6-naphthalen-2-yl-isoquinolin-3-yl)-urea;
   - 1-ethyl-3-[8-methyl-6-(4-methyl-thiophen-3-yl)-isoquinolin-3-yl]-urea;
   - *N*-{4-[3-(3-ethyl-ureido)-8-methyl-isoquinolin-6-yl]-benzyl}-acetamide;
   - 1-[6-(2-chloro-pyridin-3-yl)-8-methyl-isoquinolin-3-yl]-3-ethyl-urea;
   - 1-[6-(2,3-dihydro-benzofuran-5-yl)-8-methyl-isoquinolin-3-yl]-3-ethyl-urea;
   - 3-[3-(3-ethyl-ureido)-8-methyl-isoquinolin-6-yl]-benzoic acid ethyl ester;
   - 2-[3-(3-ethyl-ureido)-8-methyl-isoquinolin-6-yl]-benzoic acid ethyl ester;
   - *N*-{2-[3-(3-ethyl-ureido)-8-methyl-isoquinolin-6-yl]-phenyl}-acetamide;
   - 5-[3-(3-ethyl-ureido)-8-methyl-isoquinolin-6-yl]-nicotinic acid methyl ester;
   - 1-ethyl-3-[6-(3-fluoro-phenyl)-8-methyl-isoquinolin-3-yl]-urea;
   - 1-ethyl-3-[6-(6-methoxy-pyridin-3-yl)-8-methyl-isoquinolin-3-yl]-urea;
   - 1-ethyl-3-[6-(1*H-*indol-4-yl)-8-methyl-isoquinolin-3-yl]-urea;
   - 1-ethyl-3-(8'-methyl-[4,6']biisoquinolinyl-3'-yl)-urea;
   - 1-[6-(4-(cyanomethyl)-phenyl)-8-methyl-isoquinolin-3-yl]-3-ethyl-urea;
   - 1-ethyl-3-(8-methyl-6-thiophen-3-yl-isoquinolin-3-yl)-urea;
   - 1-ethyl-3-[6-(4-methanesulfonyl-phenyl)-8-methyl-isoquinolin-3-yl]-urea;
   - 1-ethyl-3-[6-(4-isopropyl-pyrimidin-5-yl)-8-methyl-isoquinolin-3-yl]-urea;
   - 1-ethyl-3-[8-methyl-6-(5-methylsulfanyl-pyridin-3-yl)-isoquinolin-3-yl]-urea;
   - 1-ethyl-3-[6-(3-fluoro-pyridin-4-yl)-8-methyl-isoquinolin-3-yl]-urea;
   - 1-[6-(3-chloro-pyridin-4-yl)-8-methyl-isoquinolin-3-yl]-3-ethyl-urea;
   - 1-ethyl-3-[6-(6-fluoro-pyridin-3-yl)-8-methyl-isoquinolin-3-yl]-urea;
   - 1-ethyl-3-[6-(2-fluoro-pyridin-3-yl)-8-methyl-isoquinolin-3-yl]-urea;
   - 1-ethyl-3-[6-(6-hydroxymethyl-pyridin-3-yl)-8-methyl-isoquinolin-3-yl]-urea;
   - 1-ethyl-3-[6-(5-fluoro-pyridin-3-yl)-8-methyl-isoquinolin-3-yl]-urea;
   - 1-ethyl-3-[6-(5-methanesulfonyl-pyridin-3-yl)-8-methyl-isoquinolin-3-yl]-urea;
   - 1-ethyl-3-(8'-methyl-[5,6']biisoquinolinyl-3'-yl)-urea;
   - 1-ethyl-3-(8-methyl-6-o-tolyl-isoquinolin-3-yl)-urea;
   - *N-*[3-(3-ethyl-ureido)-8-methyl-isoquinolin-6-yl]-benzamide;
   - 1-[6-(3-cyano-phenyl)-8-methyl-isoquinolin-3-yl]-3-ethyl-urea;
   - 1-[6-(4-acetyl-phenyl)-8-methyl-isoquinolin-3-yl]-3-ethyl-urea;
   - 1-ethyl-3-[6-(2-hydroxy-phenyl)-8-methyl-isoquinolin-3-yl]-urea;
   - 1-[6-benzylamino-5-fluoro-8-(2-methoxy-pyrimidin-5-yl)-isoquinolin-3-yl]-3-ethyl-urea;
   - 4-[8-chloro-3-(3-ethyl-ureido)-5-fluoro-isoquinolin-6-yl]-benzenesulfonamide;
   - 1-ethyl-3-(5-fluoro-6,8-di-quinolin-3-yl-isoquinolin-3-yl)-urea;
   - *N-*{3-[3-(3-ethyl-ureido)-5-fluoro-8-pyridin-3-yl-isoquinolin-6-yl]-phenyl}-acetamide;
   - *N-*{3-[3-(3-ethyl-ureido)-5-fluoro-8-(2-methoxy-pyrimidin-5-yl)-isoquinolin-6-yl]-phenyl} -acetamide;
   - 1-ethyl-3-(8-methyl-6-propylamino-isoquinolin-3-yl)-urea;
   - *N-*[3-(3-ethyl-ureido)-8-methyl-isoquinolin-6-yl]-isonicotinamide;
   - *N-*[3-(3-ethyl-ureido)-8-methyl-isoquinolin-6-yl]-4-methanesulfonyl-benzamide;
   - 3-dimethylamino-*N-*[3-(3-ethyl-ureido)-8-methyl-isoquinolin-6-yl]-benzamide;
   - *N-*[3-(3-ethyl-ureido)-8-methyl-isoquinolin-6-yl]-nicotinamide;
   - *N-*[3-(3-ethyl-ureido)-8-methyl-isoquinolin-6-yl]-4-methoxy-benzamide;
   - 4-dimethylamino-*N-*[3-(3-ethyl-ureido)-8-methyl-isoquinolin-6-yl]-benzamide;
   - 1-ethyl-3-{8-methyl-6-[(pyridin-4-ylmethyl)-amino]-isoquinolin-3-yl}-urea;
   - 4-[3-(3-ethyl-ureido)-isoquinolin-8-yl]-benzoic acid;
   - 4-[3-(3-ethyl-ureido)-isoquinolin-5-yl]-benzoic acid;
   - 1-ethyl-3-(8-methoxy-5-pyridin-4-yl-isoquinolin-3-yl)-urea;
   - 3-{[3-(3-ethyl-ureido)-8-methyl-isoquinolin-6-ylamino]-methyl}-benzoic acid methyl ester;
   - 4-{[3-(3-ethyl-ureido)-8-methyl-isoquinolin-6-ylamino]-methyl}-benzoic acid;
   - 3-{[3-(3-ethyl-ureido)-8-methyl-isoquinolin-6-ylamino]-methyl}-benzoic acid;
   - 1-ethyl-3-{6-[3-(2-hydroxy-ethoxy)-benzylamino]-8-methyl-isoquinolin-3-yl} -urea;
   - 1-ethyl-3-{8-methyl-6-[(4-methyl-3,4-dihydro-2*H-*benzo[1,4]oxazin-7-ylmethyl)-amino]-isoquinolin-3-yl}-urea;
   - 1-ethyl-3-{8-methyl-6-[4-(3-methyl-[1,2,4]triazol-1-yl)-benzylamino]-isoquinolin-3-yl}-urea;
   - 1-[6-(2-benzyloxy-ethylamino)-8-methyl-isoquinolin-3-yl]-3-ethyl-urea;
   - 1-{6-[(cyclopropylmethyl)-amino]-8-methyl-isoquinolin-3-yl}-3-ethyl-urea;
   - 1-ethyl-3-{6-[(1*H*-indol-6-ylmethyl)-amino]-8-methyl-isoquinolin-3-yl}-urea;
   - 1-ethyl-3-{8-methyl-6-[((1-methyl-1*H-*indol-6-yl)methyl)-amino]-isoquinolin-3-yl}-urea;
   - 1-ethyl-3-(6-isobutylamino-8-methyl-isoquinolin-3-yl)-urea;
   - *N*-(4-{[3-(3-ethyl-ureido)-8-methyl-isoquinolin-6-ylamino]-methyl}-phenyl)-acetamide;
   - 1-(2-fluoro-ethyl)-3-(8-methyl-5-pyridin-4-yl-isoquinolin-3-yl)-urea;
   - 1-cyclopropyl-3-(8-methyl-5-pyridin-4-yl-isoquinolin-3-yl)-urea;
   - 1-ethyl-3-[5-(pyridin-4-yl)-8-vinylisoquinolin-3-yl]-urea;
   - 1-ethyl-3-[5-fluoro-8-methyl-6-(5-methylpyridin-3-yl)isoquinolin-3-yl]-urea;
   - *N*-{3-[3-(3-ethyl-ureido)-5-fluoro-8-methyl-isoquinolin-6-yl]-phenyl} -acetamide;
   - 1-ethyl-3-[5-fluoro-8-methyl-6-(quinolin-3-yl)-isoquinolin-3-yl]-urea;
   - 4-[3-(3-ethyl-ureido)-5-fluoro-8-methyl-isoquinolin-6-yl]-benzenesulfonamide;
   - 1-ethyl-3-[8-(pyridin-3-ylamino)-isoquinolin-3-yl]-urea;
   - 1-ethyl-3-[8-(pyridin-2-ylamino)-isoquinolin-3-yl]-urea;
   - 1-ethyl-3-[8-(pyridin-3-yloxy)-isoquinolin-3-yl]-urea;
   as well as the salts (in particular the pharmaceutically acceptable salts) thereof.
82) Further particularly preferred compounds are the following compounds of formula I as defined in embodiment 1) or 2):
   - 1-ethyl-3-[8-(pyrazin-2-ylamino)-isoquinolin-3-yl]-urea;
   - 1-ethyl-3-[8-(pyrimidin-5-ylamino)-isoquinolin-3-yl]-urea;
   - 1-ethyl-3-[8-(imidazo[1,2-*a*]pyridin-7-ylamino)-isoquinolin-3-yl]-urea;
   - 1-ethyl-3-[8-(pyrimidin-4-ylamino)-isoquinolin-3-yl]-urea;
   - 1-ethyl-3-[8-(pyrimidin-2-ylamino)-isoquinolin-3-yl]-urea;
   - 1-ethyl-3-[8-(pyridin-4-ylamino)-isoquinolin-3-yl]-urea;
   - 1-ethyl-3-[8-(5-methyl-[1,3,4]oxadiazol-2-ylamino)-isoquinolin-3-yl]-urea;
   - 1-ethyl-3-[8-(6-methyl-pyridin-2-ylamino)-isoquinolin-3-yl]-urea;
   - 6-[3-(3-ethyl-ureido)-isoquinolin-8-ylamino]-nicotinamide;
   - 1-ethyl-3-[8-(3-fluoro-pyridin-4-ylamino)-isoquinolin-3-yl]-urea;
   - 1-ethyl-3-[8-(4-fluoro-pyridin-2-ylamino)-isoquinolin-3-yl]-urea;
   - 1-ethyl-3-[8-(5-fluoro-pyridin-3-ylamino)-isoquinolin-3-yl]-urea;
   - 1-ethyl-3-[8-(4-methoxy-pyridin-2-ylamino)-isoquinolin-3-yl]-urea;
   - *N*-{5-[3-(3-ethyl-ureido)-isoquinolin-8-ylamino]-pyridin-2-yl}-acetamide;
   - 1-ethyl-3-[8-(4-methyl-pyridin-2-ylamino)-isoquinolin-3-yl]-urea;
   - 1-ethyl-3-[8-(5-methyl-pyridin-2-ylamino)-isoquinolin-3-yl]-urea;
   - 1-ethyl-3-[8-(6-methyl-pyridin-3-ylamino)-isoquinolin-3-yl]-urea;
   - 6-[3-(3-ethyl-ureido)-isoquinolin-8-ylamino]-nicotinic acid methyl ester;
   - 6-[3-(3-ethyl-ureido)-isoquinolin-8-ylamino]-pyridine-2-carboxylic acid methyl ester;
   - 1-ethyl-3-[8-(3-methyl-pyridin-2-ylamino)-isoquinolin-3-yl]-urea;
   - 1-ethyl-3-[8-(3-methoxy-phenylamino)-isoquinolin-3-yl]-urea;
   - 1-ethyl-3-(8-phenylamino-isoquinolin-3-yl)-urea;
   - 1-ethyl-3-[8-(3-hydroxy-phenylamino)-isoquinolin-3-yl]-urea;
   - 3-[3-(3-ethyl-ureido)-isoquinolin-8-ylamino]-benzenesulfonamide;
   - *N*-{3-[3-(3-ethyl-ureido)-isoquinolin-8-ylamino]-phenyl}-acetamide;
   - *N*-{4-[3-(3-ethyl-ureido)-isoquinolin-8-ylamino]-phenyl}-acetamide;
   - 1-[8-(4-cyano-phenylamino)-isoquinolin-3-yl]-3-ethyl-urea;
   - 1-ethyl-3-[8-(4-hydroxy-phenylamino)-isoquinolin-3-yl]-urea;
   - 3-[3-(3-ethyl-ureido)-isoquinolin-8-ylamino]-benzoic acid methyl ester;
   - 1-ethyl-3-[8-(5-methoxy-pyridin-3-ylamino)-isoquinolin-3-yl]-urea;
   - 1-ethyl-3-[8-(6-methoxy-pyridin-2-ylamino)-isoquinolin-3-yl]-urea;
   - 4-[3-(3-ethyl-ureido)-isoquinolin-8-ylamino]-benzoic acid methyl ester;
   - 1-ethyl-3-[8-(2-methoxy-pyridin-4-ylamino)-isoquinolin-3-yl]-urea;
   - 1-ethyl-3-[8-(5-methoxy-pyridin-2-ylamino)-isoquinolin-3-yl]-urea;
   - 1*H-*pyrrole-2-carboxylic acid [3-(3-ethyl-ureido)-isoquinolin-8-yl]-amide;
   - 3*H-*[1,2,3]triazole-4-carboxylic acid [3-(3-ethyl-ureido)-isoquinolin-8-yl]-amide;
   - 1-ethyl-3-{6-[(1*H*-indazol-6-ylmethyl)-amino]-8-methyl-isoquinolin-3-yl}-urea;
   - 1-ethyl-3-{8-methyl-6-[(2-morpholin-4-yl-pyridin-4-ylmethyl)-amino]-isoquinolin-3-yl}-urea;
   - 2-(4-{[3-(3-ethyl-ureido)-8-methyl-isoquinolin-6-ylamino]-methyl}-phenoxy)-acetamide;
   - 1-ethyl-3-[8-methyl-6-(3-[1,2,4]triazol-1-yl-benzylamino)-isoquinolin-3-yl]-urea;
   - 1-{6-[(3-((1*H-*1,2,4-triazol-1-yl)methyl)benzyl)amino]-8-methylisoquinolin-3-yl}-3-ethylurea;
   - 4-{[3-(3-ethyl-ureido)-8-methyl-isoquinolin-6-ylamino]-methyl}-*N*-methyl-benzenesulfonamide;
   - 1-ethyl-3-[8-(pyridin-3-ylaminomethyl)-isoquinolin-3-yl]-urea;
   - 1-{8-[(2,5-dimethyl-2*H*-pyrazol-3-ylamino)-methyl]-isoquinolin-3-yl}-3-ethyl-urea;
   - 1-ethyl-3- {8-[(3-methyl-isothiazol-5-ylamino)-methyl]-isoquinolin-3 yl}-urea;
   - *N-*[3-(3-ethyl-ureido)-8-methyl-isoquinolin-6-yl]-terephthalamic acid methyl ester;
   - *N-*[3-(3-ethyl-ureido)-8-methyl-isoquinolin-6-yl]-isophthalamic acid methyl ester;
   - *N-*[3-(3-ethyl-ureido)-8-methyl-isoquinolin-6-yl]-2-methoxy-acetamide;
   - 1-ethyl-3- {6-[(6-methoxy-pyridin-3-ylmethyl)-amino]-8-methyl-isoquinolin-3-yl}-urea;
   - 1-ethyl-3-[6-(3-methoxy-benzylamino)-8-methyl-isoquinolin-3-yl]-urea;
   - 3-[3-(3-ethyl-ureido)-isoquinolin-8-ylamino]-benzoic acid;
   - 3-[3-(3-ethyl-ureido)-isoquinolin-8-ylamino]-*N*,*N-*dimethyl-benzamide;
   - 3-[3-(3-ethyl-ureido)-isoquinolin-8-ylamino]-*N-*methyl-benzamide;
   - *N-*benzyl-3-[3-(3-ethyl-ureido)-isoquinolin-8-ylamino]-benzamide;
   - 4-[3-(3-ethyl-ureido)-isoquinolin-8-ylamino]-benzoic acid;
   - 4-[3-(3-ethyl-ureido)-isoquinolin-8-ylamino]-*N,N-*dimethyl-benzamide;
   - 4-[3-(3-ethyl-ureido)-isoquinolin-8-ylamino]-*N-*methyl-benzamide;
   - {4-[3-(3-ethyl-ureido)-isoquinolin-8-ylamino]-phenyl}-acetic acid;
   - 2-{4-[3-(3-ethyl-ureido)-isoquinolin-8-ylamino]-phenyl}-N,N-dimethyl-acetamide;
   - 2-{4-[3-(3-ethyl-ureido)-isoquinolin-8-ylamino]-phenyl}-*N-*methyl-acetamide;
   - *N*-benzyl-2-{4-[3-(3-ethyl-ureido)-isoquinolin-8-ylamino]-phenyl}-acetamide;
   - {3-[3-(3-ethyl-ureido)-isoquinolin-8-ylamino]-phenyl}-acetic acid;
   - *N*-benzyl-2-{3-[3-(3-ethyl-ureido)-isoquinolin-8-ylamino]-phenyl}-acetamide;
   - 2-{3-[3-(3-ethyl-ureido)-isoquinolin-8-ylamino]-phenyl}-*N*,*N-*dimethyl-acetamide;
   - 4-[3-(3-ethyl-ureido)-isoquinolin-8-ylmethoxy]-nicotinic acid methyl ester;
   - 1-ethyl-3-[8-(3-hydroxy-phenoxymethyl)-isoquinolin-3-yl]-urea;
   - 1-ethyl-3-[8-(6-methoxy-pyridin-3-ylamino)-isoquinolin-3-yl]-urea;
   - 1-ethyl-3-(5-fluoro-8-pyridin-3-yl-isoquinolin-3-yl)-urea;
   - 1-ethyl-3-(8-methyl-6-vinyl-isoquinolin-3-yl)-urea;
   - 1-ethyl-3-[8-(6-methoxy-pyridin-2-ylamino)-5-pyridin-4-yl-isoquinolin-3-yl]-urea;
   - 1-ethyl-3-[8-(6-methyl-pyridin-3-ylamino)-5-pyridin-4-yl-isoquinolin-3-yl]-urea;
   - 1-ethyl-3-[8-(6-methyl-pyridin-2-ylamino)-5-pyridin-4-yl-isoquinolin-3-yl]-urea;
   - 3-[3-(3-ethyl-ureido)-5-pyridin-4-yl-isoquinolin-8-ylamino]-benzoic acid methyl ester;
   - 1-ethyl-3-[5-(4-hydroxymethyl-phenyl)-8-(pyridin-3-yloxy)-isoquinolin-3-yl]-urea;
   - 1-ethyl-3-[5-pyridin-4-yl-8-(pyridin-3-yloxy)-isoquinolin-3-yl]-urea;
   - 1-ethyl-3-[5-pyridin-3-yl-8-(pyridin-3-yloxy)-isoquinolin-3-yl]-urea;
   - 1-ethyl-3-[5-pyridin-3-yl-8-(pyridin-3-ylamino)-isoquinolin-3-yl]-urea;
   - 1-ethyl-3-[5-(2-methoxy-pyridin-4-yl)-8-(pyridin-3-ylamino)-isoquinolin-3-yl]-urea;
   - 1-ethyl-3-[5-(4-hydroxymethyl-phenyl)-8-(pyridin-3-ylamino)-isoquinolin-3-yl]-urea;
   - 1-ethyl-3-[5-pyridin-4-yl-8-(pyridin-3-ylamino)-isoquinolin-3-yl]-urea;
   - 1-ethyl-3-[5-(4-hydroxy-phenyl)-8-(pyridin-3-ylamino)-isoquinolin-3-yl]-urea;
   - 1-ethyl-3-[5-(2-methyl-pyridin-4-yl)-8-(pyridin-3-ylamino)-isoquinolin-3-yl]-urea;
   - 4-[3-(3-ethyl-ureido)-8-(pyridin-3-ylamino)-isoquinolin-5-yl]-benzenesulfonamide;
   - 1-[5-(2,6-dimethyl-pyridin-4-yl)-8-(pyridin-3-ylamino)-isoquinolin-3-yl]-3-ethyl-urea;
   - 1-(5-(3-(aminomethyl)phenyl)-8-(pyridin-3-ylamino)isoquinolin-3-yl)-3-ethylurea;
   - 1-[5-(2-amino-pyridin-4-yl)-8-(pyridin-3-ylamino)-isoquinolin-3-yl]-3-ethyl-urea;
   - *N*-{3-[3-(3-ethyl-ureido)-5-fluoro-8-(pyridin-3-ylamino)-isoquinolin-6-yl]-phenyl}-acetamide;
   - 1-ethyl-3-[5-fluoro-6-(5-methyl-pyridin-3-yl)-8-(pyridin-3-ylamino)-isoquinolin-3-yl]-urea;
   - *N*-{3-[3-(3-ethyl-ureido)-8-(pyridin-3-ylamino)-isoquinolin-6-yl]-phenyl}-acetamide;
   - 1-ethyl-3-[6-(5-methyl-pyridin-3-yl)-8-(pyridin-3-ylamino)-isoquinolin-3-yl]-urea;
   - 1-ethyl-3-[6-propylamino-8-(pyridin-3-ylamino)-isoquinolin-3-yl]-urea;
   - 1-[6-benzylamino-8-(pyridin-3-ylamino)-isoquinolin-3-yl]-3-ethyl-urea;
   - 1-[6-benzylamino-5-fluoro-8-(pyridin-3-ylamino)-isoquinolin-3-yl]-3-ethyl-urea;
   - 3-dimethylamino-*N-*[3-(3-ethyl-ureido)-8-(pyridin-3-ylamino)-isoquinolin-6-yl]-benzamide;
   - 5-[3-(3-ethyl-ureido)-isoquinolin-8-ylamino]-2-methoxy-benzamide;
   - 5-[3-(3-ethyl-ureido)-isoquinolin-8-ylamino]-2-methoxy-*N-*methyl-benzamide;
   - 5-[3-(3-ethyl-ureido)-isoquinolin-8-ylamino]-2-methoxy-*N*,*N-*dimethyl-benzamide;
   - 1-ethyl-3-[5-(2-methyl-pyridin-4-yl)-8-(6-methyl-pyridin-3-ylamino)-isoquinolin-3-yl]-urea;
   - 3-[3-(3-ethyl-ureido)-5-(2-methyl-pyridin-4-yl)-isoquinolin-8-ylamino]-benzoic acid;
   - 3-[3-(3-ethyl-ureido)-5-(2-methyl-pyridin-4-yl)-isoquinolin-8-ylamino]-N-methyl-benzamide;
   - 3-[3-(3-ethyl-ureido)-5-(2-methyl-pyridin-4-yl)-isoquinolin-8-ylamino]-*N,N*-dimethyl-benzamide;
   - 3-[3-(3-ethyl-ureido)-5-(4-hydroxymethyl-phenyl)-isoquinolin-8-ylamino]-benzoic acid;
   - 3-[3-(3-ethyl-ureido)-5-(4-hydroxymethyl-phenyl)-isoquinolin-8-ylamino]-*N-*methyl-benzamide;
   - 3-[3-(3-ethyl-ureido)-5-(4-hydroxymethyl-phenyl)-isoquinolin-8-ylamino]-*N*,*N-*dimethyl-benzamide;
   - 4-[3-(3-ethyl-ureido)-isoquinolin-8-ylamino]-benzamide;
   - 2-{4-[3-(3-ethyl-ureido)-isoquinolin-8-ylamino]-phenyl}-acetamide;
   - 3-[3-(3-ethyl-ureido)-isoquinolin-8-ylamino]-benzamide;
   - 2-{3-[3-(3-ethyl-ureido)-isoquinolin-8-ylamino]-phenyl}-acetamide;
   - 1-ethyl-3-[8-(6-methyl-pyridin-3-yloxymethyl)-isoquinolin-3-yl]-urea;
   - 1-ethyl-3-[8-(5-methyl-pyridin-2-yloxymethyl)-isoquinolin-3-yl]-urea;
   - 2-[3-(3-ethyl-ureido)-isoquinolin-8-ylmethoxy]-benzoic acid methyl ester;
   - 1-ethyl-3-[8-(2-methyl-pyridin-3-yloxymethyl)-isoquinolin-3-yl]-urea;
   - 1-[8-(3-amino-phenoxymethyl)-isoquinolin-3-yl]-3-ethyl-urea;
   - 1-ethyl-3-[8-(pyridin-2-yloxymethyl)-isoquinolin-3-yl]-urea;
   - 1-ethyl-3-[8-(pyridin-4-yloxymethyl)-isoquinolin-3-yl]-urea;
   - 1-ethyl-3-[8-(pyridin-2-ylaminomethyl)-isoquinolin-3-yl]-urea;
   as well as the salts (in particular the pharmaceutically acceptable salts) thereof.
83) Yet further particularly preferred compounds are the following compounds of formula I as defined in embodiment 1):
   - 5-[3-(3-ethyl-ureido)-isoquinolin-8-ylamino]-2-methyl-benzoic acid methyl ester;
   - 5-[3-(3-ethyl-ureido)-isoquinolin-8-ylamino]-2-methyl-benzoic acid;
   - 4-[3-(3-ethyl-ureido)-isoquinolin-8-ylamino]-2-methyl-benzoic acid methyl ester;
   - 4-[3-(3-ethyl-ureido)-isoquinolin-8-ylamino]-2-methyl-benzoic acid;
   - 1-[5,8-*bis*-(pyridin-3-ylamino)-isoquinolin-3-yl]-3-ethyl-urea;
   - 1-ethyl-3-(8-methyl-5-methylaminomethyl-isoquinolin-3-yl)-urea;
   - 1-ethyl-3- {5-[(methylamino)methyl]-8-(pyridin-3-ylamino)-isoquinolin-3-yl} -urea;
   - 1-ethyl-3- {8-[(6-methoxy-pyridin)-2-ylamino]-5-[(methylamino)methyl]-isoquinolin-3-yl}-urea;
   - 3-{[3-(3-ethyl-ureido)-5-[(methylamino)methyl]-isoquinolin-8-yl]amino}-benzoic acid;
   - 1-{5-[(dimethylamino)methyl]-8-[(6-methoxy-pyridin-2-yl)amino]-isoquinolin-3-yl} - 3-ethyl-urea;
   - 1-ethyl-3-[5-morpholin-4-yl-8-(pyridin-3-ylamino)-isoquinolin-3-yl]-urea;
   - 1-ethyl-3-{8-[(6-methoxy-pyridin-2-yl)amino]-5-(morpholin-4-ylmethyl)-isoquinolin-3-yl}-urea;
   - 1-ethyl-3-[8-(6-methoxy-pyridin-2-ylamino)-5-(4-methyl-piperazin-1-ylmethyl)-isoquinolin-3-yl]-urea;
   - 1-ethyl-3-[5-(4-methyl-piperazin-1-yl)-8-(pyridin-3-ylamino)-isoquinolin-3-yl]-urea;
   - 1-ethyl-3-(8-methyl-5-pyrimidin-4-yl-isoquinolin-3-yl)-urea;
   - 1-ethyl-3-(8-methyl-5-pyridazin-4-yl-isoquinolin-3-yl)-urea;
   - 1-ethyl-3-[5-(1*H-*imidazol-4-yl)-8-methyl-isoquinolin-3-yl]-urea;
   - 1-ethyl-3-[8-methyl-5-(1*H-*pyrazol-4-yl)-isoquinolin-3-yl]-urea;
   - 1-ethyl-3-(8-methyl-5-pyridazin-3-yl-isoquinolin-3-yl)-urea;
   - 1-ethyl-3-[8-methyl-5-(1-methyl-1*H-*imidazol-4-yl)-isoquinolin-3-yl]-urea;
   - 1-ethyl-3-[8-methyl-5-(1-methyl-1*H*-pyrazol-4-yl)-isoquinolin-3-yl]-urea;
   - 1-ethyl-3-(8-methyl-5-thiazol-4-yl-isoquinolin-3-yl)-urea;
   - 1-(8-chloro-5-pyrimidin-4-yl-isoquinolin-3-yl)-3-ethyl-urea;
   - 1-ethyl-3-[8-(6-methyl-pyridin-3-ylamino)-5-pyrimidin-4-yl-isoquinolin-3-yl]-urea;
   - 1-[8-chloro-5-(1*H*-imidazol-4-yl)-isoquinolin-3-yl]-3-ethyl-urea;
   - 1-(8-chloro-5-pyrimidin-4-yl-isoquinolin-3-yl)-3-ethyl-urea;
   - 1-ethyl-3-[8-(6-methoxy-pyridin-2-ylamino)-5-thiazol-4-yl-isoquinolin-3-yl]-urea;
   - 1-ethyl-3-[8-(2-methoxy-pyridin-3-ylamino)-isoquinolin-3-yl]-urea;
   - 1-ethyl-3-[8-(4-hydroxymethyl-pyrazol-1-ylmethyl)-isoquinolin-3-yl]-urea;
   - 1-ethyl-3-[8-(5-hydroxymethyl-imidazol-1-ylmethyl)-isoquinolin-3-yl]-urea;
   - 1-ethyl-3-[8-(4-hydroxymethyl-imidazol-1-ylmethyl)-isoquinolin-3-yl]-urea;
   - 1-[3-(3-ethyl-ureido)-isoquinolin-8-ylmethyl]-1*H*-imidazole-2-carboxylic acid;
   - 1-ethyl-3-(8-pyrazol-1-ylmethyl-isoquinolin-3-yl)-urea;
   - 1-[8-(2,4-dimethyl-imidazol-1-ylmethyl)-isoquinolin-3-yl]-3-ethyl-urea;
   - 1-[8-(2,5-dimethyl-imidazol-1-ylmethyl)-isoquinolin-3-yl]-3-ethyl-urea;
   - 1-ethyl-3-[8-(2-methyl-imidazol-1-ylmethyl)-isoquinolin-3-yl]-urea;
   - 1-ethyl-3-(8-[1,2,3]triazol-1-ylmethyl-isoquinolin-3-yl)-urea;
   - 1-ethyl-3-(8-imidazol-1-ylmethyl-isoquinolin-3-yl)-urea;
   - 1-ethyl-3-[8-(4-methyl-pyrazol-1-ylmethyl)-isoquinolin-3-yl]-urea;
   - 1-ethyl-3-{8-[(5-methyl-isoxazol-3-ylamino)-methyl]-isoquinolin-3-yl}-urea;
   - 1-ethyl-3-{8-[(6-methoxy-pyridin-3-ylamino)-methyl]-isoquinolin-3-yl}-urea;
   - 1-ethyl-3-{8-[(2-methyl-2*H*-pyrazol-3-ylamino)-methyl]-isoquinolin-3-yl}-urea;
   - 1-ethyl-3-[8-(pyrimidin-2-ylaminomethyl)-isoquinolin-3-yl]-urea;
   as well as the salts (in particular the pharmaceutically acceptable salts) thereof.
84) The invention further relates to the groups of compounds of formula I selected from the group consisting of the compounds listed in one of embodiments 81) to 83), whereby each of said groups of compounds furthermore corresponds to one of embodiments 2) to 80), as well as to the salts (in particular the pharmaceutically acceptable salts) of such compounds.
85) The invention moreover relates to the compounds of formula I as defined in embodiment 1) which are selected from the group consisting of the compounds listed in embodiment 81), the compounds listed in embodiment 82) and the compounds listed in embodiment 83), and to the salts (in particular the pharmaceutically acceptable salts) of such compounds.
86) The invention moreover relates to any individual compound of formula I selected from the group consisting of the compounds listed in embodiments 81) to 83) and to the salts (in particular the pharmaceutically acceptable salts) of such individual compound.

The compounds of formula I according to the present invention, i.e. according to one of embodiments 1) to 86), are suitable for the use as chemotherapeutic active compounds in human and veterinary medicine and as substances for preserving inorganic and organic materials in particular all types of organic materials for example polymers, lubricants, paints, fibres, leather, paper and wood.

The compounds of formula I according to the invention are particularly active against bacteria and bacteria-like organisms. They are therefore particularly suitable in human and veterinary medicine for the prophylaxis and chemotherapy of local and systemic infections caused by these pathogens as well as disorders related to bacterial infections comprising pneumonia, otitis media, sinusitis, bronchitis, tonsillitis, and mastoiditis related to infection by *Streptococcus pneumoniae, Haemophilus influenzae, Moraxella catarrhalis, Staphylococcus aureus, Enterococcus faecalis, Enterococcus faecium, Enterococcus casseliflavus, Staphylococcus epidermidis, Staphylococcus haemolyticus,* or *Peptostreptococcus spp.;* pharyngitis, rheumatic fever, and glomerulonephritis related to infection by *Streptococcus pyogenes,* Groups C and G streptococci, *Corynebacterium diphtheria,* or *Actinobacillus haemolyticum;* respiratory tract infections related to infection by *Mycoplasma pneumoniae, Legionella pneumophila, Streptococcus pneumoniae, Haemophilus influenzae,* or *Chlamydia pneumoniae;* blood and tissue infections, including endocarditis and osteomyelitis, caused by *Staphylococcus aureus, Staphylococcus haemolyticus, Enterococcus faecalis, Enterococcus faecium, Enterococcus durans,* including strains resistant to known antibacterials such as, but not limited to, betalactams, vancomycin, aminoglycosides, quinolones, chloramphenicol, tetracyclines and macrolides; uncomplicated skin and soft tissue infections and abscesses, and puerperal fever related to infection by *Staphylococcus aureus,* coagulase-negative staphylococci (i.e., *Staphylococcus epidermidis, Staphylococcus haemolyticus,* etc.), *Streptococcus pyogenes, Streptococcus agalactiae,* Streptococcal groups C-F, viridans streptococci, *Corynebacterium* spp. or *Clostridium spp.,* uncomplicated acute urinary tract infections related to infection by *Staphylococcus aureus,* coagulase-negative staphylococcal species, or *Enterococcus spp.;* urethritis and cervicitis; sexually transmitted diseases related to infection by *Chlamydia trachomatis, Haemophilus ducreyi**,** Treponema pallidum, Ureaplasma urealyticum,* or *Neiserria gonorrheae;* toxin diseases related to infection by *Staphylococcus aureus* (food poisoning and toxic shock syndrome), or Groups A, B and C streptococci; ulcers related to infection by *Helicobacter pylori;* conjunctivitis, keratitis, and dacrocystitis related to infection by *Chlamydia trachomatis, Neisseria gonorrhoea, Staphylococcus aureus, Streptococcus pneumoniae, Streptococcus pyogenes, Haemophilus influenzae,* or *Listeria* spp.; disseminated *Mycobacterium avium* complex (MAC) disease related to infection by *Mycobacterium avium,* or *Mycobacterium intracellulare;* infections caused by *Mycobacterium* such as *Mycobacterium tuberculosis;* gastroenteritis related to infection by *Campylobacter jejuni;* odontogenic infection related to infection by viridans streptococci; persistent cough related to infection by *Bordetella pertussis;* gas gangrene related to infection by *Clostridium perfringens* or *Bacteroides* **s**pp.; and atherosclerosis or cardiovascular disease related to infection by *Helicobacter pylori* or *Chlamydia pneumoniae.*

The compounds of formula I according to the present invention are further useful for the preparation of a medicament and are suitable for the treatment of infections that are mediated by bacteria such as *Clostridium difficile, Corynebacterium spp., Propionibacterium acnes* and *Bacteroides spp.* They can be used for example in the treatment of, *inter alia,* Gram positive infections (notably those caused by *Staphylococcus aureus**,*** enterococci and streptococci), community acquired pneumonias, skin and skin structure infections, acne vulgaris and infected atopic dermatitis.

The present list of pathogens is to be interpreted merely as examples and in no way as limiting.

The compounds of formula I according to one of embodiments 1) to 86), or the pharmaceutically acceptable salts thereof, may be used for the preparation of a medicament, and are suitable, for the prevention or treatment of a bacterial infection.

As well as in humans, bacterial infections can also be treated using compounds of formula I according to one of embodiments 1) to 86) (or pharmaceutically acceptable salts thereof) in other species like pigs, ruminants, horses, dogs, cats and poultry.

Accordingly, the compounds of formula I according to one of embodiments 1) to 86), or the pharmaceutically acceptable salts thereof, may be used for the preparation of a medicament, and are suitable, for the prevention or treatment of a bacterial infection selected from the group consisting of respiratory tract infections, otitis media, meningitis, skin and soft tissue infections (whether complicated or uncomplicated), pneumonia (including hospital acquired pneumonia), bacteremia, endocarditis, gastrointestinal infections, *Clostridium difficile* infections, sexually transmitted infections, foreign body infections, osteomyelitis, topical infections, opthalmological infections and tuberculosis, and notably for the prevention or treatment of a bacterial infection selected from the group consisting of respiratory tract infections, otitis media, meningitis, skin and soft tissue infections (whether complicated or uncomplicated), pneumonia (including hospital acquired pneumonia) and bacteremia.

In particular, the compounds of formula I according to one of embodiments 1) to 86), or the pharmaceutically acceptable salts thereof, may be used for the preparation of a medicament, and are suitable, for the prevention or treatment of a bacterial infection caused by bacteria selected from the group consisting of *Staphylococcus aureus,* enterococci, pneumococci, streptococci, *Haemophilus influenzae, Moraxella catarrhalis* and *Clostridium difficile.*

The present invention also relates to pharmacologically acceptable salts and to compositions and formulations of compounds of formula I according to one of embodiments 1) to 86).

Any reference to a compound of formula I in this text (and notably in the embodiments presented above) is to be understood as referring also to the salts (and especially the pharmaceutically acceptable salts) of such compounds, as appropriate and expedient.

A pharmaceutical composition according to the present invention contains at least one compound of formula I according to one of embodiments 1) to 86) (or a pharmaceutically acceptable salt thereof) as the active agent and optionally carriers and/or diluents and/or adjuvants, and may also contain additional known antibiotics.

The compounds of formula I according to one of embodiments 1) to 86) and their pharmaceutically acceptable salts can be used as medicaments, e.g. in the form of pharmaceutical compositions for enteral or parenteral administration. The production of the pharmaceutical compositions can be effected in a manner which will be familiar to any person skilled in the art (see for example Remington, The Science and Practice of Pharmacy, 21st Edition (2005), Part 5, "Pharmaceutical Manufacturing" [published by Lippincott Williams & Wilkins]) by bringing the described compounds of formula I or their pharmaceutically acceptable salts, optionally in combination with other therapeutically valuable substances, into a galenical administration form together with suitable, non-toxic, inert, therapeutically compatible solid or liquid carrier materials and, if desired, usual pharmaceutical adjuvants.

The invention also relates to a method for the prevention or the treatment of a bacterial infection, and in particular a method for the treatment of a bacterial infection caused by *Streptococcus pneumoniae* bacteria, in a patient comprising the administration to said patient of a pharmaceutically active amount of a compound of formula I according to one of embodiments 1) to 86) or a pharmaceutically acceptable salt thereof.

Moreover, the compounds of formula I according to one of embodiments 1) to 86) may also be used for cleaning purposes, e.g. to remove pathogenic microbes and bacteria from surgical instruments or to make a room or an area aseptic. For such purposes, the compounds of formula I could be contained in a solution or in a spray formulation.

The compounds of formula I can be manufactured in accordance with the present invention using the procedures described hereafter.

### PREPARATION OF COMPOUNDS OF FORMULA I

### Abbreviations:

The following abbreviations are used throughout the specification and the examples:
- Ac: acetyl
- AcOH: acetic acid
- anhydr.: anhydrous
- aq.: aqueous
- BINAP: (±)-(1,1'-binaphthalene-2,2'-diyl)bis(diphenylphosphine)
- bippyphos: 5-(di-*tert*-butylphosphino)-1',3',5'-triphenyl-1'*H-*[1,4']bipyrazole
- Boc: *tert*-butoxycarbonyl
- BrettPhos: 2-(dicyclohexylphosphino)3,6-dimethoxy-2',4',6'-triisopropyl-1,1'-biphenyl
- Cbz: benzyloxycarbonyl
- CC: column chromatography over silica gel
- conc.: concentrated
- DBU: 1,8-diazabicyclo[5.4.0]undec-7-ene
- DCC: *N,N*'-dicyclohexylcarbodiimide
- DCE: 1,2-dichloroethane
- DCM: dichloromethane
- DEAD: diethyl azodicarboxylate
- DIAD: diisopropyl azodicarboxylate
- DIPEA: *N,N-*diisopropylethylamine
- DMAP: 4-(dimethylamino)pyridine
- DME: 1,2-dimethoxyethane
- DMF: *N,N-*dimethylformamide
- DMSO: dimethylsulfoxide
- DPPA: diphenyl phosphoryl azide
- EA: ethyl acetate
- EDC: 1-ethyl-3-(3-dimethylaminopropyl) carbodiimide hydrochloride
- ESI: Electron Spray Ionisation
- eq.: equivalent
- Et: ethyl
- EtOH: ethanol
- HATU: *O*-(7-azabenzotriazol-1-yl)-*N,N,N'N'*-tetramethyluronium hexafluorophosphate
- Hex: hexane
- Hept: heptane
- HOAT: 1-hydroxy-7-aza-benzotriazole
- HOBT: 1-hydroxybenzotriazole
- HV: high vacuum conditions
- IPr: 1,3-bis(2,6-diisopropylphenyl)-1,3-dihydro-2*H*-imidazol-2-ylidene
- JosiPhos ligands: (*R*)-1-[(SP)-2-(di-*tert*-butylphosphino)ferrocenyl]ethylbis(2-methylphenyl)phosphine or (*R*)-1-[(SP)-2-(di-*tert-*butylphosphino)ferrocenyl]ethyldiphenylphosphine or (*R*)-1-[(SP)-2-(dicyclohexylphosphino)ferrocenyl]ethyldi-*tert*-butylphosphine
- LC: liquid chromatography
- Me: methyl
- MeCN: acetonitrile
- MeOH: methanol
- MS: Mass Spectroscopy
- Ms: methanesulfonyl (mesyl)
- org.: organic
- NMO: 4-methylmorpholine *N*-oxide
- NMP: *N-*methyl-2-pyrrolidone
- PCy₃: tricyclohexylphosphine
- Pd/C: palladium on carbon
- Pd₂(dba)₃: tris[dibenzylideneacetone]dipalladium(0)
- Pd(PPh₃)₄: tetrakis(triphenylphosphine)palladium(0)
- PEPPSI™-IPr: [1,3-bis(2,6-diisopropylphenyl)imidazol-2-ylidene](3-chloropyridyl)palladium(II) dichloride
- Ph: phenyl
- prep-HPLC: preparative HPLC
- prep-TLC: preparative TLC
- Pyr: pyridine
- Q-phos: 1,2,3,4,5-pentaphenyl-1'-(di-*tert*-butylphosphino)ferrocene
- RaNi: Raney-nickel
- rt: room temperature
- sat.: saturated
- S-Phos: 2-dicyclohexylphosphino-2',6'-dimethoxybiphenyl
- SK-CC01-A: 2'-(dimethylamino)-2-biphenylyl-palladium(II) chloride dinorbornylphosphine complex
- SK-CC02-A: 2-(dimethylaminomethyl)ferrocen-1-yl-palladium(II) chloride dinorbornylphosphine complex
- T3P: propylphosphonic anhydride
- tBu: *tert*-butyl
- TEA: triethylamine
- TFA: trifluoroacetic acid
- THF: tetrahydrofuran
- TLC: thin layer chromatography
- TsOH: *p*-toluenesulfonic acid monohydrate
- v/v: proportion by volume
- X-Phos: 2-dicyclohexylphosphino-2',4',6'-triisopropylbiphenyl
- XantPhos: 4,5-bis(diphenylphosphino)-9,9-dimethylxanthene

### General reaction techniques:

### General reaction technique 1 (Suzuki, coupling):

The aromatic halide (typically a bromide) is reacted with the required boronic acid derivative or its boronate ester equivalent (e.g. pinacol ester) in the presence of a palladium catalyst and a base such as K₂CO₃, Cs₂CO₃, K₃PO₄, tBuONa or tBuOK between 20 and 120°C in a solvent such as toluene, THF, dioxane, DME or DMF, usually in the presence of water (20 to 50%). Examples of typical palladium catalysts are triarylphosphine palladium complexes such as Pd(PPh₃)₄. These catalysts can also be prepared *in situ* from a common palladium source such as Pd(OAc)₂ or Pd₂(dba)₃ and a ligand such as trialkylphosphines (e.g. PCy₃ or P(tBu)₃), dialkylphosphinobiphenyls (e.g. S-Phos) or ferrocenylphosphines (e.g. Q-phos). Alternatively, one can use a commercially available precatalyst based on palladacycle (e.g. SK-CC01-A) or *N*-heterocyclic carbene complexes (e.g. PEPPSI™-IPr). The reaction can also be performed by using the corresponding aromatic triflate. Further variations of the reaction are described in Chem. Rev. (1995), 95, 2457-2483, Synthesis (2004), 2419-2440, Aldrichimica Acta (2006), 39, 17-24 and 97-111, Acc. Chem. Res. (2008), 41, 1555-1564, and references cited therein.

### General reaction technique 2 (Negishi coupling);

The aromatic halide (typically a bromide) is reacted with the required organozinc reagent in the presence of a palladium catalyst between 20 and 120°C in a solvent such as toluene, THF, dioxane, DME or DMF. Examples of typical palladium catalysts are triarylphosphine palladium complexes such as Pd(PPh₃)₄. These catalysts can also be prepared *in situ* from a common palladium source such as Pd(OAc)₂ or Pd₂(dba)₃ and a ligand such as trialkylphosphines (e.g. PCy₃ or P(tBu)₃), dialkylphosphinobiphenyls (e.g. S-Phos) or ferrocenylphosphines (e.g. Josiphos). Alternatively, one can use a commercially available precatalyst based on palladacycle (e.g. SK-CC01-A) or *N*-heterocyclic carbene complexes (e.g. PEPPSI™-IPr). The reaction can also be performed by using the corresponding aromatic triflate. Further variations of the reaction are described in Chem. Rev. (1993), 93, 2117-2188, Aldrichimica Acta (2006), 39, 17-24 and 97-111, Acc. Chem. Res. (2008), 41, 1555-1564, Chem. Soc. Rev. (2009), 38, 1598-1607, and references cited therein. In the particular case wherein the methyl organozinc derivative is requested, the reaction is performed starting from dimethyl zinc.

### General reaction technigue 3 (Goldberg-type coupling):

The aromatic halide is reacted with the required amide or lactam in presence of a copper catalyst such as CuI, a 1,2-diamine ligand such as *N,N'*-dimethylethylenediamine or (*R,R*)-(-)-trans-*N,N'*-dimethyl-1,2-cyclohexanediamine, a base such as K₂CO₃ or K₃PO₄ between 20 and 120°C in a solvent such as toluene, THF, dioxane or DMF, as described in J. Am. Chem. Soc. (2002), 124, 7421-7428.

### General reaction technique 4 (Buchwald-Hartwig amination):

The aromatic halide is reacted with the required amine in the presence of a palladium catalyst and a base such as K₂CO₃, Cs₂CO₃, K₃PO₄, tBuONa or tBuOK between 20 and 120°C in a solvent such as toluene, THF, dioxane, DME or DMF. Examples of typical palladium catalysts are triarylphosphine palladium complexes such as Pd(PPh₃)₄. These catalysts can also be prepared *in situ* from a common palladium source such as Pd(OAc)₂ or Pd₂(dba)₃ and a ligand such as trialkylphosphines (e.g. PCy₃ or P(tBu)₃), dialkylphosphinobiphenyls (e.g. X-Phos or BrettPhos), chelating diphosphines (e.g. BINAP, XantPhos) or ferrocenylphosphines (e.g. Q-phos). Alternatively, one can use a commercially available precatalyst based on palladacycle (e.g. SK-CC02-A) or *N-*heterocyclic carbene complexes (e.g. PEPPSI™-IPr). The reaction can also be performed by using the corresponding aromatic triflate. Further variations of the reaction are described in J. Org. Chem. (2000), 65, 1144-1157, Angew. Chem. Int. Ed. (2005), 44, 1371-1375, Aldrichimica Acta (2006), 39, 17-24 and 97-111, Angew. Chem. Int. Ed. (2008), 47, 6338-6361, and references cited therein.

### General reaction technique 5 (Heck coupling):

The aromatic halide is reacted with the required alkene in the presence of a palladium catalyst and a base such as TEA, K₂CO₃, Cs₂CO₃, K₃PO₄, tBuONa or tBuOK between 20 and 120°C in a solvent such as toluene, THF, dioxane, DME or DMF. Examples of typical palladium catalysts are triarylphosphine palladium complexes such as Pd(PPh₃)₄. These catalysts can also be prepared *in situ* from a common palladium source such as Pd(OAc)₂ or Pd₂(dba)₃ and a ligand such as trialkylphosphines (e.g. PCy₃ or P(tBu)₃), dialkylphosphinobiphenyls (e.g. S-Phos), chelating diphosphines (e.g. BINAP) or ferrocenylphosphines (e.g. Q-phos). Alternatively, one can use a commercially available precatalyst based on palladacycle (e.g. SK-CC01-A) or *N*-heterocyclic carbene complexes (e.g. PEPPSI™-IPr). The reaction can also be performed by using the corresponding aromatic triflate. Further variations of the reaction are described in Org. React. (1982), 27, 345-390, Angew. Chem. Int. Ed. Engl. (1994), 33, 2379-2411, Adv. Synth. Catal. (2006), 348, 609-679, Acc. Chem. Res. (2008), 41, 1555-1564, and references cited therein.

### General reaction technique 6 (amide formation):

The carboxylic acid is reacted with the required amine in presence of an activating agent such as DCC, EDC, HOBT, HOAT, T3P, HATU or di-(*N-*succinimidyl)-carbonate, in a dry aprotic solvent such as DCM, MeCN or DMF between -20 and 60°C (see G. Benz in Comprehensive Organic Synthesis, B.M. Trost, I. Fleming, Eds; Pergamon Press: New York (1991), vol. 6, p. 381). Alternatively, the carboxylic acid can first be activated by conversion into its corresponding acid chloride by reaction with oxalyl chloride or thionyl chloride neat or in a solvent such as DCM between -20° and 60°C. Further activating agents can be found in Comprehensive Organic Transformations. A guide to Functional Group Preparations; 2nd Edition, R. C. Larock, Wiley-VC; New York, Chichester, Weinheim, Brisbane, Singapore, Toronto, 1999; Section nitriles, carboxylic acids and derivatives, p. 1941-1949.

### General reaction technique 7 (alcohol activation):

The alcohol is reacted with a sulfonyl chloride derivative such as MsCl, TfCl or TsCl in presence of a base such as TEA in a dry aprotic solvent such as Pyr, THF or DCM between -30 and 50°C. In the case of the triflate or mesylate, Tf₂O or Ms₂O can also be used. These sulfonates can be reacted with a sodium halide such as NaI or NaBr in MeCN or DMF between 40 and 120°C, delivering the corresponding iodide or bromide derivatives. Alternatively, the corresponding chlorides or bromides can also be obtained respectively by reaction of the corresponding alcohol derivatives with POCl₃ either neat or in a solvent such as DCM, MeCN or toluene between 20 and 120°C, or by reaction of the corresponding alcohol derivatives with PBr₃ in a solvent such as DCM, THF or toluene between 20 and 120°C. Further variations of this transformation can be found in Comprehensive Organic Transformations. A guide to Functional Group Preparations; 2nd Edition, R. C. Larock, Wiley-VC; New York, Chichester, Weinheim, Brisbane, Singapore, Toronto, 1999; Section halides, p. 689-703.

### General reaction technique 8 (formation of azides):

The activated alcohol (activated either as a sulfonate or a iodide derivative) is reacted with sodium azide in presence of an organic base such as DIPEA or TEA or an inorganic base such as Na₂CO₃ in a solvent such as DMSO or DMF between 20 and 100°C. Alternatively, the azide can also be obtained by activation of the alcohol under Mitsunobu conditions in presence of PPh₃ and DEAD or DIAD in a solvent such as THF, DMF, DCM or DME between -20 and +60°C as reviewed in Synthesis (1981), 1-28. Alternatively, the alcohol is directly reacted with DPPA in presence of a base such as TEA or DBU in a solvent such as THF between -20 and +60°C as described in J. Org. Chem. (1993), 58, 5886-5888.

### General reaction technique 9 (formation of phthalimides);

The activated alcohol (activated either as a sulfonate or a iodide derivative) is reacted with potassium phthalimide in a solvent such as DMSO or DMF between 20 and 100°C.

### General reaction technique 10 (formation of amines):

The azides are hydrogenated over a noble metal catalyst such as Pd/C in a solvent such as MeOH or EA. In case the molecule is containing an unsaturated double or triple bond, the reduction can be performed using PPh₃ in the presence of water as described in J. Med. Chem. (1993), 36, 2558-68. Besides, the phthalimide derivatives are treated between 50 and 120°C with a hydrazine derivative such as hydrazine hydrate, methylhydrazine or an amine such as *N¹,N¹*-dimethylpropane-1,3-diamine in a solvent such as MeOH or EtOH. Further general methods have been described in Protecting Groups in Organic Synthesis, 3rd Ed (1999), 564-566; T.W. Greene, P.G.M. Wuts (Publisher: John Wiley and Sons, Inc., New York).

### General reaction technique 11 (addition of benzyl amines on an imidate derivative):

The required benzyl amine is reacted with 2,2-diethoxy-ethanimidic acid methyl ester in a solvent such as MeOH between 0 and 70°C as described in WO 2007/125405. If not commercially available, the imidate is obtained by reacting NaOMe with diethoxyacetonitrile in MeOH between 0 and 70°C.

### General reaction technique 12 (isoquinoline formation by cyclization):

The crude intermediate from general reaction technique 11 undergoes a cyclization reaction in conc. H₂SO₄ between 0 and 100°C as described in WO 2007/125405.

### General reaction techniques 13 (reductive amination):

The reaction between the amine and the aldehyde or ketone is performed in a solvent system allowing the removal of the formed water through physical or chemical means (e.g. distillation of the solvent-water azeotrope or presence of drying agents such as molecular sieves, MgSO₄ or Na₂SO₄). Such solvent is typically toluene, Hex, THF, NMP, DCM or DCE or a mixture of solvents such as DCE/MeOH. The reaction can be catalyzed by traces or a stoichiometric amount of acid (usually AcOH or TsOH). The intermediate imine is reduced with a suitable reducing agent (e.g. NaBH₄, NaBHCN₃, or NaBH(OAc)₃) or by hydrogenation over a noble metal catalyst such as Pd/C. The reaction is carried out between -10 and +110°C, preferably between 0 and 60°C. The reaction can also be carried out in one pot. It can also be performed in protic solvents such as MeOH or water in the presence of a picoline-borane complex (Tetrahedron (2004), 60, 7899-7906).

### General reaction technique 1.4 (Ullmann-type coupling):

The aromatic halide is reacted with the required phenol or alcohol in presence of a copper catalyst such as CuI or CuCl, a ligand such as *N,N-*dimethylglycine, a β-diketone (e.g. 2,2,6,6-tetramethyl-3,5-heptanedione) or a phenanthroline derivative (e.g. 3,4,7,8-tetramethyl-1,10-phenanthroline), a base such as Cs₂CO₃ or K₃PO₄ between 20 and 150°C in a solvent such as toluene, dioxane, DMSO or DMF, as described in Angew. Chem. Int. Ed. (2009), 48, 6954-6971.

### General reaction technique 15 (ester hydrolysis):

Representative carboxy protecting groups are alkyl, e.g. methyl, ethyl or *tert-butyl,* haloalkyl, e.g. trichloroethyl, arylalkyl, e.g. benzyl or para nitrobenzyl, alkenyl, e.g. allyl, trialkylsilyl, e.g. trimethylsilyl, *tert*-butyldimethylsilyl or di *tert-*butylmethylsilyl, alkylthioalkyl, e.g. methylthiomethyl (MTM), alkoxyalkoxyalkyl, e.g. methoxyethoxymethyl (MEM), arylalkoxyalkyl, e.g. benzyloxymethyl (BOM), trialkylsilylalkoxyalkyl, e.g. 2-(trimethylsilyl)ethoxymethyl (SEM), trialkylsilylalkyl, e.g. 2-(trimethylsilyl)ethyl (TMSE). Further examples of protecting groups to mask carboxylic acids and the conditions to regenerate them are well known to those skilled in the art, and are listed in reference book such as P.J. Kocienski 'Protecting Groups', Georg Thieme Verlag Stuttgart, New-York, 1994 pp. 118-143. In particular, methyl and ethyl esters are deprotected by saponification with an alkali OH such as NaOH or KOH, benzyl ester by hydrogenolysis over a noble metal catalyst such as Pd/C, and *tert*-butyl ester by treatment with TFA (neat or diluted in an organic solvent such as DCM) or a solution of HCl in an organic solvent such as dioxane.

### General reaction technique 16 (ether formation):

The phenol or the alcohol is first reacted with NaH in a solvent such as THF or DMF between 0 and 80°C. The resulting mixture is treated with the required halide or sulfonate derivative (e.g. mesylate, tosylate or triflate) between 0 and 100°C.

### General reaction technique 17 (amine formation):

The chloride derivative is reacted with an amine in a solvent such as THF, MeCN, DMF or NMP between 0 and 120°C.

### General reaction technique 18 (Stille coupling):

The aromatic halide is reacted with the required organostannane reagent in the presence of a palladium catalyst between 20 and 120°C in a solvent such as toluene, THF, dioxane, DME or DMF. Examples of typical palladium catalysts are triarylphosphine palladium complexes such as Pd(PPh₃)₄. These catalysts can also be prepared *in situ* from a common palladium source such as Pd(OAc)₂ or Pd₂(dba)₃ and a ligand such as trialkylphosphines (e.g. PCy₃ or P(tBu)₃), dialkylphosphinobiphenyls (e.g. X-Phos), ferrocenylphosphines (e.g. Josiphos) or *N-*heterocyclic carbenes (e.g. IPr). The reaction can also be run in presence of copper and fluoride additives (e.g. CuI and CsF) when less reactive organistannanes are used. The reaction can also be performed by using the corresponding aromatic triflate. Further variations of the reaction are described in Angew. Chem. Int. Ed. Engl. (2004), 43, 4704-4734, Acc. Chem. Res. (2008), 41, 1555-1564, Aldrichimica Acta (2006), 39, 97-111, and references cited therein.

### General reaction technigue 19 (removal of amine protecting groups):

The benzyl or benzyl carbamate protecting groups are removed by hydrogenolysis over a noble metal catalyst (e.g. Pd/C or Pd(OH)₂/C). The Boc group is removed under acidic conditions such as HCl in an organic solvent such as MeOH or dioxane, or TFA neat or diluted in a solvent such as DCM. Further general methods to remove amine protecting groups have been described in Protecting Groups in Organic Synthesis, 3rd Ed (1999), 494-653; T.W. Greene, P.G.M. Wuts (Publisher: John Wiley and Sons, Inc., New York).

### General preparation methods:

### Preparation of the compounds of formula I:

The compounds of formula I can be manufactured by the methods given below, by the methods given in the examples or by analogous methods. Optimum reaction conditions may vary with the particular reactants or solvents used, but such conditions can be determined by a person skilled in the art by routine optimisation procedures.

The compounds of formula I can be manufactured in accordance with the present invention by
a) reacting the compounds of formula II 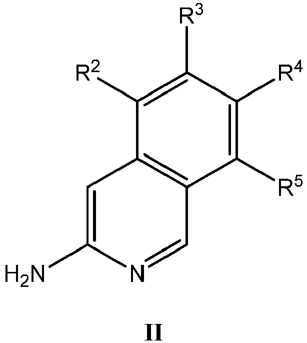 wherein R², R³, R⁴ and R⁵ are as defined in formula I with the compounds of formula III or III*a*

   R¹-N=C=O **III**

   R¹-NH-C(=O)-L⁰ **III*a***

   wherein R¹ is as defined in formula I and L⁰ represents halogen such as chlorine; or
b) reacting the compounds of formula II as defined in item a) with the compounds of formula IV

   L¹-C(=O)-L² **IV**

   wherein L¹ represents halogen such as chlorine and L² represents trichloromethoxy or L¹ and L² both represent trichloromethoxy, *N*-succinimidyloxy, imidazol-1-yl or halogen such as chlorine, followed by reaction with the amines of formula V

   R¹-NH₂ **V**

   wherein R¹ is as defined in formula I; or
c) reacting the compounds of formula VI 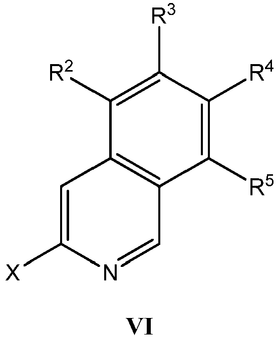 wherein R², R³, R⁴ and R⁵ are as defined in formula I and X represents halogen such as chlorine, with the compounds of formula VII

   R¹-NH-CONH₂ **VII**

   wherein R¹ is as defined in formula I in the presence of a catalyst such as Pd₂(dba)₃, a ligand such as bippyphos and a base such as K₃PO₄ in a solvent such as DME between 60 and 100°C (e.g. as described in Org. Lett. (2009), 11, 947-950); or
d) reacting the compounds of formula VIII 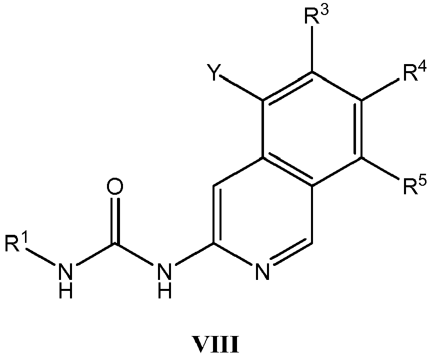 wherein R¹, R³, R⁴ and R⁵ are as defined in formula I and Y represents a halogen such as chlorine or bromine either with the compounds of formula IX

   L³-B(OH)₂ **IX**

   wherein L³ represents vinyl or one of the aromatic or heteroaromatic groups mentioned in the possible meanings for R² in formula I, or with the corresponding boronate esters (e.g. pinacol ester) using general reaction technique 1 (vinylboronic anhydride pyridine complex being however used when L³ represents vinyl); or
e) reacting the compounds of formula X 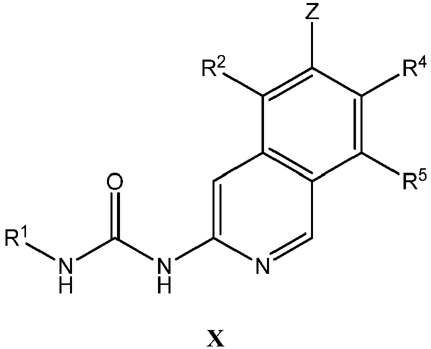 wherein R¹, R², R⁴ and R⁵ are as defined in formula I and Z represents a halogen such as chlorine or bromine either with the compounds of formula XI

   L⁴-B(OH)₂ **XI**

   wherein L⁴ represents vinyl or one of the aromatic or heteroaromatic groups mentioned in the possible meanings for R³ in formula I or with the corresponding boronate esters (e.g. pinacol ester) using general reaction technique 1 (vinylboronic anhydride pyridine complex being however used when L⁴ represents vinyl); or
f) reacting the compounds of formula **XII** 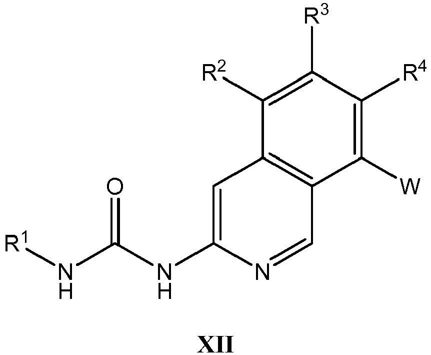 wherein R¹, R², R³ and R⁴ are as defined in formula I and W represents a halogen such as chlorine or bromine either with the compounds of formula XIII

   L⁵-B(OH)₂ **XIII**

   wherein L⁵ represents vinyl or one of the aromatic or heteroaromatic groups mentioned in the possible meanings for R⁵ in formula I, or with the corresponding boronate esters (e.g. pinacol ester) using general reaction technique 1 (vinylboronic anhydride pyridine complex being however used when L⁵ represents vinyl); or
g) reacting the compounds of formula XII as defined in item f) with the compounds of formula XIV

   L⁶-CONH₂ **XIV**

   wherein L⁶ represents a possibly substituted phenyl group using general reaction technique 3; or
h) reacting the compounds of formula X as defined in item e) with the compounds of formula XV

   L⁷-CONH₂ **XV**

   wherein L⁷ represents a possibly substituted phenyl group using general reaction technique 3; or
i) reacting the compounds of formula XII as defined in item f) with the compounds of formula XVI

   L⁸-NH₂ **XVI**

   wherein L⁸ represents R⁸ as defined in formula I or pyridin-3-ylmethyl, using general reaction technique 4; or
j) reacting the compounds of formula X as defined in item e) with the compounds of formula XVII

   L⁹-NH₂ **XVII**

   wherein L⁹ is such that L⁹-NH- corresponds to one of the possible meanings of R³, and in particular wherein L⁹ represents alkyl, cyclopropylmethyl, a possibly substituted benzyl group or pyridin-4-ylmethyl, using general reaction technique 4; or
k) reacting the compounds of formula VIII as defined in item d) with the compounds of formula XVIII

   L¹⁰-NH₂ **XVIII**

   wherein L¹⁰ represents C₁-C₃ alkyl such as ethyl using general reaction technique 4; or
l) reacting the compounds of formula XIX*a* or XIX*b* 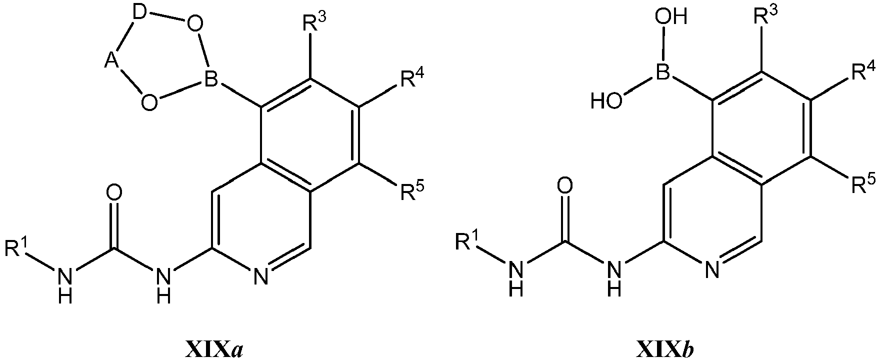 wherein R¹, R³, R⁴ and R⁵ are as defined in formula I and -A-D- represents -C(Me)₂C(Me)₂- or -CH₂C(Me)₂CH₂- with the compounds of formula XX

   L¹¹-X^{a} **XX**

   wherein L¹¹ represents one of the aromatic or heteroaromatic groups mentioned in the possible meanings for R² in formula I and X^{a} represents halogen (such as chlorine and bromine) using general reaction technique 1; or
m) reacting the compounds of formula XXI*a* or XXI*b* 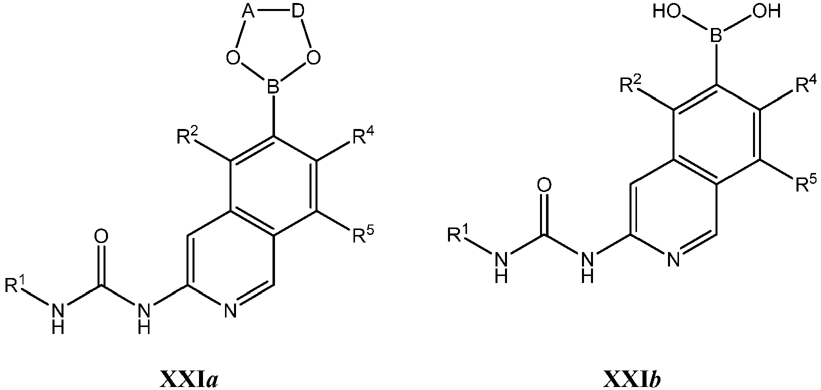 wherein R¹, R², R⁴ and R⁵ are as defined in formula I and -A-D- represents -C(Me)₂C(Me)₂- or -CH₂C(Me)₂CH₂- with the compounds of formula XXII

   L¹²-X^{b} **XXII**

   wherein L¹² represents one of the aromatic or heteroaromatic groups mentioned in the possible meanings for R³ in formula I and X^{b} represents halogen (such as chlorine and bromine) using general reaction technique 1; or
n) reacting the compounds of formula XXIII*a* or XXIII*b* 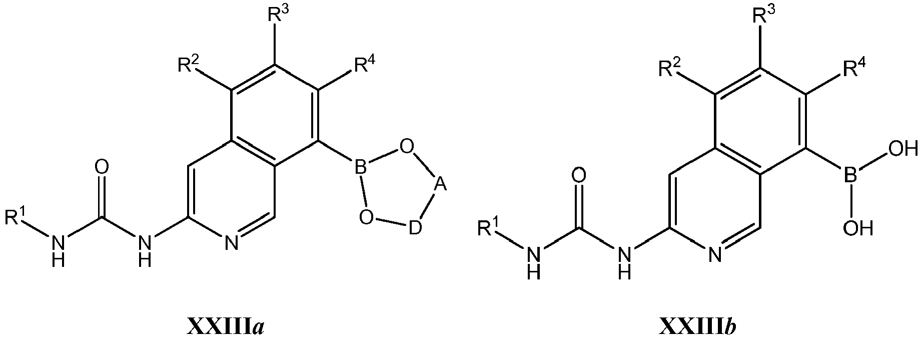 wherein R¹, R², R³ and R⁴ are as defined in formula I and -A-D- represents -C(Me)₂C(Me)₂- or -CH₂C(Me)₂CH₂- with the compounds of formula XXIV

   L¹³-X^{c} **XXIV**

   wherein L¹³ represents one of the aromatic or heteroaromatic groups mentioned in the possible meanings for R⁵ in formula I and X^{c} represents halogen (such as chlorine and bromine) using general reaction technique 1; or
o) reacting the compounds of formula XXV 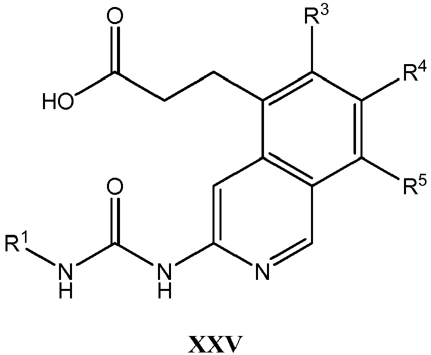 wherein R¹, R³, R⁴ and R⁵ are as defined in formula I with ammonia following general reaction technique 6; or
p) reacting the compounds of formula XXVI 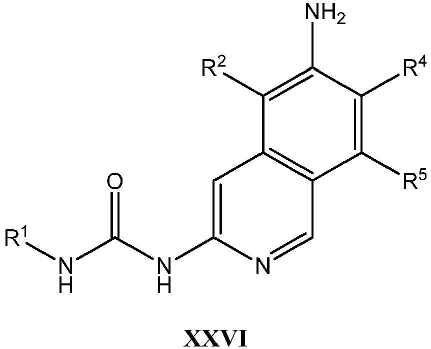 wherein R¹, R², R⁴ and R⁵ are as defined in formula I with the compounds of formula XXVII

   L¹⁴-COOH **XXVII**

   wherein L¹⁴ is such that L¹⁴-CONH- corresponds to one of the possible meanings of R³, and in particular wherein L¹⁴ represents methoxymethyl, thiazol-5-yl, pyridin-3-yl, pyridin-4-yl or a phenyl group substituted by the groups B⁶³ and B⁶⁴ as defined in formula I, following general reaction technique 6; or
q) reacting the compounds of formula XXVIII 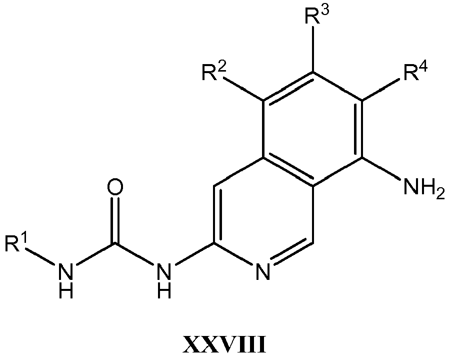 wherein R¹, R², R³ and R⁴ are as defined in formula I with the compounds of formula XXIX

   L¹⁵-COOH **XXIX**

   wherein L¹⁵ is such that L¹⁵-CONH- corresponds to one of the possible meanings of R⁵, and in particular wherein L¹⁵ represents phenyl, pyrrol-2-yl or *1H-*1,2,3-triazol-5-yl, following general reaction technique 6; or
r) reacting the compounds of formula XXX 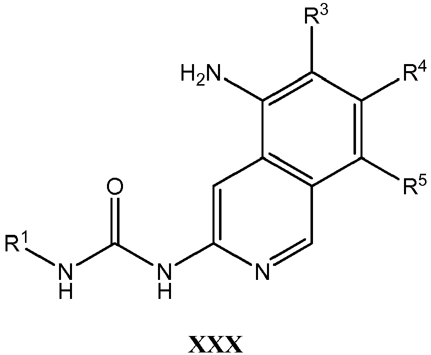 wherein R¹, R³, R⁴ and R⁵ are as defined in formula I with the compounds of formula XXXI

   L¹⁶-CHO **XXXI**

   wherein L¹⁶ is such that -NH-CH₂-L¹⁶ corresponds to one of the possible meanings for R² in formula I following general reaction technique 13; or
s) reacting the compounds of formula XXVI as defined in item p) with the compounds of formula XXXII

   L¹⁷-CHO **XXXII**

   wherein L¹⁷ is such that -NH-CH₂-L¹⁷ corresponds to one of the possible meanings for R³ in formula I following general reaction technique 13; or
t) reacting the compounds of formula XXVIII as defined in item q) with the compounds of formula XXXIII

   L¹⁸-CHO **XXXIII**

   wherein L¹⁸ is such that -NH-CH₂-L¹⁸ corresponds to one of the possible meanings for R⁵ in formula I following general reaction technique 13; or
u) reacting the compounds of formula XII as defined in item f) with the compounds of formula XXXIV

   L¹⁹-ZnX^{d} **XXXIV**

   wherein L¹⁹ represents methyl or benzyl and X^{d} represents a halogen such as bromine using general reaction technique 2, Me₂Zn being however used when L¹⁹ is methyl; or
v) reacting the compounds of formula XII as defined in item f) with the compounds of formula XXXV

   L²⁰-OH **XXXV**

   wherein L²⁰ represents pyridin-3-yl following general reaction technique 14; or
w) reacting the compounds of formula XXXVI 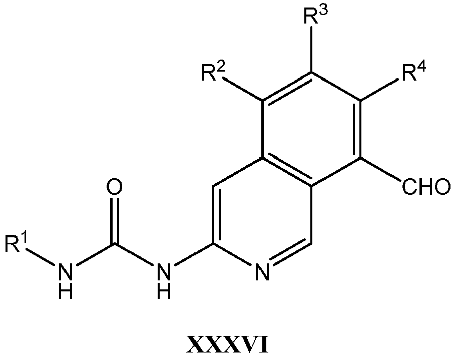 wherein R¹, R², R³ and R⁴ are as defined in formula I with the compounds of formula XXXVII

   L²¹-NH₂ **XXXVII**

   wherein L²¹ is such that L²¹-NHCH₂- corresponds to one of the possible meanings of R⁵, and in particular wherein L²¹ represents pyridin-2-yl, pyridin-3-yl, 1,3-dimethyl-1*H*-pyrazol-5-yl or 3-methylisothiazol-5-yl, using general reaction technique 13; or
x) deprotecting the compounds of formula XXXVIII 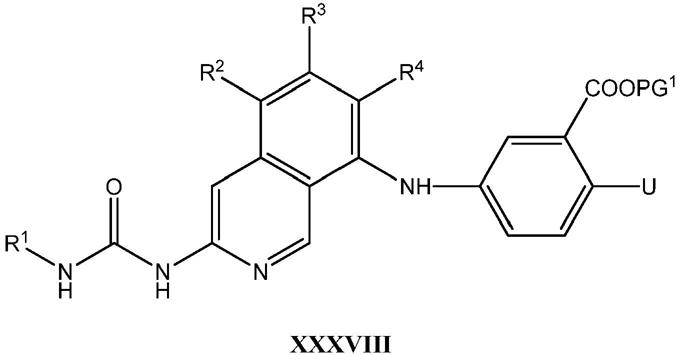 wherein R¹, R², R³ and R⁴ are as defined in formula I, U represents H or methyl and PG¹ represents a carboxylic acid protecting group (in particular methyl or *tert*-butyl), using general reaction technique 15; or
y) deprotecting the compounds of formula XXXIX 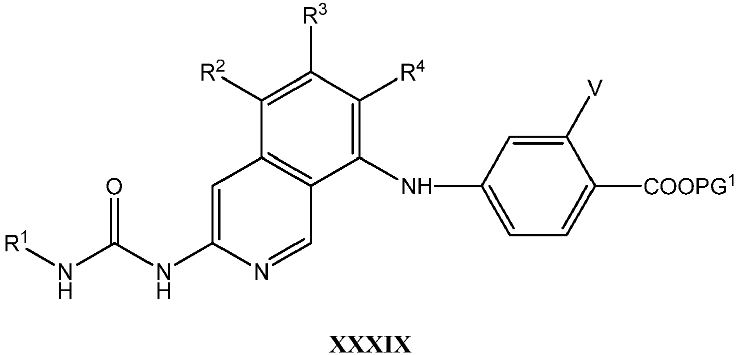 wherein R¹, R², R³ and R⁴ are as defined in formula I, V represents H or methyl and PG¹ represents a carboxylic acid protecting group (in particular methyl or *tert*-butyl), using general reaction technique 15; or
z) reacting the compounds of formula XL 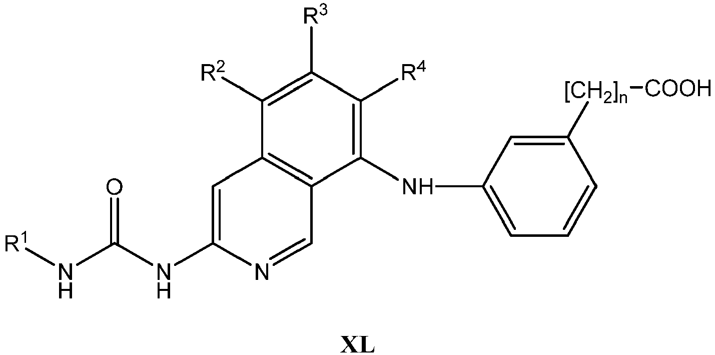 wherein R¹, R², R³ and R⁴ are as defined in formula I and n represents 0 or 1 with dimethylamine or an amine of formula XLI

   L²²-NH₂ **XLI**

   wherein L²² represents H, (C₁-C₃)alkyl or benzyl, using general reaction technique 6; or
aa) reacting the compounds of formula XLII 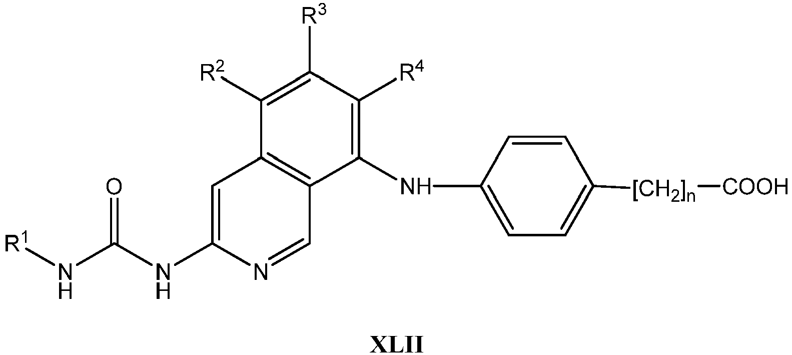 wherein R¹, R², R³ and R⁴ are as defined in formula I and n represents 0 or 1 with dimethylamine or an amine of formula XLI

   L²²-NH₂ **XLI**

   wherein L²² represents H, (C₁-C₃)alkyl or benzyl, using general reaction technique 6; or
bb) reacting the compounds of formula XLIII 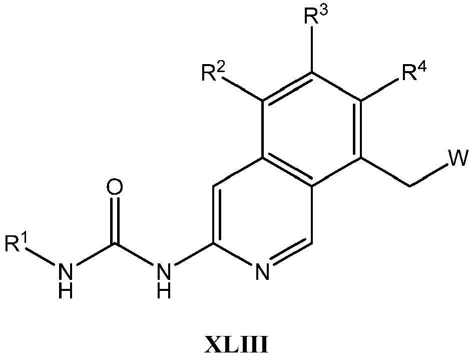 wherein R¹, R², R³ and R⁴ are as defined in formula I and W represents a halogen such as chlorine or bromine with an alcohol of formula XLIV

   L²³-OH **XLIV**

   wherein L²³ is such that CH₂O-L²³ corresponds to one of the possible meanings of R⁵, and in particular wherein L²³ represents 3-hydroxyphenyl, 3-aminophenyl, 2-methoxycarbonylphenyl, pyridin-2-yl, pyridin-4-yl, 5-methyl-pyridin-2-yl, 6-methyl-pyridin-3-yl, 2-methyl-pyridin-3-yl or 2-methoxycarbonylpyridin-4-yl, using general reaction technique 16; or
cc) reacting the compounds of formula XLIII as defined in item bb) wherein R¹, R², R³ and R⁴ are as defined in formula I and W represents a halogen such as chlorine or bromine with an amine of formula XLV

   L²⁴-NH₂ **XLV**

   wherein L²⁴ is such that CH₂NH-L²⁴ corresponds to one of the possible meanings of R⁵, and in particular wherein L²⁴ represents pyridin-2-yl, pyridin-3-yl, 1,3-dimethyl-1*H*-pyrazol-5-yl or 3-methylisothiazol-5-yl, using general reaction technique 17; or
dd) reacting the compounds of formula XXVIII as defined in item q) with the compounds of formula XLVI

   L²⁵-Br **XLVI**

   wherein L²⁵ represents R⁸ as defined in formula I using general reaction technique 4; or
ee) reacting the compounds of formula XLVII 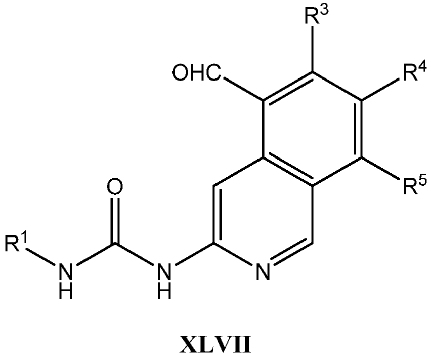 wherein R¹, R³, R⁴ and R⁵ are as defined in formula I with the compounds of formula XLVIII*a* or XLVIII*b*

   L^{26a}-NH₂ **XLVIII*a***

   L^{26b}-NH-L_{26c} **XLVIII*b***

   wherein L^{26a} is such that L^{26a}-NHCH₂- corresponds to one of the possible meanings of R² (and in particular wherein L^{26a} represents methyl) and L^{26b}-NH-L^{26c} is *N*,*N-*dimethylamino, morpholino or 4-methyl-piperazin-1-yl, using general reaction technique 13; or
ff) reacting the compounds of formula XLIX 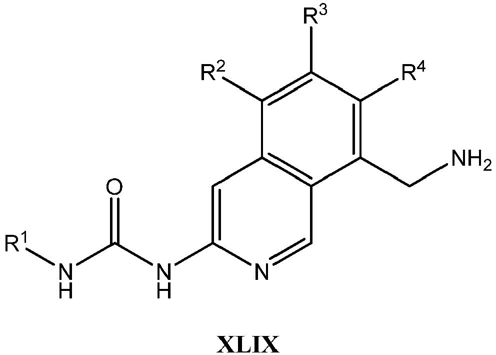 wherein R¹, R³, R⁴ and R⁵ are as defined in formula I with the compounds of formula L

   L²⁷-X^{e} **L**

   wherein L²⁷ corresponds to one of the possible meanings of R⁹ (and in particular wherein L²⁷ represents pyrimidin-2-yl) and X^{e} represents a halogen such as bromine using general reaction technique 4; or
gg) reacting the compounds of formula LI 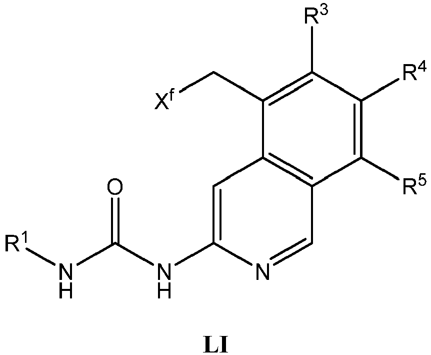 wherein R¹, R³, R⁴ and R⁵ are as defined in formula I and X^{f} represents a halogen such as chlorine or bromine with the compounds of formula LII*a* or LII*b*

   L^{28a}-NH₂ **LII*a***

   L^{28b}-NH-L_{28c} **LII*b***

   wherein L^{28a} is (C₁-C₃)alkyl (and in particular methyl) and L^{28b}-NH-L^{28c} is N,N dimethylamino, morpholino or 4-methyl-piperazin-1-yl using general reaction technique 17; or
hh) deprotecting the compounds of formula LIII 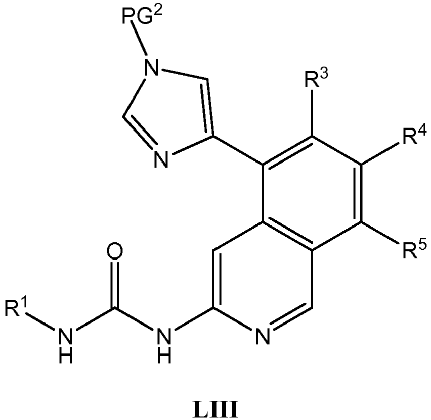 wherein PG² represents an amine protecting group such as benzyl, Boc or Cbz (and in particular Boc), using general reaction technique 19.

The compounds of formula I thus obtained may, if desired, be converted into their salts, and notably into their pharmaceutically acceptable salts.

Besides, whenever the compounds of formula I are obtained in the form of mixtures of diastereomers, the diastereomers can be separated using methods known to one skilled in the art, e.g. by HPLC over a chiral stationary phase such as a Regis Whelk-Ol(R,R) (10 µm) column, a Daicel ChiralCel OD-H (5-10 µm) column, or a Daicel ChiralPak IA (10 µm) or AD-H (5 µm) column. Typical conditions of chiral HPLC are an isocratic mixture of eluent A (EtOH, in presence or absence of an amine such as TEA or diethylamine) and eluent B (Hex), at a flow rate of 0.8 to 150 mL/min. The mixtures of diasteromers may also be separated by an appropriate combination of silica gel chromatography, HPLC and crystallization techniques.

### Preparation of the intermediates used in the preparation the compounds of formula I:

The compounds of formula III, IIIa, IV, V, VII, IX, XI, XIII, XIV, XV, XVI, XVII, XVIII, XX, XXII, XXIV, XXVII, XXIX, XXXI, XXXII, XXXIII, XXXIV, XXXV, XXXVII, XLI, XLIV, XLV, XLVI, XLVIII*a*, XLVIII*b*, L, LII*a* or LII*b* are commercially available or can be obtained by methods known to someone skilled in the art. The other intermediates can be prepared (for example) as described hereafter.

### Compounds of formula II:

The compounds of formula II can be prepared as summarised in Scheme 1 hereafter.

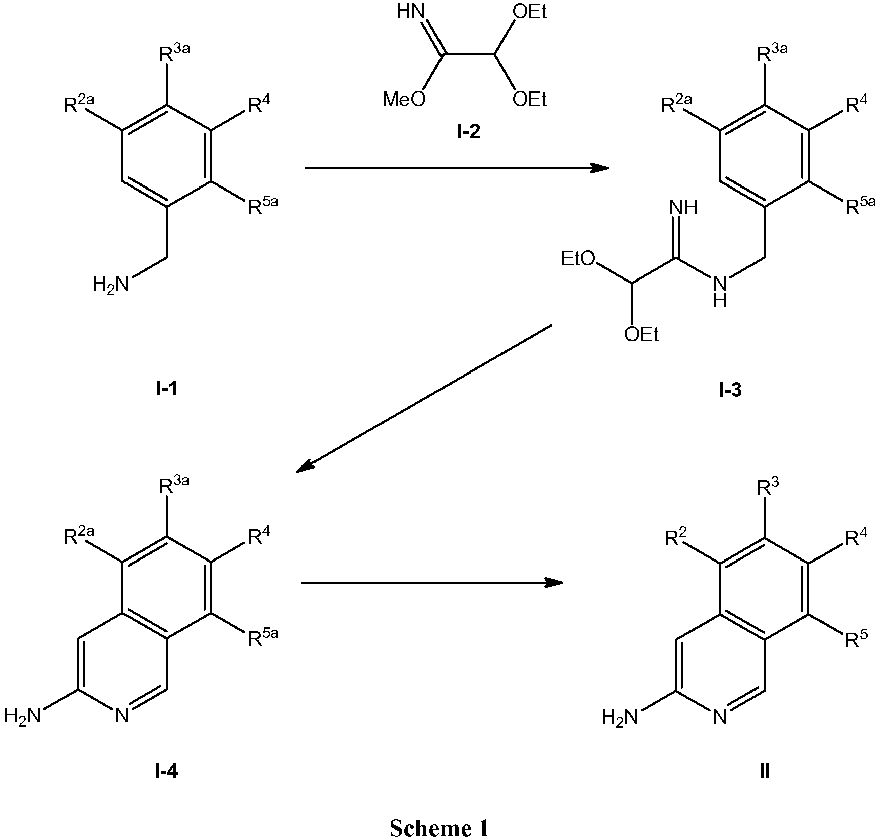

In Scheme 1, R², R³, R⁴ and R⁵ are as defined in formula I, R^{2a} represents H or halogen (e.g. fluorine, chlorine or bromine), R^{3a} represents H or halogen such as bromine, R^{5a} represents H, halogen (e.g. chlorine or bromine) or alkyl (e.g. Me).

The benzyl amines of formula I-1 can be reacted (Scheme 1) with 2,2-diethoxy-ethanimidic acid methyl ester (I-2; commercially available or prepared according to WO 2007/125405) according to general reaction technique 11, affording the intermediates of formula I-3. The latter can then be ring closed using general reaction technique 12. The isoquinoline derivatives of formula I-4 can then be transformed, if required, into the corresponding further substituted isoquinoline derivatives of formula II using one of general reaction techniques 1 to 5, 14 and 18.

Alternatively, the compounds of formula II can be prepared as summarised in Scheme 2 hereafter.

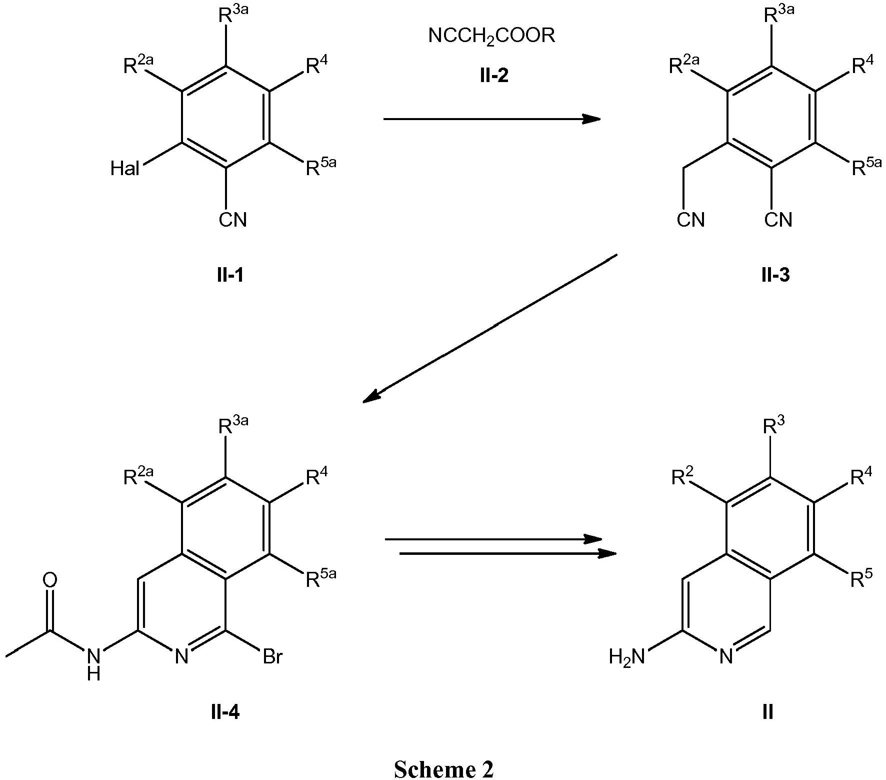

In Scheme 2, R², R³, R⁴ and R⁵ are as defined in formula I, R represents alkyl, R^{2a} represents H or halogen (e.g. fluorine, chlorine or bromine), R^{3a} represents H or halogen (e.g. bromine), R^{5a} represents H, halogen (e.g. chlorine or bromine) or alkyl (e.g. Me) and Hal represents halogen such as bromine, chlorine or fluorine.

The compounds of formula II can thus also be obtained (Scheme 2) by reacting the commercially available cyano derivatives of formula II-1 with the methyl cyanoacetates of formula II-2 before decarboxylating, as described in WO 2009/103966. The resulting dicyano derivatives of formula II-3 can be cyclised in the presence of HBr in AcOH and treated with acetyl chloride to afford the acetamide derivatives of formula II-4. The bromide and the acetyl group of the latter can be removed by sequential treatment with PPh₃ in presence of Pd(OAc)₂ and a base such as K₂C0₃ followed by heating in aq. 2M HC1 between 30 and 100°C. The compounds thus obtained can then be transformed, if required, into the further substituted derivatives of formula II using one of general reaction techniques 1 to 5, 14 and 18.

### Compounds of formula VI:

The compounds of formula VI can be prepared as summarised in Scheme 3 hereafter.

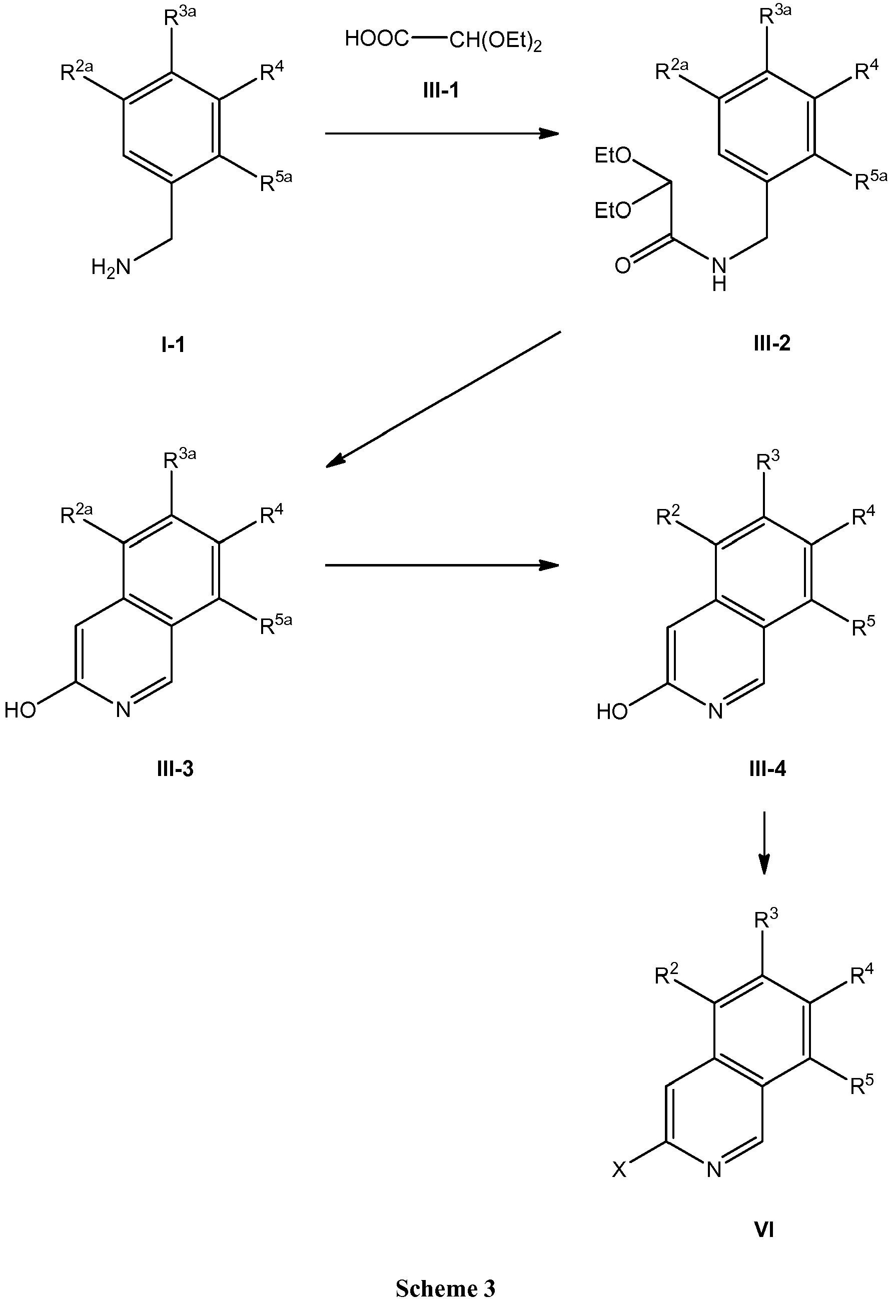

In Scheme 3, R², R³, R⁴, R⁵ are as defined in formula I, X is as defined in formula VI, R^{2a} represents H or halogen such as fluorine, chlorine or bromine, R^{3a} represents H or halogen such as bromine, R^{5a} represents H, halogen such as chlorine or bromine or alkyl such as methyl.

The amines of formula I-1 can be reacted with diethoxyacetic acid (111-1; commercially available or prepared according to WO 03/080578) using general reaction technique 6, followed by ring closure in presence of conc. sulfuric acid between -5 and +100°C. The resulting isoquinoline derivatives of formula III-3 can be transformed, if required, into their corresponding further substituted isoquinoline derivatives of formula III-4 using one of general reaction techniques 1 to 5, 14 and 18. The isoquinoline derivatives of formula III-4 can then be transformed into the corresponding isoquinoline derivatives of formula VI using general reaction technique 7.

### Compounds of formula VIII, X or XII:

The isoquinoline derivatives of formula I-4 wherein R^{2a} is Y, R^{3a} is R³ and R^{5a} is R⁵ or R^{2a} is R², R^{3a} is Z and R^{5a} is R⁵ or R^{2a} is R², R^{3a} is R³ and R^{5a} is W (see Scheme 1) can be respectively transformed into the corresponding alkyl urea derivatives of formula VIII, X or XII either by reaction with the appropriate alkyl isocyanates of formula III, by reaction with the appropriate carbamic chlorides of formula III*a* or by reaction with the appropriate compounds of formula IV followed by reaction with the amines of formula V.

### Compounds of formula XIXa or XIXb:

The compounds of formula XIXa or XIXb can be prepared as summarised in Scheme 4 hereafter.

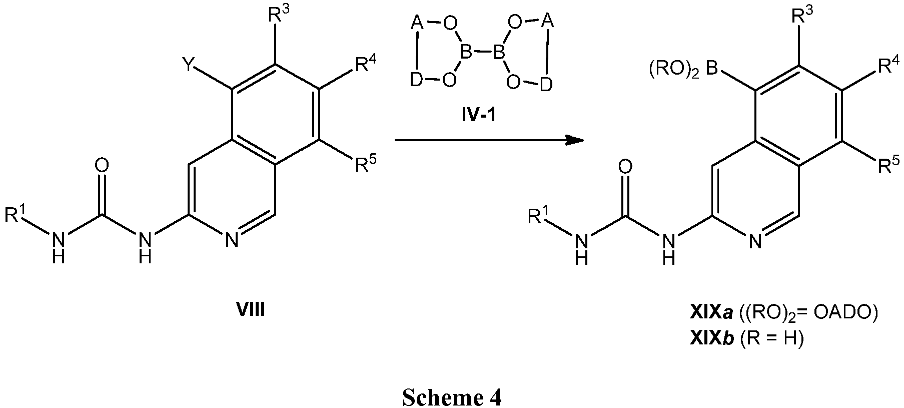

In Scheme 4, R¹, R³, R⁴, R⁵ are as defined in formula I, Y represents halogen such as bromine and A-D represents C(Me)₂C(Me)₂ or CH₂C(Me)₂CH₂.

The compounds of formula VIII can be reacted with the borane derivatives of formula IV-1 wherein A-D represents C(Me)₂C(Me)₂ or CH₂C(Me)₂CH₂ using general reaction technique 1. The resulting derivatives of formula XIXa can be hydrolysed in acidic medium, affording the derivatives of formula XIXb.

### Compounds of formula XXIa or XXIb:

Starting from the compounds of formula X, the compounds of formula XXI*a* or XXIb can be prepared in a manner analogous to the preparation of the compounds of formula XIX*a* or XIX*b* (see above).

### Compounds of formula XXIIIa or XXIIIb:

Starting from the compounds of formula XII, the compounds of formula XXIII*a* or XXIII*b* can be prepared in a manner analogous to the preparation of the compounds of formula XIX*a* or XIX*b* (see above).

### Compounds of formula XXV:

The compounds of formula XXV can be prepared as summarised in Scheme 5 hereafter.

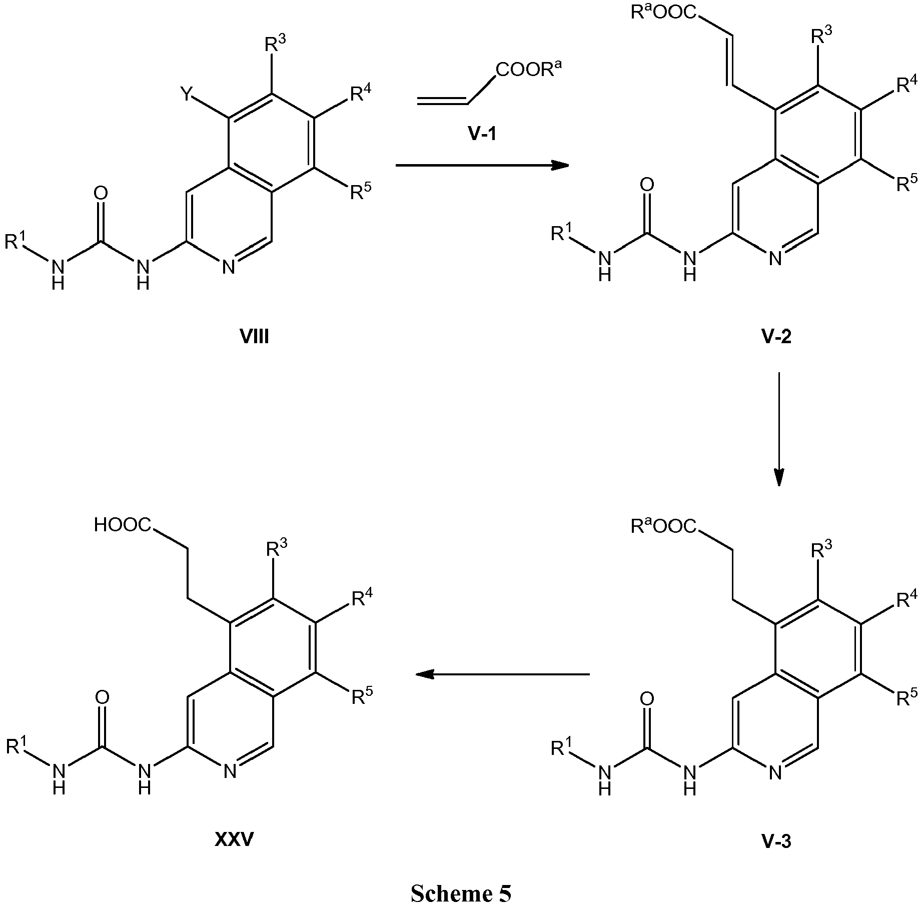

In Scheme 5, R¹, R³, R⁴, R⁵ are as defined in formula I, Y represents halogen such as chlorine or bromine and R^{a} represents alkyl.

The compounds of formula VIII can be reacted with the alkyl acrylate derivatives of formula V-1 following general reaction technique 5. The resulting compounds of formula V-2 can be hydrogenated over a noble metal catalyst such as Pd/C or reduced using NaBH₄ in presence of NiCl₂, affording the ester derivatives of formula V-3 which can then be hydrolysed into the corresponding acid derivatives of formula XXV by treatment with an alkali hydroxide such as KOH or NaOH.

### Compounds of formula XXVI:

The compounds of formula XXVI can be prepared as summarised in Scheme 6 hereafter.

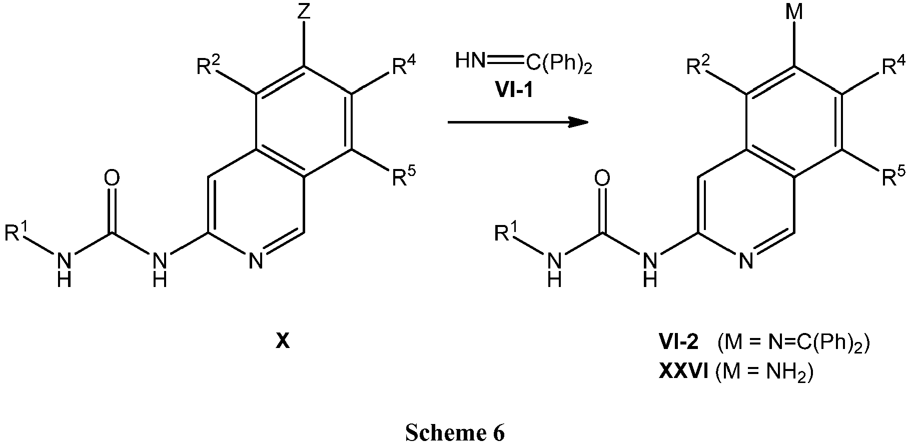

In Scheme 6, R¹, R², R⁴, R⁵ are as defined in formula I and Z represents halogen such as chlorine or bromine.

The intermediates of formula X can be reacted (Scheme 6) with benzophenone imine (VI-1; commercially available) following general reaction technique 4. The resulting derivatives of formula VI-2 can then be reacted with hydroxylamine hydrochloride in the presence of sodium acetate to afford the compounds of formula XXVI.

Alternatively the compounds of formula X can be reacted with NH₄OH in the presence of CuI or in the presence of a Pd catalyst, such as (XPhos) palladium(II) phenethylamine chloride, using general reaction technique 3 or 4.

### Compounds of formula XXVIII:

The compounds of formula XXVIII can be obtained from the compounds of formula XII using methods similar to those described above for preparing the compounds of formula XXVI.

### Compounds of formula XXX:

The compounds of formula XXX can be obtained from the compounds of formula VIII using methods similar to those described above for preparing the compounds of formula XXVI.

### Compounds of formula XXXVI, XLIII or XLIX:

The compounds of formula XXXVI, XLIII or XLIX can be prepared as summarised in Scheme 7 hereafter.

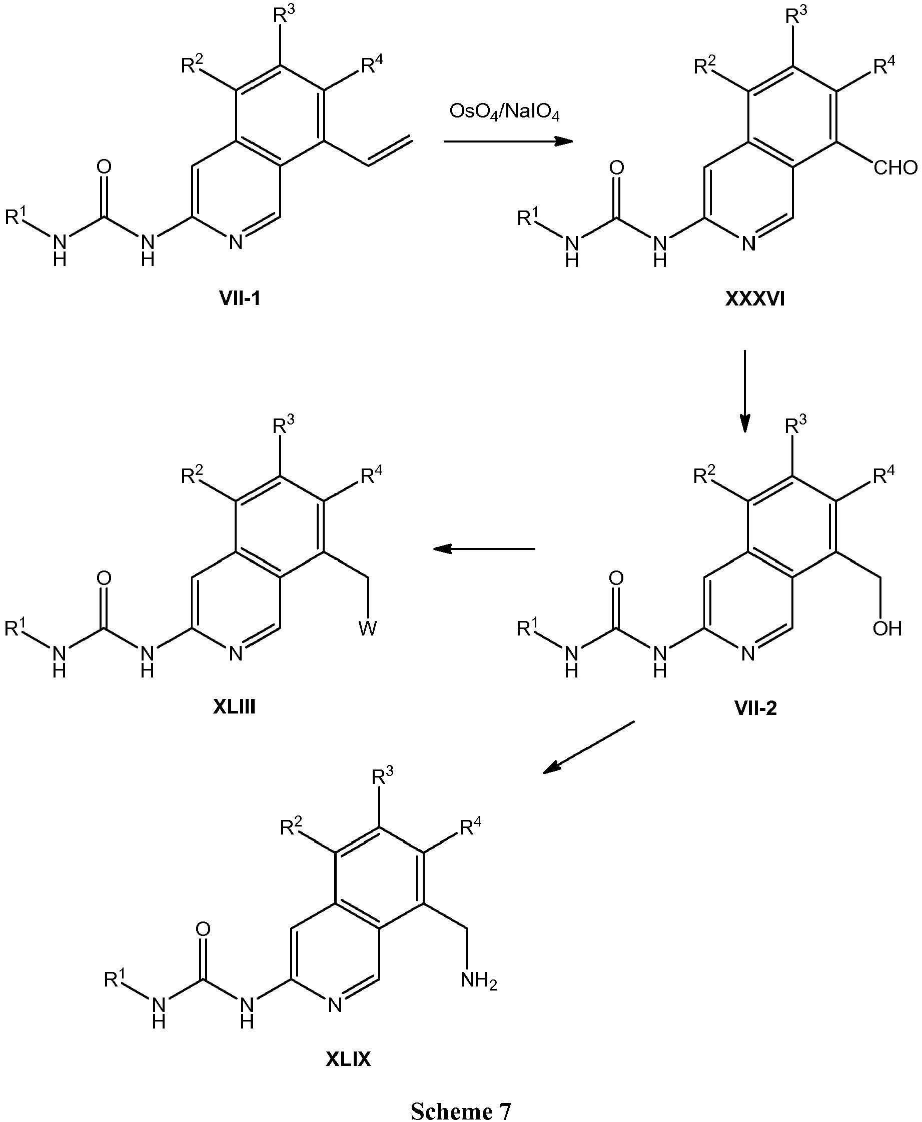

In Scheme 7, R¹, R², R³, R⁴ are as defined in formula I and W represents a halogen such as chlorine or bromine.

The compounds of formula VII-1, obtained from the compounds of formula XII following the general preparation method f), can be dihydroxylated using OsO₄/NMO as described in Tetrahedron Letters (1976), 23, 1973-1976 and subsequently transformed into the corresponding aldehydes of formula XXXVI with NaIO₄. Alternatively, this reaction sequence can be performed in a one-pot process as described in Org. Lett. (2000), 2, 3975-3977. The resulting aldehydes can be transformed into the corresponding alcohol derivatives of formula VII-2 by reduction with a hydride reagent such as LiAlH₄ or NaBH₄. The halide derivatives of formula XLIII can be obtained either directly through reaction with thionyl chloride (W = Cl) or with CBr₄ and PPh₃ (W = Br) or via transformation of the compounds of formula VII-2 into the corresponding mesylates through reaction with mesyl chloride in presence of TEA and subsequent reaction with sodium iodide or lithium bromide. Besides, the compounds of formula VII-2 can be sequentially reacted with alkyl or arylsulfonyl chlorides using general reaction technique 7, followed either by reaction with sodium azide using general reaction technique 8 or by reaction with potassium phthalimide using general reaction technique 9, and subsequently deprotected using general reaction technique 10, affording the amines of formula XLIX. Alternatively, the compounds of formula VII-2 can be directly reacted with DPPA using general reaction technique 8 and the corresponding azides can be transformed *in situ* into the amines of formula XLIX using general reaction technique 10.

### Compounds of formula XXXVIII or XXXIX:

The compounds of formula XXXVIII or XXXIX can be prepared by reacting the compounds of formula XII as defined in item f) above with the anilines of formula 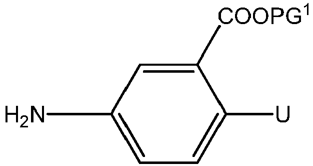 or 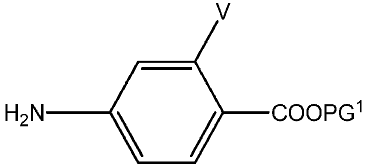 using general reaction technique 4.

### Compounds of formula XL or XLII:

The compounds of formula XL or XLII can be obtained by deprotection of the corresponding esters using general reaction technique 15.

### Compounds of formula XLVII or LI:

The compounds of formula XLVII or LI can be prepared as summarised in Scheme 8 hereafter.

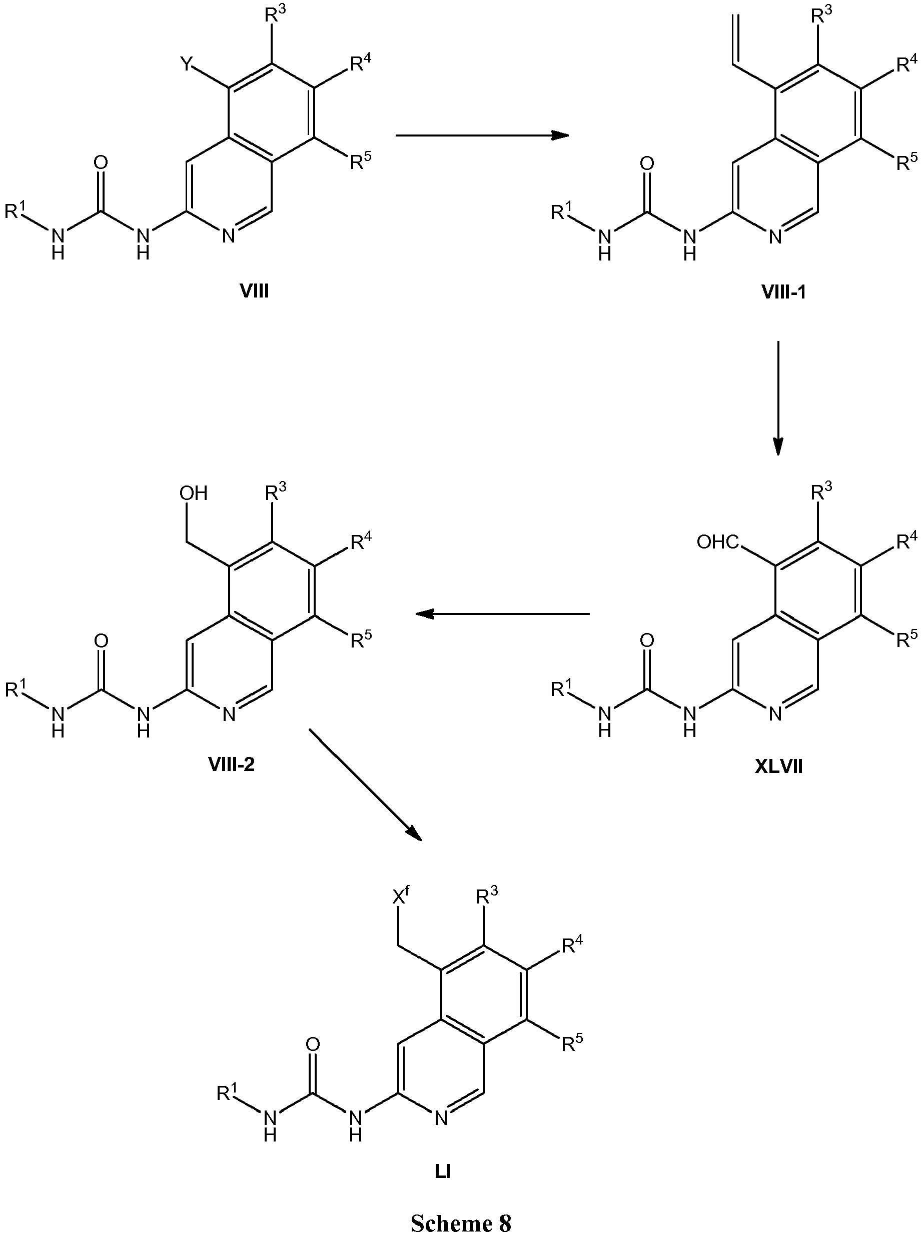

In Scheme 8, R¹, R³, R⁴, R⁵ are as defined in formula I, Y represents a halogen such as chlorine or bromine and X^{f} represents a halogen such as chlorine, bromine or iodine.

The compounds of formula VIII-1, obtained from the compounds of formula VIII following the general preparation method d), can be dihydroxylated using OsO₄/NMO as described in Tetrahedron Letters (1976), 23, 1973-1976 and subsequently transformed into the corresponding aldehydes of formula XLVII with NaIO₄. Alternatively, this reaction sequence can be performed in a one-pot process as described in Org. Lett. (2000), 2, 3975-3977. The resulting aldehydes of formula XLVII can be transformed into the corresponding alcohol derivatives of formula VIII-2 by reduction with a hydride reagent such as LiAlH₄ or NaBH₄. The halide derivatives of formula LI can then be obtained from the compounds of formula VIII-2 using general reaction technique 7.

### Compounds of formula LIII:

The compounds of formula LIII can be obtained from compounds of formula XIXa or XIXb using general reaction technique 1.

### Compounds of formula I-1:

The compounds of formula I-1, if not commercially available, can be prepared as summarised in Scheme 9 hereafter.

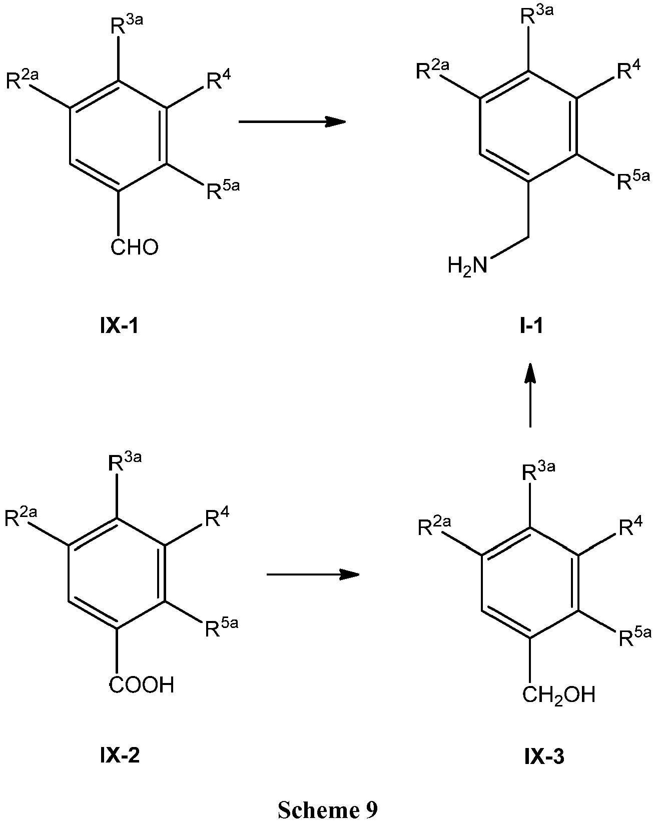

In Scheme 9, R^{2a} represents H or halogen (e.g. fluorine, chlorine or bromine), R^{3a} represents H or halogen (e.g. chlorine or bromine), R⁴ represents H, R^{5a} represents H, halogen (e.g. fluorine, chlorine or bromine) or alkyl (e.g. Me).

The non-commercial benzyl amines of formula I-1 can be obtained (Scheme 9) by reaction of the aldehydes of formula IX-1 (commercially available) with hydroxylamine followed by reduction over RaNi. Alternatively the commercially available carboxylic acid derivatives of formula IX-2 can be reduced using BH₃. The resulting benzylic alcohols of formula IX-3 can then be activated using general reaction technique 7 and transformed into the benzyl amine derivatives of formula I-1 through conversion into either the corresponding azides or the corresponding phthalimides using general reaction technique 8 or 9 and subsequently converted into the corresponding amines using general reaction technique 10.

Particular embodiments of the invention are described in the following Examples, which serve to illustrate the invention in more detail without limiting its scope in any way.

### EXAMPLES

All temperatures are stated in °C. Unless otherwise indicated, the reactions take place at rt.

Analytical TLC characterisations were performed with 0.2 mm plates: Merck, Silica gel 60 F₂₅₄. Elution was performed with EA, Hept, DCM, MeOH or mixtures thereof. Detection was done with UV or with a solution of KMnO₄ (3 g), K₂CO₃ (20 g), 5% NaOH (3 mL) and H₂O (300 mL) with subsequent heating.

Prep-TLCs were performed with 2.0 mm plates: Merck, Silica gel 60 F₂₅₄. Elution was performed with EA, Hept, DCM, MeOH or mixtures thereof. Detection was done with UV.

CCs were performed using Brunschwig 60A silica gel (0.032-0.63mm), SNAP KP-Sil^{™} cartridges from Biotage or EasyVarioFlash^{®} cartridges from Merck; elution was performed with EA, Hept, DCM, MeOH or mixtures thereof. In the cases of compounds containing a basic function (e.g. amine), 1% of NH₄OH (25% aq.) was added to the eluent(s).

Prep-HPLCs were performed on XBridge Prep C18 columns from Waters. The following conditions were used:
- Eluents: A: H₂O + 0.1% acidic or basic additive; B: MeCN + 0.1% acidic or basic additive;
- Gradient: 5% B → 95% B over 5 min.
- Detection: UV/Vis and/or MS and/or ELSD.
- Prep-HPLC (acidic conditions): additive in A and B is 0.1% HCO₂H.
- Prep-HPLC (basic conditions): additive in A and B is 0.1% NH₄OH.

LC-MSs were performed on Sciex API 2000 with Agilent 1100 Binary Pump with DAD and ELSD; or Agilent quadrupole MS 6140 with Agilent 1200 Binary Pump, DAD and ELSD; or Thermo Finnigan MSQ Surveyor MS with Agilent 1100 Binary Pump, DAD and ELSD; or Thermo MSQ Plus with Dionex GHP 3200 Binary Pump, DAD and ELSD. The number of decimals given for the [M+H⁺] peak of each tested compound depends upon the accuracy of the LC-MS device actually used.

NMR spectra were recorded on a Varian Mercury 300 (300 MHz) spectrometer unless indicated otherwise ("400 MHz" being used to mean a Bruker Avance 400 (400 MHz) spectrometer). Chemical shifts are given in ppm relative to the solvent used; multiplicities: s = singlet, d = doublet, t = triplet, q = quadruplet, p = pentuplet, hex = hextet, hept = heptet, m = multiplet; br. = broad; coupling constants are given in Hz.

### DETAILED SYNTHETIC PROCEDURES

### Procedure A (Suzuki coupling with polymer-supported Pd reagents):

To the aromatic halide (0.1 mmol; 1.0 eq.), the required boronic acid derivative (2.0 eq.) and dibenzylideneacetone palladium(0) phosphaadamantane ethyl silica (0.1 eq.; PhosphonicS PAPd2r; loading: 0.01-0.03 mmol/g; particle size: 60-200 µm; pore diameter: 110 Å) in a glass vial, under inert atmosphere (Ar), are added degassed dioxane (1.0 mL) and degassed aq. 1M K₂CO₃ solution (1.5 eq.; the quantity is increased to 2.5 eq. when a hydrochloride salt of the boronic acid is used). The reaction mixture is stirred at 115°C for 4 h, then allowed to cool down to rt and further stirred at rt for 20 h. The reaction mixture is treated with AcOH to reach pH 8, stirred at rt for 15 min and treated with a 1:1 mixture (60 mg) of methyl thiourea ethyl sulfide ethyl silica (PhosphonicS MTCf; loading: 0.6 mmol/g; particle size: 60-200 µm; pore diameter: 90 Å) and triamine ethyl sulfide amide silica (PhosphonicS STA3; loading: 0.8 mmol/g; particle size: 60-200 µm; pore diameter: 60 Å). The suspension is shaken at 50°C for 20 h, diluted with 1:1 MeOH/DCM (5.0 mL), filtered and concentrated under reduced pressure. The residue is purified by prep-HPLC. Purification of the residue gives the desired product.

### Procedure B (Suzuki coupling without polymer-supported Pd reagents):

To the aromatic halide (0.1 mmol; 1.0 eq.), the required boronic acid (1.5 eq.) and Pd(PPh₃)₄ (0.1 eq) in a glass vial, under inert atmosphere (N₂), are added dioxane (0.8 mL) and an aq. 1*N* K₂CO₃ solution (0.2 mL; 2.0 eq.). The reaction mixture is purged with N₂ for 5 min, stirred at 80°C and monitored by LC-MS. Upon reaction completion, the reaction mixture is diluted with DCM and a sat. aq. NaHCO₃ solution. The layers are separated and the aq. layer is extracted with 9:1 DCM/MeOH (3x). The combined org. layers are washed with a sat. aq. NaHCO₃ solution, water and brine, dried over MgSO₄, filtered and concentrated under reduced pressure. Purification of the residue gives the desired product.

### Procedure C (Suzuki coupling with scavenger treatment):

To the aromatic halide (0.1 mmol; 1.0 eq.), the required boronic acid (1.5 eq.) and Pd(PPh₃)₄ (0.1 eq.) in a glass vial, under inert atmosphere (N₂), are added dioxane (0.8 mL) and an aq. 1*N* K₂CO₃ solution (0.2 mL; 2.0 eq.). The reaction mixture is purged with N₂ for 5 min, stirred at 80°C and monitored by LC-MS. Upon reaction completion, the reaction mixture is diluted with DCM and a sat. aq. NaHCO₃ solution. The layers are separated and the aq. layer is extracted with 9:1 DCM/MeOH (3x). The combined org. layers are washed with a sat. aq. NaHCO₃ solution, water and brine, dried over MgSO₄, filtered and concentrated under reduced pressure. The residue is dissolved in 9:1 DCM/MeOH (2.0 mL) and treated with a 1:1 mixture (40 mg) of triamine ethyl sulfide amide silica (PhosphonicS STA3; loading: 0.8 mmol/g; particle size: 60-200 µm; pore diameter: 60 Å) and methyl thiourea ethylsulfide ethyl silica (PhosphonicS MTCf; loading: 0.6 mmol/g; particle size: 60-200 µm; pore diameter: 90 Å). The mixture is shaken at rt overnight and filtered. The scavengers are washed with 9:1 DCM/MeOH and the filtrate is concentrated under reduced pressure. Purification of the residue gives the desired product.

### Procedure D (Negishi coupling with bis(triphenylphosphine)palladium(II) dichloride):

To the aromatic halide (0.1 mmol, 1.0 eq.) and bis(triphenylphosphine)palladium(II) dichloride (0.1 eq.) in a glass vial, under inert atmosphere (N₂), are added THF (0.5 mL) and the required organozinc reagent (*e.g*. benzylzinc bromide; 0.5M solution in THF; 5.0 eq.). The reaction mixture is purged with N₂ for 5 min, stirred at 60°C and monitored by LC-MS. Upon reaction completion, the reaction mixture is diluted with DCM and a sat. aq. NaHCO₃ solution. The layers are separated and the aq. layer is extracted with 9:1 DCM/MeOH (3x). The combined org. layers are washed with a sat. aq. NaHCO₃ solution, water and brine, dried over MgSO₄, filtered and concentrated under reduced pressure. Purification of the residue gives the desired product.

### Procedure E (Negishi coupling with [1,1'-bis(diphenylphosphino)ferrocene]dichloro palladium(II), complex with dichloromethane):

To the appropriate aromatic halide (0.1 mmol, 1.0 eq.) and a [1,1'-bis(diphenylphosphino)ferrocene]dichloropalladium(II) complex with DCM (0.01 eq.) in a glass vial under inert atmosphere (N₂) are added dioxane (0.5 mL) and dimethylzinc (1.2M in toluene; 1.6 eq.). The reaction mixture is purged with N₂ for 5 min, stirred at 80°C and monitored by LC-MS. Upon reaction completion, the reaction mixture is diluted with DCM and a sat. aq. NaHCO₃ solution. The layers are separated and the aq. layer is extracted with 9:1 DCM/MeOH (3x). The combined org. layers are washed with a sat. aq. NaHCO₃ solution, water and brine, dried over MgSO₄, filtered and concentrated under reduced pressure. Purification of the residue gives the desired product.

### Procedure F (Goldberg-type coupling):

To the aromatic halide (0.1 mmol, 1.0 eq.), the required amide (1.2 eq.), copper iodide (0.3 eq.), K₂CO₃ (2.0 eq.) and *trans*-*N,N*'-dimethyl-1,2-cyclohexanediamine (0.1 eq.) in a glass vial, under inert atmosphere (N₂), is added dry dioxane (0.5 mL). The reaction mixture is purged with N₂ for 5 min, stirred at 110°C and monitored by LC-MS. Upon reaction completion, the reaction mixture is diluted with DCM and a sat. aq. NaHCO₃ solution. The layers are separated and the aq. layer is extracted with 9:1 DCM/MeOH (3x). The combined org. layers are washed with a sat. aq. NaHCO₃ solution, water and brine, dried over MgSO₄, filtered and concentrated under reduced pressure. Purification of the residue gives the desired product.

### Procedure G (Buchwald-Hartwig amination with SK-CC02-A):

To the aromatic halide (0.1 mmol, 1.0 eq.) and tBuONa (1.4 eq.) in a glass vial, under inert atmosphere (N₂), are added dry dioxane (0.2 mL) and the required amine derivative (1.5 eq.). The reaction mixture is stirred at rt for 10 min and a solution of SK-CC02-A (0.16 eq.) in dry dioxane (0.3 mL) is added. The reaction mixture is purged with N₂ for 5 min, stirred at 80°C and monitored by LC-MS. Upon reaction completion, the reaction mixture is diluted with DCM and a sat. aq. NaHCO₃ solution. The layers are separated and the aq. layer is extracted with 9:1 DCM/MeOH (3x). The combined org. layers are washed with brine, dried over MgSO₄, filtered and concentrated under reduced pressure. Purification of the residue gives the desired product.

### Procedure H (Buchwald-Hartwig amination with Pd/BINAP):

To the aromatic halide (0.1 mmol, 1.0 eq.) and tBuONa (1.4 eq.) in a glass vial, under inert atmosphere (N₂), are added dry dioxane (0.5 mL) and the required amine derivative (2.0 eq.). BINAP (0.15 eq.) is dissolved in dry dioxane (0.5 mL) in a glass vial, under inert atmosphere (N₂), at 80°C and upon cooling to rt, treated with Pd(OAc)₂ (0.1 eq) and stirred at rt for 5 min. This freshly prepared solution of premixed catalyst is then added to the reaction mixture. The resulting reaction mixture is purged with N₂ for 5 min, stirred at 80°C and monitored by LC-MS. Upon reaction completion, the reaction mixture is diluted with DCM and a sat. aq. NaHCO₃ solution. The layers are separated and the aq. layer is extracted with 9:1 DCM/MeOH (3x). The combined org. layers are washed with brine, dried over MgSO₄, filtered and concentrated under reduced pressure. Purification of the residue gives the desired product.

### Procedure I (amide formation using HA TU):

To a solution of the required acid (0.1 mmol, 1.0 eq.) in DMF (0.4 mL) in a round-bottomed flask, under inert atmosphere (N₂), are added DIPEA (3.0 eq.) and 0.5M ammonia in dioxane (3.0 eq.). The mixture is stirred at rt for 10 min and HATU (1.5 eq.) is added at once. The reaction mixture is stirred at rt and monitored by LC-MS. Upon reaction completion, the reaction mixture is concentrated under reduced pressure. Purification of the residue gives the desired product.

### Procedure J (amide formation using HOBT):

To a solution of the amine (0.1 mmol, 1.0 eq.) and HOBT hydrate (1.5 eq.) in DCM (0.5 mL) in a round-bottomed flask, under inert atmosphere (N₂), are added DIPEA (3.0 eq), the required acid (1.2 eq.) and EDC HCl (1.5 eq.). The reaction mixture is stirred at rt and monitored by LC-MS. Upon reaction completion, a sat. aq. NH₄Cl solution is added to the reaction mixture. The layers are separated and the aq. layer is extracted with 9:1 DCM/MeOH (3x). The combined org. layers are washed with a sat. aq. NaHCO₃ solution, water and brine, dried over MgSO₄, filtered and concentrated under reduced pressure. Purification of the residue gives the desired product.

### Procedure K (amide formation using HOAT):

A solution of the amine (0.075 mmol; 1 eq.), the required acid (0.113 mmol; 1.5 eq.), HOAT (1M solution in DMF; 0.041 mmol; 0.5 eq.) and Si-DCC (0.150 mmol; 2 eq.) in 1:1 DCM/DMF (400 µL), in a glass vial under inert atmosphere (N₂), is shaken overnight at rt. The reaction mixture is filtered over a phase separator and the resin is washed twice with DCM (1 mL). The filtrate is concentrated under reduced pressure. Purification of the residue gives the desired product..

### Procedure L (addition of benzyl amine on imidate derivatives):

To the required benzyl amine derivative (10.0 mmol, 1.0 eq.) in a round-bottomed flask, under inert atmosphere (N₂), are added dry MeOH (50.0 mL) and 2,2-diethoxy-ethanimidic acid methyl ester (1.2 eq.; prepared as described in WO 2007/125405). The reaction mixture is stirred at rt and monitored by LC-MS. Upon reaction completion, the reaction mixture is concentrated under reduced pressure. The residue is dissolved in DCM and the org. layer is washed with water and brine, dried over MgSO₄, filtered and concentrated under reduced pressure. The crude product is used without further purification in the next step.

### Procedure M (isoquinoline formation by cyclization):

The crude product of Procedure L is placed in a round-bottomed flask, at 0°C under inert atmosphere (N₂) and conc. H₂SO₄ (35 eq) is added. The reaction mixture is stirred at rt and monitored by LC-MS. Upon reaction completion, the reaction mixture is slowly poured into water at 0°C. The resulting acidic aq. solution is then poured slowly into a 12N aq. NaOH solution (40 eq.) at 0°C. The resulting basic aq. layer is extracted with 9:1 DCM/MeOH (3x). The combined org. layers are washed with brine, dried over MgS0₄, filtered and concentrated under reduced pressure. Purification of the residue gives the desired product.

### Procedure N (urea formation using isocyanate):

To the required aminoisoquinoline derivative (1.0 mmol, 1.0 eq.) in a round-bottomed flask, under inert atmosphere (N₂), are added dry dioxane (4.0 mL) and ethyl isocyanate (2.5 eq.). The reaction mixture is stirred at 50°C and monitored by LC-MS. Upon reaction completion, the reaction mixture is cooled to 10°C. The precipitate is filtered, washed with a minimum amount of dioxane and dried to give the desired product.

### Procedure O (urea formation using CDI):

To a solution of the required aminoisoquinoline derivative (1.0 mmol, 1.0 eq.) in DCM (10 mL) in a round-bottomed flask, under inert atmosphere (N₂), is added CDI (1.5 eq.). The reaction mixture is stirred overnight at rt. The resulting suspension is treated with the corresponding amine (6.0 eq.) and further stirred at rt for 24 h. The reaction mixture is diluted with 9:1 DCM/MeOH (50 mL), washed with a 1N aq. HCl solution, a 1*N* aq. NaOH solution, water and brine, dried over MgSO₄, filtered and concentrated under reduced pressure. Purification of the residue gives the desired product.

### Procedure P (reductive amination with NaBH₄):

The amine derivative (0.075 mmol; 1.0 eq.) and the required aldehyde (1.5 eq.) are dissolved in MeOH (1 mL) in a glass vial under inert atmosphere (N₂).. The mixture is shaken overnight at rt. The reaction mixture is treated with NaBH₄ (2.0 eq.) and shaken 1 h at rt. The reaction mixture is treated with a 25% HCl solution (8 eq.) and shaken 1 h at rt. It is then diluted with MeOH (2 mL) and dioxane (1 mL) and is concentrated under reduced pressure. Purification of the residue gives the desired product.

### Procedure Q (reductive amination with NaBH(OAc)₃):

The amine derivative (0.1 mmol, 1.0 eq.) and the required aldehyde (1.2 eq.) are dissolved in 7:3 DCM/DMF (1 mL) in a glass vial, under inert atmosphere (N₂) and treated with AcOH (1.5 eq.). The reaction mixture is heated to 40°C for dissolution and, upon cooling to rt, is treated with NaBH(OAc)₃ (1.5 eq.). The reaction mixture is further shaken overnight at rt and is treated with PL-HCO₃ (97 mg; Polymer Laboratories; loading: 1.8 mmol/g; particle size: 150-300 µm; pore diameter: 100 Å). It is shaken 1 h at rt, filtered and concentrated under reduced pressure. Purification of the residue gives the desired product.

### Procedure R (Buchwald-Hartwig amination with Pd/BINAP with scavenger treatment):

To the aromatic halide (0.1 mmol, 1.0 eq.) and tBuONa (1.4 eq.) in a glass vial, under inert atmosphere (N₂), are added dry dioxane (0.5 mL) and the required amine derivative (2.0 eq.). BINAP (0.15 eq.) is dissolved in dry dioxane (0.5 mL) in a glass vial, under inert atmosphere (N₂), at 80°C and upon cooling to rt, treated with Pd(OAc)₂ (0.1 eq) and stirred at rt for 5 min. This freshly prepared solution of premixed catalyst is then added to the reaction mixture. The resulting reaction mixture is purged with N₂ for 5 min, stirred at 80°C and monitored by LC-MS. Upon reaction completion, the reaction mixture is diluted with DCM and a sat. aq. NaHCO₃ solution. The layers are separated and the aq. layer is extracted with 9:1 DCM/MeOH (3x). The combined org. layers are washed with brine, dried over MgSO₄, filtered and concentrated under reduced pressure. The residue is dissolved in 9:1 DCM/MeOH (2.0 mL) and treated with a 1:1 mixture (40 mg) of triamine ethyl sulfide amide silica (PhosphonicS STA3; loading: 0.8 mmol/g; particle size: 60-200 µm; pore diameter: 60 Å) and methyl thiourea ethylsulfide ethyl silica (PhosphonicS MTCf; loading: 0.6 mmol/g; particle size: 60-200 µm; pore diameter: 90 Å). The mixture is shaken at rt overnight and filtered. The solids are washed with 9:1 DCM/MeOH and the filtrate is concentrated under reduced pressure. Purification of the residue gives the desired product.

### Procedure S (Negishi coupling with scavenger treatment):

To the appropriate aromatic halide (0.1 mmol, 1.0 eq.) and a [1,1'-bis(diphenylphosphino)ferrocene]dichloropalladium(II) complex with DCM (0.01 eq.) in a glass vial under inert atmosphere (N₂) are added dioxane (0.5 mL) and dimethylzinc (1.2*M* in toluene; 1.6 eq.). The reaction mixture is purged with N₂ for 5 min, stirred at 80°C and monitored by LC-MS. Upon reaction completion, the reaction mixture is diluted with DCM and a sat. aq. NaHCO₃ solution. The layers are separated and the aq. layer is extracted with 9:1 DCM/MeOH (3x). The combined org. layers are washed with a sat. aq. NaHCO₃ solution, water and brine, dried over MgSO₄, filtered and concentrated under reduced pressure. The residue is dissolved in 9:1 DCM/MeOH (2.0 mL) and treated with a 1:1 mixture (40 mg) of triamine ethyl sulfide amide silica (PhosphonicS STA3; loading: 0.8 mmol/g; particle size: 60-200 µm; pore diameter: 60 Å) and methyl thiourea ethylsulfide ethyl silica (PhosphonicS MTCf; loading: 0.6 mmol/g; particle size: 60-200 µm; pore diameter: 90 Å). The mixture is shaken at rt overnight and filtered. The solids are washed with 9:1 DCM/MeOH and the filtrate is concentrated under reduced pressure. Purification of the residue gives the desired product.

### Procedure T (Ullmann-type coupling):

To the aromatic halide (0.1 mmol, 1.0 eq.), the required alcohol (2.0 eq.), copper(I) chloride (0.5 eq.), Cs₂CO₃ (2.0 eq.) and 2,2,6,6-tetramethyl-3,5-heptanedione (0.1 eq.) in a glass vial, under inert atmosphere (N₂), is added dry DMF (2.0 mL). The reaction mixture is purged with N₂ for 5 min, stirred at 120°C and monitored by LC-MS. Upon reaction completion, the reaction mixture is concentrated under reduced pressure, diluted with 9:1 DCM/MeOH and a sat. aq. NaHCO₃ solution. The layers are separated and the aq. layer is extracted with 9:1 DCM/MeOH (3x). The combined org. layers are washed with water and brine, dried over MgSO₄, filtered and concentrated under reduced pressure. Purification of the residue gives the desired product.

### Procedure U (Buchwald-Hartwig amination with Pd/BINAP and SCX treatment):

To the aromatic halide (0.1 mmol, 1.0 eq.) and tBuONa (1.4 eq.) in a glass vial, under inert atmosphere (N₂), are added dry dioxane (0.5 mL) and the required amine derivative (2.0 eq.). BINAP (0.2 eq.) is dissolved in dry dioxane (0.5 mL) at 90°C in a glass vial, under inert atmosphere (N₂), and after cooling to rt, treated with Pd(OAc)₂ (0.1 eq) and stirred at rt for 5 min. This freshly prepared solution of premixed catalyst is then added to the reaction mixture. The resulting reaction mixture is purged with N₂ for 5 min, stirred at 90°C and monitored by LC-MS. Upon reaction completion, the reaction mixture is diluted with MeOH and a few drops of AcOH are added to obtain a solution. This solution is either treated with silica gel-supported sulfonic acid (5.0 eq.; Silicycle SiliaBond^{®} Tosic Acid; SCX; R60530B; 0.8 mmol/g), shaken 1 h at rt and filtered, or loaded on a corresponding cartridge (Silicycle SiliaPrep™ Tosic Acid Si-SCX). In both cases, the resin is washed with DCM, 1:1 DCM/MeOH and MeOH, and the product eventually released from the resin with 7M ammonia solution in MeOH. The solution of crude product is concentrated under reduced pressure and purification of the residue gives the desired product.

### Procedure V (Buchwald-Hartwig amination with Pd₂(dba)₃/XPhos and SCX treatment):

To the aromatic halide (0.1 mmol, 1.0 eq.), the required amine derivative (1.5 eq.), Pd₂(dba)₃ (0.05 eq.), X-Phos (0.1 eq.) and tBuONa (2.5 eq.) in a glass vial, under inert atmosphere (N₂), is added dry dioxane (0.5 mL). The resulting reaction mixture is purged with N₂ for 5 min, stirred at 90°C and monitored by LC-MS. Upon reaction completion, the reaction mixture is diluted with MeOH and a few drops of AcOH are added to obtain a solution. This solution is either treated with silica gel-supported sulfonic acid (5.0 eq.; Silicycle SiliaBond^{®} Tosic Acid; SCX; R60530B; 0.8 mmol/g), shaken 1 h at rt and filtered, or loaded on a corresponding cartridge (Silicycle SiliaPrep™ Tosic Acid Si-SCX). In both cases, the resin is washed with DCM, 1:1 DCM/MeOH and MeOH, and the product eventually released from the resin with 7M ammonia solution in MeOH. The solution of crude product is concentrated under reduced pressure and purification of the residue gives the desired product.

### Procedure W (Buchwald-Hartwig amination with Pd/BINAP and SCX treatment, inversed stoichiometry):

To the aromatic amine (0.1 mmol, 1.0 eq.) and tBuONa (1.4 eq.) in a glass vial, under inert atmosphere (N₂), are added dry dioxane (0.5 mL) and the required halide derivative (2.0 eq.). BINAP (0.2 eq.) is dissolved in dry dioxane (0.5 mL) at 90°C in a glass vial, under inert atmosphere (N₂), and upon cooling to rt, treated with Pd(OAc)₂ (0.1 eq.) and stirred at rt for 5 min. This freshly prepared solution of premixed catalyst is then added to the reaction mixture. The resulting reaction mixture is purged with N₂ for 5 min, stirred at 90°C and monitored by LC-MS. Upon reaction completion, the reaction mixture is diluted with MeOH and a few drops of AcOH are added to obtain a solution. This solution is either treated with silica gel-supported sulfonic acid (5.0 eq.; Silicycle SiliaBond^{®} Tosic Acid; SCX; R60530B; 0.8 mmol/g), shaken 1 h at rt and filtered, or loaded on a corresponding cartridge (Silicycle SiliaPrep™ Tosic Acid Si-SCX). In both cases, the resin is washed with DCM, 1:1 DCM/MeOH and MeOH, and the product released from the resin with 7M ammonia solution in MeOH. The solution of crude product is concentrated under reduced pressure and purification of the residue gives the desired product.

### Procedure X (amide formation using HATU):

To a solution of the amine (0.1 mmol, 1.0 eq.) in DMF (0.4 mL) in a round-bottomed flask, under inert atmosphere (N₂), are added DIPEA (2.7 eq.) and the required acid (2.5 eq.). The mixture is stirred at rt for 10 min and HATU (1.05 eq.) is added at once. The reaction mixture is stirred at rt and monitored by LC-MS. Upon reaction completion, the reaction mixture is treated with PL-HCO₃ (194 mg; Polymer Laboratories; loading: 2.06 mmol/g; particle size: 150-300 µm; pore diameter: 100 Å). It is shaken 2 h at rt, filtered and concentrated under reduced pressure. Purification of the residue gives the desired product.

### Procedure Y (reductive amination using NaBH₃CN):

The aldehyde derivative (0.1 mmol, 1.0 eq.) and the required amine (1.3 eq.) are dissolved in 95:5 MeOH/NMP (0.5 mL) in a glass vial, under inert atmosphere (N₂) and treated with AcOH (2.0 eq.) and NaBH₃CN (1.5 eq.). The reaction mixture is stirred at rt and monitored by LC-MS. Upon reaction completion, the reaction mixture is concentrated under reduced pressure. Purification of the residue gives the desired product.

### Procedure Z (reductive amination using NaBH₃CN, inversed stoichiometry):

The aldehyde derivative (0.1 mmol, 1.0 eq.) and the required amine (5.0 eq.) are dissolved in 1:1 MeOH/THF (3.0 mL) in a glass vial, under inert atmosphere (N₂) and treated with TsOH (0.5 eq.). The reaction mixture is stirred at 70°C for 2 h, cooled to rt and treated with AcOH (6.0 eq.) and NaBH₃CN (5.0 eq.). The reaction mixture is stirred at rt and monitored by LC-MS. Upon reaction completion, the reaction mixture is concentrated under reduced pressure. Purification of the residue gives the desired product.

### Procedure AA (amide formation using HA TU):

To a solution of the acid (0.1 mmol, 1.0 eq.) in DMF (0.25 mL) in a glass vial, under inert atmosphere (N₂), are added DIPEA (2.5 eq.) and the required amine (2.5 eq.). The mixture is stirred at rt for 10 min and treated with a solution of HATU (1.05 eq.) in DMF (0.25 mL). The reaction mixture is stirred at rt and monitored by LC-MS. Upon reaction completion, the reaction mixture is treated with PL-HCO₃ (194 mg; Polymer Laboratories; loading: 2.06 mmol/g; particle size: 150-300 µm; pore diameter: 100 Å). It is shaken 2 h at rt, filtered and concentrated under reduced pressure. Purification of the residue gives the desired product.

### Procedure AB (Buchwald-Hartwig amination with Pd₂(dba)₃/XPhos and scavenger treatment):

To the aromatic halide (0.1 mmol, 1.0 eq.), the required amine derivative (1.5 eq.), Pd₂(dba)₃ (0.05 eq.), X-Phos (0.1 eq.) and tBuONa (1.2 eq.) in a glass vial, under inert atmosphere (N₂), is added dry dioxane (0.5 mL). The resulting reaction mixture is purged with N₂ for 5 min, stirred at 90°C and monitored by LC-MS. Upon reaction completion, the reaction mixture is diluted with DCM and a sat. aq. NaHCO₃ solution. The layers are separated and the aq. layer is extracted with 9:1 DCM/MeOH (3x). The combined org. layers are washed with a sat. aq. NaHCO₃ solution, water and brine, dried over MgSO₄, filtered and concentrated under reduced pressure. The residue is dissolved in 9:1 DCM/MeOH (2.0 mL) and treated with a 1:1 mixture (40 mg) of triamine ethyl sulfide amide silica (PhosphonicS STA3; loading: 0.8 mmol/g; particle size: 60-200 µm; pore diameter: 60 Å) and methyl thiourea ethylsulfide ethyl silica (PhosphonicS MTCf; loading: 0.6 mmol/g; particle size: 60-200 µm; pore diameter: 90 Å). The mixture is shaken at rt overnight and filtered. The scavengers are washed with 9:1 DCM/MeOH and the filtrate is concentrated under reduced pressure. Purification of the residue gives the desired product.

### Procedure AC (nucleophilic substitution of chloride with alcohols):

A solution of the required alcohol (0.30 mmol; 3 eq.) in DMF (0.2 mL), in a glass vial under inert atmosphere (N₂), is treated with NaH (3.3 eq.) at rt. After 10 min, it is treated with a solution of the chloride (0.10 mmol; 1 eq.) in DMF (0.8 mL) and the reaction mixture is stirred at rt and monitored by LC-MS. Upon reaction completion, the reaction mixture is either treated with silica gel-supported sulfonic acid (5.0 eq.; Silicycle SiliaBond^{®} Tosic Acid; SCX; R60530B; 0.8 mmol/g), shaken 1 h at rt and filtered, or loaded on a corresponding cartridge (Silicycle SiliaPrep™ Tosic Acid Si-SCX). In both cases, the resin is then washed with DCM, 1:1 DCM/MeOH and MeOH, and the product eventually released from the resin with 7*M* ammonia solution in MeOH. The solution of crude product is concentrated under reduced pressure and purification of the residue gives the desired product.

### Procedure AD (one-pot dihydroxylation periodate cleavage):

To a solution of alkene (1.0 mmol, 1.0 eq.) in 4:1 acetone/water (10 mL) in a round-bottomed flask, are added NMO (1.7 eq.) and potassium osmate dihydrate (0.005 eq.) at rt. The reaction mixture is stirred at rt for 16 h and is then treated with a solution of sodium periodate (2.0 eq.) in water (10 mL). The reaction mixture was stirred at rt for 2 h and diluted with 9:1 DCM/MeOH. The layers are separated and the aq. layer is extracted with 9:1 DCM/MeOH. The combined org. layers are washed with water (2x) and brine, dried over MgSO₄, filtered and concentrated under reduced pressure. Purification of the residue gives the desired product.

### Procedure AE (Buchwald-Hartwig amination with BrettPhos precatalyst and scavenger treatment):

To the aromatic halide (0.1 mmol, 1.0 eq.), the required amine derivative (2.0 eq.), (BrettPhos) palladium(II) phenethylamine chloride (0.05 eq.) and tBuONa (1.3 eq.) in a glass vial, under inert atmosphere (N₂), is added dry dioxane (0.5 mL). The resulting reaction mixture is purged with N₂ for 5 min, stirred at 90°C and monitored by LC-MS. Upon reaction completion, the reaction mixture is diluted with DCM and a sat. aq. NaHCO₃ solution. The layers are separated and the aq. layer is extracted with 9:1 DCM/MeOH (3x). The combined org. layers are washed with a sat. aq. NaHCO₃ solution, water and brine, dried over MgSO₄, filtered and concentrated under reduced pressure. The residue is dissolved in 9:1 DCM/MeOH (2.0 mL) and treated with a 1:1 mixture (40 mg) of triamine ethyl sulfide amide silica (PhosphonicS STA3; loading: 0.8 mmol/g; particle size: 60-200 µm; pore diameter: 60 Å) and methyl thiourea ethylsulfide ethyl silica (PhosphonicS MTCf; loading: 0.6 mmol/g; particle size: 60-200 µm; pore diameter: 90 Å). The mixture is shaken at rt overnight and filtered. The scavengers are washed with 9:1 DCM/MeOH and the filtrate is concentrated under reduced pressure. Purification of the residue gives the desired product.

### Procedure AF (reduction of carboxylic acid):

A solution of the acid (1.0 mmol; 1.0 eq.) in dry THF (4 mL) in a round-bottomed flask, under inert atmosphere (N₂), is treated with a solution of borane-THF complex (1M in THF; 1.5 eq.) at 0°C. The reaction mixture is stirred at rt and monitored by LC-MS. Upon reaction completion, the reaction mixture is concentrated under reduced pressure and diluted with EA and 1*N* HCl. The layers are separated and the aq. layer is extracted with EA (3x). The combined org. layers are washed with 1*N* HCl, 1*M* NaOH, water and brine, dried over MgSO₄, filtered and concentrated under reduced pressure. Purification of the residue gives the desired product.

### Procedure AG (mesylation):

A solution of the alcohol (1.0 mmol; 1.0 eq.) in dry THF (4 mL) in a round-bottomed flask, under inert atmosphere (N₂), is treated with TEA (1.5 eq.) and a solution of methanesulfonic anhydride (1.5 eq.) in dry THF (1 mL) at 0°C. The reaction mixture is stirred at rt and monitored by LC-MS. Upon reaction completion, the reaction mixture is concentrated under reduced pressure and diluted with EA and water. The layers are separated and the aq. layer is extracted with EA. The combined org. layers are washed with water and brine, dried over MgSO₄, filtered and concentrated under reduced pressure. Purification of the residue gives the desired product.

### Procedure AH (nucleophilic substitution of mesylate with phthalimide):

A solution of the mesylate (1.0 mmol; 1.0 eq.) in dry DMF (5 mL) in a round-bottomed flask, under inert atmosphere (N₂), is treated with phthalimide potassium salt (1.2 eq.) at rt. The reaction mixture is stirred at rt and monitored by LC-MS. Upon reaction completion, the reaction mixture is concentrated under reduced pressure and diluted with DCM and water. The layers are separated and the aq. layer is extracted with DCM. The combined org. layers are washed with water and brine, dried over MgSO₄, filtered and concentrated under reduced pressure. Purification of the residue gives the desired product.

### Procedure AI (phthalimide hydrolysis):

A solution of the phthalimide derivative (1.0 mmol; 1.0 eq.) in dry MeOH (5 mL) in a round-bottomed flask, under inert atmosphere (N₂), is treated with hydrazine monohydrate (2.0 eq.) at rt. The reaction mixture is stirred at 65°C and monitored by LC-MS. Upon reaction completion, the reaction mixture is cooled to rt and treated with water. Then most of the MeOH is removed under reduced pressure. The resulting aq. suspension is acidified with 1N HCl and washed twice with DCM. The aq. layer is treated with 1N NaOH until pH 12 and extracted with 9:1 DCM/MeOH (3x). The combined org. layers are washed with brine, dried over MgSO₄, filtered and concentrated under reduced pressure. Purification of the residue gives the desired product.

### Procedure AJ (Ullmann-type coupling using N,N-dimethylglycine):

To the aromatic halide (0.1 mmol, 1.0 eq.), the required alcohol (1.5 eq.), copper(I) iodide (0.1 eq.), K₃PO₄ (2.0 eq.) and *N,N*-dimethylglycine (0.2 eq.) in a glass vial, under inert atmosphere (N₂), is added dry DMSO (0.2 mL). The reaction mixture is purged with N₂ for 5 min, stirred at 90°C and monitored by LC-MS. Upon reaction completion, the reaction mixture is cooled down to rt, diluted with 9:1 DCM/MeOH and a sat. aq. NaHCO₃ solution. The layers are separated and the aq. layer is extracted with 9:1 DCM/MeOH (3x). The combined org. layers are washed with water and brine, dried over MgSO₄, filtered and concentrated under reduced pressure. Purification of the residue gives the desired product.

### Procedure AK (Suzuki coupling with tricyclohexylphosphine and scavenger treatment):

To the aromatic halide (0.1 mmol; 1.0 eq.), the required boronic acid (2.0 eq.), Pd₂(dba)₃ (0.1 eq.) and PCy₃ (0.2 eq.) in a glass vial, under inert atmosphere (N₂), are added dioxane (0.8 mL) and an aq. 1*N* K₂CO₃ solution (0.2 mL; 2.0 eq.). The reaction mixture is purged with N₂ for 5 min, stirred at 100°C and monitored by LC-MS. Upon reaction completion, the reaction mixture is diluted with DCM and a sat. aq. NaHCO₃ solution. The layers are separated and the aq. layer is extracted with 9:1 DCM/MeOH (3x). The combined org. layers are washed with a sat. aq. NaHCO₃ solution, water and brine, dried over MgSO₄, filtered and concentrated under reduced pressure. The residue is dissolved in 9:1 DCM/MeOH (2.0 mL) and treated with a 1:1 mixture (40 mg) of triamine ethyl sulfide amide silica (PhosphonicS STA3; loading: 0.8 mmol/g; particle size: 60-200 µm; pore diameter: 60 Å) and methyl thiourea ethylsulfide ethyl silica (PhosphonicS MTCf; loading: 0.6 mmol/g; particle size: 60-200 µm; pore diameter: 90 Å). The mixture is shaken at rt overnight and filtered. The scavengers are washed with 9:1 DCM/MeOH and the filtrate is concentrated under reduced pressure. Purification of the residue gives the desired product.

### Procedure AL (amide formation using HA TU):

To a solution of the required acid (0.1 mmol, 1.0 eq.) in DMF (0.4 mL) in a round-bottomed flask, under inert atmosphere (N₂), are added DIPEA (3.0 eq.) and the required amine (1.1 eq.). The mixture is stirred at rt for 10 min and HATU (1.5 eq.) is added at once. The reaction mixture is stirred at rt and monitored by LC-MS. Upon reaction completion, the reaction mixture is concentrated under reduced pressure. Purification of the residue gives the desired product.

### Procedure AM (nucleophilic substitution of chloride with amines and SCX treatment):

A solution of the chloride (0.10 mmol; 1 eq.) in NMP (0.5 mL) is treated with the required amine (1.0 eq.), in a glass vial under inert atmosphere (N₂) at rt. The reaction mixture is stirred at rt and monitored by LC-MS. Upon reaction completion, the reaction mixture is diluted with MeOH and is either treated with silica gel-supported sulfonic acid (5.0 eq.; Silicycle SiliaBond^{®} Tosic Acid; SCX; R60530B; 0.8 mmol/g), shaken 1 h at rt and filtered, or loaded on a corresponding cartridge (Silicycle SiliaPrep™ Tosic Acid Si-SCX). In both cases, the resin is then washed with DCM, 1:1 DCM/MeOH and MeOH, and the product eventually released from the resin with 7*M* ammonia solution in MeOH. The solution of crude product is concentrated under reduced pressure and purification of the residue gives the desired product.

### Procedure AN (nucleophilic substitution of chloride with amines):

A solution of the chloride (0.25 mmol; 1.0 eq.) in DMF (1.25 mL), in a glass vial under inert atmosphere (N₂), is treated with the required amine (2.0 eq.) at rt. The reaction mixture is stirred at rt and monitored by LC-MS. Upon reaction completion, the reaction mixture is diluted with 9:1 DCM/MeOH and a sat. aq. NaHCO₃ solution. The layers are separated and the aq. layer is extracted with 9:1 DCM/MeOH (3x). The combined org. layers are dried over MgSO₄, filtered and concentrated under reduced pressure. Purification of the residue gives the desired product.

### Procedure AO (preparation of boronate ester):

To a mixture of the aromatic halide (1.0 mmol; 1.0 eq.), bis(neopentyl glycolato)diboron (1.2 eq.), AcOK (3.0 eq.) and [1,1'-bis(diphenylphosphino)ferrocene]dichloropalladium(II) complex with DCM (0.1 eq.) in a glass vial, under inert atmosphere (Ar), is added degassed DMSO (5.0 mL). The resulting reaction mixture is purged at rt with N₂ for 5 min, stirred at 90°C and monitored by LC-MS. Upon reaction completion, the reaction mixture is concentrated under reduced pressure, the residue diluted with 9:1 DCM/MeOH and a sat. aq. NH₄Cl solution is added. The layers are separated and the aq. layer is extracted with 9:1 DCM/MeOH (3x). The combined org. layers are dried over MgSO₄, filtered and concentrated under reduced pressure. Purification of the residue gives the desired product.

### Procedure AP (Suzuki coupling with SCX treatment):

To the aromatic boronic ester (0.1 mmol; 1.0 eq.), the required halide derivative (2.0 eq.) and Pd(PPh₃)₄ (0.1 eq.) in a glass vial, under inert atmosphere (Ar), are added degassed dioxane (0.8 mL) and degassed aq. 1M K₂CO₃ solution (0.3 mL). The reaction mixture is stirred at 115°C for 4 h, then allowed to cool down to rt and further stirred at rt for 20 h. The reaction mixture is diluted with MeOH and a few drops of AcOH are added to obtain a solution. This solution is either treated with silica gel-supported sulfonic acid (5.0 eq.; Silicycle SiliaBond^{®} Tosic Acid; SCX; R60530B; 0.8 mmol/g), shaken 1 h at rt and filtered, or loaded on a corresponding cartridge (Silicycle SiliaPrep™ Tosic Acid Si-SCX). In both cases, the resin is washed with DCM, 1:1 DCM/MeOH and MeOH, and the product eventually released from the resin with 7M ammonia solution in MeOH. The solution of crude product is concentrated under reduced pressure and purification of the residue gives the desired product.

### Procedure AQ (nucleophilic substitution of chloride with amines using NaH):

A solution of the required amine (0.20 mmol; 2 eq.) in DMF (0.2 mL), in a glass vial under inert atmosphere (N₂), is treated with NaH (3.3 eq.) at rt. After 10 min, it is treated with a solution of the chloride (0.10 mmol; 1 eq.) in DMF (0.8 mL) and the reaction mixture is stirred at rt and monitored by LC-MS. Upon reaction completion, the reaction mixture is quenched by the addition of a sat. aq. KH₂PO₄ solution and the reaction mixture is stirred at rt for 20 h. The reaction mixture is concentrated under reduced pressure and purification of the residue gives the desired product.

### PREPARATION OF SYNTHETIC INTERMEDIATES

### Preparation A: 5-bromo-8-methyl-isoquinolin-3-ylamine:

### A.1. N-(5-bromo-2-methyl-benzyl)-2,2-diethoxy-acetamidine:

Starting from 5-bromo-2-methyl-benzylamine (21.21 g; prepared according to WO 2009/100168) and 2,2-diethoxy-ethanimidic acid methyl ester (23.93 g; commercial) and proceeding in analogy to Procedure L, the title compound was obtained as a beige solid (37.19 g).
MS (ESI, m/z): 329.23 and 331.2 [M+H⁺ of the two main isotopes].

### A.2. 5-bromo-8-methyl-isoquinolin-3-ylamine:

Starting from intermediate A.1 (37.19 g) and proceeding in analogy to Procedure M, the title compound was obtained, after purification by CC (DCM/MeOH 100:0 to 97:3), as a yellow solid (9.70 g; 39% yield over 2 steps).
¹H NMR (d6-DMSO) δ: 8.93 (d, J = 0.6 Hz, 1H); 7.66 (d, J = 7.3 Hz, 1H); 6.84 (dd, J = 7.6, 1.2 Hz, 1H); 6.79 (d, J = 0.9 Hz, 1H); 6.24 (br. s, 2H); 2.57 (d, J = 0.9 Hz, 3H). MS (ESI, m/z): 237.1 and 239.0 [M+H⁺ of the two main isotopes].

### Preparation B: 1-(6-bromo-8-methyl-isoquinolin-3-yl)-3-ethyl-urea:

### B.1. N-(4-bromo-2-methyl-benzyl)-2,2-diethoxy-acetamidine:

Starting from 4-bromo-2-methyl-benzenemethanamine (24.42 g; commercial) and 2,2-diethoxy-ethanimidic acid methyl ester (26.18 g) and proceeding in analogy to Procedure L, the title compound was obtained as a yellow oil (43.86 g).
MS (ESI, m/z): 329.4 and 331.3 [M+H⁺ of the two main isotopes].

### B.2. 5-bromo-8-methyl-isoquinolin-3-ylamine:

Starting from intermediate B.1 (43.86 g) and proceeding in analogy to Procedure M, the title compound was obtained, after purification by CC (Hept/EA 60:40 to 0:100), as a brown solid (6.22 g; 22% yield over 2 steps).
¹H NMR (d6-DMSO) δ: 8.90 (s, 1H); 7.63-7.60 (m, 1H); 7.05-7.03 (m, 1H); 6.53 (s, 1H); 6.05 (br. s, 2H); 2.57 (s, 3H).
MS (ESI, m/z): 237.1 and 239.0 [M+H⁺ of the two main isotopes].

### B.3. 1-(6-bromo-8-methyl-isoquinolin-3-yl)-3-ethyl-urea:

Starting from intermediate B.2 (6.22 g) and proceeding in analogy to Procedure N, the title compound was obtained as a pale yellow solid (6.13 g; 76% yield).
¹H NMR (d6-DMSO) δ: 9.12 (s, 1H); 9.09 (s, 1H); 7.95 (s, 1H); 7.87 (br. s, 1H); 7.36-7.32 (m, 1H); 7.02 (t, J = 5.6 Hz, 1H); 3.22-3.10 (m, 2H); 2.66 (s, 3H); 1.07 (t, J = 7.3 Hz, 3H).
MS (ESI, m/z): 308.3 and 310.4 [M+H⁺ of the two main isotopes].

### Preparation C: 8-bromo-5-fluoro-isoquinolin-3-ylamine:

### C.1. N-(2-bromo-5-fluoro-benzyl)-2,2-diethoxy-acetamidine:

Starting from 2-bromo-5-fluorobenzylamine (5.40 g) and proceeding in analogy to Procedure L, the title compound was obtained as an orange oil (9.35 g).
MS (ESI, m/z): 333.2 and 335.1 [M+H⁺ of the two main isotopes].

### C.2. 8-bromo-5 fluoro-isoquinolin-3-ylamine:

Starting from intermediate C.1 (9.35 g) and proceeding in analogy to Procedure M, the reaction mixture being however heated to 40°C, the title compound was obtained, after purification by CC (Hept/EA 100:0 to 10:90), as a yellow solid (3.11 g; 49% yield over 2 steps).
¹H NMR (d6-DMSO) δ: 8.95-8.92 (m, 1H); 7.34 (dd, J = 8.2, 4.7 Hz, 1H); 7.21 (dd, J = 10.8, 8.2 Hz, 1H); 6.67 (d, J = 0.6 Hz, 1H); 6.47 (br. s, 2H).
MS (ESI, m/z): 241.2 and 243.0 [M+H⁺ of the two main isotopes].

### Preparation D: 1-(8-chloro-5-fluoro-6-pyridin-3-yl-isoquinolin-3-yl)-3-ethyl-urea:

### D.1. (4-bromo-2-chloro-5-fluoro-phenyl)-methanol:

A solution of borane-THF complex (1*M* in THF; 200 mL) was added to a solution of 4-bromo-2-chloro-5-fluorobenzoic acid (25.35 g; commercial) in dry THF (200 mL) at rt. The reaction mixture was stirred at rt for 2 h. The reaction mixture was concentrated under reduced pressure and diluted with EA and water. The org. layer was washed with aq. 1*N* HCl, aq. 1*M* NaOH, water and brine, dried over MgSO₄, filtered, concentrated under reduced pressure and dried to give the title compound as a white solid (22.35 g; 93% yield).
¹H NMR (d6-DMSO) δ: 7.81 (d, J = 6.2 Hz, 1H); 7.43 (d, J = 9.7 Hz, 1H); 5.60 (t, J = 5.6 Hz, 1H); 4.48 (d, J = 5.6 Hz, 2H).

### D.2. 2-(4-bromo-2-chloro-5-fluoro-benzyl)-isoindole-1,3-dione:

DIAD (24.08 mL) was added dropwise to a suspension of intermediate D.1 (22.35 g), PPh₃ (29.41 g) and phthalimide (16.50 g) in dry THF (500 mL) at rt. The reaction mixture was stirred at rt overnight. The reaction mixture was concentrated under reduced pressure and the crude residue was purified twice by CC (eluents for first CC: Hept/EA 100:0 to 50:50; eluents for second CC: Hept/DCM 50:50 to 0:100), affording the title compound as a beige solid (31.25 g; 91% yield).
¹H NMR (d6-DMSO) δ: 7.94-7.82 (m, 4H); 7.87 (d, J = 5.6 Hz, 1H); 7.48 (d, J = 9.4 Hz, 1H); 4.76 (s, 2H).

### D.3. 4-bromo-2-chloro-5-fluoro-benzylamine hydrochloride:

Hydrazine monohydrate (8.54 mL) was added dropwise to a solution of intermediate D.2 (31.25 g) in MeOH (600 mL) at rt. The reaction mixture was stirred at 65°C for 2.5 h. The reaction mixture was cooled to rt, diluted with water (200 mL) and most of the MeOH was removed under reduced pressure. The resulting aq. suspension was basified with 1*N* HCl (50 mL) and filtered. The filtrate was washed twice with DCM. The aq. layer was treated with 1*N* NaOH until pH 12 and extracted with DCM and 9:1 DCM/MeOH (3x). The combined org. layers were washed with brine, dried over MgSO₄ and concentrated under reduced pressure. The residue was dissolved in MeCN (220 mL) and treated with conc. HCl (22 mL). The precipitated solid was collected by fitration and dried to give the title compound as a white solid (17.23 g; 74% yield).
¹H NMR (d6-DMSO) δ: 8.78 (br. s, 3H); 7.98 (d, J = 6.4 Hz, 1H); 7.78 (d, J = 9.7 Hz, 1H); 4.08 (s, 2H).

### D.4. N-(4-bromo-2-chloro-5-fluoro-benzyl)-2,2-diethoxy-acetamidine:

Starting from intermediate D.3 (17.23 g) and 2,2-diethoxy-ethanimidic acid methyl ester (15.50 g) and proceeding in analogy to Procedure L, the title compound was obtained as a yellow oil (22.12 g).
MS (ESI, m/z): 366.9 and 369.3 [M+H⁺ of the two main isotopes].

### D.5. 6-bromo-8-chloro-5 fluoro-isoquinolin-3-ylamine:

Starting from intermediate D.4 (22.12 g) and proceeding in analogy to Procedure M, the reaction mixture being however heated to 40°C, the title compound was obtained, after purification by CC (Hept/EA 100:0 to 50:50), as a yellow solid (5.10 g; 30% yield over 2 steps).
¹H NMR (d6-DMSO) δ: 9.01-8.98 (m, 1H); 7.45 (d, J = 5.9 Hz, 1H); 6.66 (s, 1H); 6.65 (br. s, 2H).
MS (ESI, m/z): 275.0 and 277.0 [M+H⁺ of the two main isotopes].

### D.6. 1-(6-bromo-8-chloro-5-fluoro-isoquinolin-3-yl)-3-ethyl-urea:

Starting from intermediate D.5 (3.79 g) and ethyl isocyanate (6.60 mL) and proceeding in analogy to Procedure N, TEA (2.00 mL) being however added, the title compound was obtained as a white solid (2.57 g; 56% yield).
¹H NMR (d6-DMSO) δ: 9.43 (s, 1H); 9.22-9.20 (m, 1H); 8.23 (s, 1H); 7.82 (d, J = 5.9 Hz, 1H); 6.89 (t, J = 5.3 Hz, 1H); 3.22-3.11 (m, 2H); 1.08 (t, J = 7.0 Hz, 3H).
MS (ESI, m/z): 346.3 and 348.0 [M+H⁺ of the two main isotopes].

### D.7. 1-(8-chloro-5-fluoro-6-pyridin-3-yl-isoquinolin-3-yl)-3-ethyl-urea:

Starting from intermediate D.6 (944 mg) and 3-pyridineboronic acid (670 mg) and proceeding in analogy to Procedure B, the title compound was obtained, after purification by CC (DCM/MeOH 100:0 to 90:10), as a yellow solid (770 mg; 83% yield).
¹H NMR (d6-DMSO) δ: 9.41 (s, 1H); 9.28-9.26 (m, 1H); 8.90 (s, 1H); 8.67 (dd, J = 4.7, 1.5 Hz, 1H); 8.32 (d, J = 0.6 Hz, 1H); 8.17-8.11 (m, 1H); 7.75 (d, J = 6.4 Hz, 1H); 7.57 (ddd, J = 7.9, 5.0, 0.9 Hz, 1H); 6.93 (t, J = 5.6 Hz, 1H); 3.23-3.12 (m, 2H); 1.09 (t, J = 7.0 Hz, 3H).
MS (ESI, m/z): 345.4 [M+H⁺].

### Preparation E: 1-ethyl-3-[8-methyl-5-(4,4,5,5-tetramethyl-[1,3,2]dioxaborolan-2-yl)-isoquinolin-3-yl]-urea:

A mixture of PCy₃ (88 mg) and Pd₂(dba)₃ (60 mg) in dioxane (6.5 mL) was purged with N₂ and stirred at rt for 30 min. Bis(pinacolato)diboron (1.09 g), the compound of Example 1 (400 mg) and AcOK (193 mg) were added to the solution. The reaction mixture was then purged with N₂ and heated to 95°C for 19 h. The reaction mixture was cooled to rt and was partitioned between EA and water. The aq. layer was extracted with EA (3x). The combined org. layers were dried over MgSO₄, filtered and concentrated under reduced pressure. The title compound was obtained, after purification by CC (DCM/MeOH 100:0 to 98:2) followed by a second CC (Hept/EA 100:0 to 60:40), as a yellow solid (164 mg; 70% purity).
¹H NMR (d6-DMSO) δ: 9.16-9.12 (m, 2H); 8.53 (s, 1H); 7.87 (d, J = 6.7 Hz, 1H); 7.64-7.54 (m, 1H); 7.19 (dd, J = 7.0, 0.6 Hz, 1H); 3.25-3.12 (m, 2H); 2.69 (s, 3H); 1.34 (s, 12H); 1.10 (t, J = 7.0 Hz, 3H).
MS (ESI, m/z): 356.2 [M+H⁺].

### Preparation F: 1-(8-chloro-5-fluoro-6-pyridin-2-yl-isoquinolin-3-yl)-3-ethyl-urea:

Starting from intermediate D.6 (100 mg) and a solution of 2-pyridinylzinc bromide (0.5*M* in THF; 3.0 mL) and proceeding in analogy to Procedure D, the reaction mixture being however heated to 80°C, the title compound was obtained, after purification by CC (Hept/EA 100:0 to 0:100), as a yellow solid (88 mg; 88% yield).
¹H NMR (d6-DMSO) δ: 9.42 (s, 1H); 9.26-9.24 (m, 1H); 8.81-8.77 (m, 1H); 8.37 (s, 1H); 8.06-7.96 (m, 2H); 7.64-7.46 (m, 2H); 7.00-6.92 (m, 1H); 3.23-3.12 (m, 2H); 1.09 (t, J = 7.0 Hz, 3H).
MS (ESI, m/z): 345.4 [M+H⁺].

### Preparation G: 1-(5-bromo-8-chloro-isoquinolin-3-yl)-3-ethyl-urea:

### G.1. N-(5-bromo-2-chloro-benzyl)-2,2-diethoxy-acetamidine:

Starting from 5-bromo-2-chlorobenzylamine (15.09 g; commercial) and 2,2-diethoxy-ethanimidic acid methyl ester (15.14 g) and proceeding in analogy to Procedure L, the title compound was obtained as an orange viscous oil (23.95 g).
MS (ESI, m/z): 349.2 and 351.0 [M+H⁺ of the two main isotopes].

### G.2. 5-bromo-8-chloro-isoquinolin-3-ylamine:

Starting from intermediate G.1 (23.95 g) and proceeding in analogy to Procedure M, the title compound was obtained, after purification by CC (Hept/EA 100:0 to 0:100), as a yellow solid (11.29 g; 64% yield over 2 steps).
¹H NMR (d6-DMSO) δ: 9.02 (d, J = 0.9 Hz, 1H); 7.76 (d, J = 7.9 Hz, 1H); 7.14 (d, J = 7.9 Hz, 1H); 6.82 (d, J = 0.6 Hz, 1H); 6.58 (br. s, 2H).
MS (ESI, m/z): 257.2 and 259.1 [M+H⁺ of the two main isotopes].

### G.3. 1-(5-bromo-8-chloro-isoquinolin-3-yl)-3-ethyl-urea:

Starting from intermediate G.2 (5.00 g) and ethyl isocyanate (7.70 mL) and proceeding in analogy to Procedure N, the title compound was obtained as a pale yellow solid (4.25 g; 67% yield).
¹H NMR (d6-DMSO) δ: 9.37 (s, 1H); 9.24 (d, J = 0.6 Hz, 1H); 8.45 (d, J = 0.9 Hz, 1H); 7.97 (d, J = 8.2 Hz, 1H); 7.45 (d, J = 8.2 Hz, 1H); 6.91 (t, J = 5.3 Hz, 1H); 3.23-3.12 (m, 2H); 1.08 (t, J = 7.0 Hz, 3H).
MS (ESI, m/z): 328.3 and 330.01 [M+H⁺ of the two main isotopes].

### Preparation H: 1-(8-chloro-5-pyridin-3-yl-isoquinolin-3-yl)-3-ethyl-urea:

Starting from the compound of Preparation G (1.00 g) and 3-pyridineboronic acid (748 mg) and proceeding in analogy to Procedure B, the title compound was obtained, after purification by CC (DCM/MeOH 100:0 to 90:10), as a yellow solid (663 mg; 67% yield).
¹H NMR (d6-DMSO) δ: 9.32 (d, J = 0.6 Hz, 1H); 9.19 (s, 1H); 8.71-8.65 (m, 2H); 8.11 (d, J = 0.9 Hz, 1H); 7.92 (dt, J =7.9, 2.1 Hz, 1H); 7.64-7.55 (m, 3H); 6.92 (t, J =5.6 Hz, 1H); 3.10 (qd, J =7.3, 5.9 Hz, 2H); 1.03 (t, J = 7.0 Hz, 3H).
MS (ESI, m/z): 327.4 [M+H⁺].

### Preparation I: 1-(6-bromo-isoquinolin-3-yl)-3-ethyl-urea:

### 1.1. N-(4-bromo-benzyl)-2,2-diethoxy-acetamidine:

Starting from 4-bromobenzylamine (25.00 g; commercial) and 2,2-diethoxy-ethanimidic acid methyl ester (30.00 g), and proceeding in analogy to Procedure L, the title compound was obtained as a yellow oil (46.80 g).
MS (ESI, m/z): 315.2 and 317.0 [M+H⁺ of the two main isotopes].

### I.2. 6-bromo-isoquinolin-3-ylamine:

Starting from intermediate I.1 (46.80 g) and proceeding in analogy to Procedure M, the title compound was obtained, after filtration of the basic aq. layer and wash of the precipitate with water (2 L), as a yellow solid (20.61 g; 69% yield over 2 steps).
¹H NMR (d6-DMSO) δ: 8.79 (s, 1H); 7.77 (d, J = 1.8 Hz, 1H); 7.71 (d, J = 8.8 Hz, 1H); 7.20 (dd, J = 8.5, 1.8 Hz, 1H); 6.54 (s, 1H); 6.08 (br. s, 2H).
MS (ESI, m/z): 223.0 and 225.3 [M+H⁺ of the two main isotopes].

### I.3. 1-(6-bromo-isoquinolin-3-yl)-3-ethyl-urea:

Intermediate 1.2 (5.00 g) and ethyl isocyanate (7.10 mL) were reacted proceeding in analogy to Procedure N. The reaction mixture was then concentrated, diluted in 9:1 DCM/MeOH, washed with a sat. aq. NaHCO₃ solution, water and brine, dried over MgSO₄, filtered and concentrated under reduced pressure. The title compound was obtained, after purification by CC (DCM/MeOH 100:0 to 90:10), a beige solid (4.03 g; 61% yield).
¹H NMR (d6-DMSO) δ: 9.07 (s, 1H); 9.02 (s, 1H); 8.05 (d, J = 1.8 Hz, 1H); 8.00 (s, 1H); 7.91 (d, J = 8.8 Hz, 1H); 7.51 (dd, J = 8.8, 2.1 Hz, 1H); 6.95 (t, J = 5.6 Hz, 1H); 3.16 (qd, J = 7.3, 5.9 Hz, 2H); 1.07 (t, J = 7.0 Hz, 3H).
MS (ESI, m/z): 294.4 and 296.4 [M+H⁺ of the two main isotopes].

### Preparation J: 1-[6-(benzhydrylidene-amino)-8-methyl-isoquinolin-3-yl]-3-ethyl-urea:

To the compound of Preparation B (6.60 g), benzophenone imine (5.40 g), tBuONa (3.00 g), Pd₂dba₃ (200 mg) and BINAP (400 mg) in a round-bottomed flask under inert atmosphere (N₂) was added dry dioxane (180 mL) at rt. The reaction mixture was purged with N₂ for 5 min and stirred at 80°C for 18 h. The reaction mixture was diluted with DCM and a sat. aq. NH₄Cl solution. The layers were separated and the aq. layer was extracted with DCM (3x). The combined org. layers were washed with brine, dried over MgSO₄, filtered and concentrated under reduced pressure. Purification of the residue by CC (DCM/EA 50:50 to 0:100) followed by a second CC (DCM/MeOH 100:0 to 90:10) gave a yellow residue. The title compound was obtained, after trituration of the residue in Et₂O, as a yellow solid (7.13 g; 82% yield).
MS (ESI, m/z): 409.5 [M+H⁺].

### Preparation K: 1-(8-bromo-isoquinolin-3-yl)-3-ethyl-urea:

### K.1. N-(2-bromo-benzyl)-2,2-diethoxy-acetamidine:

Starting from 2-bromobenzylamine (25.00 g; commercial) and 2,2-diethoxy-ethanimidic acid methyl ester (26.00 g) and proceeding in analogy to Procedure L, the title compound was obtained as an orange oil (43.45 g).
MS (ESI, m/z): 315.2 and 317.0 [M+H⁺ of the two main isotopes].

### K.2. 8-bromo-isoquinolin-3-yl-amine:

Starting from intermediate K.1 (43.45 g) and proceeding in analogy to Procedure M, the title compound was obtained, after purification by CC (Hept/EA 100:0 to 30:70), as a yellow solid (11.85 g; 40% yield over 2 steps).
¹H NMR (d6-DMSO) δ: 8.92 (s, 1H); 7.55-7.50 (m, 1H); 7.41-7.37 (m, 1H); 7.33-7.26 (m, 1H); 6.61 (s, 1H); 6.18 (br. s, 2H).
MS (ESI, m/z): 223.1 and 225.4 [M+H⁺ of the two main isotopes].

### K.3. 1-(8-bromo-isoquinolin-3-yl)-3-ethyl-urea:

Starting from intermediate K.2 (10.84 g) and ethyl isocyanate (11.50 mL) and proceeding in analogy to Procedure N, a first batch of product was obtained (6.93 g). The mother liquors were concentrated under reduced pressure and the residue purified by CC (DCM/MeOH 100:0 to 95:5) to give a second batch of product (4.68 g). The title compound was obtained, after combining the two batches, as a yellow solid (11.62 g; 81% yield).
¹H NMR (d6-DMSO) δ: 9.19 (s, 1H); 9.14 (s, 1H); 8.09 (s, 1H); 7.81 (d, J = 8.5 Hz, 1H); 7.69 (d, J = 7.6 Hz, 1H); 7.51 (dd, J = 8.5, 7.6 Hz, 1H); 6.94 (t, J = 5 .6 Hz, 1H); 3.22-3.11 (m, 2H); 1.08 (t, J = 7.3 Hz, 3H).
MS (ESI, m/z): 294.4 and 296.6 [M+H⁺ of the two main isotopes].

### Preparation L: 1-(8-chloro-5-pyridin-4-yl-isoquinolin-3-yl)-3-ethyl-urea:

Starting from the compound of Preparation G (3.00 g) and 4-pyridineboronic acid (2.24 g) and proceeding in analogy to Procedure B, the title compound was obtained, after purification by CC (DCM/MeOH +1% NH₄OH 100:0 to 96:4), as a yellow solid (2.19 g; 74% yield).
¹H NMR (d6-DMSO) δ: 9.32 (d, J = 0.9 Hz, 1H); 9.20 (s, 1H); 8.75-8.71 (m, 2H); 8.15 (d, J = 0.9 Hz, 1H); 7.65-7.56 (m, 2H); 7.52-7.48 (m, 2H); 6.92 (t, J = 5.6 Hz, 1H); 3.16-3.04 (m, 2H); 1.03 (t, J = 7.3 Hz, 3H).
MS (ESI, m/z): 327.2 [M+H⁺].

### Preparation M: 5-bromo-8-methoxy-isoquinolin-3-ylamine:

### M.1. N-(3-bromo-6-methoxy-benzyl)-2,2-diethoxy-acetamidine:

Starting from 5-bromo-2-methoxybenzylamine (4.75 g; commercial) and 2,2-diethoxy-ethanimidic acid methyl ester (5.01 g) and proceeding in analogy to Procedure L, the title compound was obtained as a beige solid (7.60 g).
MS (ESI, m/z): 345.4 and 347.3 [M+H⁺ of the two main isotopes].

### M.2. 5-bromo-8-methoxy-isoquinolin-3-ylamine:

Starting from intermediate M.1 (7.60 g) and proceeding in analogy to Procedure M, the title compound was obtained, after purification by CC (DCM/MeOH 100:0 to 99:1), as a yellow solid (306 mg; 6% yield over 2 steps).
¹H NMR (d6-DMSO) δ: 8.97 (s, 1H); 7.67 (d, J = 8.2 Hz, 1H); 6.70 (d, J = 0.6 Hz, 1H); 6.51 (d, J = 8.5 Hz, 1H); 6.28 (br. s, 2H); 3.91 (s, 3H).
MS (ESI, m/z): 253.1 and 255.1 [M+H⁺ of the two main isotopes].

### Preparation N: N-{3-[8-chloro-3-(3-ethyl-ureido)-5-fluoro-isoquinolin-6-yl]-phenyl}-acetamide:

Starting from intermediate D.6 (500 mg) and 3-acetylaminophenylboronic acid (380 mg) and proceeding in analogy to Procedure C, the title compound was obtained, after purification by CC (DCM/MeOH 100:0 to 90:10), as a beige solid (229 mg, 40% yield).
¹H NMR (d6-DMSO) δ: 10.08 (s, 1H); 9.38 (s, 1H); 9.25 (s, 1H); 8.30 (s, 1H); 7.92 (d, J = 0.6 Hz, 1H); 7.69-7.63 (m, 1H); 7.59 (d, J = 6.4 Hz, 1H); 7.45 (t, J = 7.9 Hz, 1H); 7.38-7.32 (m, 1H); 6.99-6.91 (m, 1H); 3.23-3.12 (m, 2H); 2.06 (s, 3H); 1.08 (t, J = 7.0 Hz, 3H).
MS (ESI, m/z): 401.4 [M+H⁺].

### Preparation O: 1-(5-bromo-isoquinolin-3-yl)-3-ethyl-urea:

### O.1. N-(3-bromo-benzyl)-2,2-diethoxy-acetamidine:

Starting from 3-bromobenzylamine hydrochloride (37.50 g; commercial) and 2,2-diethoxy-ethanimidic acid methyl ester (31.11 g) and proceeding in analogy to Procedure L, the title compound was obtained as an orange oil (43.10 g).
MS (ESI, m/z): 315.3 and 317.3 [M+H⁺ of the two main isotopes].

### 0.2. 5-bromo-isoquinolin-3-ylamine:

Starting from intermediate O.1 (43.10 g) and proceeding in analogy to Procedure M, the title compound was obtained, after purification by 2 CC (Hept/EA 100:0 to 40:60), as a yellow solid (4.36 g; 12% yield over 2 steps).
¹H NMR (d6-DMSO) δ: 8.82 (s, 1H); 7.84-7.77 (m, 2H); 7.04 (dd, J = 7.9, 7.3 Hz, 1H); 6.77-6.75 (m, 1H); 6.27 (br. s, 2H).
MS (ESI, m/z): 223.1 and 225.1 [M+H⁺ of the two main isotopes].

### 0.3. 1-(5-bromo-isoquinolin-3-yl)-3-ethyl-urea:

Starting from intermediate 0.2 (2.91 g) and ethyl isocyanate (2.58 mL) and proceeding in analogy to Procedure N, a first batch of product (1.52 g) was obtained. The mother liquors were concentrated under reduced pressure and the residue purified by CC (Hept/EA 100:0 to 50:50) to give a second batch of product (1.37 g). The title compound was obtained, after combining the two batches, as a yellow solid (2.89 g; 75% yield).
¹H NMR (d6-DMSO) δ: 9.17 (s, 1H); 9.05 (d, J= 0.6 Hz, 1H); 8.35 (s, 1H); 8.04-7.96 (m, 2H); 7.32 (dd, J = 7.9, 7.3 Hz, 1H); 6.94 (t, J = 5.6 Hz, 1H); 3.23-3.11 (m, 2H); 1.08 (t, J = 7.3 Hz, 3H).
MS (ESI, m/z): 294.4 and 296.6 [M+H⁺ of the two main isotopes].

### Preparation P: 1-(8-amino-isoquinolin-3-yl)-3-ethyl-urea:

### P.1. 1-[8-(benzhydrylidene-amino)-isoquinolin-3 yl]-3-ethyl-urea:

To the compound of Preparation K (3.44 g), benzophenone imine (2.62 g), tBuONa (1.60 g), Pd₂dba₃ (120 mg) and BINAP (230 mg) in a round-bottomed flask under inert atmosphere (N₂) was added dry dioxane (30 mL) at rt. The reaction mixture was purged with N₂ for 5 min and stirred at 80°C for 18 h. The reaction mixture was cooled to rt, diluted with DCM and a sat. aq. NH₄Cl solution. The layers were separated and the aq. layer was extracted with DCM (3x). The combined org. layers were washed with brine, dried over MgSO₄, filtered and concentrated under reduced pressure. The title compound was obtained, after purification by CC (DCM/MeOH 100:0 to 90:10), as a yellow solid (4.55 g; 98% yield).
¹H NMR (d6-DMSO) δ: 9.04 (s, 1H); 8.97 (s, 1H); 7.86 (s, 1H); 7.78 (s, 1H); 7.76 (s, 1H); 7.61-7.47 (m, 3H); 7.41-7.06 (m, 8H); 6.43 (dd, J = 6.5, 1.5 Hz, 1H); 3.21-3.09 (m, 2H); 1.07 (t, J = 7.2 Hz, 3H).
MS (ESI, m/z): 395.2 [M+H⁺].

### P.2. 1-(8-amino-isoquinolin-3-yl)-3-ethyl-urea:

To a solution of intermediate P.1 (4.55 g) in MeOH (100 mL) in a round-bottomed flask, under inert atmosphere (N₂), were added AcONa (2.30 g) and hydroxylamine hydrochloride (1.40 g) at rt. The reaction mixture was stirred at rt for 3 h and then partitioned between 0.1*M* NaOH (150 mL) and DCM (150 mL). The org. layer was washed once with brine, dried over MgSO₄, filtered and concentrated under reduced pressure. After purification by 2 CC (DCM/MeOH +1% NH₄OH 100:0 to 90:10), the title compound was obtained as a yellow solid (1.37 g; 52% yield).
¹H NMR (d6-DMSO) δ: 9.15 (s, 1H); 8.86 (s, 1H); 7.70 (s, 1H); 7.26 (t, J = 7.9 Hz, 1H); 7.20 (t, J = 5.3 Hz, 1H); 6.80 (d, J = 8.0 Hz, 1H); 6.47 (d, J = 7.5 Hz, 1H); 6.07 (br. s, 2H); 3-21-3.10 (m, 2H); 1.07 (t, J = 7.2 Hz, 3H).
MS (ESI, m/z): 231.2 [M+H⁺].

### Preparation Q: 1-ethyl-3-(8-formyl-isoquinolin-3-yl)-urea:

### Q.1. 1-ethyl-3-(8-vinyl-isoquinolin-3-yl)-urea:

Starting from the compound of Preparation K (3.00 g) and vinylboronic anhydride pyridine complex (1.72 g) and proceeding in analogy to Procedure B, the title compound was obtained, after purification by CC (DCM/MeOH +1% NH₄OH 100:0 to 95:5), as a yellow solid (2.25 g; 92% yield).
¹H NMR (d6-DMSO) δ: 9.30 (s, 1H); 9.03 (s, 1H); 8.00 (s, 1H); 7.72-7.65 (m, 1H); 7.64-7.52 (m, 3H); 7.04 (t, J = 5.5 Hz, 1H); 5.91 (dd, J = 17.2, 1.4 Hz, 1H); 5.53 (dd, J = 11.0, 1.4 Hz, 1H); 3.21-3.10 (m, 2H); 1.08 (t, J = 7.2 Hz, 3H).
MS (ESI, m/z): 242.4 [M+H⁺].

### Q.2. 1-ethyl-3-(8-formyl-isoquinolin-3 yl)-urea:

Starting from intermediate Q.1 (2.25 g) and proceeding in analogy to Procedure AD, the title compound was obtained, after filtration of the solid that precipitated from the reaction mixture, as a yellow solid (1.54 g; 68% yield).
¹H NMR (d6-DMSO) δ: 10.32 (s, 1H); 10.05 (s, 1H); 9.19 (s, 1H); 8.16 (br. s, 1H); 8.14-8.09 (m, 1H); 8.08-8.03 (m, 1H); 7.83 (dd, J = 8.4, 7.0 Hz, 1H); 6.99 (t, J = 5.4 Hz, 1H); 3.17 (m, 2H); 1.08 (t, J = 7.1 Hz, 3H).
MS (ESI, m/z): 244.2 [M+H⁺].

**Preparation R: 1-[8-(chloromethyl)isoquinolin-3-yl]-3-ethyl-urea hydrochloride:**

### R.1. Methyl 3-(3-ethyl-ureido)-isoquinoline-8-carboxylate:

A suspension of the compound of Preparation K (10.30 g), sodium acetate (22.97 g) and [1,1'-bis(diphenylphosphino)ferrocene]dichloropalladium(II) complex with DCM (1.00 g) in MeOH (150 mL) was stirred under a CO atmosphere (30 atm) at 80°C for 20 h. Upon reaction completion, water (300 mL) was added and the aq. layer was extracted with EA (5x). The combined org. layers were washed with brine, dried over MgSO₄ and concentrated under reduced pressure. The title compound was obtained, after purification by CC (Hept/EA 50:50 to 0:100), as a brown solid (8.40 g; 87% yield).
¹H NMR (d6-DMSO) δ: 9.78 (s, 1H); 9.18 (s, 1H); 8.14 (s, 1H); 8.08-7.96 (m, 2H); 7.76-7.67 (m, 1H); 7.01 (t, J = 5.2 Hz, 1H); 3.96 (s, 3H); 3.24-3.12 (m, 2H); 1.10 (t, J = 7.2 Hz, 3H).

### R.2. 1-ethyl-3-(8-hydroxymethyl-isoquinolin-3-yl)-urea:

A solution of intermediate R.1 (8.47 g) in THF (500 mL) was added dropwise to a solution of LiAlH₄ (3.53 g) in THF (250 mL) at 0°C over 45 min. The reaction mixture was stirred at 0°C for 2 h and then treated with a sat. aq. NH₄Cl solution. The mixture was extracted with EA (5x). The combined org. layers were washed with brine, dried over MgSO₄, filtered and concentrated under reduced pressure. The title compound was obtained, after trituration of the residue in THF, as a pale yellow solid (6.2 g; 81 % yield).
¹H NMR (d6-DMSO) δ: 9.23 (s, 1H); 9.05 (s, 1H); 8.00 (s, 1H); 7.69-7.64 (m, 1H); 7.61-7.55 (m, 1H); 7.42-7.38 (m, 1H); 7.13 (t, J = 5.2 Hz, 1H); 5.39 (t, J = 5.5 Hz, 1H); 5.00 (d, J = 5.5 Hz, 2H); 3.24-3.15 (m, 2H); 1.11 (t, J = 7.2 Hz, 3H).
MS (ESI, m/z): 246.26 [M+H⁺].

### R.3. 1-[8-(chloromethyl)isoquinolin-3-yl]-3-ethyl-urea hydrochloride:

A suspension of intermediate R.2 (4.0 g) in thionyl chloride (80 mL) in a round-bottomed flask, under inert atmosphere (N₂), was stirred at 80°C until a solution was obtained. The reaction mixture was further stirred at rt for 30 min. The title compound was obtained, after concentration of the reaction mixture under reduced pressure, as a yellow solid (5.0 g; quantitative yield).
¹H NMR (d6-DMSO) δ: 9.36 (br. s, 1H); 9.30 (s, 1H); 8.05 (s, 1H); 7.80 (d, J = 8.3 Hz, 1H); 7.64-7.57 (m, 1H); 7.54-7.49 (m, 1H); 5.29 (s, 2H); 3.17 (q, J = 7.2 Hz, 2H); 1.08 (t, J = 7.2 Hz, 3H). One NH, HCl and water were not observed.
MS (ESI, m/z): 264.11 [M+H⁺].

### Preparation S: 1-(8-bromo-5-chloro-isoquinolin-3-yl)-3-ethyl-urea:

### S.1. (2-bromo-5-chloro-phenyl)-methanol:

Starting from 2-bromo-5-chlorobenzoic acid (25.0 g) and proceeding in analogy to Procedure AF, the title compound was obtained, without additional purification, as a white solid (23.48 g).
¹H NMR (d6-DMSO) δ: 7.58 (d, J = 8.5 Hz, 1H); 7.52-7.49 (d, J = 2.8 Hz, 1H); 7.26 (dd, J = 8.5, 2.8 Hz, 1H); 5.56 (t, J = 5.7 Hz, 1H); 4.47 (d, J = 5.7 Hz, 2H).

### S.2. 2-bromo-5-chlorobenzyl methanesulfonate:

Starting from intermediate S.1 (23.48 g) and proceeding in analogy to Procedure AG, the title compound was obtained, without additional purification, as a white solid (34.53 g). ¹H NMR (d6-DMSO) δ: 7.72 (d, J = 8.5 Hz, 1H); 7.67 (d, J = 2.6 Hz, 1H); 7.44 (dd, J = 8.5, 2.6 Hz, 1H); 5.27 (s, 2H); 3.29 (s, 3 H).

### S.3. 2-(2-bromo-5-chloro-benzyl)-isoindole-1,3-dione:

Starting from intermediate S.2 (34.53 g) and proceeding in analogy to Procedure AH, the title compound was obtained, without additional purification, as a white solid (39.22 g). ¹H NMR (d6-DMSO) δ: 7.93-7.80 (m, 4H); 7.67 (d, J = 8.5 Hz, 1H); 7.38 (d, J = 2.5 Hz, 1H); 7.31 (dd, J = 8.5, 2.5 Hz, 1H); 4.76 (s, 2H).

### S. 4. 2-bromo-5-chloro-benzylamine:

Starting from intermediate S.3 (39.22 g) and proceeding in analogy to Procedure AI, the title compound was obtained, without additional purification, as a yellow oil which solidified upon standing (13.27 g).
¹H NMR (d6-DMSO) δ: 7.60 (d, J = 2.7 Hz, 1H); 7.55 (d, J = 8.4 Hz, 1H); 7.21 (dd, J= 8.4, 2.7 Hz, 1H); 3.70 (s, 2H); 1.96 (br. s, 2H).

### 5.5. N-(2-bromo-5-chloro-benzyl)-2,2-diethoxy-acetamidine:

Starting from intermediate S.4 (13.27 g) and 2,2-diethoxy-ethanimidic acid methyl ester (13.70 g) and proceeding in analogy to Procedure L, the title compound was obtained as a yellow oil (22.62 g).
MS (ESI, m/z): 348.7 and 350.8 [M+H⁺ of the two main isotopes].

### 5.6. 8-bromo-5-chloro-isoquinolin-3-ylamine:

Starting from intermediate S.5 (22.62 g) and proceeding in analogy to Procedure M, the title compound was obtained, after purification by CC (Hept/EA 100:0 to 70:30), as a yellow solid (7.65 g; 28% yield over the 6 steps S.1 to S.6).
¹H NMR (d6-DMSO) δ: 8.97 (s, 1H); 7.52 (d, J = 7.9 Hz, 1H); 7.37 (d, J = 7.9 Hz, 1H); 6.82 (s, 1H); 6.57 (s, 2H).
MS (ESI, m/z): 256.9 and 259.0 [M+H⁺ of the two main isotopes].

### S.7. 1-(8-bromo-5-chloro-isoquinolin-3-yl)-3-ethyl-urea:

Starting from intermediate S.6 (7.65 g) and ethyl isocyanate (7.10 mL) and proceeding in analogy to Procedure N, more ethyl isocyanate (7.10 mL) being however added after 2 days, the title compound was obtained, after combining the precipitate from the reaction mixture with the solid obtained from the purification of the reaction mixture filtrate by CC (Hept/EA 100:0 to 60:40 to DCM/MeOH 90:10), as a yellow solid (7.11 g; 73% yield).
¹H NMR (d6-DMSO) δ: 9.38 (s, 1H); 9.18 (s, 1H); 8.42 (s, 1H); 7.70 (q, J = 8.0 Hz, 2H); 6.92 (t, J = 5.5 Hz, 1H); 3.22-3.12 (m, 2H); 1.08 (t, J = 7.2 Hz, 3H).
MS (ESI, m/z): 328.1 and 330.2 [M+H⁺ of the two main isotopes].

### Preparation T: 1-[5-chloro-8-(pyridin-3-yloxy)-isoquinolin-3-yl]-3-ethyl-urea:

Starting from the compound of Preparation S (200 mg) and 3-hydroxypyridine (87 mg) and proceeding in analogy to Procedure AJ, adding however all reagents again after 6 h, the title compound was obtained, after purification by CC (Hept/EA 100:0 to 50:50), as a yellow solid (144 mg; 69% yield).
¹H NMR (d6-DMSO) δ: 9.32 (s, 1H); 9.30 (s, 1H); 8.55 (d, J = 2.7 Hz, 1H); 8.46 (dd, J = 4.5, 1.0 Hz, 1H); 8.40 (s, 1H); 7.72 (d, J = 8.2 Hz, 1H); 7.66-7.60 (m, 1H); 7.48 (dd, J = 8.4, 4.6 Hz, 1H); 6.95 (t, J = 5.5 Hz, 1H); 6.69 (d, J = 8.3 Hz, 1H); 3.23-3.12 (m, 2H); 1.08 (t, J = 7.1 Hz, 3H).
MS (ESI, m/z): 343.07 [M+H⁺].

### Preparation U: 1-[5-chloro-8-(pyridin-3-ylamino)-isoquinolin-3-yl]-3-ethyl-urea:

Starting from the compound of Preparation S (1.50 g) and 3-aminopyridine (644 mg) and proceeding in analogy to Procedure AB, using however tBuONa (2.5 eq.), the title compound was obtained, after purification by CC (DCM/MeOH +1% NH₄OH 100:0 to 96:4), as a yellow solid (1.26 g; 81% yield).
¹H NMR (d6-DMSO) δ: 9.34 (s, 1H); 9.23 (s, 1H); 8.81 (s, 1H); 8.47 (d, J = 2.6 Hz, 1H); 8.29 (s, 1H); 8.14 (dd, J = 4.6, 1.3 Hz, 1H); 7.61 (d, J = 8.3 Hz, 1H); 7.55 (ddd, J = 8.3, 2.7, 1.4 Hz, 1H); 7.29 (dd, J = 8.4, 4.6 Hz, 1H); 7.03 (d, J = 8.3 Hz, 1H); 6.99 (t, J = 5.5 Hz, 1H); 3.22-3.12 (m, 2H); 1.08 (t, J = 7.2 Hz, 3H).
MS (ESI, m/z): 342.12 [M+H⁺].

### Preparation V: 1-(6-bromo-8-chloro-isoquinolin-3-yl)-3-ethyl-urea:

### V.1. N-(4-bromo-2-chloro-benzyl)-2,2-diethoxy-acetamidine:

Starting from 4-bromo-2-chlorobenzylamine (17.67 g; commercial) and 2,2-diethoxy-ethanimidic acid methyl ester (20.00 g) and proceeding in analogy to Procedure L, the title compound was obtained as a yellow viscous oil (31.12 g).
MS (ESI, m/z): 348.8 and 350.9 [M+H⁺ of the two main isotopes].

### V.2. 6-bromo-8-chloro-isoquinolin-3-ylamine:

Starting from intermediate V.1 (31.12 g) and proceeding in analogy to Procedure M, the title compound was obtained, after purification by CC (DCM/MeOH 100:0 to 90:10) followed by a second CC (DCM/MeOH 100:0 to 95:5), as a brown solid (4.03 g; 20% yield over 2 steps).
¹H NMR (d6-DMSO) δ: 8.97 (s, 1H); 7.81 (s, 1H); 7.36 (d, J = 1.7 Hz, 1H); 6.59 (s, 1H); 6.39 (br. s, 2H).
MS (ESI, m/z): 257.0 and 259.1 [M+H⁺ of the two main isotopes].

### V.3. 1-(6-bromo-8-chloro-isoquinolin-3-yl)-3-ethyl-urea:

Starting from intermediate V.2 (4.03 g) and ethyl isocyanate (3.14 mL) and proceeding in analogy to Procedure N, adding however more ethyl isocyanate (2.50 mL) after 2 days, the title compound was obtained as a white solid (2.05 g; 40% yield).
¹H NMR (d6-DMSO) δ: 9.26 (s, 1H); 9.19 (s, 1H); 8.13-8.10 (m, 2H); 7.71 (d, J = 1.7 Hz, 1H); 6.90 (t, J = 5.4 Hz, 1H); 3.21-3.10 (m, 2H); 1.07 (t, J = 7.2 Hz, 3H).
MS (ESI, m/z): 328.00 and 329.76 [M+H⁺ of the two main isotopes].

### Preparation W: 1-(8-chloro-5-(chloromethyl)-isoquinolin-3-yl)-3-ethyl-urea:

### W.1. 1-(8-chloro-5-vinyl-isoquinolin-3-yl)-3-ethyl-urea:

Starting from the compound of Preparation G (2.62 g) and vinylboronic anhydride pyridine complex (960 mg) and proceeding in analogy to Procedure B, the title compound was obtained, after purification by CC (Hept/EA 100:0 to 50:50), as a yellow solid (1.93 g; 89% yield).
¹H NMR (d6-DMSO) δ: 9.24 (d, J = 0.7 Hz, 1H); 9.23 (s, 1H); 8.38 (d, J = 0.6 Hz, 1H); 7.78-7.74 (m, 1H); 7.51 (d, J = 7.8 Hz, 1H); 7.21 (dd, J = 17.4, 11.1 Hz, 1H); 6.97 (t, J = 5.5 Hz, 1H); 5.89 (dd, J = 17.3, 1.3 Hz, 1H); 5.56 (dd, J = 11.0, 1.3 Hz, 1H); 3.22-3.12 (m, 2H); 1.08 (t, J = 7.2 Hz, 3H).
MS (ESI, m/z): 276.07 [M+H⁺].

### W.2. 1-(8-chloro-5-formyl-isoquinolin-3-yl)-3-ethyl-urea:

Starting from intermediate W.1 (1.16 g) and proceeding in analogy to Procedure AD, the title compound was obtained, without additional purification, as a yellow solid (926 mg; 79% yield).
¹H NMR (d6-DMSO) δ: 10.23 (s, 1H); 9.38 (br. s, 1H); 9.34 (s,1H); 9.33 (s, 1H); 8.25 (d, J = 7.7 Hz, 1H); 7.74 (d, J = 7.7 Hz, 1H); 7.06 (t, J = 5 .5 Hz, 1H); 3.24-3.12 (m, 2H); 1.09 (t, J = 7.2 Hz, 3H).
MS (ESI, m/z): 278.04 [M+H⁺].

### W.3. 1-(8-chloro-5-hydroxymethyl-isoquinolin-3-yl)-3-ethyl-urea:

To a suspension of intermediate W.2 (164 mg) in THF (6 mL) at 0°C in a round-bottomed flask, under inert atmosphere (N₂), was added a 1M solution of LiAlH₄ in THF (0.6 mL). The reaction mixture was stirred at 0°C for 2 h. The reaction mixture was quenched by the addition of sat. aq. NH₄Cl solution and allowed to warm to rt. It was diluted with DCM and the layers separated. The aq. layer was extracted with 9:1 DCM/MeOH (3x) and the combined org. layers were washed with brine, dried over MgSO₄, filtered and concentrated under reduced pressure. The title compound was obtained, after drying, as a yellow solid
(158 mg; 96% yield).
¹H NMR (d6-DMSO) δ: 9.23 (s, 1H); 9.20 (s, 1H); 8.20 (s, 1H); 7.65-7.59 (m, 1H); 7.52-7.46 (m, 1H); 7.09-7.01 (m, 1H); 5.39 (t, J = 5.0 Hz, 1H); 4.81 (d, J = 5.0 Hz, 2H); 3.23-3.10 (m, 2H); 1.08 (t, J = 7.2 Hz, 3H).
MS (ESI, m/z): 280.16 [M+H⁺].

### W.4. 1-(8-chloro-5-(chloromethyl)-isoquinolin-3-yl)-3-ethyl-urea:

A suspension of intermediate W.3 (203 mg) in thionyl chloride (1.8 mL) in a round-bottomed flask, under inert atmosphere (N₂), was stirred at rt for 45 min. The reaction mixture was concentrated under reduced pressure. The title compound was obtained, after purification by CC (DCM/MeOH 100:0 to 98:2), as a yellow solid (140 mg; 64% yield).
¹H NMR (d6-DMSO) δ: 9.29 (s, 1H); 9.27 (d, J = 0.6 Hz, 1H); 8.40 (d, J = 0.6 Hz, 1H); 7.76 (d, J = 7.7 Hz, 1H); 7.50 (d, J = 7.7 Hz, 1H); 6.96 (t, J = 5.5 Hz, 1H); 5.07 (s, 2H); 3.23-3.12 (m, 2H); 1.09 (t, J = 7.2 Hz, 3H).
MS (ESI, m/z): 298.08 [M+H⁺].

### Preparation X: 1-[5-(5,5-dimethyl-[1,3,2]dioxaborinan-2-yl)-8-methyl-isoquinolin-3-yl]-3-ethyl-urea:

Starting from the compound of Example 1 (679 mg), and proceeding in analogy to Procedure AO, the title compound was obtained, after purification by CC (DCM/MeOH 100:0 to 96:4) followed by trituration in EA, as a yellow solid (544 mg; 72% yield).
¹H NMR (d6-DMSO) δ: 9.12 (d, J = 0.5 Hz, 1H); 9.03 (s, 1H); 8.67 (s, 1H); 7.90 (d, J = 7.0 Hz, 1H); 7.53 (t, J = 5 .2 Hz, 1H); 7.16 (dd, J = 7.0, 0.7 Hz, 1H); 3.82 (s, 4H); 3.24-3.13 (m, 2H); 2.67 (s, 3H); 1.09 (t, J = 7.2 Hz, 3H); 1.00 (s, 6H).
MS (ESI, m/z): 274.00 [M+H⁺ of the corresponding boronic acid].

### Preparation Y: 1-[8-chloro-5-(5,5-dimethyl-[1,3,2]dioxaborinan-2-yl)-isoquinolin-3-yl]-3-ethyl-urea:

Starting from the compound of Preparation G (1 g), and proceeding in analogy to Procedure AO, the title compound was obtained, after purification by CC (DCM/MeOH 100:0 to 98:2) followed by trituration in DCM, as a yellow solid (556 mg; 51% yield).
¹H NMR (d6-DMSO) δ: 9.24 (d, J = 0.8 Hz, 1H); 9.17 (s, 1H); 8.84 (d, J = 0.8 Hz, 1H); 7.94 (d, J = 7.5 Hz, 1H); 7.48 (d, J = 7.5 Hz, 1H); 7.24 (t, J = 5.3 Hz, 1H); 3.84 (s, 4H); 3.23-3.12 (m, 2H); 1.09 (t, J = 7.2 Hz, 3H); 1.00 (s, 6H).
MS (ESI, m/z): 294.16 [M+H⁺ of the corresponding boronic acid].

### Preparation Z: 1-[8-(aminomethyl)isoquinolin-3-yl]-3-ethyl-urea:

A suspension of intermediate R.2 (1.96 g) in anhydrous THF (40 mL) in a round-bottomed flask under inert atmosphere (N₂) at rt was treated with DPPA (2.07 mL) and DBU (1.43 mL). The reaction mixture was stirred at rt for 2 h, treated with water (5 mL) and PPh₃ (2.62 g) and heated to 60°C. It was stirred for 2 h at 60°C, cooled down to rt and concentrated under reduced pressure. The title compound was obtained, after purification by CC (DCM/MeOH +1% NH₄OH 100:0 to 96:4), as a white solid (995 mg; 51% yield).
¹H NMR (d6-DMSO) δ: 9.25 (s, 1H); 9.00 (s, 1H); 7.95 (d, J = 0.5 Hz, 1H); 7.63-7.57 (m, 1H); 7.57-7.50 (m, 1H); 7.38 (dd, J = 6.7, 1.2 Hz, 1H); 7.13 (t, J = 5.4 Hz, 1H); 4.21 (s, 2H); 3.21-3.11 (m, 2H); 1.88 (br. s, 2H); 1.08 (t, J = 7.2 Hz, 3H).
MS (ESI, m/z): 245.20 [M+H⁺].

### PREPARATION OF THE EXAMPLE COMPOUNDS

### Example 1: 1-(5-bromo-8-methyl-isoquinolin-3-yl)-3-ethyl-urea:

Starting from the compound of Preparation A and proceeding in analogy to Procedure N, the title compound was obtained as a yellow solid (82% yield).
¹H NMR (d6-DMSO) δ: 9.20 (s, 1H); 9.15 (s, 1H); 8.33 (s, 1H); 7.85 (d, J = 7.6 Hz, 1H); 7.12 (dd, J = 7.6, 0.9 Hz, 1H); 7.01 (t, J = 5.6 Hz, 1H); 3.23-3.12 (m, 2H); 2.66 (s, 3H); 1.08 (t, J = 7.0 Hz, 3H).
MS (ESI, m/z): 308.4 and 310.4 [M+H⁺ of the two main isotopes].

### Example 2: 1-ethyl-3-[5-(4-hydroxy-phenyl)-8-methyl-isoquinolin-3-yl]-urea:

Starting from the compound of Example 1 and 4-hydroxyphenylboronic acid and proceeding in analogy to Procedure A, the title compound was obtained, after purification by prep-HPLC (acidic conditions), as an amorphous material (33% yield).
MS (ESI, m/z): 322.05 [M+H⁺].

### Example 3: 1-ethyl-3-[5-(2-hydroxy-phenyl)-8-methyl-isoquinolin-3-yl]-urea:

Starting from the compound of Example 1 and 2-hydroxyphenylboronic acid and proceeding in analogy to Procedure A, the title compound was obtained, after purification by prep-HPLC (acidic conditions), as an amorphous solid (33% yield).
MS (ESI, m/z): 322.05 [M+H⁺].

### Example 4: N-{3-[3-(3-ethyl-ureido)-8-methyl-isoquinolin-5-yl]-phenyl}-acetamide:

Starting from the compound of Example 1 and 3-acetylaminophenylboronic acid and proceeding in analogy to Procedure A, the title compound was obtained, after purification by prep-HPLC (acidic conditions), as an amorphous solid (20% yield).
MS (ESI, m/z): 363.07 [M+H⁺].

### Example 5: 4-[3-(3-ethyl-ureido)-8-methyl-isoquinolin-5-yl]-N,N-dimethyl-benzamide:

Starting from the compound of Example 1 and 4-(*N*,*N*-dimethylaminocarbonyl)phenylboronic acid and proceeding in analogy to Procedure A, the title compound was obtained, after purification by prep-HPLC (acidic conditions), as an amorphous solid (43% yield).
MS (ESI, m/z): 377.08 [M+H⁺].

### Example 6: 4-[3-(3-ethyl-ureido)-8-methyl-isoquinolin-5-yl]-N-methyl-benzamide:

Starting from the compound of Example 1 and 4-(*N*-methylaminocarbonyl)phenylboronic acid and proceeding in analogy to Procedure A, the title compound was obtained, after purification by prep-HPLC (acidic conditions), as an amorphous solid (9% yield).
MS (ESI, m/z): 363.08 [M+H⁺].

### Example 7: 1-ethyl-3-(5-imidazo[1,2-a]pyridin-6-yl-8-methyl-isoquinolin-3-yl)-urea:

Starting from the compound of Example 1 and imidazo[1,2-*a*]pyridine-6-boronic acid and proceeding in analogy to Procedure A, the title compound was obtained, after purification by prep-HPLC (acidic conditions), as an amorphous solid (7% yield).
MS (ESI, m/z): 346.09 [M+H⁺].

### Example 8: 1-ethyl-3-[5-(6-hydroxymethyl-pyridin-3-yl)-8-methyl-isoquinolin-3-yl]-urea:

Starting from the compound of Example 1 and 6-(hydroxymethyl)pyridine-3-boronic acid and proceeding in analogy to Procedure A, the title compound was obtained, after purification by prep-HPLC (acidic conditions), as an amorphous solid (11% yield).
MS (ESI, m/z): 337.08 [M+H⁺].

### Example 9: 1-ethyl-3-[5-(5-fluoro-pyridin-3-yl)-8-methyl-isoquinolin-3-yl]-urea:

Starting from the compound of Example 1 and 5-fluoropyridine-3-boronic acid and proceeding in analogy to Procedure A, the title compound was obtained, after purification by prep-HPLC (acidic conditions), as an amorphous solid (35% yield).
MS (ESI, m/z): 325.02 [M+H⁺].

### Example 10: 1-ethyl-3-[8-methyl-5-(5-methyl-pyridin-3-yl)-isoquinolin-3-yl]-urea:

Starting from the compound of Example 1 and 5-methylpyridine-3-boronic acid and proceeding in analogy to Procedure A, the title compound was obtained, after purification by prep-HPLC (acidic conditions), as an amorphous solid (25% yield).
MS (ESI, m/z): 321.06 [M+H⁺].

### Example 11: 1-[5-(3-(aminomethyl)-phenyl)-8-methyl-isoquinolin-3-yl]-3-ethyl-urea:

Starting from the compound of Example 1 and 3-aminomethylphenylboronic acid hydrochloride and proceeding in analogy to Procedure A, the title compound was obtained, after purification by prep-HPLC (acidic conditions), as an amorphous solid (13% yield). MS (ESI, m/z): 335.09 [M+H⁺].

### Example 12: 1-ethyl-3-[5-(3-hydroxymethyl-phenyl)-8-methyl-isoquinolin-3-yl]-urea:

Starting from the compound of Example 1 and 3-hydroxymethylphenylboronic acid and proceeding in analogy to Procedure A, the title compound was obtained, after purification by prep-HPLC (acidic conditions), as an amorphous solid (29% yield).
MS (ESI, m/z): 336.06 [M+H⁺].

### Example 13: 3-[3-(3-ethyl-ureido)-8-methyl-isoquinolin-5-yl]-benzenesulfonamide:

Starting from the compound of Example 1 and 3-aminosulfonylphenylboronic acid and proceeding in analogy to Procedure A, the title compound was obtained, after purification by prep-HPLC (acidic conditions), as an amorphous solid (2% yield).
MS (ESI, m/z): 385.02 [M+H⁺].

### Example 14: 1-ethyl-3-(6-furan-3-yl-8-methyl-isoquinolin-3-yl)-urea:

Starting from the compound of Preparation B and 3-furanylboronic acid and proceeding in analogy to Procedure A, the title compound was obtained, after purification by prep-HPLC (acidic conditions), as an amorphous solid (9% yield).
MS (ESI, m/z): 296.02 [M+H⁺].

### Example 15: 1-ethyl-3-[6-(4-hydroxymethyl-phenyl)-8-methyl-isoquinolin-3-yl]-urea:

Starting from the compound of Preparation B and 4-hydroxymethylphenylboronic acid and proceeding in analogy to Procedure A, the title compound was obtained, after purification by prep-HPLC (acidic conditions), as an amorphous solid (5% yield).
MS (ESI, m/z): 336.07 [M+H⁺].

### Example 16: 1-ethyl-3-(8-methyl-6-pyridin-3-yl-isoquinolin-3-yl)-urea:

Starting from the compound of Preparation B and pyridine-3-boronic acid and proceeding in analogy to Procedure A, the title compound was obtained, after purification by prep-HPLC (acidic conditions), as an amorphous material (36% yield).
MS (ESI, m/z): 307.01 [M+H⁺].

### Example 17: N-{3-[3-(3-ethyl-ureido)-8-methyl-isoquinolin-6-yl]-benzyl}-acetamide:

Starting from the compound of Preparation B and 3-acetamidomethylphenylboronic acid and proceeding in analogy to Procedure A, the title compound was obtained, after purification by prep-HPLC (acidic conditions), as an amorphous solid (32% yield).
MS (ESI, m/z): 377.08 [M+H⁺].

### Example 18: N-{4-[3-(3-ethyl-ureido)-8-methyl-isoquinolin-6-yl]-phenyl}-acetamide:

Starting from the compound of Preparation B and 4-acetylaminophenylboronic acid and proceeding in analogy to Procedure A, the title compound was obtained, after purification by prep-HPLC (acidic conditions), as an amorphous solid (3% yield).
MS (ESI, m/z): 363.06 [M+H⁺].

### Example 19: 1-ethyl-3-[6-(3-hydroxymethyl-phenyl)-8-methyl-isoquinolin-3-yl]-urea:

Starting from the compound of Preparation B and 3-hydroxymethylphenylboronic acid and proceeding in analogy to Procedure A, the title compound was obtained, after purification by prep-HPLC (acidic conditions), as an amorphous solid (25% yield).
MS (ESI, m/z): 336.07 [M+H⁺].

### Example 20: 1-ethyl-3-[6-(3-hydroxy-phenyl)-8-methyl-isoquinolin-3-yl]-urea:

Starting from the compound of Preparation B and 3-hydroxyphenylboronic acid and proceeding in analogy to Procedure A, the title compound was obtained, after purification by prep-HPLC (acidic conditions), as an amorphous solid (21 % yield).
MS (ESI, m/z): 322.04 [M+H⁺].

### Example 21: 1-ethyl-3-(8-methyl-6-pyridin-4-yl-isoquinolin-3-yl)-urea:

Starting from the compound of Preparation B and pyridine-4-boronic acid and proceeding in analogy to Procedure A, the title compound was obtained, after purification by prep-HPLC (acidic conditions), as an amorphous solid (22% yield).
MS (ESI, m/z): 307.01 [M+H⁺].

### Example 22: 3-[3-(3-ethyl-ureido)-8-methyl-isoquinolin-6-yl]-benzenesulfonamide:

Starting from the compound of Preparation B and 3-aminosulfonylphenylboronic acid and proceeding in analogy to Procedure A, the title compound was obtained, after purification by prep-HPLC (acidic conditions), as an amorphous solid (10% yield).
MS (ESI, m/z): 385.02 [M+H⁺].

### Example 23: 2-[3-(3-ethyl-ureido)-8-methyl-isoquinolin-6-yl]-N,N-dimethyl-benzamide:

Starting from 2-(*N,N*-dimethylaminocarbonyl)phenylboronic acid and the compound of Preparation B and proceeding in analogy to Procedure A, the title compound was obtained, after purification by prep-HPLC (acidic conditions), as an amorphous solid (32% yield).
MS (ESI, m/z): 377.09 [M+H⁺].

### Example 24: 4-[3-(3-ethyl-ureido)-8-methyl-isoquinolin-6-yl]-N,N-dimethyl-benzamide:

Starting from 4-(*N*,*N*-dimethylaminocarbonyl)phenylboronic acid and the compound of Preparation B and proceeding in analogy to Procedure A, the title compound was obtained, after purification by prep-HPLC (acidic conditions), as an amorphous solid (36% yield).
MS (ESI, m/z): 377.10 [M+H⁺].

### Example 25: 1-ethyl-3-(6-imidazo[1,2-a]pyridin-6-yl-8-methyl-isoquinolin-3-yl)-urea:

Starting from the compound of Preparation B and imidazo[1,2-a]pyridine-6-boronic acid and proceeding in analogy to Procedure A, the title compound was obtained, after purification by prep-HPLC (acidic conditions), as an amorphous solid (30% yield).
MS (ESI, m/z): 346.07 [M+H⁺].

### Example 26: 1-[6-(3-(aminomethyl)-phenyl)-8-methyl-isoquinolin-3-yl]-3-ethyl-urea:

Starting from the compound of Preparation B and 3-aminomethylphenylboronic acid hydrochloride and proceeding in analogy to Procedure A, the title compound was obtained, after purification by prep-HPLC (acidic conditions), as an amorphous solid (15% yield).
MS (ESI, m/z): 335.08 [M+H⁺].

### Example 27: 1-[5-(2-chloro-pyridin-4-yl)-8-methyl-isoquinolin-3-yl]-3-ethyl-urea:

Starting from the compound of Example 1 and 2-chloropyridine-4-boronic acid and proceeding in analogy to Procedure A, the title compound was obtained, after purification by prep-TLC (Hept/EA 1:2), as an amorphous solid (13% yield).
MS (ESI, m/z): 340.6 [M+H⁺].

### Example 28: 1-ethyl-3-[8-methyl-6-(1H-pyrazol-4-yl)-isoquinolin-3-yl]-urea:

Starting from the compound of Preparation B and 4,4,5,5-tetramethyl-2-(1*H*-pyrazol-4-yl)-1,3,2-dioxaborolane and proceeding in analogy to Procedure A, the title compound was obtained, after purification by prep-TLC (EA), as an amorphous solid (18% yield).
MS (ESI, m/z): 296.06 [M+H⁺].

### Example 29: 1-[6-(3-acetyl-phenyl)-8-methyl-isoquinolin-3-yl]-3-ethyl-urea:

Starting from the compound of Preparation B and 3-acetylphenylboronic acid and proceeding in analogy to Procedure A, the title compound was obtained, after purification by prep-TLC (Hept/EA 1:2), as an amorphous solid (23% yield).
MS (ESI, m/z): 348.09 [M+H⁺].

### Example 30: 4-[3-(3-ethyl-ureido)-8-methyl-isoquinolin-6-yl]-benzamide:

Starting from the compound of Preparation B and 4-aminocarbonylphenylboronic acid and proceeding in analogy to Procedure A, the title compound was obtained, after purification by prep-TLC (EA), as an amorphous solid (7% yield).
MS (ESI, m/z): 349.10 [M+H⁺].

### Example 31: 3-[3-(3-ethyl-ureido)-8-methyl-isoquinolin-6-yl]-benzamide:

Starting from the compound of Preparation B and 3-aminocarbonylphenylboronic acid and proceeding in analogy to Procedure A, the title compound directly precipitated from the reaction mixture and was obtained as an amorphous solid (17% yield).
MS (ESI, m/z): 349.06 [M+H⁺].

### Example 32: 1-(8-bromo-5-fluoro-isoquinolin-3-yl)-3-ethyl-urea:

Starting from the compound of Preparation C and ethyl isocyanate (8 eq.) and proceeding in analogy to Procedure N, a first batch of product was obtained. The mother liquors were concentrated under reduced pressure and the residue was purified by CC (Hept/EA 100:0 to 40:60) to give a second batch of product. The title compound was obtained, after combining the two batches, as a yellow solid (96% yield).
¹H NMR (d6-DMSO) δ: 9.36 (s, 1H); 9.16 (d, J = 1.5 Hz, 1H); 8.22 (s, 1H); 7.67 (dd, J = 8.2, 4.7 Hz, 1H); 7.43 (dd, J = 10.5, 8.2 Hz, 1H); 6.91 (t, J = 5.3 Hz, 1H); 3.22-3.11 (m, 2H); 1.08 (t, J = 7.0 Hz, 3H).
MS (ESI, m/z): 312.6 and 314.2 [M+H⁺ of the two main isotopes].

### Example 33: 4-[3-(3-ethyl-ureido)-5-fluoro-6-pyridin-3-yl-isoquinolin-8-yl]-benzoic acid:

Starting from the compound of Preparation D and 4-carboxyphenylboronic acid (4 eq.) and proceeding in analogy to Procedure B, using however 7 eq. of aq. 1*N* K₂CO₃ solution, the title compound was obtained, after purification by CC (DCM/MeOH +1% formic acid 100:0 to 90:10) and prep-HPLC (acidic conditions), as a yellow solid (1% yield).
MS (ESI, m/z): 431.04 [M+H⁺].

### Example 34: 1-ethyl-3-[5-(4-hydroxymethyl-phenyl)-8-methyl-isoquinolin-3-yl]-urea:

Starting from the compound of Example 1 and 4-hydroxymethylphenylboronic acid and proceeding in analogy to Procedure C, the title compound was obtained, after purification by CC (DCM/MeOH +1% NH₄OH 100:0 to 96:4), as a beige solid (55% yield).
¹H NMR (d6-DMSO) δ: 9.19 (s, 1H); 8.96 (s, 1H); 8.11 (s, 1H); 7.47-7.36 (m, 5H); 7.25 (d, J = 7.9 Hz, 1H); 7.04 (t, J = 5.6 Hz, 1H); 5.26 (t, J = 5.9 Hz, 1H); 4.59 (d, J = 5.9 Hz, 2H); 3.16-3.04 (m, 2H); 2.71 (s, 3H); 1.03 (t, J = 7.0 Hz, 3H).
MS (ESI, m/z): 336.2 [M+H⁺].

### Example 35: 4-[3-(3-ethyl-ureido)-8-methyl-isoquinolin-5-yl]-benzenesulfonamide:

Starting from the compound of Example 1 and 4-aminosulfonylphenylboronic acid and proceeding in analogy to Procedure C, the title compound was obtained, after purification by prep-HPLC (acidic conditions), as a yellow solid (5% yield).
¹H NMR (d6-DMSO) δ: 9.22 (s, 1H); 9.01 (s, 1H); 8.08 (s, 1H); 7.95 (d, J = 8.4 Hz, 2H); 7.64 (d, J = 8.4 Hz, 2H); 7.49-7.44 (m, 3H); 7.32-7.27 (m, 1H); 7.03 (t, J = 5.3 Hz, 1H); 3.16-3.05 (m, 2H); 2.73 (s, 3H); 1.03 (t, J = 7.2 Hz, 3H).
MS (ESI, m/z): 385.08 [M+H⁺].

### Example 36: 1-ethyl-3-(8-methyl-6-quinolin-3-yl-isoquinolin-3-yl)-urea:

Starting from the compound of Preparation B and 3-quinolineboronic acid and proceeding in analogy to Procedure C, the title compound was obtained, after purification by CC (DCM/MeOH 100:0 to 90:10), as a white solid (65% yield).
¹H NMR (d6-DMSO) δ: 9.40 (d, J=2.3 Hz, 1H); 9.19 (s, 1H); 9.10 (s, 1H); 8.83 (d, J = 2.1 Hz, 1H); 8.15 (s, 1H); 8.10 (s, 1H); 8.11-8.05 (m, 2H); 7.83-7.78 (m, 1H); 7.78-7.75 (m, 1H); 7.70-7.63 (m, 1H); 7.16 (t, J = 5.3 Hz, 1H); 3.25-3.13 (m, 2H); 2.79 (s, 3H); 1.10 (t, J = 7.3 Hz, 3H).
MS (ESI, m/z): 357.2 [M+H⁺].

### Example 37: N-{3-[3-(3-ethyl-ureido)-8-methyl-isoquinolin-6-yl]-phenyl}-acetamide:

Starting from the compound of Preparation B and 3-acetylaminophenylboronic acid and proceeding in analogy to Procedure C, the title compound was obtained, after purification by CC (DCM/MeOH 100:0 to 90:10), as a beige solid (85% yield).
¹H NMR (d6-DMSO) δ: 10.03 (s, 1H); 9.14 (s, 1H); 9.06 (s, 1H); 8.01 (s, 1H); 8.00-7.97 (m, 1H); 7.76 (s, 1H); 7.64-7.59 (m, 1H); 7.49-7.37 (m, 3H); 7.22-7.15 (m, 1H); 3.23-3.13 (m, 2H); 2.74 (s, 3H); 2.06 (s, 3H); 1.09 (t, J = 7.2 Hz, 3H).
MS (ESI, m/z): 363.2 [M+H⁺].

### Example 38: 4-[3-(3-ethyl-ureido)-8-methyl-isoquinolin-6-yl]-benzenesulfonamide:

Starting from the compound of Preparation B and 4-aminosulfonylphenylboronic acid, and proceeding in analogy to Procedure C, the title compound was obtained, after purification by CC (DCM/MeOH 100:0 to 90:10), as a yellow solid (95% yield).
¹H NMR (d6-DMSO) δ: 9.16 (s, 1H); 9.09 (s, 1H); 8.06 (s, 1H); 8.04-7.99 (m, 2H); 7.96 (s, 1H); 7.95-7.89 (m, 2H); 7.59 (s, 1H); 7.40 (s, 2H); 7.13 (t, J = 5.4 Hz, 1H); 3.24-3.12 (m, 2H); 2.75 (s, 3H); 1.09 (t, J = 7.2 Hz, 3H).
MS (ESI, m/z): 385.0 [M+H⁺].

### Example 39: 1-ethyl-3-[8-methyl-6-(5-methyl-pyridin-3-yl)-isoquinolin-3-yl]-urea:

Starting from the compound of Preparation B and 5-methylpyridine-3-boronic acid and proceeding in analogy to Procedure C, the title compound was obtained, after purification by CC (DCM/MeOH 100:0 to 90:10), as a pale yellow solid (91% yield).
¹H NMR (d6-DMSO) δ: 9.15 (s, 1H); 9.08 (s, 1H); 8.83 (d, J = 2.2 Hz, 1H); 8.45 (d, J = 1.6 Hz, 1H); 8.07-8.03 (m, 2H); 7.95 (s, 1H); 7.58 (s, 1H); 7.15 (t, J = 5.4 Hz, 1H); 3.23-3.12 (m, 2H); 2.74 (s, 3H); 2.38 (s, 3H); 1.09 (t, J = 7.2 Hz, 3H).
MS (ESI, m/z): 321.2 [M+H⁺].

### Example 40: 1-ethyl-3-[8-methyl-5-(2-methyl-pyridin-4-yl)-isoquinolin-3-yl]-urea:

Starting from the compound of Example 1 and 2-methylpyridine-4-boronic acid and proceeding in analogy to Procedure C, the title compound was obtained, after purification by CC (DCM/MeOH +1% NH₄OH 100:0 to 96:4), as a beige solid (43% yield).
¹H NMR (d6-DMSO) δ: 9.22 (s, 1H); 9.03 (s, 1H); 8.55 (d, J = 5.1 Hz, 1H); 8.02 (s, 1H); 7.47 (d, J = 7.2 Hz, 1H); 7.33 (s, 1H); 7.31-7.23 (m, 2H); 7.16 (t, J = 5 .4 Hz, 1H); 3.17-3.06 (m, 2H); 2.73 (s, 3H); 2.54 (s, 3H); 1.04 (t, J = 7.2 Hz, 3H).
MS (ESI, m/z): 321.2 [M+H⁺].

### Example 41: 1-ethyl-3-[5-(2-fluoro-pyridin-4-yl)-8-methyl-isoquinolin-3-yl]-urea:

Starting from the compound of Example 1 and 2-fluoropyridine-4-boronic acid and proceeding in analogy to Procedure C, the title compound was obtained, after purification by CC (DCM/MeOH +1% NH₄OH 100:0 to 98:2) followed by trituration in EA, as a pale yellow solid (36% yield).
¹H NMR (d6-DMSO) δ: 9.24 (s, 1H); 9.06 (s, 1H); 8.37 (d, J = 5.1 Hz, 1H); 8.04 (s, 1H); 7.55 (d, J = 7.2 Hz, 1H); 7.48-7.43 (m, 1H); 7.33-7.28 (m, 2H); 7.12 (t, J = 5.5 Hz, 1H); 3.17-3.06 (m, 2H); 2.74 (s, 3H); 1.04 (t, J = 7.2 Hz, 3H).
MS (ESI, m/z): 325.2 [M+H⁺].

### Example 42: 1-ethyl-3-[5-(2-methoxy-pyridin-4-yl)-8-methyl-isoquinolin-3-yl]-urea:

Starting from the compound of Example 1 and 2-methoxypyridine-4-boronic acid, and proceeding in analogy to Procedure C, the title compound was obtained, after purification by CC (DCM/MeOH +1% NH₄OH 100:0 to 96:4), as a yellow solid (44% yield).
¹H NMR (d6-DMSO) δ: 9.21 (d, J = 0.7 Hz, 1H); 9.03 (s, 1H); 8.27 (dd, J = 5.3, 0.4 Hz, 1H); 8.04 (d, J = 0.3 Hz, 1H); 7.47 (d, J = 7.2 Hz, 1H); 7.28 (dd, J = 7.3, 0.8 Hz, 1H); 7.10 (t, J = 5.3 Hz, 1H); 7.06 (dd, J = 5.2, 1.4 Hz, 1H); 6.87-6.85 (m, 1H); 3.92 (s, 3H); 3.16-3.06 (m, 2H); 2.72 (s, 3H); 1.04 (t, J = 7.2 Hz, 3H).
MS (ESI, m/z): 337.2 [M+H⁺].

### Example 43: 1-ethyl-3-(5-phenyl-isoquinolin-3-yl)-urea:

Starting from the compound of Preparation O and phenylboronic acid and proceeding in analogy to Procedure B, the title compound was obtained, after purification by prep-HPLC (acidic conditions), as a beige solid (11% yield).
¹H NMR (d6-DMSO) δ: 9.08 (d, J = 0.8 Hz, 1H); 8.95 (s, 1H); 8.10 (s, 1H); 8.01-7.96 (m, 1H); 7.57-7.42 (m, 7H); 6.99 (t, J = 5.6 Hz, 1H); 3.15-3.04 (m, 2H); 1.03 (t, J = 7.2 Hz, 3H).
MS (ESI, m/z): 292.15 [M+H⁺].

### Example 44: 1-[5-(2,6-dimethyl-pyridin-4-yl)-8-methyl-isoquinolin-3-yl]-3-ethyl-urea:

Starting from the compound of Preparation E and 4-bromo-2,6-dimethylpyridine and proceeding in analogy to Procedure C, the title compound was obtained, after purification by CC (DCM/MeOH +1% NH₄OH 100:0 to 96:4), as a beige solid (57% yield).
¹H NMR (d6-DMSO) δ: 9.21 (s, 1H); 9.04 (s, 1H); 7.96 (s, 1H); 7.44 (d, J = 7.2 Hz, 1H); 7.29-7.23 (m, 2H); 7.11 (s, 2H); 3.18-3.06 (m, 2H); 2.72 (s, 3H); 2.49 (s, 6H); 1.05 (t, J = 7.2 Hz, 3H).
MS (ESI, m/z): 335.2 [M+H⁺].

### Example 45: 1-[5-(2-amino-pyridin-4-yl)-8-methyl-isoquinolin-3-yl]-3-ethyl-urea:

Starting from the compound of Preparation E and 2-amino-4-bromopyridine, and proceeding in analogy to Procedure C, the title compound was obtained, after purification by CC (DCM/MeOH +1% NH₄OH 100:0 to 96:4) followed by trituration in DCM, as a yellow solid (58% yield).
¹H NMR (d6-DMSO) δ: 9.19 (d, J = 0.4 Hz, 1H); 9.01 (s, 1H); 8.06 (s, 1H); 7.99 (d, J = 5.1 Hz, 1H); 7.41-7.37 (m, 1H); 7.27-7.23 (m, 1H); 7.11 (t, J = 5.6 Hz, 1H); 6.52 (dd, J = 5.2, 1.4 Hz, 1H); 6.46 (s, 1H); 5.99 (s, 2H); 3.12 (dd, J = 7.3, 5.7 Hz, 2H); 2.71 (s, 3H); 1.04 (t, J = 7.2 Hz, 3H).
MS (ESI, m/z): 322.2 [M+H⁺].

### Example 46: 1-(8-benzyl-5-fluoro-isoquinolin-3-yl)-3-ethyl-urea:

Starting from the compound of Example 32 and benzylzinc bromide and proceeding in analogy to Procedure D, the title compound was obtained, after purification by prep-HPLC (acidic conditions), as a white solid (32% yield).
¹H NMR (d6-DMSO) δ: 9.20-9.17 (m, 1H); 9.11 (s, 1H); 8.14 (d, J = 0.5 Hz, 1H); 7.40 (dd, J = 10.7, 7.8 Hz, 1H); 7.30-7.12 (m, 6H); 7.02 (t, J = 5.5 Hz, 1H); 4.44 (s, 2H); 3.21-3.10 (m, 2H); 1.07 (t, J = 7.2 Hz, 3H).
MS (ESI, m/z): 324.21 [M+H⁺].

### Example 47: 1-ethyl-3-(5-fluoro-8-methyl-6-pyridin-3-yl-isoquinolin-3-yl)-urea:

Starting from the compound of Preparation D and proceeding in analogy to Procedure E, the title compound was obtained, after purification by prep-HPLC (acidic conditions), as a beige solid (23% yield).
¹H NMR (d6-DMSO) δ: 9.24 (s, 1H); 9.21-9.19 (m, 1H); 8.87 (s, 1H); 8.64 (dd, J = 4.8, 1.6 Hz, 1H); 8.22 (d, J = 0.6 Hz, 1H); 8.12-8.06 (m, 1H); 7.56 (ddd, J = 7.9, 4.9, 0.8 Hz, 1H); 7.38 (dd, J = 7.3, 0.9 Hz, 1H); 7.04 (t, J = 5.5 Hz, 1H); 3.23-3.12 (m, 2H); 2.70 (s, 3H); 1.09 (t, J = 7.2 Hz, 3H).
MS (ESI, m/z): 325.14 [M+H⁺].

### Example 48: 1-ethyl-3-(5-fluoro-8-methyl-6-pyridin-2-yl-isoquinolin-3-yl)-urea:

Starting from the compound of Preparation F and proceeding in analogy to Procedure E, the title compound was obtained, after purification by prep-HPLC (acidic conditions), as a beige solid (8% yield).
¹H NMR (d6-DMSO) δ: 9.23 (s, 1H); 9.20-9.18 (m, 1H); 8.77 (dt, J = 4.8, 1.4 Hz, 1H); 8.25 (s, 1H); 7.99-7.95 (m, 2H); 7.70 (dd, J = 7.3, 0.9 Hz, 1H); 7.50-7.42 (m, 1H); 7.07-7.00 (m, 1H); 3.23-3.12 (m, 2H); 2.70 (s, 3H); 1.09 (t, J = 7.2 Hz, 3H).
MS (ESI, m/z): 325.34 [M+H⁺].

### Example 49: 1-(6-benzylamino-8-chloro-5-fluoro-isoquinolin-3-yl)-3-ethyl-urea:

Starting from intermediate D.6 and benzylamine and proceeding in analogy to Procedure H, the title compound was obtained, after purification by CC (Hept/EA 100:0 to 0:100), as a yellow solid (74% yield).
¹H NMR (d6-DMSO) δ: 9.05 (s, 1H); 8.87-8.84 (m, 1H); 7.85 (d, J = 0.5 Hz, 1H); 7.39-7.27 (m, 4H); 7.25-7.11 (m, 2H); 7.08-7.00 (m, 2H); 4.51 (d, J = 6.4 Hz, 2H); 3.20-3.09 (m, 2H); 1.07 (t, J = 7.2 Hz, 3H).
MS (ESI, m/z): 373.3 [M+H⁺].

### Example 50: 1-ethyl-3-(8-methyl-5-pyridin-3-yl-isoquinolin-3-yl)-urea:

Starting from the compound of Preparation H and proceeding in analogy to Procedure E, the title compound was obtained, after trituration in DCM, as a yellow solid (21 % yield).
MS (ESI, m/z): 307.6 [M+H⁺].

### Example 51: N-[3-(3-ethyl-ureido)-5-fluoro-isoquinolin-8-yl]-benzamide:

Starting from the compound of Example 32 and benzamide and proceeding in analogy to Procedure F, the title compound was obtained, after purification by prep-HPLC (basic conditions), as a yellow solid (19% yield).
MS (ESI, m/z): 353.09 [M+H⁺].

### Example 52: N-[3-(3-ethyl-ureido)-isoquinolin-6-yl]-benzamide:

Starting from the compound of Preparation I and benzamide and proceeding in analogy to Procedure F, the title compound was obtained, after purification by prep-HPLC (basic conditions), as a beige solid (45% yield).
MS (ESI, m/z): 335.13 [M+H⁺].

### Example 53: 1-ethyl-3-(5-fluoro-6,8-di-pyridin-3-yl-isoquinolin-3-yl)-urea:

Starting from intermediate D.6 and pyridine-3-boronic acid and proceeding in analogy to Procedure B, the title compound was obtained, after purification by CC (DCM/MeOH 100:0 to 90:10), as a yellow solid (39% yield).
¹H NMR (d6-DMSO) δ: 9.33 (s, 1H); 8.97 (s, 1H); 8.87-8.85 (m, 1H); 8.83 (dd, J = 2.4, 0.7 Hz, 1H); 8.71 (dd, J = 4.8, 1.6 Hz, 1H); 8.66 (dd, J = 4.8, 1.6 Hz, 1H); 8.33 (s, 1H); 8.23-8.18 (m, 1H); 8.11-8.05 (m, 1H); 7.62-7.53 (m, 3H); 7.01 (s, 1H); 3.23-3.12 (m, 2H); 1.08 (t, J = 7.1 Hz, 3H).
MS (ESI, m/z): 388.06 [M+H⁺].

### Example 54: 1-(6-amino-8-methyl-isoquinolin-3-yl)-3-ethyl-urea:

To a solution of the compound of Preparation J (7.13 g) in MeOH (150 mL) in a round-bottomed flask, under inert atmosphere (N₂), were added AcONa (3.52 g) and hydroxylamine hydrochloride (2.20 g) at rt. The reaction mixture was stirred at rt for 3 h and then partitioned between 0.1*M* NaOH (150 mL) and DCM (150 mL). The org. layer was washed once with brine, dried over MgSO₄, filtered and concentrated under reduced pressure. After purification by 2 CC (DCM/MeOH +1% NH₄OH 100:0 to 90:10), the title compound was obtained as a yellow solid (3.44 g; 81 % yield).
¹H NMR (d6-DMSO) δ: 8.74 (s, 1H); 8.68 (s, 1H); 7.63-7.55 (m, 1H); 7.32 (s, 1H); 6.58 (d, J = 0.9 Hz, 1H); 6.35 (d, J = 1.8 Hz, 1H); 5.77 (br. s, 2H); 3.21-3.10 (m, 2H); 2.49 (s, 3H); 1.07 (t, J = 7.3 Hz, 3H).
MS (ESI, m/z): 245.2 [M+H⁺].

### Example 55: 1-ethyl-3-{5-fluoro-8-[(pyridin-3-ylmethyl)-amino]-isoquinolin-3-yl}-urea:

Starting from the compound of Example 32 and 3-picolylamine and proceeding in analogy to Procedure G, the title compound was obtained, after purification by prep-HPLC (basic conditions), as a yellow solid (8% yield).
¹H NMR (d6-DMSO) δ: 9.35 (d, J = 0.7 Hz, 1H); 9.11 (s, 1H); 8.62 (d, J = 1.7 Hz, 1H); 8.43 (dd, J = 4.8, 1.6 Hz, 1H); 7.97 (s, 1H); 7.78 (dt, J = 7.8, 1.9 Hz, 1H); 7.32 (ddd, J = 7.8, 4.7, 0.6 Hz, 1H); 7.24 (t, J = 5 . 8 Hz, 1H); 7.14 (dd, J = 10.7, 8.5 Hz, 1H); 7.04 (t, J = 5.5 Hz, 1H); 6.14 (dd, J = 8.6, 3.9 Hz, 1H); 4.47 (d, J = 5.7 Hz, 2H); 3.22-3.11 (m, 2H); 1.08 (t, J = 7.2 Hz, 3H).
MS (ESI, m/z): 340.30 [M+H⁺].

### Example 56: 1-(8-benzylamino-5-fluoro-isoquinolin-3-yl)-3-ethyl-urea:

Starting from the compound of Example 32 and benzylamine and proceeding in analogy to Procedure G, the title compound was obtained, after purification by prep-HPLC (basic conditions), as a yellow solid (8% yield).
¹H NMR (d6-DMSO) δ: 9.37 (s, 1H); 9.10 (s, 1H); 7.96 (s, 1H); 7.42-7.35 (m, 2H); 7.34-7.16 (m, 4H); 7.16-7.01 (m, 2H); 6.07 (dd, J = 8.6, 3.8 Hz, 1H); 4.44 (d, J = 5.6 Hz, 2H); 3.23-3.11 (m, 2H); 1.08 (t, J = 7.2 Hz, 3H).
MS (ESI, m/z): 339.29 [M+H⁺].

### Example 57: 1-(6-benzylamino-5-fluoro-8-methyl-isoquinolin-3-yl)-3-ethyl-urea:

Starting from the compound of Example 49 and proceeding in analogy to Procedure E, the title compound was obtained, after purification by CC (Hept/EA 100:0 to 0:100), as a yellow solid (44% yield).
¹H NMR (d6-DMSO) δ: 8.91 (s, 1H); 8.78 (s, 1H); 7.69 (s, 1H); 7.38-7.26 (m, 5H); 7.23-7.15 (m, 1H); 6.83-6.75 (m, 2H); 4.49 (d, J = 6.3 Hz, 2H); 3.22-3.09 (m, 2H); 2.48 (s, 3H); 1.07 (t, J = 7.2 Hz, 3H).
MS (ESI, m/z): 353.2 [M+H⁺].

### Example 58: 1-(6-benzylamino-8-methyl-isoquinolin-3-yl)-3-ethyl-urea:

Starting from the compound of Preparation B and benzylamine, and proceeding in analogy to Procedure H, the title compound was obtained, after purification by CC (Hept/EA 100:0 to 0:100) followed by a second CC (DCM/MeOH +1% NH₄OH 100:0 to 90:10), as a beige solid (44% yield).
¹H NMR (d6-DMSO) δ: 8.76 (s, 1H); 8.68 (s, 1H); 7.65-7.56 (m, 1H); 7.40-7.27 (m, 5H); 7.25-7.17 (m, 1H); 6.92 (t, J = 6.0 Hz, 1H); 6.71 (d, J = 0.8 Hz, 1H); 6.21 (d, J = 1.7 Hz, 1H); 4.35 (d, J = 5.9 Hz, 2H); 3.20-3.09 (m, 2H); 2.49 (s, 3H); 1.06 (t, J = 7.2 Hz, 3H). MS (ESI, m/z): 335.2 [M+H⁺].

### Example 59: 3-[3-(3-ethyl-ureido)-8-methyl-isoquinolin-5-yl]-propionamide:

### 59.1. (E)-3-[3-(3-ethyl-ureido)-8-methyl-isoquinolin-5-yl]-acrylic acid methyl ester:

To a solution of the compound of Example 1 (726 mg), Pd(OAc)₂ (17 mg) and P(*o*-tolyl)₃ (72 mg) in dry DMF (12 mL), in a round-bottomed flask under inert atmosphere (N₂), were added TEA (1.0 mL) and methyl acrylate (1.1 mL). The reaction mixture was purged at rt with N₂ for 5 min, and then stirred at 120°C for 1 h. The reaction mixture was cooled to rt, concentrated under reduced pressure and diluted with 9:1 DCM/MeOH and water. The aq. layer was extracted with 9:1 DCM/MeOH (3x). The combined org. layers were washed with brine, dried over Na₂SO₄, filtered and concentrated under reduced pressure. The title compound was obtained, after trituration in 9:1 DCM/MeOH, as a yellow solid (335 mg, 45% yield).
¹H NMR (d6-DMSO) δ: 9.20 (d, J = 0.4 Hz, 1H); 9.15 (s, 1H); 8.34 (s, 1H); 8.17 (d, J = 15.9 Hz, 1H); 8.01 (d, J = 7.4 Hz, 1H); 7.25 (d, J = 7.6 Hz, 1H); 7.14 (t, J = 5.5 Hz, 1H); 6.68 (d, J = 15.8 Hz, 1H); 3.76 (s, 3H); 3.24-3.13 (m, 2H); 2.71 (s, 3H); 1.09 (t, J = 7.2 Hz, 3H).
MS (ESI, m/z): 314.3 [M+H⁺].

### 59.2. 3-[3-(3-ethyl-ureido)-8-methyl-isoquinolin-5-yl]-propionic acid methyl ester:

A suspension of intermediate 59.1 (335 mg) and 10% Pd/C (57 mg) in DMF (5 mL) was hydrogenated at rt for 5 h. The catalyst was filtered off and washed with 9:1 DCM/MeOH. The filtrate was concentrated under reduced pressure to give a brown residue. The title compound was obtained, after purification by CC (Hept/EA 100:0 to 50:50), as a white solid (195 mg; 58% yield).
¹H NMR (d6-DMSO) δ: 9.12 (d, J = 0.6 Hz, 1H); 9.03 (s, 1H); 8.11 (s, 1H); 7.33 (d, J = 7.1 Hz, 1H); 7.21 (t, J = 5 .5 Hz, 1H); 7.10 (dd, J = 7.1, 0.8 Hz, 1H); 3.58 (s, 3H); 3.23-3.09 (m, 4H); 2.71 (t, J = 7.9 Hz, 2H); 2.63 (s, 3H); 1.09 (t, J = 7.2 Hz, 3H).
MS (ESI, m/z): 316.2 [M+H⁺].

### 59.3. 3-[3-(3-ethyl-ureido)-8-methyl-isoquinolin-5-yl]-propionic acid:

To a solution of intermediate 59.2 (190 mg) in dioxane (3 mL) was added 1*N* NaOH (2.4 mL). The reaction mixture was stirred at rt for 30 min. The aq. layer was acidified with 1*N* HCl until pH reached 4-5 and EA and water were added. The layers were separated and the aq. layer was extracted twice with EA. The combined org. layers were dried over Na₂SO₄, filtered and concentrated under reduced pressure to give a pale green solid (177 mg; 97% yield).
MS (ESI, m/z): 302.2 [M+H⁺].

### 59.4. 3-[3-(3-ethyl-ureido)-8-methyl-isoquinolin-5-yl]-propionamide:

Starting from intermediate 59.3 (69 mg) and 0.5*M* ammonia in dioxane (3.1 eq.) and proceeding in analogy to Procedure I, the title compound was obtained, after trituration in 5:4:1 DCM/water/MeOH, as a white solid (20 mg; 29% yield).
1H NMR (d6-DMSO) δ: 9.12 (s, 1H); 9.03 (s, 1H); 8.12 (s, 1H); 7.32 (d, J =7.1 Hz, 1H); 7.29-7.20 (m, 2H); 7.10 (d, J =6.9 Hz, 1H); 6.76 (br. s, 1H); 3.24-3.12 (m, 2H); 3.12-3.02 (m, 2H); 2.63 (s, 3H); 2.48 (m, 2H); 1.09 (t, J = 7.2 Hz, 3H).
MS (ESI, m/z): 301.2 [M+H⁺].

### Example 60: 1-ethyl-3-(5-fluoro-8-methyl-isoquinolin-3-yl)-urea:

Starting from the compound of Example 32 and proceeding in analogy to Procedure E, the title compound was obtained, after trituration in 1:1 MeOH/DMF, as a white solid (29% yield).
¹H NMR (d6-DMSO) δ: 9.19-9.15 (m, 2H); 8.13 (d, J = 0.6 Hz, 1H); 7.33 (dd, J = 10.8, 7.8 Hz, 1H); 7.18-7.11 (m, 1H); 7.01 (t, J = 5 .6 Hz, 1H); 3.22-3.11 (m, 2H); 2.64 (s, 3H); 1.08 (t, J = 7.2 Hz, 3H).
MS (ESI, m/z): 248.3 [M+H⁺].

### Example 61: 1-ethyl-3-(8-methyl-5-pyridin-4-yl-isoquinolin-3-yl)-urea:

Starting from the compound of Example 1 and pyridine-4-boronic acid and proceeding in analogy to Procedure C, the title compound was obtained, after purification by CC (DCM/MeOH +1% NH₄OH 100:0 to 98:2), as a yellow solid (86% yield).
¹H NMR (d6-DMSO) δ: 9.22 (s, 1H); 9.03 (s, 1H); 8.70 (d, J = 5.9 Hz, 2H); 8.06 (s, 1H); 7.51-7.46 (m, 3H); 7.30 (d, J = 7.1 Hz, 1H); 7.07 (t, J = 5.0 Hz, 1H); 3.16-3.05 (m, 2H); 2.73 (s, 3H); 1.04 (t, J = 7.2 Hz, 3H).
MS (ESI, m/z): 307.6 [M+H⁺].

### Example 62: thiazole-5-carboxylic acid [3-(3-ethyl-ureido)-8-methyl-isoquinolin-6-yl]-amide:

Starting from the compound of Example 54 and thiazole-5-carboxylic acid (2.4 eq.) and proceeding in analogy to Procedure J, using however twice as much of all other reagents and heating to 50°C, the title compound was obtained, after purification by CC (DCM/MeOH 100:0 to 95:5), as a beige solid (14% yield).
¹H NMR (d6-DMSO) δ: 10.59 (s, 1H); 9.32 (s, 1H); 9.03 (s, 1H); 9.00 (s, 1H); 8.73 (s, 1H); 8.04 (d, J = 0.8 Hz, 1H); 7.83 (s, 1H); 7.45 (d, J = 0.6 Hz, 1H); 7.27-7.19 (m, 1H); 3.23-3.11 (m, 2H); 2.67 (s, 3H); 1.08 (t, J = 7.2 Hz, 3H).
MS (ESI, m/z): 356.2 [M+H⁺].

### Example 63: 1-ethyl-3-(5-ethylamino-8-methyl-isoquinolin-3-yl)-urea:

Starting from the compound of Example 1 and a 2M solution of ethylamine in MeOH (4 eq.) and proceeding in analogy to Procedure R, using however twice as much of all other reagents, the title compound was obtained, after purification by CC (DCM/MeOH +1% NH₄OH 100:0 to 90:10) followed by trituration in Et₂O, as a yellow solid (22% yield).
MS (ESI, m/z): 273.2 [M+H⁺].

### Example 64: 1-ethyl-3-[8-methyl-5-(4-methyl-thiophen-3-yl)-isoquinolin-3-yl]-urea:

Starting from the compound of Example 1 and 4-methyl-3-thiopheneboronic acid and proceeding in analogy to Procedure A, the title compound was obtained, after purification by prep-HPLC (acidic conditions), as an amorphous solid (36% yield).
MS (ESI, m/z): 326.04 [M+H⁺].

### Example 65: {4-[3-(3-ethyl-ureido)-8-methyl-isoquinolin-5-yl]-phenyl}-carbamic acid methyl ester:

Starting from the compound of Example 1 and 4-methoxycarbonylaminophenylboronic acid and proceeding in analogy to Procedure A, the title compound was obtained, after purification by prep-HPLC (acidic conditions), as an amorphous solid (25% yield).
MS (ESI, m/z): 379.07 [M+H⁺].

### Example 66: 1-ethyl-3-[5-(2-fluoro-pyridin-3-yl)-8-methyl-isoquinolin-3-yl]-urea:

Starting from the compound of Example 1 and 2-fluoropyridine-3-boronic acid and proceeding in analogy to Procedure A, the title compound was obtained, after purification by prep-HPLC (acidic conditions), as an amorphous solid (12% yield).
MS (ESI, m/z): 325.04 [M+H⁺].

### Example 67: 1-ethyl-3-(8-methyl-[5,5']biisoquinolinyl-3-yl)-urea:

Starting from the compound of Example 1 and isoquinoline-5-boronic acid and proceeding in analogy to Procedure A, the title compound was obtained, after purification by prep-HPLC (acidic conditions), as an amorphous solid (25% yield).
MS (ESI, m/z): 357.07 [M+H⁺].

### Example 68: 1-ethyl-3-[5-(3-hydroxy-phenyl)-8-methyl-isoquinolin-3-yl]-urea:

Starting from the compound of Example 1 and 3-hydroxyphenylboronic acid and proceeding in analogy to Procedure A, the title compound was obtained, after purification by prep-HPLC (acidic conditions), as an amorphous solid (25% yield).
MS (ESI, m/z): 322.03 [M+H⁺].

### Example 69: 1-ethyl-3-[5-(1H-indol-4-yl)-8-methyl-isoquinolin-3-yl]-urea:

Starting from the compound of Example 1 and indole-4-boronic acid and proceeding in analogy to Procedure A, the title compound was obtained, after purification by prep-HPLC (acidic conditions), as an amorphous solid (30% yield).
MS (ESI, m/z): 345.08 [M+H⁺].

### Example 70: N-[3-(3-ethyl-ureido)-isoquinolin-8-yl]-benzamide:

Starting from the compound of Preparation K and benzamide and proceeding in analogy to Procedure F, the title compound was obtained, after purification by prep-HPLC (basic conditions), as a white solid (11% yield).
MS (ESI, m/z): 335.12 [M+H⁺].

### Example 71: 1-ethyl-3-{8-[(pyridin-3-ylmethyl)-amino]-isoquinolin-3-yl}-urea:

Starting from the compound of Preparation K and 3-picolylamine and proceeding in analogy to Procedure G, the title compound was obtained, after purification by prep-HPLC (basic conditions), as a yellow solid (23% yield).
MS (ESI, m/z): 322.20 [M+H⁺].

### Example 72: 1-(5,8-di-pyridin-4-yl-isoquinolin-3-yl)-3-ethyl-urea:

Starting from the compound of Preparation L and pyridine-4-boronic acid, and proceeding in analogy to Procedure C, the title compound was obtained, after purification by CC (DCM/MeOH +1% NH₄OH 100:0 to 97:3), as a yellow solid (73% yield).
MS (ESI, m/z): 370.4 [M+H⁺].

### Example 73: 1-(5-bromo-8-methoxy-isoquinolin-3-yl)-3-ethyl-urea:

Starting from the compound of Preparation M and proceeding in analogy to Procedure N, the title compound was obtained as a yellow solid (65% yield).
¹H NMR (d6-DMSO) δ: 9.19 (s, 1H); 9.17 (s, 1H); 8.26 (s, 1H); 7.87 (d, J = 8.3 Hz, 1H); 6.97 (t, J = 5.6 Hz, 1H); 6.79 (d, J = 8.4 Hz, 1H); 3.96 (s, 3H); 3.22-3.11 (m, 2H); 1.08 (t, J = 7.2 Hz, 3H).
MS (ESI, m/z): 324.2 and 326.3 [M+H⁺ of the two main isotopes].

### Example 74: 4-[3-(3-ethyl-ureido)-5-fluoro-isoquinolin-8-yl]-benzoic acid:

Starting from the compound of Example 32 and 4-carboxyphenylboronic acid and proceeding in analogy to Procedure B, the title compound was obtained, after trituration in 1:1 MeOH/DMF, as a yellow solid (18% yield).
MS (ESI, m/z): 354.3 [M+H⁺].

### Example 75: 1-ethyl-3-(8-methyl-6-pyrimidin-5-yl-isoquinolin-3-yl)-urea:

Starting from the compound of Preparation B and pyrimidine-5-boronic acid and proceeding in analogy to Procedure A, the title compound was obtained, after purification by prep-HPLC (acidic conditions), as an amorphous solid (22% yield).
MS (ESI, m/z): 307.72 [M+H⁺].

### Example 76: 1-ethyl-3-[6-(3-methoxy-phenyl)-8-methyl-isoquinolin-3-yl]-urea:

Starting from the compound of Preparation B and 3-methoxyphenylboronic acid and proceeding in analogy to Procedure A, the title compound was obtained, after purification by prep-HPLC (acidic conditions), as an amorphous solid (8% yield).
MS (ESI, m/z): 336.07 [M+H⁺].

### Example 77: 1-(6-(benzo[1,3]dioxol-5-yl)-8-methyl-isoquinolin-3-yl)-3-ethyl-urea:

Starting from the compound of Preparation B and 3,4-methylenedioxyphenylboronic acid and proceeding in analogy to Procedure A, the title compound was obtained, after purification by prep-HPLC (acidic conditions), as an amorphous solid (3% yield).
MS (ESI, m/z): 350.05 [M+H⁺].

### Example 78: 1-ethyl-3-(6-furan-2-yl-8-methyl-isoquinolin-3-yl)-urea:

Starting from the compound of Preparation B and furan-2-boronic acid pinacol ester and proceeding in analogy to Procedure A, the title compound was obtained, after purification by prep-HPLC (acidic conditions), as an amorphous solid (37% yield).
MS (ESI, m/z): 296.00 [M+H⁺].

### Example 79: 1-ethyl-3-(8-methyl-6-naphthalen-2-yl-isoquinolin-3-yl)-urea:

Starting from the compound of Preparation B and 2-naphthaleneboronic acid and proceeding in analogy to Procedure A, the title compound was obtained, after purification by prep-HPLC (acidic conditions), as an amorphous solid (3% yield).
MS (ESI, m/z): 356.07 [M+H⁺].

### Example 80: 1-ethyl-3-[8-methyl-6-(4-methyl-thiophen-3-yl)-isoquinolin-3-yl]-urea:

Starting from the compound of Preparation B and 4-methyl-3-thiopheneboronic acid and proceeding in analogy to Procedure A, the title compound was obtained, after purification by prep-HPLC (acidic conditions), as an amorphous solid (18% yield).
MS (ESI, m/z): 326.00 [M+H⁺].

### Example 81: N-{4-[3-(3-ethyl-ureido)-8-methyl-isoquinolin-6-yl]-benzyl}-acetamide:

Starting from the compound of Preparation B and 4-acetamidomethylphenylboronic acid and proceeding in analogy to Procedure A, the title compound was obtained, after purification by prep-HPLC (acidic conditions), as an amorphous solid (28% yield).
MS (ESI, m/z): 377.09 [M+H⁺].

### Example 82: 1-[6-(2-chloro-pyridin-3-yl)-8-methyl-isoquinolin-3-yl]-3-ethyl-urea:

Starting from the compound of Preparation B and 2-chloropyridine-3-boronic acid and proceeding in analogy to Procedure A, the title compound was obtained, after purification by prep-HPLC (acidic conditions), as an amorphous solid (28% yield).
MS (ESI, m/z): 340.6 [M+H⁺].

### Example 83: 1-[6-(2,3-dihydro-benzofuran-5-yl)-8-methyl-isoquinolin-3-yl]-3-ethyl-urea:

Starting from the compound of Preparation B and 2,3-dihydrobenzofuran-5-boronic acid and proceeding in analogy to Procedure A, the title compound was obtained, after purification by prep-HPLC (acidic conditions), as an amorphous solid (34% yield).
MS (ESI, m/z): 348.06 [M+H⁺].

### Example 84: 3-[3-(3-ethyl-ureido)-8-methyl-isoquinolin-6-yl]-benzoic acid ethyl ester:

Starting from the compound of Preparation B and 3-ethoxycarbonylphenylboronic acid and proceeding in analogy to Procedure A, the title compound was obtained, after purification by prep-HPLC (acidic conditions), as an amorphous solid (9% yield).
MS (ESI, m/z): 378.06 [M+H⁺].

### Example 85: 2-[3-(3-ethyl-ureido)-8-methyl-isoquinolin-6-yl]-benzoic acid ethyl ester:

Starting from the compound of Preparation B and 2-ethoxycarbonylphenylboronic acid and proceeding in analogy to Procedure A, the title compound was obtained, after purification by prep-HPLC (acidic conditions), as an amorphous solid (36% yield).
MS (ESI, m/z): 378.07 [M+H⁺].

### Example 86: N-{2-[3-(3-ethyl-ureido)-8-methyl-isoquinolin-6-yl]-phenyl}-acetamide:

Starting from the compound of Preparation B and 2-acetylaminophenylboronic acid and proceeding in analogy to Procedure A, the title compound was obtained, after purification by prep-HPLC (acidic conditions), as an amorphous solid (31% yield).
MS (ESI, m/z): 363.07 [M+H⁺].

### Example 87: 5-[3-(3-ethyl-ureido)-8-methyl-isoquinolin-6-yl]-nicotinic acid methyl ester:

Starting from the compound of Preparation B and 5-(methoxycarbonyl)pyridine-3-boronic acid and proceeding in analogy to Procedure A, the title compound was obtained, after purification by prep-HPLC (acidic conditions), as an amorphous solid (8% yield).
MS (ESI, m/z): 365.05 [M+H⁺].

### Example 88: 1-ethyl-3-[6-(3-fluoro-phenyl)-8-methyl-isoquinolin-3-yl]-urea:

Starting from the compound of Preparation B and 3-fluorophenylboronic acid and proceeding in analogy to Procedure A, the title compound was obtained, after purification by prep-HPLC (acidic conditions), as an amorphous solid (9% yield).
MS (ESI, m/z): 324.05 [M+H⁺].

### Example 89: 1-ethyl-3-[6-(6-methoxy-pyridin-3-yl)-8-methyl-isoquinolin-3-yl]-urea:

Starting from the compound of Preparation B and 2-methoxypyridine-5-boronic acid and proceeding in analogy to Procedure A, the title compound was obtained, after purification by prep-HPLC (acidic conditions), as an amorphous solid (6% yield).
MS (ESI, m/z): 337.06 [M+H⁺].

### Example 90: 1-ethyl-3-[6-(1H-indol-4-yl)-8-methyl-isoquinolin-3-yl]-urea:

Starting from the compound of Preparation B and indole-4-boronic acid and proceeding in analogy to Procedure A, the title compound was obtained, after purification by prep-HPLC (acidic conditions), as an amorphous solid (36% yield).
MS (ESI, m/z): 345.06 [M+H⁺].

### Example 91: 1-ethyl-3-(8'-methyl-[4,6']biisoquinolinyl-3'-yl)-urea:

Starting from the compound of Preparation B and isoquinoline-4-boronic acid and proceeding in analogy to Procedure A, the title compound was obtained, after purification by prep-HPLC (acidic conditions), as an amorphous solid (25% yield).
MS (ESI, m/z): 357.06 [M+H⁺].

### Example 92: 1-[6-(4-(cyanomethyl)-phenyl)-8-methyl-isoquinolin-3-yl]-3-ethyl-urea:

Starting from the compound of Preparation B and 4-cyanomethylphenylboronic acid and proceeding in analogy to Procedure A, the title compound was obtained, after purification by prep-HPLC (acidic conditions), as an amorphous solid (35% yield).
MS (ESI, m/z): 345.07 [M+H⁺].

### Example 93: 1-ethyl-3-(8-methyl-6-thiophen-3-yl-isoquinolin-3-yl)-urea:

Starting from the compound of Preparation B and thiophene-3-boronic acid and proceeding in analogy to Procedure A, the title compound was obtained, after purification by prep-HPLC (acidic conditions), as an amorphous solid (15% yield).
MS (ESI, m/z): 311.99 [M+H⁺].

### Example 94: 1-ethyl-3-[6-(4-methanesulfonyl-phenyl)-8-methyl-isoquinolin-3-yl]-urea:

Starting from the compound of Preparation B and 4-methylsulfonylphenylboronic acid and proceeding in analogy to Procedure A, the title compound was obtained, after purification by prep-HPLC (acidic conditions), as an amorphous solid (19% yield).
MS (ESI, m/z): 384.01 [M+H⁺].

### Example 95: 1-ethyl-3-[6-(4-isopropyl-pyrimidin-5-yl)-8-methyl-isoquinolin-3-yl]-urea:

Starting from the compound of Preparation B and 4-isopropylpyrimidine-5-boronic acid and proceeding in analogy to Procedure A, the title compound was obtained, after purification by prep-HPLC (acidic conditions), as an amorphous solid (34% yield).
MS (ESI, m/z): 350.08 [M+H⁺].

### Example 96: 1-ethyl-3-[8-methyl-6-(5-methylsulfanyl-pyridin-3-yl)-isoquinolin-3-yl]-urea:

Starting from the compound of Preparation B and 5-(methylthio)pyridine-3-boronic acid and proceeding in analogy to Procedure A, the title compound was obtained, after purification by prep-HPLC (acidic conditions), as an amorphous solid (24% yield).
MS (ESI, m/z): 353.04 [M+H⁺].

### Example 97: 1-ethyl-3-[6-(3-fluoro-pyridin-4-yl)-8-methyl-isoquinolin-3-yl]-urea:

Starting from the compound of Preparation B and 3-fluoropyridine-4-boronic acid and proceeding in analogy to Procedure A, the title compound was obtained, after purification by prep-HPLC (acidic conditions), as an amorphous solid (7% yield).
MS (ESI, m/z): 325.03 [M+H⁺].

### Example 98: 1-[6-(3-chloro-pyridin-4-yl)-8-methyl-isoquinolin-3-yl]-3-ethyl-urea:

Starting from the compound of Preparation B and 3-chloropyridine-4-boronic acid and proceeding in analogy to Procedure A, the title compound was obtained, after purification by prep-HPLC (acidic conditions), as an amorphous solid (15% yield).
MS (ESI, m/z): 341.07 [M+H⁺].

### Example 99: 1-ethyl-3-[6-(6-fluoro-pyridin-3-yl)-8-methyl-isoquinolin-3-yl]-urea:

Starting from the compound of Preparation B and 6-fluoropyridine-3-boronic acid and proceeding in analogy to Procedure A, the title compound was obtained, after purification by prep-HPLC (acidic conditions), as an amorphous solid (22% yield).
MS (ESI, m/z): 325.03 [M+H⁺].

### Example 100: 1-ethyl-3-[6-(2-fluoro-pyridin-3-yl)-8-methyl-isoquinolin-3-yl]-urea:

Starting from the compound of Preparation B and 2-fluoropyridine-3-boronic acid and proceeding in analogy to Procedure A, the title compound was obtained, after purification by prep-HPLC (acidic conditions), as an amorphous solid (16% yield).
MS (ESI, m/z): 325.05 [M+H⁺].

### Example 101: 1-ethyl-3-[6-(6-hydroxymethyl-pyridin-3-yl)-8-methyl-isoquinolin-3-yl]-urea:

Starting from the compound of Preparation B and 6-(hydroxymethyl)pyridine-3-boronic acid and proceeding in analogy to Procedure A, the title compound was obtained, after purification by prep-HPLC (acidic conditions), as an amorphous solid (23% yield).
MS (ESI, m/z): 337.07 [M+H⁺].

### Example 102: 1-ethyl-3-[6-(5-fluoro-pyridin-3-yl)-8-methyl-isoquinolin-3-yl]-urea:

Starting from the compound of Preparation B and 5-fluoropyridine-3-boronic acid and proceeding in analogy to Procedure A, the title compound was obtained, after purification by prep-HPLC (acidic conditions), as an amorphous solid (13% yield).
MS (ESI, m/z): 325.03 [M+H⁺].

### Example 103: 1-ethyl-3-[6-(5-methanesulfonyl-pyridin-3-yl)-8-methyl-isoquinolin-3-yl]-urea:

Starting from the compound of Preparation B and 5-(methylsulfonyl)pyridine-3-boronic acid and proceeding in analogy to Procedure A, the title compound was obtained, after purification by prep-HPLC (acidic conditions), as an amorphous solid (15% yield).
MS (ESI, m/z): 385.01 [M+H⁺].

### Example 104: 1-ethyl-3-(8'-methyl-[5,6']biisoquinolinyl-3'-yl)-urea:

Starting from the compound of Preparation B and isoquinoline-5-boronic acid hydrochloride and proceeding in analogy to Procedure A, the title compound was obtained, after purification by prep-HPLC (acidic conditions), as an amorphous solid (31% yield). MS (ESI, m/z): 357.06 [M+H⁺].

### Example 105: 1-ethyl-3-(8-methyl-6-o-tolyl-isoquinolin-3-yl)-urea:

Starting from the compound of Preparation B and 2-tolylboronic acid and proceeding in analogy to Procedure A, the title compound was obtained, after purification by prep-HPLC (acidic conditions), as an amorphous solid (29% yield).
MS (ESI, m/z): 320.03 [M+H⁺].

### Example 106: N-[3-(3-ethyl-ureido)-8-methyl-isoquinolin-6-yl]-benzamide:

This compound was obtained in two different ways:
a) Starting from the compound of Preparation B and benzamide and proceeding in analogy to Procedure F, the title compound was obtained, after purification by CC (DCM/MeOH 100:0 to 90:10) followed by trituration in Et₂O, as a beige solid (52% yield).
b) Starting from the compound of Example 54 and benzoic acid (3.0 eq.), and proceeding in analogy to Procedure J, using however twice as much of all other reagents and heating to 50°C, the title compound was obtained, after purification by CC (DCM/MeOH 100:0 to 90:10), as a white solid (34% yield).
   ¹H NMR (d6-DMSO) δ: 10.41 (s, 1H); 9.02 (s, 1H); 8.99 (s, 1H); 8.12 (d, J = 0.9 Hz, 1H); 8.00-7.95 (m, 2H); 7.81 (s, 1H); 7.64-7.50 (m, 4H); 7.32-7.24 (m, 1H); 3.23-3.12 (m, 2H); 2.66 (s, 3H); 1.09 (t, J = 7.2 Hz, 3H).
   MS (ESI, m/z): 349.2 [M+H⁺].

### Example 107: 1-[6-(3-cyano-phenyl)-8-methyl-isoquinolin-3-yl]-3-ethyl-urea:

Starting from the compound of Preparation B and 3-cyanophenylboronic acid and proceeding in analogy to Procedure A, the title compound was obtained, after purification by prep-TLC (Hept/EA 1:2), as an amorphous solid (18% yield).
MS (ESI, m/z): 331.07 [M+H⁺].

### Example 108: 1-[6-(4-acetyl-phenyl)-8-methyl-isoquinolin-3-yl]-3-ethyl-urea:

Starting from the compound of Preparation B and 4-acetylphenylboronic acid and proceeding in analogy to Procedure A, the title compound was obtained, after purification by prep-TLC (Hept/EA 1:2), as an amorphous solid (21 % yield).
MS (ESI, m/z): 348.09 [M+H⁺].

### Example 109: 1-ethyl-3-[6-(2-hydroxy-phenyl)-8-methyl-isoquinolin-3-yl]-urea:

Starting from the compound of Preparation B and 2-hydroxyphenylboronic acid and proceeding in analogy to Procedure A, the title compound was obtained, after purification by prep-TLC (Hept/EA 1:2), as an amorphous solid (15% yield).
MS (ESI, m/z): 322.05 [M+H⁺].

### Example 110: 1-[6-benzylamino-5-fluoro-8-(2-methoxy-pyrimidin-5-yl)-isoquinolin-3-yl]-3-ethyl-urea:

Starting from the compound of Example 49 and 2-methoxypyrimidine-5-boronic acid (3 eq.) and proceeding in analogy to Procedure B, the title compound was obtained, after purification by CC (DCM/MeOH 100:0 to 90:10) followed by a second CC (Hept/EA 100:0 to 0:100), as a yellow solid (22% yield).
MS (ESI, m/z): 447.2 [M+H⁺].

### Example 111: 4-[8-chloro-3-(3-ethyl-ureido)-5-fluoro-isoquinolin-6-yl]-benzenesulfonamide:

Starting from intermediate D.6 and 4-aminosulfonylphenylboronic acid and proceeding in analogy to Procedure C, the title compound was obtained, after purification by CC (DCM/MeOH 100:0 to 90:10), as a yellow solid (46% yield).
MS (ESI, m/z): 423.4 [M+H⁺].

### Example 112: 1-ethyl-3-(5-fluoro-6,8-di-quinolin-3-yl-isoquinolin-3-yl)-urea:

Starting from intermediate D.6 and 3-quinolineboronic acid (2 eq.) and proceeding in analogy to Procedure C, the title compound was obtained, after purification by CC (Hept/EA 100:0 to 0:100), as a yellow solid (13% yield).
MS (ESI, m/z): 488.2 [M+H⁺].

### Example 113: N-{3-[3-(3-ethyl-ureido)-5-fluoro-8-pyridin-3-yl-isoquinolin-6-yl]-phenyl}-acetamide:

Starting from the compound of Preparation N and pyridine-3-boronic acid and proceeding in analogy to Procedure C, the title compound was obtained, after purification by CC (Hept/EA 100:0 to 0:100), as a yellow solid (48% yield).
MS (ESI, m/z): 444.2 [M+H⁺].

### Example 114: N-{3-[3-(3-ethyl-ureido)-5-fluoro-8-(2-methoxy-pyrimidin-5-yl)-isoquinolin-6-yl]-phenyl}-acetamide:

Starting from the compound of Preparation N and 2-methoxypyrimidine-5-boronic acid (4 eq.) and proceeding in analogy to Procedure C, the title compound was obtained, after purification by CC (DCM/MeOH 100:0 to 98:2), as a yellow solid (34% yield).
MS (ESI, m/z): 475.2 [M+H⁺].

### Example 115: 1-ethyl-3-(8-methyl-6-propylamino-isoquinolin-3-yl)-urea:

Starting from the compound of Example 54 and propanal and proceeding in analogy to Procedure P, the title compound was obtained, after purification by prep-HPLC (acidic conditions), as an amorphous solid (41% yield).
MS (ESI, m/z): 287.28 [M+H⁺].

### Example 116: N-[3-(3-ethyl-ureido)-8-methyl-isoquinolin-6-yl]-isonicotinamide:

Starting from the compound of Example 54 and isonicotic acid and proceeding in analogy to Procedure K, the title compound was obtained, after purification by prep-HPLC (basic conditions), as an amorphous solid (27% yield).
MS (ESI, m/z): 350.32 [M+H⁺].

### Example 117: N-[3-(3-ethyl-ureido)-8-methyl-isoquinolin-6-yl]-4-methanesulfonyl-benzamide:

Starting from the compound of Example 54 and 4-(methylsulfonyl)benzoic acid and proceeding in analogy to Procedure K, the title compound was obtained, after purification by prep-HPLC (basic conditions), as an amorphous solid (41% yield).
MS (ESI, m/z): 427.53 [M+H⁺].

### Example 118: 3-dimethylamino-N-[3-(3-ethyl-ureido)-8-methyl-isoquinolin-6-yl]-benzamide:

Starting from the compound of Example 54 and 3-(dimethylamino)benzoic acid and proceeding in analogy to Procedure K, adding however DIPEA (3 eq.) after 2 days and stirring for additional 72 h at 40°C, the title compound was obtained, after purification by prep-HPLC (basic conditions), as an amorphous solid (45% yield).
MS (ESI, m/z): 392.38 [M+H⁺].

### Example 119: N-[3-(3-ethyl-ureido)-8-methyl-isoquinolin-6-yl]-nicotinamide:

Starting from the compound of Example 54 and nicotinic acid and proceeding in analogy to Procedure K, the title compound was obtained, after purification by prep-HPLC (basic conditions), as an amorphous solid (42% yield).
MS (ESI, m/z): 350.26 [M+H⁺].

### Example 120: N-[3-(3-ethyl-ureido)-8-methyl-isoquinolin-6-yl]-4-methoxybenzamide:

Starting from the compound of Example 54 and 4-methoxybenzoic acid, and proceeding in analogy to Procedure K, adding however DIPEA (3 eq.) after 2 days and stirring for additional 72 h at 40°C, the title compound was obtained, after purification by prep-HPLC (basic conditions), as an amorphous solid (36% yield).
MS (ESI, m/z): 379.33 [M+H⁺].

### Example 121: 4-dimethylamino-N-[3-(3-ethyl-ureido)-8-methyl-isoquinolin-6-yl]-benzamide:

Starting from the compound of Example 54 and 3-(dimethylamino)benzoic acid and proceeding in analogy to Procedure K, adding however DIPEA (3 eq.) after 2 days and stirring for additional 72 h at 40°C, the title compound was obtained, after purification by prep-HPLC (basic conditions), as an amorphous solid (66% yield).
MS (ESI, m/z): 392.41 [M+H⁺].

### Example 122: 1-ethyl-3-{8-methyl-6-[(pyridin-4-ylmethyl)-amino]-isoquinolin-3-yl}-urea hydrochloride:

Starting from the compound of Example 54 and 4-pyridinecarboxaldehyde (3 eq.) and proceeding in analogy to Procedure P, shaking however the reaction mixture overnight at 50°C for the imine formation and then using NaBH₄ (4 eq.), the title compound was obtained, after purification by prep-HPLC (basic conditions) and treatment with HCl, as an amorphous solid (29% yield).
MS (ESI, m/z): 336.27 [M+H⁺].

### Example 123: 4-[3-(3-ethyl-ureido)-isoquinolin-8-yl]-benzoic acid:

Starting from the compound of Preparation K and 4-carboxyphenylboronic acid and proceeding in analogy to Procedure B, the title compound was obtained, after purification by prep-HPLC (acidic conditions), as an amorphous solid (23% yield).
MS (ESI, m/z): 336.08 [M+H⁺].

### Example 124: 4-[3-(3-ethyl-ureido)-isoquinolin-5-yl]-benzoic acid:

Starting from the compound of Preparation O and 4-carboxybenzeneboronic acid and proceeding in analogy to Procedure B, the title compound was obtained, after purification by prep-HPLC (acidic conditions), as an amorphous solid (6% yield).
MS (ESI, m/z): 336.14 [M+H⁺].

### Example 125: 1-ethyl-3-(8-methoxy-5-pyridin-4-yl-isoquinolin-3-yl)-urea:

Starting from the compound of Example 73 and pyridine-4-boronic acid and proceeding in analogy to Procedure C, the title compound was obtained, after purification by CC (DCM/MeOH +1% NH₄OH 100:0 to 97:3), as a yellow solid (66% yield).
¹H NMR (d6-DMSO) δ: 9.25 (s, 1H); 9.04 (s, 1H); 8.70-8.66 (m, 2H); 8.08 (s, 1H); 7.56 (d, J = 8.0 Hz, 1H); 7.48-7.45 (m, 2H); 7.03 (t, J = 5.5 Hz, 1H); 6.95 (d, J = 8.1 Hz, 1H); 4.01 (s, 3H); 3.17-3.05 (m, 2H); 1.03 (t, J = 7.2 Hz, 3H).
MS (ESI, m/z): 323.4 [M+H⁺].

### Example 126: 3-{[3-(3-ethyl-ureido)-8-methyl-isoquinolin-6-ylamino]-methyl}-benzoic acid methyl ester:

Starting from the compound of Example 54 and methyl 3-formylbenzoate and proceeding in analogy to Procedure P, the title compound was obtained, after purification by prep-HPLC (acidic conditions), as an amorphous solid (24% yield).
MS (ESI, m/z): 393.19 [M+H⁺].

### Example 127: 4-{[3-(3-ethyl-ureido)-8-methyl-isoquinolin-6-ylamino]-methyl}-benzoic acid:

Starting from the compound of Example 54 and 4-formylbenzoic acid and proceeding in analogy to Procedure P, the title compound was obtained, after purification by prep-HPLC (basic conditions), as an amorphous solid (50% yield).
MS (ESI, m/z): 379.18 [M+H⁺].

### Example 128: 3-{[3-(3-ethyl-ureido)-8-methyl-isoquinolin-6-ylamino]-methyl}-benzoic acid hydrochloride:

Starting from the compound of Example 54 and 3-formylbenzoic acid and proceeding in analogy to Procedure P, the title compound was obtained, after purification by prep-HPLC (acidic conditions) and treatment with HCl, as an amorphous solid (40% yield).
MS (ESI, m/z): 379.18 [M+H⁺].

### Example 129: 1-ethyl-3-{6-[3-(2-hydroxy-ethoxy)-benzylamino]-8-methyl-isoquinolin-3-yl}-urea hydrochloride:

Starting from the compound of Example 54 and 3-(2-hydroxyethoxy)benzaldehyde, and proceeding in analogy to Procedure P, the title compound was obtained, after purification by prep-HPLC (acidic conditions) and treatment with HCl, as an amorphous solid (44% yield).
MS (ESI, m/z): 395.22 [M+H⁺].

### Example 130: 1-ethyl-3-{8-methyl-6-[(4-methyl-3,4-dihydro-2H-benzo[1,4]oxazin-7-ylmethyl)-amino]-isoquinolin-3-yl}-urea hydrochloride:

Starting from the compound of Example 54 and 4-methyl-3,4-dihydro-2*H*-1,4-benzoxazine-7-carbaldehyde, and proceeding in analogy to Procedure P, the title compound was obtained, after purification by prep-HPLC (acidic conditions) and treatment with HCl, as an amorphous solid (64% yield).
MS (ESI, m/z): 406.23 [M+H⁺].

### Example 131: 1-ethyl-3-{8-methyl-6-[4-(3-methyl-[1,2,4]triazol-1-yl)-benzylamino]-isoquinolin-3-yl}-urea hydrochloride:

### 131.1. 4-(3-methyl-[1,2,4]triazol-1-yl)-benzaldehyde:

To a solution of 4-fluorobenzaldehyde (3.63 g) and 3-methyl-1*H*-1,2,4-triazole (2.92 g; prepared according to US 2006/293304) in dry DMF (35 mL) was added K₂CO₃ (24.25 g) at rt. The reaction mixture was stirred at 120°C overnight, then cooled to rt and diluted with EA and water. The aq. layer was extracted with EA (2x) and the combined org. layers were washed with water and brine, dried over MgSO₄, filtered and concentrated under reduced pressure. The title compound was obtained, after purification by CC (Hept/EA 80:20 to 50:50), as a yellow solid (36% yield).
¹H NMR (CDCl₃) δ: 10.06 (s, 1H); 8.58 (s, 1H); 8.04 (d, J = 8.0 Hz, 2H); 7.88 (d, J = 8.0 Hz, 2H); 2.53 (s, 3H).

### 131.2. 1-ethyl-3-{8-methyl-6-[4-(3-methyl-[1,2,4]triazol-1-yl)-benzylamino]-isoquinolin-3-yl}-urea hydrochloride:

Starting from the compound of Example 54 and intermediate 131.1 and proceeding in analogy to Procedure P, the title compound was obtained, after purification by prep-HPLC (acidic conditions) and treatment with HCl, as an amorphous solid (41% yield).
MS (ESI, m/z): 416.19 [M+H⁺].

### Example 132: 1-[6-(2-benzyloxy-ethylamino)-8-methyl-isoquinolin-3-yl]-3-ethyl-urea hydrochloride:

Starting from the compound of Example 54 and benzyloxyacetaldehyde and proceeding in analogy to Procedure Q, the title compound was obtained, after purification by prep-HPLC (acidic conditions) and treatment with HCl, as an amorphous solid (22% yield).
MS (ESI, m/z): 379.32 [M+H⁺].

### Example 133: 1-{6-[(cyclopropylmethyl)-amino]-8-methyl-isoquinolin-3-yl}-3-ethyl-urea hydrochloride:

Starting from the compound of Example 54 and cyclopropanecarboxaldehyde and proceeding in analogy to Procedure Q, the title compound was obtained, after purification by prep-HPLC (acidic conditions) and treatment with HCl, as an amorphous solid (7% yield).
MS (ESI, m/z): 299.29 [M+H⁺].

### Example 134: 1-ethyl-3-{6-[(1H-indol-6-ylmethyl)-amino]-8-methyl-isoquinolin-3-yl}-urea hydrochloride:

Starting from the compound of Example 54 and indole-6-carboxaldehyde and proceeding in analogy to Procedure Q, the title compound was obtained, after purification by prep-HPLC (acidic conditions) and treatment with HCl, as an amorphous solid (37% yield).
MS (ESI, m/z): 373.88 [M+H⁺].

### Example 135: 1-ethyl-3-{8-methyl-6-[((1-methyl-1H-indol-6-yl)methyl)-amino]-isoquinolin-3-yl}-urea hydrochloride:

Starting from the compound of Example 54 and 1-methyl-1*H*-indole-6-carbaldehyde and proceeding in analogy to Procedure Q, the title compound was obtained, after purification by prep-HPLC (acidic conditions) and treatment with HCl, as an amorphous solid (75% yield).
MS (ESI, m/z): 388.29 [M+H⁺].

### Example 136: 1-ethyl-3-(6-isobutylamino-8-methyl-isoquinolin-3-yl)-urea hydrochloride:

Starting from the compound of Example 54 and isobutyraldehyde and proceeding in analogy to Procedure Q, the title compound was obtained, after purification by prep-HPLC (acidic conditions) and treatment with HCl, as an amorphous solid (65% yield).
MS (ESI, m/z): 301.24 [M+H⁺].

### Example 137: N-(4-{[3-(3-ethyl-ureido)-8-methyl-isoquinolin-6-ylamino]-methyl}-phenyl)-acetamide:

Starting from the compound of Example 54 and 4-acetamidobenzaldehyde and proceeding in analogy to Procedure Q, the title compound was obtained, after trituration in 1:1 MeOH/DMSO, as a yellow solid (59% yield).
MS (ESI, m/z): 392.26 [M+H⁺]

### Example 138: 1-(2-fluoro-ethyl)-3-(8-methyl-5-pyridin-4-yl-isoquinolin-3-yl)-urea:

### 138.1. 8-methyl-5-(pyridin-4 yl)isoquinolin-3-amine:

Starting from the compound of Preparation A and pyridine-4-boronic acid and proceeding in analogy to Procedure C, the title compound was obtained, after purification by CC (DCM/MeOH +1% NH₄OH 100:0 to 90:10), as a yellow solid (86% yield).
¹H NMR (d6-DMSO) δ: 9.00 (d, J= 0.5 Hz, 1H); 8.69-8.65 (m, 2H); 7.46-7.42 (m, 2H); 7.29 (d, J = 7.1 Hz, 1H); 7.02 (dd, J = 7.1, 0. Hz, 1H); 6.57 (d, J = 0.6 Hz, 1H); 5.96 (br. s, 2H); 2.64 (s, 3H).
MS (ESI, m/z): 236.2 [M+H⁺].

### 138.2. 1-(2-fluoro-ethyl)-3-(8-methyl-5-pyridin-4-yl-isoquinolin-3-yl)-urea:

Starting from intermediate 138.1 and 2-fluoroethylamine hydrochloride and proceeding in analogy to Procedure O, the title compound was obtained, after purification by CC (DCM/MeOH 100:0 to 95:5), as a beige solid (13% yield).
¹H NMR (d6-DMSO) δ: 9.24 (s, 1H); 9.18 (s, 1H); 8.71-8.68 (m, 2H); 8.07 (s, 1H); 7.53-7.46 (m, 3H); 7.37 (t, J = 5.6 Hz, 1H); 7.32 (d, J = 7.3 Hz, 1H); 4.44 (dt, J = 47.6, 4.9 Hz, 2H); 3.41 (dq, J = 27.7, 5.3 Hz, 2H); 2.74 (s, 3H).
MS (ESI, m/z): 325.2 [M+H⁺].

### Example 139: 1-cyclopropyl-3-(8-methyl-5-pyridin-4-yl-isoquinolin-3-yl)-urea:

Starting from intermediate 138.1 and cyclopropylamine and proceeding in analogy to Procedure O, the title compound was obtained, after purification by CC (DCM/MeOH 100:0 to 96:4), as a white solid (13% yield).
¹H NMR (d6-DMSO) δ: 9.22 (s, 1H); 8.92 (s, 1H); 8.71 (d, J = 5.9 Hz, 2H); 8.11 (s, 1H); 7.52-7.46 (m, 3H); 7.31 (d, J = 7.4 Hz, 1H); 7.19-7.14 (m, 1H); 2.73 (s, 3H); 2.57-2.45 (m, 1H); 0.66-0.57 (m, 2H); 0.41-0.34 (m, 2H).
MS (ESI, m/z): 319.2 [M+H⁺].

### Example 140: 1-ethyl-3-[5-(pyridin-4-yl)-8-vinylisoquinolin-3-yl]-urea:

Starting from the compound of Preparation L and vinylboronic anhydride pyridine complex and proceeding in analogy to Procedure C, the title compound was obtained, after purification by CC (DCM/MeOH +1% NH₄OH 100:0 to 96:4), as an orange solid (64% yield).
¹H NMR (d6-DMSO) δ: 9.39 (d, J = 0.3 Hz, 1H); 9.05 (s, 1H); 8.75-8.69 (m, 2H); 8.09 (d, J = 0.4 Hz, 1H); 7.76-7.57 (m, 3H); 7.53-7.47 (m, 2H); 7.01 (t, J = 5.6 Hz, 1H); 5.98 (dd, J = 17.2, 1.2 Hz, 1H); 5.60 (dd, J = 11.0, 1.1 Hz, 1H); 3.17-3.05 (m, 2H); 1.03 (t, J = 7.2 Hz, 3H).
MS (ESI, m/z): 319.3 [M+H⁺].

### Example 141: 1-ethyl-3-[5-fluoro-8-methyl-6-(5-methylpyridin-3-yl)isoquinolin-3-yl]-urea:

### 141.1. 1-[8-chloro-5-fluoro-6-(5-methylpyridin-3-yl)isoquinolin-3-yl]-3-ethyl-urea:

Starting from intermediate D.6 and 5-methylpyridine-3-boronic acid and proceeding in analogy to Procedure C, the title compound was obtained, after purification by CC (DCM/MeOH 100:0 to 90:10), as a beige solid (75% yield).
¹H NMR (d6-DMSO) δ: 9.40 (s, 1H); 9.26 (s, 1H); 8.72-8.68 (m, 1H); 8.51 (d, J = 1.8 Hz, 1H); 8.31 (s, 1H); 7.96 (s, 1H); 7.73 (d, J = 6.4 Hz, 1H); 6.94 (t, J = 5.4 Hz, 1H); 3.23-3.12 (m, 2H); 2.39 (s, 3H); 1.08 (t, J = 7.0 Hz, 3H).
MS (ESI, m/z): 359.2 [M+H⁺].

### 141.2. 1-ethyl-3-[5-fluoro-8-methyl-6-(5-methylpyridin-3 yl)isoquinolin-3 yl]-urea:

Starting from intermediate 141.1 and proceeding in analogy to Procedure S, the title compound was obtained, after purification by CC (Hept/EA 100:0 to 0:100), as a beige solid (26% yield).
¹H NMR (d6-DMSO) δ: 9.23 (s, 1H); 9.19 (s, 1H); 8.67 (s, 1H); 8.48 (d, J = 1.1 Hz, 1H); 8.21 (s, 1H); 7.91 (s, 1H); 7.36 (d, J = 7.2 Hz, 1H); 7.04 (t, J = 5.4 Hz, 1H); 3.23-3.12 (m, 2H); 2.70 (s, 3H); 2.39 (s, 3H); 1.09 (t, J = 7.2 Hz, 3H).
MS (ESI, m/z): 339.2 [M+H⁺].

### Example 142: N-{3-[3-(3-ethyl-ureido)-5-fluoro-8-methyl-isoquinolin-6-yl]-phenyl}-acetamide:

Starting from the compound of Preparation N and proceeding in analogy to Procedure S, the title compound was obtained, after purification by CC (DCM/MeOH 100:0 to 90:10), as a beige solid (23% yield).
MS (ESI, m/z): 381.2 [M+H⁺].

### Example 143: 1-ethyl-3-[5-fluoro-8-methyl-6-(quinolin-3-yl)-isoquinolin-3-yl]-urea:

### 143.1. 1-[8-chloro-5 fluoro-6-(quinolin-3-yl)-isoquinolin-3-yl]-3-ethylurea:

Starting from intermediate D.6 and 3-quinolineboronic acid and proceeding in analogy to Procedure C, the title compound was obtained, after purification by CC (DCM/MeOH 100:0 to 90:10), as a yellow solid (76% yield).
MS (ESI, m/z): 395.2 [M+H⁺].

### 143.2. 1-ethyl-3-[5-fluoro-8-methyl-6-(quinolin-3-yl)-isoquinolin-3-yl]urea:

Starting from intermediate 143.1 and proceeding in analogy to Procedure S, the title compound was obtained, after purification by CC (Hept/EA 100:0 to 0:100), as a white solid (14% yield).
¹H NMR (d6-DMSO) δ: 9.25 (s, 1H); 9.22 (s, 1H); 9.19 (t, J = 2.1 Hz, 1H); 8.70 (d, J = 2.0 Hz, 1H); 8.25 (s, 1H); 8.12-8.06 (m, 2H); 7.86-7.79 (m, 1H); 7.71-7.64 (m, 1H); 7.55-7.49 (m, 1H); 7.05 (t, J = 5.2 Hz, 1H); 3.24-3.11 (m, 2H); 2.74 (s, 3H); 1.09 (t, J = 7.2 Hz, 3H).
MS (ESI, m/z): 375.2 [M+H⁺].

### Example 144: 4-[3-(3-ethyl-ureido)-5-fluoro-8-methyl-isoquinolin-6-yl]-benzenesulfonamide:

Starting from the compound of Example 111 and proceeding in analogy to Procedure S, the title compound was obtained, after purification by CC (DCM/MeOH 100:0 to 90:10), as a yellow solid (16% yield).
¹H NMR (d6-DMSO) δ: 9.23 (s, 1H); 9.20 (s, 1H); 8.22 (s, 1H); 7.99-7.92 (m, 2H); 7.89-7.82 (m, 2H); 7.43 (br. s, 2H); 7.38-7.32 (m, 1H); 7.07-7.00 (m, 1H); 3.23-3.12 (m, 2H); 2.70 (s, 3H); 1.08 (t, J = 7.2 Hz, 3H).
MS (ESI, m/z): 403.2 [M+H⁺].

### Example 145: 1-ethyl-3-[8-(pyridin-3-ylamino)-isoquinolin-3-yl]-urea:

Starting from the compound of Preparation K and 3-aminopyridine and proceeding in analogy to Procedure R, the title compound was obtained, after purification by CC (DCM/MeOH +1% NH₄OH 100:0 to 96:4), as an orange solid (20% yield).
¹H NMR (d6-DMSO) δ: 9.27 (s, 1H); 9.04 (s, 1H); 8.66 (s, 1H); 8.46-8.43 (m, 1H); 8.10 (dd, J = 4.6, 1.4 Hz, 1H); 7.93 (s, 1H); 7.54-7.43 (m, 2H); 7.32-7.23 (m, 2H); 7.11-7.03 (m, 2H); 3.22-3.11 (m, 2H); 1.08 (t, J = 7.2 Hz, 3H).
MS (ESI, m/z): 308.2 [M+H⁺].

### Example 146: 1-ethyl-3-[8-(pyridin-2-ylamino)-isoquinolin-3-yl]-urea:

Starting from the compound of Preparation K and 2-aminopyridine and proceeding in analogy to Procedure R, the title compound was obtained, after purification by CC (DCM/MeOH +1% NH₄OH 100:0 to 96:4), as a yellow solid (13% yield).
¹H NMR (d6-DMSO) δ: 9.27 (s, 1H); 9.12 (s, 1H); 9.01 (s, 1H); 8.15-8.10 (m, 1 H); 7.92 (s, 1H); 7.85 (d, J = 7.6 Hz, 1H); 7.63-7.56 (m, 1H); 7.53 (t, J = 7.8 Hz, 1H); 7.35 (d, J = 8.2 Hz, 1H); 7.09 (t, J = 5.4 Hz, 1H); 7.05-7.00 (m, 1H); 6.82-6.75 (m, 1H); 3.22-3.11 (m, 2H); 1.08 (t, J = 7.2 Hz, 3H).
MS (ESI, m/z): 308.2 [M+H⁺].

### Example 147: 1-ethyl-3-[8-(pyridin-3-yloxy)-isoquinolin-3-yl]-urea:

Starting from the compound of Preparation K and 3-hydroxypyridine and proceeding in analogy to Procedure T, the title compound was obtained, after purification by CC (DCM/MeOH +1% NH₄OH 100:0 to 98:2), as a white solid (15% yield).
¹H NMR (d6-DMSO) δ: 9.22 (s, 1H); 9.12 (s, 1H); 8.52 (d, J = 2.8 Hz, 1H); 8.43 (d, J = 4.6 Hz, 1H); 8.09-8.07 (m, 1H); 7.60-7.52 (m, 3H); 7.49-7.43 (m, 1H); 6.99 (t, J = 5.5 Hz, 1H); 6.76-6.69 (m, 1H); 3.22-3.11 (m, 2H); 1.08 (t, J = 7.2 Hz, 3H).
MS (ESI, m/z): 309.2 [M+H⁺].

### Example 148: 1-ethyl-3-[8-(pyrazin-2-ylamino)-isoquinolin-3-yl]-urea:

Starting from the compound of Preparation K and aminopyrazine and proceeding in analogy to Procedure U, the title compound was obtained, after purification by prep-HPLC (basic conditions), as an amorphous solid (26% yield).
MS (ESI, m/z): 308.2 [M+H⁺].

### Example 149: 1-ethyl-3-[8-(pyrimidin-5-ylamino)-isoquinolin-3-yl]-urea:

Starting from the compound of Preparation K and 5-aminopyrimidine and proceeding in analogy to Procedure U, the title compound was obtained, after purification by prep-HPLC (basic conditions), as an amorphous solid (66% yield).
¹H NMR (d6-DMSO) δ: 9.26 (s, 1H); 9.07 (s, 1H); 8.81 (s, 1H); 8.69 (s, 1H); 8.61 (s, 2H); 7.97 (s, 1H); 7.53-7.46 (m, 1H); 7.40-7.34 (m, 1H); 7.21-7.16 (m, 1H); 7.03 (t, J = 5.5 Hz, 1H); 3.22-3.11 (m, 2H); 1.08 (t, J = 7.2 Hz, 3H).
MS (ESI, m/z): 308.2 [M+H⁺].

### Example 150: 1-ethyl-3-[8-(imidazo[1,2-a]pyridin-7-ylamino)-isoquinolin-3-yl]-urea:

Starting from the compound of Preparation K and imidazo[1,2-a]pyridin-7-amine and proceeding in analogy to Procedure U, the title compound was obtained, after purification by prep-HPLC (basic conditions), as an amorphous solid (23% yield).
MS (ESI, m/z): 347.14 [M+H⁺].

### Example 151: 1-ethyl-3-[8-(pyrimidin-4-ylamino)-isoquinolin-3-yl]-urea:

Starting from the compound of Preparation K and 4-aminopyrimidine and proceeding in analogy to Procedure U, the title compound was obtained, after purification by prep-HPLC (basic conditions), as an amorphous solid (68% yield).
MS (ESI, m/z): 308.2 [M+H⁺].

### Example 152: 1-ethyl-3-[8-(pyrimidin-2-ylamino)-isoquinolin-3-yl]-urea:

Starting from the compound of Preparation K and 2-aminopyrimidine and proceeding in analogy to Procedure U, the title compound was obtained, after purification by prep-HPLC (basic conditions), as an amorphous solid (31% yield).
MS (ESI, m/z): 308.2 [M+H⁺].

### Example 153: 1-ethyl-3-[8-(pyridin-4-ylamino)-isoquinolin-3-yl]-urea:

Starting from the compound of Preparation K and 4-aminopyridine and proceeding in analogy to Procedure U, the title compound was obtained, after purification by prep-HPLC (basic conditions), as an amorphous solid (53% yield).
MS (ESI, m/z): 308.03 [M+H⁺].

### Example 154: 1-ethyl-3-[8-(5-methyl-[1,3,4]oxadiazol-2-ylamino)-isoquinolin-3-yl]-urea:

Starting from the compound of Preparation K and 5-methyl-1,3,4-oxadiazol-2-ylamine and proceeding in analogy to Procedure U, the title compound was obtained, after purification by prep-HPLC (basic conditions), as an amorphous solid (10% yield).
MS (ESI, m/z): 313.09 [M+H⁺].

### Example 155: 1-ethyl-3-[8-(6-methyl-pyridin-2-ylamino)-isoquinolin-3-yl]-urea hydrochloride:

Starting from the compound of Preparation K and 2-amino-6-methylpyridine and proceeding in analogy to Procedure U, the title compound was obtained, after purification by prep-HPLC (acidic conditions) and treatment with HCl, as an amorphous solid (63% yield).
MS (ESI, m/z): 322.2 [M+H⁺].

### Example 156: 6-[3-(3-ethyl-ureido)-isoquinolin-8-ylamino]-nicotinamide hydrochloride:

Starting from the compound of Preparation K and 6-aminonicotinamide and proceeding in analogy to Procedure U, the title compound was obtained, after purification by prep-HPLC (acidic conditions) and treatment with HCl, as an amorphous solid (41% yield).
MS (ESI, m/z): 351.2 [M+H⁺].

### Example 157: 1-ethyl-3-[8-(3-fluoro-pyridin-4-ylamino)-isoquinolin-3-yl]-urea hydrochloride:

Starting from the compound of Preparation K and 4-amino-3-fluoropyridine and proceeding in analogy to Procedure U, the title compound was obtained, after purification by prep-HPLC (acidic conditions) and treatment with HCl, as an amorphous solid (38% yield).
MS (ESI, m/z): 325.94 [M+H⁺].

### Example 158: 1-ethyl-3-[8-(4-fluoro-pyridin-2-ylamino)-isoquinolin-3-yl]-urea hydrochloride:

Starting from the compound of Preparation K and 2-amino-4-fluoropyridine and proceeding in analogy to Procedure U, the title compound was obtained, after purification by prep-HPLC (acidic conditions) and treatment with HCl, as an amorphous solid (14% yield).
MS (ESI, m/z): 325.97 [M+H⁺].

### Example 159: 1-ethyl-3-[8-(5-fluoro-pyridin-3-ylamino)-isoquinolin-3-yl]-urea hydrochloride:

Starting from the compound of Preparation K and 3-amino-5-fluoropyridine and proceeding in analogy to Procedure U, the title compound was obtained, after purification by prep-HPLC (acidic conditions) and treatment with HCl, as an amorphous solid (62% yield).
MS (ESI, m/z):325.97 [M+H⁺].

### Example 160: 1-ethyl-3-[8-(4-methoxy-pyridin-2-ylamino)-isoquinolin-3-yl]-urea hydrochloride:

Starting from the compound of Preparation K and 2-amino-4-methoxypyridine and proceeding in analogy to Procedure U, the title compound was obtained, after purification by prep-HPLC (acidic conditions) and treatment with HCl, as an amorphous solid (43% yield).
MS (ESI, m/z): 338.19 [M+H⁺].

### Example 161: N-{5-[3-(3-ethyl-ureido)-isoquinolin-8-ylamino]-pyridin-2-yl}-acetamide hydrochloride:

Starting from the compound of Preparation K and 2-acetamido-5-aminopyridine and proceeding in analogy to Procedure U, the title compound was obtained, after purification by prep-HPLC (acidic conditions) and treatment with HCl, as an amorphous solid (12% yield).
MS (ESI, m/z): 365.21 [M+H⁺].

### Example 162: 1-ethyl-3-[8-(4-methyl-pyridin-2-ylamino)-isoquinolin-3-yl]-urea hydrochloride:

Starting from the compound of Preparation K and 2-amino-4-picoline and proceeding in analogy to Procedure U, the title compound was obtained, after purification by prep-HPLC (acidic conditions) and treatment with HCl, as an amorphous solid (32% yield).
MS (ESI, m/z): 322.19 [M+H⁺].

### Example 163: 1-ethyl-3-[8-(5-methyl-pyridin-2-ylamino)-isoquinolin-3-yl]-urea hydrochloride:

Starting from the compound of Preparation K and 6-amino-3-picoline and proceeding in analogy to Procedure U, the title compound was obtained, after purification by prep-HPLC (acidic conditions) and treatment with HCl, as an amorphous solid (67% yield).
MS (ESI, m/z): 322.2 [M+H⁺].

### Example 164: 1-ethyl-3-[8-(6-methyl-pyridin-3-ylamino)-isoquinolin-3-yl]-urea hydrochloride:

Starting from the compound of Preparation K and 5-amino-2-picoline and proceeding in analogy to Procedure U, the title compound was obtained, after purification by prep-HPLC (acidic conditions) and treatment with HCl, as an amorphous solid (3% yield).
MS (ESI, m/z): 322.2 [M+H⁺].

### Example 165: 6-[3-(3-ethyl-ureido)-isoquinolin-8-ylamino]-nicotinic acid methyl ester hydrochloride:

Starting from the compound of Preparation K and methyl 6-aminopyridine-3-carboxylate and proceeding in analogy to Procedure U, the title compound was obtained, after purification by prep-HPLC (acidic conditions) and treatment with HCl, as an amorphous solid (18% yield).
MS (ESI, m/z): 366.22 [M+H⁺].

### Example 166: 6-[3-(3-ethyl-ureido)-isoquinolin-8-ylamino]-pyridine-2-carboxylic acid methyl ester hydrochloride:

Starting from the compound of Preparation K and methyl 6-aminopyridine-2-carboxylate and proceeding in analogy to Procedure U, the title compound was obtained, after purification by prep-HPLC (acidic conditions) and treatment with HCl, as an amorphous solid (3% yield).
MS (ESI, m/z): 366.2 [M+H⁺].

### Example 167: 1-ethyl-3-[8-(3-methyl-pyridin-2-ylamino)-isoquinolin-3-yl]-urea hydrochloride:

Starting from the compound of Preparation K and 2-amino-3-picoline and proceeding in analogy to Procedure U, the title compound was obtained, after purification by prep-HPLC (acidic conditions) and treatment with HCl, as an amorphous solid (19% yield).
MS (ESI, m/z): 322.2 [M+H⁺].

### Example 168: 1-ethyl-3-[8-(3-methoxy-phenylamino)-isoquinolin-3-yl]-urea:

Starting from the compound of Preparation K and 3-methoxyaniline and proceeding in analogy to Procedure V, the title compound was obtained, after purification by prep-HPLC (acidic conditions), as an amorphous solid (49% yield).
MS (ESI, m/z): 337.22 [M+H⁺].

### Example 169: 1-ethyl-3-(8-phenylamino-isoquinolin-3-yl)-urea:

Starting from the compound of Preparation K and aniline and proceeding in analogy to Procedure V, the title compound was obtained, after purification by prep-HPLC (acidic conditions), as an amorphous solid (46% yield).
MS (ESI, m/z): 307.18 [M+H⁺].

### Example 170: 1-ethyl-3-[8-(3-hydroxy-phenylamino)-isoquinolin-3-yl]-urea:

Starting from the compound of Preparation K and 3-aminophenol and proceeding in analogy to Procedure V, the title compound was obtained, after purification by prep-HPLC (acidic conditions), as an amorphous solid (19% yield).
MS (ESI, m/z): 323.19 [M+H⁺].

### Example 171: 3-[3-(3-ethyl-ureido)-isoquinolin-8-ylamino]-benzenesulfonamide:

Starting from the compound of Preparation K and 3-aminobenzenesulfonamide and proceeding in analogy to Procedure V, the title compound was obtained, after purification by prep-HPLC (acidic conditions), as an amorphous solid (30% yield).
MS (ESI, m/z): 386.17 [M+H⁺].

### Example 172: N-{3-[3-(3-ethyl-ureido)-isoquinolin-8-ylamino]-phenyl}-acetamide:

Starting from the compound of Preparation K and 3'-aminoacetanilide and proceeding in analogy to Procedure V, the title compound was obtained, after purification by prep-HPLC (acidic conditions), as an amorphous solid (32% yield).
MS (ESI, m/z): 364.24 [M+H⁺].

### Example 173: N-{4-[3-(3-ethyl-ureido)-isoquinolin-8-ylamino]-phenyl}-acetamide:

Starting from the compound of Preparation K and 4'-aminoacetanilide and proceeding in analogy to Procedure V, the title compound was obtained, after purification by prep-HPLC (acidic conditions), as an amorphous solid (30% yield).
¹H NMR (d6-DMSO) δ: 9.81 (s, 1H); 9.29 (s, 1H); 9.02 (s, 1H); 8.44 (br. s, 1H); 7.85 (s, 1H); 7.54-7.46 (m, 2H); 7.38 (t, J = 8.0 Hz, 1H); 7.16-7.08 (m, 4H); 6.92 (d, J = 7.5 Hz, 1H); 3.22-3.10 (m, 2H); 2.01 (s, 3H); 1.08 (t, J = 7.1 Hz, 3H).
MS (ESI, m/z): 364.24 [M+H⁺].

### Example 174: 1-[8-(4-cyano-phenylamino)-isoquinolin-3-yl]-3-ethyl-urea:

Starting from the compound of Preparation K and 4-aminobenzonitrile and proceeding in analogy to Procedure V, the title compound was obtained, after purification by prep-HPLC (acidic conditions), as an amorphous solid (32% yield).
MS (ESI, m/z): 332.2 [M+H⁺].

### Example 175: 1-ethyl-3-[8-(4-hydroxy-phenylamino)-isoquinolin-3-yl]-urea:

Starting from the compound of Preparation K and 4-aminophenol and proceeding in analogy to Procedure V, the title compound was obtained, after purification by prep-HPLC (acidic conditions), as an amorphous solid (27% yield).
MS (ESI, m/z): 323.2 [M+H⁺].

### Example 176: 3-[3-(3-ethyl-ureido)-isoquinolin-8-ylamino]-benzoic acid methyl ester:

Starting from the compound of Preparation P and methyl 3-bromobenzoate and proceeding in analogy to Procedure W, the title compound was obtained, after purification by prep-HPLC (basic conditions), as an amorphous solid (25% yield).
MS (ESI, m/z): 365.15 [M+H⁺].

### Example 177: 1-ethyl-3-[8-(5-methoxy-pyridin-3-ylamino)-isoquinolin-3-yl]-urea:

Starting from the compound of Preparation P and 3-bromo-5-methoxypyridine and proceeding in analogy to Procedure W, the title compound was obtained, after purification by prep-HPLC (basic conditions), as an amorphous solid (26% yield).
MS (ESI, m/z): 338.14 [M+H⁺].

### Example 178: 1-ethyl-3-[8-(6-methoxy-pyridin-2-ylamino)-isoquinolin-3-yl]-urea:

Starting from the compound of Preparation P and 2-bromo-6-methoxypyridine and proceeding in analogy to Procedure W, the title compound was obtained, after purification by prep-HPLC (basic conditions), as an amorphous solid (10% yield).
MS (ESI, m/z): 338.14 [M+H⁺].

### Example 179: 4-[3-(3-ethyl-ureido)-isoquinolin-8-ylamino]-benzoic acid methyl ester:

Starting from the compound of Preparation P and methyl 4-bromobenzoate and proceeding in analogy to Procedure W, the title compound was obtained, after purification by prep-HPLC (basic conditions), as an amorphous solid (38% yield).
MS (ESI, m/z): 365.18 [M+H⁺].

### Example 180: 1-ethyl-3-[8-(2-methoxy-pyridin-4-ylamino)-isoquinolin-3-yl]-urea:

Starting from the compound of Preparation P and 4-bromo-2-methoxypyridine and proceeding in analogy to Procedure W, the title compound was obtained, after purification by prep-HPLC (basic conditions), as an amorphous solid (58% yield).
MS (ESI, m/z): 338.13 [M+H⁺].

### Example 181: 1-ethyl-3-[8-(5-methoxy-pyridin-2-ylamino)-isoquinolin-3-yl]-urea:

Starting from the compound of Preparation P and 2-bromo-5-methoxypyridine and proceeding in analogy to Procedure W, the title compound was obtained, after purification by prep-HPLC (basic conditions), as an amorphous solid (53% yield).
MS (ESI, m/z): 338.17 [M+H⁺].

### Example 182: 1H-pyrrole-2-carboxylic acid [3-(3-ethyl-ureido)-isoquinolin-8-yl]-amide hydrochloride:

Starting from the compound of Preparation P and pyrrole-2-carboxylic acid and proceeding in analogy to Procedure X, the title compound was obtained, after purification by prep-HPLC (acidic conditions) and treatment with HCl, as an amorphous solid (10% yield).
MS (ESI, m/z): 324.19 [M+H⁺].

### Example 183: 3H-[1,2,3]triazole-4-carboxylic acid [3-(3-ethyl-ureido)-isoquinolin-8-yl]-amide hydrochloride:

Starting from the compound of Preparation P and 3H-[1,2,3]triazole-4-carboxylic acid and proceeding in analogy to Procedure X, the title compound was obtained, after purification by prep-HPLC (acidic conditions) and treatment with HCl, as an amorphous solid (3% yield).
MS (ESI, m/z): 326.13 [M+H⁺].

### Example 184: 1-ethyl-3-{6-[(1H-indazol-6-ylmethyl)-amino]-8-methyl-isoquinolin-3-yl}-urea:

Starting from the compound of Example 54 and 1*H-*indazole-6-carboxaldehyde and proceeding in analogy to Procedure Q, the title compound was obtained, after purification by prep-HPLC (basic conditions), as an amorphous solid (59% yield).
MS (ESI, m/z): 375.23 [M+H⁺].

### Example 185: 1-ethyl-3-{8-methyl-6-[(2-morpholin-4-yl-pyridin-4-ylmethyl)-amino]-isoquinolin-3-yl}-urea:

Starting from the compound of Example 54 and 2-morpholinoisonicotinaldehyde and proceeding in analogy to Procedure Q, the title compound was obtained, after purification by prep-HPLC (basic conditions), as an amorphous solid (42% yield).
¹H NMR (d6-DMSO) δ: 10.10 (br. s, 1H); 8.70 (s, 1H); 8.03 (d, J = 5.4 Hz, 1H); 7.83 (br. s, 1H); 7.38-7.30 (m, 1H); 7.19 (s, 1H); 6.99 (s, 1H); 6.90 (s, 1H); 6.73 (d, J = 5.5 Hz, 1H); 6.42 (s, 1H); 4.42 (m, 2H); 3.72-3.65 (m, 4H); 3.51-3.42 (m, 4H); 3.21-3.10 (m, 2H); 2.51 (s, 3H); 1.07 (t, J = 7.2 Hz, 3H).
MS (ESI, m/z): 421.23 [M+H⁺].

### Example 186: 2-(4-{[3-(3-ethyl-ureido)-8-methyl-isoquinolin-6-ylamino]-methyl}-phenoxy)-acetamide:

Starting from the compound of Example 54 and 2-(4-formylphenoxy)acetamide and proceeding in analogy to Procedure Q, the title compound was obtained, after purification by prep-HPLC (basic conditions), as an amorphous solid (33% yield).
MS (ESI, m/z): 408.23 [M+H⁺].

### Example 187: 1-ethyl-3-[8-methyl-6-(3-[1,2,4]triazol-1-yl-benzylamino)-isoquinolin-3-yl]-urea:

Starting from the compound of Example 54 and 3-(1*H*-1,2,4-triazol-1-yl)benzaldehyde and proceeding in analogy to Procedure Q, the title compound was obtained, after purification by prep-HPLC (basic conditions), as an amorphous solid (44% yield).
MS (ESI, m/z): 402.22 [M+H⁺].

### Example 188: 1-{6-[(3-((1H-1,2,4-triazol-1-yl)methyl)benzyl)amino]-8-methylisoquinolin-3-yl}-3-ethylurea:

Starting from the compound of Example 54 and 3-(1*H-*1,2,4-triazol-1-ylmethyl)benzaldehyde and proceeding in analogy to Procedure Q, the title compound was obtained, after purification by prep-HPLC (basic conditions), as an amorphous solid (57% yield).
MS (ESI, m/z): 416.23 [M+H⁺].

### Example 189: 4-[3-(3-ethyl-ureido)-8-methyl-isoquinolin-6-ylamino]-methyl}-N-methyl-benzenesulfonamide:

Starting from the compound of Example 54 and 4-formyl-*N-*methylbenzenesulfonamide and proceeding in analogy to Procedure Q, the title compound was obtained, after purification by prep-HPLC (basic conditions), as an amorphous solid (38% yield).
MS (ESI, m/z): 428.18 [M+H⁺].

### Example 190: 1-ethyl-3-[8-(pyridin-3-ylaminomethyl)-isoquinolin-3-yl]-urea:

Starting from the compound of Preparation Q and 3-aminopyridine and proceeding in analogy to Procedure Y, the title compound was obtained, after purification by prep-HPLC (basic conditions), as an amorphous solid (12% yield).
MS (ESI, m/z): 322.13 [M+H⁺].

### Example 191: 1-{8-[(2,5-dimethyl-2H-pyrazol-3-ylamino)-methyl]-isoquinolin-3-yl}-3-ethyl-urea:

Starting from the compound of Preparation Q and 1,3-dimethyl-1*H-*pyrazol-5-amine and proceeding in analogy to Procedure Y, the title compound was obtained, after purification by prep-HPLC (basic conditions), as an amorphous solid (11% yield).
MS (ESI, m/z): 339.09 [M+H⁺].

### Example 192: 1-ethyl-3-{8-[(3-methyl-isothiazol-5-ylamino)-methyl]-isoquinolin-3-yl}-urea:

Starting from the compound of Preparation Q and 3-methyl-5-aminoisothiazole hydrochloride and proceeding in analogy to Procedure Z, the title compound was obtained, after purification by prep-HPLC (basic conditions), as an amorphous solid (10% yield).
MS (ESI, m/z): 341.86 [M+H⁺].

### Example 193: N-[3-(3-ethyl-ureido)-8-methyl-isoquinolin-6-yl]-terephthalamic acid methyl ester:

Starting from the compound of Example 54 and mono-methyl terephthalate and proceeding in analogy to Procedure K, the title compound was obtained, after purification by prep-HPLC (basic conditions), as an amorphous solid (25% yield).
MS (ESI, m/z): 407.46 [M+H⁺].

### Example 194: N-[3-(3-ethyl-ureido)-8-methyl-isoquinolin-6-yl]-isophthalamic acid

### methyl ester:

Starting from the compound of Example 54 and mono-methyl isophthalate and proceeding in analogy to Procedure K, the title compound was obtained, after purification by prep-HPLC (basic conditions), as an amorphous solid (53% yield).
¹H NMR (d6-DMSO) δ: 10.81 (s, 1H); 9.76 (br. s, 1H); 9.08 (s, 1H); 8.56 (t, J= 1.7 Hz, 1H); 8.30-8.24 (m, 2H); 8.20-8.15 (m, 1H); 7.79 (s, 1H); 7.72 (t, J = 7.8 Hz, 1H); 7.65-7.61 (s, 1H); 7.32 (br. s, 1H); 3.91 (s, 3H); 3.25-3.14 (m, 2H); 2.68 (s, 3H); 1.10 (t, J = 7.2 Hz, 3H).
MS (ESI, m/z): 407.47 [M+H⁺].

### Example 195: N-[3-(3-ethyl-ureido)-8-methyl-isoquinolin-6-yl]-2-methoxy-acetamide:

Starting from the compound of Example 54 and methoxyacetic acid and proceeding in analogy to Procedure K, the title compound was obtained, after purification by prep-HPLC (basic conditions), as an amorphous solid (42% yield).
¹H NMR (d6-DMSO) δ: 10.24 (s, 1H); 9.90 (br. s, 1H); 9.06 (s, 1H); 8.14 (s, 1H); 7.72 (s, 1H); 7.50 (s, 1H); 7.32 (br. s, 1H); 4.07 (s, 3H); 3.38 (s, 2H); 3.24-3.13 (m, 2H); 2.63 (s, 3H); 1.09 (t, J = 7.2 Hz, 3H).
MS (ESI, m/z): 317.22 [M+H⁺].

### Example 196: 1-methyl-3-{6-[(6-methoxy-pyridin-3-ylmethyl)-amino]-8-methyl-isoquinolin-3-yl}-urea hydrochloride:

Starting from the compound of Example 54 and 6-methoxy-3-pyridinecarboxaldehyde (3 eq.) and proceeding in analogy to Procedure P, shaking however the reaction mixture overnight at 50°C for the imine formation and then using NaBH₄ (4 eq.), the title compound was obtained, after purification by prep-HPLC (basic conditions) and treatment with HCl, as an amorphous solid (27% yield).
MS (ESI, m/z): 366.29 [M+H⁺].

### Example 197: 1-ethyl-3-[6-(3-methoxy-benzylamino)-8-methyl-isoquinolin-3-yl]-urea hydrochloride:

Starting from the compound of Example 54 and 3-methoxybenzaldehyde (3 eq.) and proceeding in analogy to Procedure P, shaking however the reaction mixture overnight at 50°C for the imine formation and then using NaBH₄ (4 eq.), the title compound was obtained, after purification by prep-HPLC (basic conditions) and treatment with HCl, as an amorphous solid (62% yield).
MS (ESI, m/z): 365.29 [M+H⁺].

### Example 198: 3-[3-(3-ethyl-ureido)-isoquinolin-8-ylamino]-benzoic acid:

### 198.1. 3-[3-(3-ethyl-ureido)-isoquinolin-8-ylamino]-benzoic acid tert-butyl ester:

Starting from the compound of Preparation K and *tert*-butyl 3-aminobenzoate and proceeding in analogy to Procedure AB, however using BrettPhos as catalyst instead, the title compound was obtained, after purification by CC (DCM/MeOH +1% NH₄OH 100:0 to 98:2), as a yellow solid (69% yield).
MS (ESI, m/z): 407.09 [M+H⁺].

### 198.2. 3-[3-(3-ethyl-ureido)-isoquinolin-8-ylamino]-benzoic acid:

Intermediate 198.1 was dissolved in TFA and stirred at rt for 2 h. The reaction mixture was concentrated under reduced pressure and the title compound was obtained, after purification by CC (DCM/MeOH 100:0 to 90:10), as an orange solid (55% yield).
¹H NMR (d6-DMSO) δ: 9.26 (s, 1H); 9.03 (s, 1H); 8.64 (s, 1H); 7.92 (s, 1H); 7.74 (s, 1H); 7.50-7.41 (m, 2H); 7.36-7.23 (m, 3H); 7.15-7.06 (m, 2H); 3.22-3.10 (m, 2H); 1.08 (t, J = 7.2 Hz, 3H). The COOH was not observed.
MS (ESI, m/z): 351.03 [M+H⁺].

### Example 199: 3-[3-(3-ethyl-ureido)-isoquinolin-8-ylamino]-N,N-dimethyl-benzamide hydrochloride:

Starting from the compound of Example 198 and dimethylamine and proceeding in analogy to Procedure AA, the title compound was obtained, after purification by prep-HPLC (acidic conditions) and treatment with HCl, as an amorphous solid (56% yield).
¹H NMR (d6-DMSO) δ: 9.65 (br. s, 1H); 9.36 (s, 1H); 7.87 (s, 1H); 7.57 (t, J = 8.0 Hz, 1H); 7.37-7.30 (m, 2H); 7.26-7.21 (m, 1H); 7.18-7.13 (m, 2H); 6.93 (dt, J = 7.4, 1.2 Hz, 1H); 3.18 (q, J = 7.2 Hz, 2H); 2.93 (s, 6H); 1.09 (t, J = 7.2 Hz, 3H). One NH, HCl and water were not observed.
MS (ESI, m/z): 378.26 [M+H⁺].

### Example 200: 3-[3-(3-ethyl-ureido)-isoquinolin-8-ylamino]-N-methyl-benzamide hydrochloride:

Starting from the compound of Example 198 and methylamine and proceeding in analogy to Procedure AA, the title compound was obtained, after purification by prep-HPLC (acidic conditions) and treatment with HCl, as an amorphous solid (62% yield).
MS (ESI, m/z): 364.23 [M+H⁺].

### Example 201: N-benzyl-3-[3-(3-ethyl-ureido)-isoquinolin-8-ylamino]-benzamide hydrochloride:

Starting from the compound of Example 198 and benzylamine and proceeding in analogy to Procedure AA, the title compound was obtained, after purification by prep-HPLC (acidic conditions) and treatment with HCl, as an amorphous solid (62% yield).
MS (ESI, m/z): 440.29 [M+H⁺].

### Example 202: 4-[3-(3-ethyl-ureido)-isoquinolin-8-ylamino]-benzoic acid:

### 202.1. 4-[3-(3-ethyl-ureido)-isoquinolin-8-ylamino]-benzoic acid tert-butyl ester:

Starting from the compound of Preparation K and tert-butyl 4-aminobenzoate and proceeding in analogy to Procedure AB, however using BrettPhos as catalyst instead, the title compound was obtained, after purification by CC (DCM/MeOH +1% NH₄OH 100:0 to 98:2), as a yellow solid (53% yield).
MS (ESI, m/z): 407.12 [M+H⁺].

### 202.2. 4-[3-(3-ethyl-ureido)-isoquinolin-8-ylamino]-benzoic acid:

Intermediate 202.1 was dissolved in TFA and stirred at rt for 2 h. The reaction mixture was concentrated under reduced pressure and the title compound was obtained, after trituration of the residue in 2:1 Et₂O/Hept, as an orange solid (97% yield).
¹H NMR (d6-DMSO) δ: 9.19 (s, 1H); 9.13 (s, 1H); 8.99 (br. s, 1H); 7.96 (s, 1 H); 7.83-7.76 (m, 2H); 7.60-7.52 (m, 1H); 7.46-7.40 (m, 1H); 7.31-7.25 (d, J = 7.4 Hz, 1H); 7.12-7.00 (m, 3H); 3.23-3.11 (m, 2H); 1.08 (t, J = 7.2 Hz, 3H). The COOH was not observed.
MS (ESI, m/z): 351.07 [M+H⁺].

### Example 203: 4-[3-(3-ethyl-ureido)-isoquinolin-8-ylamino]-N,N-dimethyl-benzamide hydrochloride:

Starting from the compound of Example 202 and dimethylamine and proceeding in analogy to Procedure AA, the title compound was obtained, after purification by prep-HPLC (acidic conditions) and treatment with HCl, as an amorphous solid (39% yield).
MS (ESI, m/z): 378.22 [M+H⁺].

### Example 204: 4-[3-(3-ethyl-ureido)-isoquinolin-8-ylamino]-N-methyl-benzamide hydrochloride:

Starting from the compound of Example 202 and methylamine and proceeding in analogy to Procedure AA, the title compound was obtained, after purification by prep-HPLC (acidic conditions) and treatment with HCl, as an amorphous solid (65% yield).
¹H NMR (d6-DMSO) δ: 9.67 (s, 1H); 9.33 (s, 1H); 8.21 (br. s, 1H); 7.90 (s, 1 H); 7.79-7.73 (m, 2H); 7.60 (t, J = 8.0 Hz, 1H); 7.41 (d, J = 8.3 Hz, 1H); 7.29-7.24 (m, 1H); 7.19-7.12 (m, 2H); 3.18 (q, J = 7.1 Hz, 2H); 2.75 (d, J = 2.1 Hz, 3H); 1.09 (t, J = 7.2 Hz, 3H). Two NH, HCl and water were not observed.
MS (ESI, m/z): 364.22 [M+H⁺].

### Example 205: {4-[3-(3-ethyl-ureido)-isoquinolin-8-ylamino]-phenyl}-acetic acid:

Starting from the compound of Preparation K and 4-aminophenylacetic acid and proceeding in analogy to Procedure AB, however using BrettPhos as catalyst instead, the title compound was obtained, after purification by prep-HPLC (acidic conditions), as an amorphous solid (62% yield).
¹H NMR (d6-DMSO) δ: 9.27 (s, 1H); 9.00 (s, 1H); 8.48 (s, 1H); 8.12 (s, 1H); 7.88 (s, 1H); 7.41 (t, J = 8.0 Hz, 1H); 7.21-7.02 (m, 6H); 3.48 (s, 2H); 3.24-3.12 (m, 2H); 1.08 (t, J = 7.2 Hz, 3H). The COOH was not observed.
MS (ESI, m/z): 365.10 [M+H⁺].

### Example 206: 2-{4-[3-(3-ethyl-ureido)-isoquinolin-8-ylamino]-phenyl}-N,N-dimethyl-acetamide hydrochloride:

Starting from the compound of Example 205 and dimethylamine and proceeding in analogy to Procedure AA, the title compound was obtained, after purification by prep-HPLC (acidic conditions) and treatment with HCl, as an amorphous solid (70% yield).
MS (ESI, m/z): 392.29 [M+H⁺].

### Example 207: 2-{4-[3-(3-ethyl-ureido)-isoquinolin-8-ylamino]-phenyl}-N-methylacetamide hydrochloride:

Starting from the compound of Example 205 and methylamine and proceeding in analogy to Procedure AA, the title compound was obtained, after purification by prep-HPLC (acidic conditions) and treatment with HCl, as an amorphous solid (72% yield).
¹H NMR (d6-DMSO) δ: 9.66 (br. s, 1H); 9.38 (s, 1H); 7.94-7.85 (m, 1H); 7.81 (s, 1H); 7.52 (t, J = 8.0 Hz, 1H); 7.26-7.11 (m, 6H); 7.05 (dd, J = 7.6, 0.4 Hz, 1H); 3.33 (s, 2H); 3.23-3.12 (m, 2H); 2.56 (d, J = 4.6 Hz, 3H); 1.09 (t, J = 7.1 Hz, 3H). One NH, HCl and water were not observed.
MS (ESI, m/z): 378.25 [M+H⁺].

### Example 208: N-benzyl-2-{4-[3-(3-ethyl-ureido)-isoquinolin-8-ylamino]-phenyl}-acetamide hydrochloride:

Starting from the compound of Example 205 and benzylamine and proceeding in analogy to Procedure AA, the title compound was obtained, after purification by prep-HPLC (acidic conditions) and treatment with HCl, as an amorphous solid (75% yield).
MS (ESI, m/z): 453.93 [M+H⁺].

### Example 209: {3-[3-(3-ethyl-ureido)-isoquinolin-8-ylamino]-phenyl}-acetic acid:

Starting from the compound of Preparation K and 3-aminophenylacetic acid and proceeding in analogy to Procedure AB, however using BrettPhos as catalyst instead, the title compound was obtained, after purification by prep-HPLC (acidic conditions), as an amorphous solid (95% yield).
¹H NMR (d6-DMSO) δ: 9.27 (s, 1H); 9.01 (s, 1H); 8.51 (s, 1H); 7.89 (s, 1H); 7.42 (t, J= 8.0 Hz, 1H); 7.24-7.16 (m, 2H); 7.13-7.00 (m, 4H); 6.79 (d, J= 7.6 Hz, 1H); 3.50 (s, 2H); 3.24-3.12 (m, 2H); 1.08 (t, J = 7.2 Hz, 3H). The COOH was not observed.
MS (ESI, m/z): 365.08 [M+H⁺].

### Example 210: N-benzyl-2-{3-[3-(3-ethyl-ureido)-isoquinolin-8-ylamino]-phenyl}-acetamide hydrochloride:

Starting from the compound of Example 209 and benzylamine and proceeding in analogy to Procedure AA, the title compound was obtained, after purification by prep-HPLC (acidic conditions) and treatment with HCl, as an amorphous solid (77% yield).
MS (ESI, m/z): 453.9 [M+H⁺].

### Example 211: 2-{3-[3-(3-ethyl-ureido)-isoquinolin-8-ylamino]-phenyl}-N,N-dimethyl-acetamide hydrochloride:

Starting from the compound of Example 209 and dimethylamine and proceeding in analogy to Procedure AA, the title compound was obtained, after purification by prep-HPLC (acidic conditions) and treatment with HCl, as an amorphous solid (90% yield).
MS (ESI, m/z): 392.27 [M+H⁺].

### Example 212: 4-[3-(3-ethyl-ureido)-isoquinolin-8-ylmethoxy]-nicotinic acid methyl ester hydrochloride:

Starting from the compound of Preparation R and methyl 4-hydroxynicotinate and proceeding in analogy to Procedure AC, the title compound was obtained, after purification by prep-HPLC (acidic conditions) and treatment with HCl, as an amorphous solid (21% yield).
MS (ESI, m/z): 381.28 [M+H⁺].

### Example 213: 1-ethyl-3-[8-(3-hydroxy-phenoxymethyl)-isoquinolin-3-yl]-urea hydrochloride:

Starting from the compound of Preparation R and resorcinol and proceeding in analogy to Procedure AC, the title compound was obtained, after purification by prep-HPLC (acidic conditions) and treatment with HCl, as an amorphous solid (46% yield).
MS (ESI, m/z): 338.18 [M+H⁺].

### Example 214: 1-ethyl-3-[8-(6-methoxy-pyridin-3-ylamino)-isoquinolin-3-yl]-urea:

Starting from the compound of Preparation K and 5-amino-2-methoxypyridine and proceeding in analogy to Procedure AB, the title compound was obtained, after purification by CC (DCM/MeOH +1% NH₄OH 100:0 to 98:2), as a yellow solid (73% yield).
¹H NMR (d6-DMSO) δ: 9.32 (s, 1H); 9.00 (s, 1H); 8.42 (s, 1H); 8.06 (d, J = 2.6 Hz, 1H); 7.87 (s, 1H); 7.62 (dd, J = 8.8, 2.7 Hz, 1H); 7.35 (t, J = 7.9 Hz, 1H); 7.14-7.05 (m, 2H); 6.82 (d, J= 8.8 Hz, 1H); 6.68 (d, J= 7.5 Hz, 1H); 3.83 (s, 3H); 3.22-3.11 (m, 2H); 1.08 (t, J = 7.2 Hz, 3H).
MS (ESI, m/z): 338.21 [M+H⁺].

### Example 215: 1-ethyl-3-(5-fluoro-8-pyridin-3-yl-isoquinolin-3-yl)-urea:

Starting from the compound of Example 32 and pyridine-3-boronic acid and proceeding in analogy to Procedure B, the title compound was obtained, after purification by prep-HPLC (acidic conditions), as an amorphous solid (16% yield).
MS (ESI, m/z): 311.4 [M+H⁺].

### Example 216: 1-ethyl-3-(8-methyl-6-vinyl-isoquinolin-3-yl)-urea:

Starting from the compound of Preparation B and vinylboronic anhydride pyridine complex and proceeding in analogy to Procedure C, the title compound was obtained, after purification by CC (Hept/EA 10:0 to 5:5), as a yellow solid (81% yield).
¹H NMR (d6-DMSO) δ: 9.05 (s, 1H); 9.02 (s, 1H); 7.91 (s, 1H); 7.54 (s, 1H); 7.42 (s, 1H); 7.17 (t, J = 5 .2 Hz, 1H); 6.83 (dd, J = 17.6, 11.0 Hz, 1H); 6.02 (d, J = 17.6 Hz, 1H); 5.41 (d, J = 11.0 Hz, 1H); 3.22-3.11 (m, 2H); 2.66 (s, 3H); 1.08 (t, J = 7.2 Hz, 3H).
MS (ESI, m/z): 256.15 [M+H⁺].

### Example 217: 1-ethyl-3-[8-(6-methoxy-pyridin-2-ylamino)-5-pyridin-4-yl-isoquinolin-3-yl]-urea:

Starting from the compound of Preparation L and 2-amino-6-methoxypyridine and proceeding in analogy to Procedure AB, the title compound was obtained, after purification by prep-HPLC (basic conditions), as a yellow solid (84% yield).
¹H NMR (d6-DMSO) δ: 9.40 (s, 1H); 9.34 (s, 1H); 9.05 (s, 1H); 8.71-8.65 (m, 2H); 8.10 (s, 1H); 8.05 (d, J = 8.0 Hz, 1H); 7.57 (d, J = 8.0 Hz, 2H); 7.54-7.47 (m, 2H); 7.08-7.02 (m, 1H); 6.68 (d, J = 7.8 Hz, 1H); 6.25 (d, J = 7.8 Hz, 1H); 3.78 (s, 3H); 3.17-3.05 (m, 2H); 1.04 (t, J = 7.2 Hz, 3H).
MS (ESI, m/z): 415.04 [M+H⁺].

### Example 218: 1-ethyl-3-[8-(6-methyl-pyridin-3-ylamino)-5-pyridin-4-yl-isoquinolin-3-yl]-urea:

Starting from the compound of Preparation L and 5-aminopicoline and proceeding in analogy to Procedure AB, however using BrettPhos as catalyst instead, the title compound was obtained, after purification by prep-HPLC (basic conditions), as a yellow solid (39% yield).
¹H NMR (d6-DMSO) δ: 9.40 (s, 1H); 9.06 (s, 1H); 8.83 (s, 1H); 8.71-8.62 (m, 2H); 8.41 (d, J = 1.9 Hz, 1H); 8.11 (s, 1H); 7.59 (dd, J = 8.4, 2.6 Hz, 1H); 7.51-7.42 (m, 3H); 7.24 (d, J = 8.4 Hz, 1H); 7.08-6.98 (m, 2H); 3.18-3.06 (m, 2H); 2.44 (s, 3H); 1.04 (t, J = 7.2 Hz, 3H).
MS (ESI, m/z): 399.00 [M+H⁺].

### Example 219: 1-ethyl-3-[8-(6-methyl-pyridin-2-ylamino)-5-pyridin-4-yl-isoquinolin-3-yl]-urea:

Starting from the compound of Preparation L and 2-amino-6-picoline and proceeding in analogy to Procedure AB, however using BrettPhos as catalyst instead and adding all reagents again after 21 h, the title compound was obtained, after purification by prep-HPLC (basic conditions), as a yellow solid (79% yield).
MS (ESI, m/z): 399.08 [M+H⁺].

### Example 220: 3-[3-(3-ethyl-ureido)-5-pyridin-4-yl-isoquinolin-8-ylamino]-benzoic

### acid methyl ester:

Starting from the compound of Preparation L and methyl 3-aminobenzoate and proceeding in analogy to Procedure AE, the title compound was obtained, after purification by prep-HPLC (basic conditions), as a yellow solid (9% yield).
¹H NMR (d6-DMSO) δ: 9.37 (s, 1H); 9.06 (s, 1H); 8.96 (s, 1H); 8.70-8.64 (m, 2H); 8.11 (s, 1H); 7.84-7.79 (m, 1H); 7.55-7.40 (m, 6H); 7.20 (d, J = 7.9 Hz, 1H); 7.06-6.99 (m, 1H); 3.71 (s, 3H); 3.18-3.04 (m, 2H); 1.04 (t, J = 7.2 Hz, 3H).
MS (ESI, m/z): 442.04 [M+H⁺].

### Example 221: 1-ethyl-3-[5-(4-hydroxymethyl-phenyl)-8-(pyridin-3-yloxy)-isoquinolin-3-yl]-urea:

Starting from the compound of Preparation T and 4-hydroxymethylphenylboronic acid and proceeding in analogy to Procedure AK, adding however a second time catalyst and ligand after 5 h, the title compound was obtained, after purification by CC (DCM/MeOH +1% NH₄OH 100:0 to 96:4), as a yellow solid (57% yield).
¹H NMR (d6-DMSO) δ: 9.31 (s, 1H); 9.09 (s, 1H); 8.57 (d, J = 2.7Hz, 1H); 8.45 (d, J = 4.5 Hz, 1H); 8.21 (s, 1H); 7.68-7.61 (m, 1H); 7.52-7.38 (m, 6H); 6.93 (t, J = 5.3 Hz, 1H); 6.78 (d, J = 7.9 Hz, 1H); 5.27 (t, J = 5.8 Hz, 1H); 4.59 (d, J = 5.8 Hz, 2H); 3.16-3.04 (m, 2H); 1.03 (t, J = 7.1 Hz, 3H).
MS (ESI, m/z): 415.18 [M+H⁺].

### Example 222: 1-ethyl-3-[5-pyridin-4-yl-8-(pyridin-3-yloxy)-isoquinolin-3-yl]-urea:

Starting from the compound of Preparation T and pyridine-4-boronic acid and proceeding in analogy to Procedure AK, the title compound was obtained, after purification by CC (DCM/MeOH +1% NH₄OH 100:0 to 97:3), as a yellow solid (60% yield).
¹H NMR (d6-DMSO) δ: 9.36 (s, 1H); 9.16 (s, 1H); 8.71 (d, J = 5.9 Hz, 2H); 8.59 (d, J = 2. 8 Hz, 1H); 8.48 (d, J = 4.6 Hz, 1H); 8.17 (s, 1H); 7.71-7.65 (m, 1H); 7.55 (d, J = 8.0 Hz, 1H); 7.54-7.47 (m, 3H); 6.97 (t, J = 5.6 Hz, 1H); 6.77 (d, J = 8.0 Hz, 1H); 3.17-3.05 (m, 2H); 1.04 (t, J = 7.2 Hz, 3H).
MS (ESI, m/z): 385.83 [M+H⁺].

### Example 223: 1-ethyl-3-[5-pyridin-3-yl-8-(pyridin-3-yloxy)-isoquinolin-3-yl]-urea:

Starting from the compound of Preparation T and pyridine-3-boronic acid and proceeding in analogy to Procedure AK, the title compound was obtained, after purification by CC (DCM/MeOH +1% NH₄OH 100:0 to 97:3) followed by prep-HPLC (basic conditions), as a yellow solid (61% yield).
MS (ESI, m/z): 386.11 [M+H⁺].

### Example 224: 1-ethyl-3-[5-pyridin-3-yl-8-(pyridin-3-ylamino)-isoquinolin-3-yl]-urea:

Starting from the compound of Preparation U and pyridine-3-boronic acid and proceeding in analogy to Procedure AK, the title compound was obtained, after purification by CC (DCM/MeOH +1% NH₄OH 100:0 to 95:5), as a yellow solid (85% yield).
MS (ESI, m/z): 385.23 [M+H⁺].

### Example 225: 1-ethyl-3-[5-(2-methoxy-pyridin-4-yl)-8-(pyridin-3-ylamino)-isoquinolin-3-yl]-urea:

Starting from the compound of Preparation U and 2-methoxypyridine-4-boronic acid and proceeding in analogy to Procedure AK, the title compound was obtained, after purification by prep-HPLC (basic conditions), as a yellow solid (79% yield).
MS (ESI, m/z): 415.07 [M+H⁺].

### Example 226: 1-ethyl-3-[5-(4-hydroxymethyl-phenyl)-8-(pyridin-3-ylamino)-isoquinolin-3-yl]-urea:

Starting from the compound of Preparation U and 4-hydroxymethylphenylboronic acid and proceeding in analogy to Procedure AK, the title compound was obtained, after purification by CC (DCM/MeOH +1% NH₄OH 100:0 to 95:5), as a yellow solid (50% yield).
¹H NMR (d6-DMSO) δ: 9.33 (d, J = 0.6 Hz, 1H); 9.00 (s, 1H); 8.75 (s, 1H); 8.50-8.47 (m, 1H); 8.15-8.09 (m, 2H); 7.55 (ddd, J = 8.3, 2.7, 1.4 Hz, 1H); 7.46-7.36 (m, 5H); 7.28 (dd, J = 8.3, 4.7 Hz, 1H); 7.16 (d, J = 7.9 Hz, 1H); 6.98 (t, J = 5.6 Hz, 1H); 5.25 (t, J = 5.8 Hz, 1H); 4.58 (d, J = 5.8 Hz, 2H); 3.17-3.04 (m, 2H); 1.03 (t, J = 7.2 Hz, 3H).
MS (ESI, m/z): 414.18 [M+H⁺].

### Example 227: 1-ethyl-3-[5-pyridin-4-yl-8-(pyridin-3-ylamino)-isoquinolin-3-yl]-urea:

Starting from the compound of Preparation U and pyridine-4-boronic acid and proceeding in analogy to Procedure AK, the title compound was obtained, after purification by prep-HPLC (basic conditions), as a yellow solid (58% yield).
¹H NMR (d6-DMSO) δ: 9.39 (d, J = 0.4 Hz, 1H); 9.07 (s, 1H); 8.90 (s, 1H); 8.70-8.64 (m, 2H); 8.52 (d, J = 2.6 Hz, 1H); 8.17 (dd, J = 4.6, 1.3 Hz, 1H); 8.11 (s, 1H); 7.62 (ddd, J= 8.3, 2.7, 1.4 Hz, 1H); 7.51-7.46 (m, 3H); 7.32 (ddd, J= 8.2, 4.7, 0.4 Hz, 1H); 7.15 (d, J = 8.0 Hz, 1H); 7.03 (t, J = 5.6 Hz, 1H); 3.17-3.06 (m, 2H); 1.04 (t, J = 7.1 Hz, 3H).
MS (ESI, m/z): 385.10 [M+H⁺].

### Example 228: 1-ethyl-3-[5-(4-hydroxy-phenyl)-8-(pyridin-3-ylamino)-isoquinolin-3-yl]-urea:

Starting from the compound of Preparation U and 4-hydroxyphenylboronic acid and proceeding in analogy to Procedure AK, the title compound was obtained, after purification by prep-HPLC (basic conditions), as a yellow solid (36% yield).
¹H NMR (d6-DMSO) δ: 9.51 (s, 1H); 9.30 (s, 1H); 8.99 (s, 1H); 8.68 (s, 1H); 8.46 (d, J = 2.6 Hz, 1H); 8.12-8.06 (m, 2H); 7.55-7.48 (m, 1H); 7.35 (d, J = 7.8 Hz, 1H); 7.29-7.20 (m, 3H); 7.14 (d, J = 7.8 Hz, 1H); 7.05 (t, J = 5.4 Hz, 1H); 6.91-6.84 (m, 2H); 3.17-3.06 (m, 2H); 1.04 (t, J = 7.2 Hz, 3H).
MS (ESI, m/z): 400.02 [M+H⁺].

### Example 229: 1-ethyl-3-[5-(2-methyl-pyridin-4-yl)-8-(pyridin-3-ylamino)-isoquinolin-3-yl]-urea:

Starting from the compound of Preparation U and 2-methylpyridine-4-boronic acid and proceeding in analogy to Procedure AK, the title compound was obtained, after purification by prep-HPLC (basic conditions), as a yellow solid (71% yield).
MS (ESI, m/z): 399.08 [M+H⁺].

### Example 230: 4-[3-(3-ethyl-ureido)-8-(pyridin-3-ylamino)-isoquinolin-5-yl]-benzenesulfonamide:

Starting from the compound of Preparation U and 4-aminosulfonylphenylboronic acid and proceeding in analogy to Procedure AK, the title compound was obtained, after purification by prep-HPLC (basic conditions), as a yellow solid (45% yield).
¹H NMR (d6-DMSO) δ: 9.38 (d, J = 0.7 Hz, 1H); 9.05 (s, 1H); 8.85 (br. s, 1H); 8.53-8.50 (m, 1H); 8.15 (dd, J = 4.6, 1.4 Hz, 1H); 8.12 (d, J = 0.4 Hz, 1H); 7.96-7.91 (m, 2H); 7.67-7.62 (m, 2H); 7.60 (ddd, J = 8.3, 2.8, 1.5 Hz, 1H); 7.48-7.42 (m, 3H); 7.31 (ddd, J = 8.2, 4.7, 0.5 Hz, 1H); 7.16 (d, J = 7.9 Hz, 1H); 6.98 (t, J = 5.0 Hz, 1H); 3.16-3.05 (m, 2H); 1.03 (t, J = 7.2 Hz, 3H).
MS (ESI, m/z): 463.08 [M+H⁺].

### Example 231: 1-[5-(2,6-dimethyl-pyridin-4-yl)-8-(pyridin-3-ylamino)-isoquinolin-3-yl]-3-ethyl-urea:

Starting from the compound of Preparation U and 2,6-dimethylpyridine-4-boronic acid pinacol ester and proceeding in analogy to Procedure AK, the title compound was obtained, after purification by prep-HPLC (basic conditions), as a yellow solid (80% yield).
MS (ESI, m/z): 413.12 [M+H⁺].

### Example 232: 1-(5-(3-(aminomethyl)phenyl)-8-(pyridin-3-ylamino)isoquinolin-3-yl)-3-ethylurea:

Starting from the compound of Preparation U and 3-aminomethylphenylboronic acid hydrochloride and proceeding in analogy to Procedure AK, the title compound was obtained, after purification by prep-HPLC (basic conditions), as a yellow solid (32% yield).
¹H NMR (d6-DMSO) δ: 9.34 (d, J = 0.4 Hz, 1H); 9.02 (s, 1H); 8.75 (s, 1H); 8.48 (d, J = 2.5 Hz, 1H); 8.14-8.08 (m, 2H); 7.55 (ddd, J = 8.3, 2.7, 1.4 Hz, 1H); 7.45-7.36 (m, 3H); 7.37-7.33 (m, 1H); 7.32-7.25 (m, 2H); 7.17 (d, J = 7.9 Hz, 1H); 7.14-7.08 (m, 1H); 3.79 (s, 2H); 3.16-3.05 (m, 2H); 1.04 (t, J = 7.2 Hz, 3H). The NH₂ was not observed.
MS (ESI, m/z): 413.02 [M+H⁺].

### Example 233: 1-[5-(2-amino-pyridin-4-yl)-8-(pyridin-3-ylamino)-isoquinolin-3-yl]-3-ethyl-urea:

Starting from the compound of Preparation U and 2-aminopyridine-4-boronic acid pinacol ester and proceeding in analogy to Procedure AK, the title compound was obtained, after purification by prep-HPLC (basic conditions), as a yellow solid (66% yield).
¹H NMR (d6-DMSO) δ: 9.35 (s, 1H); 9.05 (s, 1H); 8.81 (s, 1H); 8.50 (d, J = 2.5 Hz, 1H); 8.14 (dd, J = 4.6, 1.0 Hz, 1H); 8.10 (s, 1H); 7.97 (d, J = 5 .2 Hz, 1H); 7.62-7.5 (m, 1H); 7.39 (d, J = 7.9 Hz, 1H); 7.30 (dd, J = 8.3, 4.6 Hz, 1H); 7.13 (d, J = 7.9 Hz, 1H); 7.10-7.04 (m, 1H); 6.54 (dd, J = 5.2, 1.0 Hz, 1H); 6.47 (s, 1H); 5.97 (s, 2H); 3.18-3.06 (m, 2H); 1.05 (t, J = 7.2 Hz, 3H).
MS (ESI, m/z): 400.10 [M+H⁺].

### Example 234: N-{3-[3-(3-ethyl-ureido)-5-fluoro-8-(pyridin-3-ylamino)-isoquinolin-6-yl]-phenyl}-acetamide:

Starting from the compound of Preparation N and 3-aminopyridine and proceeding in analogy to Procedure AB, adding however all reagents again after 18 h and 42 h, the title compound was obtained, after purification by CC (DCM/MeOH +1% NH₄OH 100:0 to 97:3) followed by prep-HPLC (basic conditions), as a brown solid (12% yield).
¹H NMR (d6-DMSO) δ: 10.03 (d, J = 0.5 Hz, 1H); 9.28 (s, 1H); 9.25 (s, 1H); 8.67 (s, 1H); 8.44 (d, J = 2.6 Hz, 1H); 8.18 (s, 1H); 8.08 (d, J = 3.5 Hz, 1H); 7.86-7.81 (m, 1H); 7.66-7.60 (m, 1H); 7.53-7.47 (m, 1H); 7.41 (t, J = 7.8 Hz, 1H); 7.30-7.21 (m, 2H); 7.08-7.04 (m, 1H); 7.04-6.97 (m, 1H); 3.22-3.14 (m, 2H); 2.04 (s, 3H); 1.08 (t, J = 7.4 Hz, 3H).
MS (ESI, m/z): 458.88 [M+H⁺].

### Example 235: 1-ethyl-3-[5-fluoro-6-(5-methyl-pyridin-3-yl)-8-(pyridin-3-ylamino)-isoquinolin-3-yl]-urea:

Starting from intermediate 141.1 and 3-aminopyridine and proceeding in analogy to Procedure AE, using however (X-Phos) palladium(II) phenethylamine chloride and tBuOH instead, the title compound was obtained, after purification by CC (DCM/MeOH 100:0 to 90:10), as a yellow solid (55% yield).
¹H NMR (d6-DMSO) δ: 9.31 (s, 1H); 9.27 (s, 1H); 8.71 (s, 1H); 8.62 (s, 1H); 8.46 (s, 1H); 8.45 (s, 1H); 8.20 (s, 1H); 8.08 (dd, J = 4.6, 1.3 Hz, 1H); 7.88-7.84 (m, 1H); 7.56-7.51 (m, 1H); 7.25 (dd, J = 8.3, 4.6 Hz, 1H); 7.12 (d, J = 6.2 Hz, 1H); 6.99 (t, J = 5.3 Hz, 1H); 3.23-3.12 (m, 2H); 2.36 (s, 3H); 1.08 (t, J = 7.2 Hz, 3H).
MS (ESI, m/z): 417.20 [M+H⁺].

### Example 236: N-{3-[3-(3-ethyl-ureido)-8-(pyridin-3-ylamino)-isoquinolin-6-yl]-phenyl}-acetamide:

### 236.1. N-{3-[8-chloro-3-(3-ethyl-ureido)-isoquinolin-6-yl]phenyl}-acetamide:

Starting from compound of Preparation V and 3-acetylaminophenylboronic acid (1.2 eq.) and proceeding in analogy to Procedure C, the title compound was obtained, after purification by CC (DCM/MeOH +1% NH₄OH 100:0 to 97:3), as a yellow solid (41% yield).
MS (ESI, m/z): 383.09 [M+H⁺].

### 236.2. N-{3-[3-(3-ethyl-ureido)-8-(pyridin-3-ylamino)-isoquinolin-6-yl]-phenyl}-acetamide:

Starting from intermediate 236.1 and 3-aminopyridine and proceeding in analogy to Procedure AB, adding however all reagents again after 18 h, the title compound was obtained, after purification by prep-HPLC (basic conditions) followed by CC (DCM/MeOH +1% NH₄OH 100:0 to 90:10), as an orange solid (8% yield).
MS (ESI, m/z): 441.14 [M+H⁺].

### Example 237: 1-ethyl-3-[6-(5-methyl-pyridin-3-yl)-8-(pyridin-3-ylamino)-isoquinolin-3-yl]-urea:

### 237.1. 1-[8-chloro-6-(5-methyl-pyriding-3-y/)-isoquinolin-3-y/]-ethyl-urea:

Starting from compound of Preparation V and 5-methylpyridine-3-boronic acid (1.2 eq.) and proceeding in analogy to Procedure C, the title compound was obtained, after purification by CC (DCM/MeOH +1% NH₄OH 100:0 to 97:3), as a yellow solid (75% yield).
MS (ESI, m/z): 341.12 [M+H⁺].

### 237.2. 1-ethyl-3-[6-(5-methyl pyridin-3 yl)-8-(pyridin-3 ylamino)-isoquinolin-3-yl]-urea:

Starting from intermediate 237.1 and 3-aminopyridine and proceeding in analogy to Procedure AB, the title compound was obtained, after purification by CC (DCM/MeOH +1% NH₄OH 100:0 to 90:10), as an orange solid (74% yield).
¹H NMR (d6-DMSO) δ: 9.30 (s, 1H); 9.10 (s, 1H); 8.81 (s, 1H); 8.72-8.69 (m, 1H); 8.52 (d, J = 2.4 Hz, 1H); 8.44-8.41 (m, 1H); 8.13 (dd, J = 4.6, 0.6 Hz, 1H); 8.03 (s, 1H); 7.97-7.92 (m, 1H); 7.67-7.61 (m, 2H); 7.35-7.26 (m, 2H); 7.12-7.05 (m, 1H); 3.24-3.12 (m, 2H); 2.36 (s, 3H); 1.09 (t, J = 7.1 Hz, 3H).
MS (ESI, m/z): 399.15 [M+H⁺].

### Example 238: 1-ethyl-3-[6-propylamino-8-(pyridin-3-ylamino)-isoquinolin-3-yl]-urea:

### 238.1. 1-(8-chloro-6-propylamino-isoquinolin-3-yl)-3-ethyl-urea:

Starting from compound of Preparation V and propylamine (1.2 eq.) and proceeding in analogy to Procedure H, the title compound was obtained, after purification by CC (DCM/MeOH +1% NH₄OH 100:0 to 90:10) followed by prep-HPLC (basic conditions), as a yellow solid (31% yield).
MS (ESI, m/z): 307.10 [M+H⁺].

### 238.2. 1-ethyl-3-[6-propylamino-8-(pyridin-3-ylamino)-isoquinolin-3-yl]-urea:

Starting from intermediate 238.1 and 3-aminopyridine and proceeding in analogy to Procedure AB, the title compound was obtained, after purification by CC (DCM/MeOH +1% NH₄OH 100:0 to 90:10) followed by prep-HPLC (basic conditions), as an orange solid (50% yield).
¹H NMR (d6-DMSO) δ: 8.83 (s, 1H); 8.80 (s, 1H); 8.43 (d, J = 2.5 Hz, 1H); 8.34 (s, 1H); 8.10-8.05 (m, 1H); 7.65-7.56 (m, 1H); 7.51-7.44 (m, 1H); 7.35 (s, 1H); 7.26 (dd, J= 8.2, 4.7 Hz, 1H); 6.63-6.58 (m, 1H); 6.33-6.25 (m, 1H); 6.07-6.03 (m, 1H); 3.33-3.10 (m, 2H); 3.06-2.96 (m, 2H); 1.63-1.49 (m, 2H); 1.07 (t, J = 7.2 Hz, 3H); 0.93 (t, J = 7.3 Hz, 3H).
MS (ESI, m/z): 365.17 [M+H⁺].

### Example 239: 1-[6-benzylamino-8-(pyridin-3-ylamino)-isoquinolin-3-yl]-3-ethyl-urea:

### 239.1. 1-(6-benzylamino-8-chloro-isoquinolin-3-yl)-3-ethyl-urea:

Starting from compound of Preparation V and benzylamine (1.2 eq.) and proceeding in analogy to Procedure H, the title compound was obtained, after purification by CC (DCM/MeOH +1% NH₄OH 100:0 to 98:2), as a yellow solid (77% yield).
MS (ESI, m/z): 355.16 [M+H⁺].

### 239.2. 1-[6-benzylamino-8-(pyridin-3-ylamino)-isoquinolin-3-yl]-3-ethyl-urea:

Starting from intermediate 239.1 and 3-aminopyridine and proceeding in analogy to Procedure AB, the title compound was obtained, after purification by CC (DCM/MeOH +1% NH₄OH 100:0 to 90:10) followed by prep-HPLC (basic conditions), as an orange solid (74% yield).
¹H NMR (d6-DMSO) δ: 8.85 (s, 1H); 8.78 (s, 1H); 8.42 (dd, J = 2.6, 0.4 Hz, 1H); 8.38 (s, 1H); 8.08 (dd, J = 4.6, 1.3 Hz, 1H); 7.55-7.48 (m, 1H); 7.46-7.41 (m, 1H); 7.37-7.28 (m, 5H); 7.26-7.18 (m, 2H); 6.94-6.88 (m, 1H); 6.67 (d, J = 1.7 Hz, 1H); 6.06 (d, J = 1.2 Hz, 1H); 4.32 (d, J = 5.7 Hz, 2H); 3.19-3.08 (m, 2H); 1.06 (t, J = 7.2 Hz, 3H).
MS (ESI, m/z): 413.10 [M+H⁺].

### Example 240: 1-[6-benzylamino-5-fluoro-8-(pyridin-3-ylamino)-isoquinolin-3-yl]-3-ethyl-urea:

Starting from the compound of Example 49 and 3-aminopyridine and proceeding in analogy to Procedure AB, the title compound was obtained, after purification by CC (DCM/MeOH +1% NH₄OH 100:0 to 90:10) followed by prep-HPLC (basic conditions), as an orange solid (48% yield).
MS (ESI, m/z): 431.00 [M+H⁺].

### Example 241: 3-dimethylamino-N-[3-(3-ethyl-ureido)-8-(pyridin-3-ylamino)-isoquinolin-6-yl]-benzamide:

### 241.1. 1-(6-amino-8-chloro-isoquinolin-3-yl)-3-ethyl-urea:

To the compound of Preparation V (150 mg), copper(I) iodide (35 mg), L-4-hydroxyproline (50 mg) and K₂CO₃ (95 mg) in a glass vial, under inert atmosphere (N₂), was added dry DMSO (3.5 mL) and a 30% ammonium hydroxide solution (0.5 mL). The reaction mixture was purged with N₂ for 5 min, heated to 80°C and stirred overnight. The reaction mixture was cooled down to rt and concentrated under reduced pressure. The title compound was obtained, after purification by CC (DCM/MeOH +1% NH₄OH 100:0 to 95:5), as a yellow solid (65 mg; 54% yield).
MS (ESI, m/z): 265.06 [M+H⁺].

### 241.2. N-[8-chloro-3-(3-ethyl-ureido)-isoquinolin-6-yl]-3-dimethylamino-benzamide:

Starting from intermediate 241.1 and 3-(dimethylamino)benzoic acid and proceeding in analogy to Procedure X, adding however all reagents again after 18 h, the title compound was obtained, after purification by CC (DCM/MeOH +1% NH₄OH 100:0 to 90:10), as an unpure brown solid which was used in the next step.
MS (ESI, m/z): 412.12 [M+H⁺].

### 241.3. 3-dimethylamino-N-[3-(3-ethyl-ureido)-8-(pyridin-3-ylamino)-isoquinolin-6-yl]-benzamide:

Starting from intermediate 241.2 and 3-aminopyridine and proceeding in analogy to Procedure AB, the title compound was obtained, after purification by two CCs (DCM/MeOH +1% NH₄OH 100:0 to 90:10), as a yellow solid (12% yield over 2 steps).
MS (ESI, m/z): 470.11 [M+H⁺].

### Example 242: 5-[3-(3-ethyl-ureido)-isoquinolin-8-ylamino]-2-methoxy-benzamide:

Starting from the compound of Preparation K and 5-amino-2-methoxybenzamide and proceeding in analogy to Procedure AB, using however BrettPhos as catalyst instead, the title compound was obtained, after purification by prep-HPLC (basic conditions), as an amorphous solid (32% yield).
MS (ESI, m/z): 380.09 [M+H⁺].

### Example 243: 5-[3-(3-ethyl-ureido)-isoquinolin-8-ylamino]-2-methoxy-N-methylbenzamide:

Starting from the compound of Preparation K and 5-amino-2-methoxy-*N*-methylbenzamide hydrochloride and proceeding in analogy to Procedure AB, however using BrettPhos as catalyst instead, the title compound was obtained, after purification by prep-HPLC (basic conditions), as an amorphous solid (53% yield).
¹H NMR (d6-DMSO) δ: 9.30 (s, 1H); 8.99 (s, 1H); 8.45 (s, 1H); 8.20-8.12 (m, 1H); 7.86 (s, 1H); 7.66 (d, J = 2.9 Hz, 1H); 7.37 (t, J = 8.0 Hz, 1H); 7.30 (dd, J = 8.8, 2.9 Hz, 1H); 7.15-7.07 (m, 3H); 6.84 (d, J = 7.4 Hz, 1H); 3.86 (s, 3H); 3.23-3.11 (m, 2H); 2.79 (d, J = 4.7 Hz, 3H); 1.08 (t, J = 7.2 Hz, 3H).
MS (ESI, m/z): 393.86 [M+H⁺].

### Example 244: 5-[3-(3-ethyl-ureido)-isoquinolin-8-ylamino]-2-methoxy-N,N-dimethyl-benzamide:

Starting from the compound of Preparation K and 5-amino-2-methoxy-*N,N*-dimethylbenzamide and proceeding in analogy to Procedure AB, using however BrettPhos as catalyst instead, the title compound was obtained, after purification by prep-HPLC (basic conditions), as an amorphous solid (42% yield).
MS (ESI, m/z): 408.08 [M+H⁺].

### Example 245: 1-ethyl-3-[5-(2-methyl-pyridin-4-yl)-8-(6-methyl-pyridin-3-ylamino)-isoquinolin-3-yl]-urea:

### 245.1. 1-[5-chloro-8-(6-methyl-pyridin-3-ylamino)-isoquinolin-3-yl]-3-ethyl-urea:

Starting from compound of Preparation S and 5-aminopicoline and proceeding in analogy to Procedure AB, using however BrettPhos as catalyst instead, the title compound was obtained, after purification by CC (DCM/MeOH +1% NH₄OH 100:0 to 95:5), as a yellow solid (40% yield).
MS (ESI, m/z): 356.04 [M+H⁺].

### 245.2. 1-ethyl-3-[5-(2-methyl-pyridin-4-yl)-8-(6-methyl-pyridin-3-ylamino)-isoquinolin-3-yl]-urea:

Starting from intermediate 245.1 and 2-methylpyridine-4-boronic acid and proceeding in analogy to Procedure AK, the title compound was obtained, after purification by prep-HPLC (basic conditions), as a yellow solid (67% yield).
MS (ESI, m/z): 413.07 [M+H⁺].

### Example 246: 3-[3-(3-ethyl-ureido)-5-(2-methyl-pyridin-4-yl)-isoquinolin-8-ylamino]-benzoic acid:

### 246.1. 3-[5-chloro-3-(3-ethyl-ureido)-isoquinolin-8-ylamino]-benzoic acid tert-butyl ester:

Starting from compound of Preparation S and *tert*-butyl 3-aminobenzoate and proceeding in analogy to Procedure AB, however using BrettPhos as catalyst instead, the title compound was obtained, after purification by CC (DCM/MeOH +1% NH₄OH 100:0 to 98:2), as a yellow solid (23% yield).
MS (ESI, m/z): 440.92 [M+H⁺].

### 246.2. 3-[3-(3-ethyl-ureido)-5-(2-methyl-pyridin-4-yl)-isoquinolin-8-ylamino]-benzoic acid tert-butyl ester:

Starting from intermediate 246.1 and 2-methylpyridine-4-boronic acid and proceeding in analogy to Procedure AK, the title compound was obtained, after purification by CC (DCM/MeOH +1% NH₄OH 100:0 to 95:5), as a yellow solid (84% yield).
MS (ESI, m/z): 498.13 [M+H⁺].

### 246.3. 3-[3-(3-ethyl-ureido)-5-(2-methyl-pyridin-4-yl)-isoquinolin-8-ylamino]-benzoic acid:

Intermediate 246.2 was dissolved in TFA and stirred at rt for 2 h. The reaction mixture was concentrated under reduced pressure and the title compound was obtained, after purification by prep-HPLC (basic conditions), as a yellow solid (87% yield).
MS (ESI, m/z): 441.89 [M+H⁺].

### Example 247: 3-[3-(3-ethyl-ureido)-5-(2-methyl-pyridin-4-yl)-isoquinolin-8-ylamino]-N-methyl-benzamide:

Starting from the compound of Example 246 and a solution of methylamine in THF and proceeding in analogy to Procedure AL, the title compound was obtained, after purification by prep-HPLC (basic conditions), as a yellow solid (19% yield).
MS (ESI, m/z): 455.06 [M+H⁺].

### Example 248: 3-[3-(3-ethyl-ureido)-5-(2-methyl-pyridin-4-yl)-isoquinolin-8-ylamino]-N,N-dimethyl-benzamide:

Starting from the compound of Example 246 and a solution of dimethylamine in THF and proceeding in analogy to Procedure AL, the title compound was obtained, after purification by prep-HPLC (basic conditions), as a yellow solid (88% yield).
¹H NMR (d6-DMSO) δ: 9.36 (s, 1H); 9.06 (s, 1H); 8.84 (s, 1H); 8.52 (d, J = 5.2 Hz, 1H); 8.06 (s, 1H); 7.47 (d, J = 7.9 Hz, 1H); 7.39-7.32 (m, 2H); 7.30-7.24 (m, 2H); 7.20-7.16 (m, 2H); 7.13 (t, J = 5.4 Hz, 1H); 6.94 (dt, J = 7.3, 1.2 Hz, 1H); 3.18-3.07 (m, 2H); 2.94 (s, 6H); 2.53 (s, 3H); 1.04 (t, J = 7.2 Hz, 3H).
MS (ESI, m/z): 469.06 [M+H⁺].

### Example 249: 3-[3-(3-ethyl-ureido)-5-(4-hydroxymethyl-phenyl)-isoquinolin-8-ylamino]-benzoic acid:

### 249.1. 3-[3-(3-ethyl-ureido)-5-(4-hydroxymethyl-phenyl)-isoquinolin-8-ylamino]-benzoic acid tert-butyl ester:

Starting from intermediate 246.1 and 4-hydroxymethylphenylboronic acid and proceeding in analogy to Procedure AK, the title compound was obtained, after purification by CC (DCM/MeOH +1% NH₄OH 100:0 to 96:4), as a yellow solid (81% yield).
MS (ESI, m/z): 513.01 [M+H⁺].

### 249.2. 3-[3-(3-ethyl-ureido)-5-(4-hydroxymethyl-phenyl)-isoquinolin-8-ylamino]-benzoic acid:

Intermediate 249.1 was dissolved in TFA and stirred at rt for 2 h. The reaction mixture was concentrated under reduced pressure and the title compound was obtained, after purification by prep-HPLC (basic conditions), as an orange solid (84% yield).
MS (ESI, m/z): 456.83 [M+H⁺].

### Example 250: 3-[3-(3-ethyl-ureido)-5-(4-hydroxymethyl-phenyl)-isoquinolin-8-ylamino]-N-methyl-benzamide:

Starting from the compound of Example 249 and a solution of methylamine in THF and proceeding in analogy to Procedure AL, the title compound was obtained, after purification by prep-HPLC (basic conditions), as a yellow solid (51% yield).
¹H NMR (d6-DMSO) δ: 9.33 (d, J = 0.4 Hz, 1H); 8.97 (s, 1H); 8.72 (s, 1H); 8.37-8.29 (m, 1H); 8.12 (s, 1H); 7.68-7.64 (m, 1H); 7.46-7.25 (m, 8H); 7.17 (d, J = 7.9 Hz, 1H); 7.00 (t, J = 5.4 Hz, 1H); 5.24 (t, J = 5.8 Hz, 1H); 4.58 (d, J = 5.7 Hz, 2H); 3.17-3.05 (m, 2H); 2.74 (d, J = 4.5 Hz, 3H); 1.03 (t, J = 7.2 Hz, 3H).
MS (ESI, m/z): 470.03 [M+H⁺].

### Example 251: 3-[3-(3-ethyl-ureido)-5-(4-hydroxymethyl-phenyl)-isoquinolin-8-ylamino]-N,N-dimethyl-benzamide:

Starting from the compound of Example 249 and a solution of dimethylamine in THF and proceeding in analogy to Procedure AL, the title compound was obtained, after purification by prep-HPLC (basic conditions), as a yellow solid (77% yield).
MS (ESI, m/z): 484.07 [M+H⁺].

### Example 252: 4-[3-(3-ethyl-ureido)-isoquinolin-8-ylamino]-benzamide hydrochloride:

Starting from the compound of Example 202 and a solution of ammonia in dioxane and proceeding in analogy to Procedure AA, adding however ammonia (4.0 eq.) and HATU (0.5 eq.) again after 20 h, the title compound was obtained, after purification by prep-HPLC (acidic conditions) and treatment with HCl, as an amorphous solid (53% yield).
MS (ESI, m/z): 350.19 [M+H⁺].

### Example 253: 2-{4-[3-(3-ethyl-ureido)-isoquinolin-8-ylamino]-phenyl}-acetamide hydrochloride:

Starting from the compound of Example 205 and a solution of ammonia in dioxane and proceeding in analogy to Procedure AA, adding however ammonia (4.0 eq.) and HATU (0.5 eq.) again after 20 h, the title compound was obtained, after purification by prep-HPLC (acidic conditions) and treatment with HCl, as an amorphous solid (80% yield).
MS (ESI, m/z): 364.27 [M+H⁺].

### Example 254: 3-[3-(3-ethyl-ureido)-isoquinolin-8-ylamino]-benzamide hydrochloride:

Starting from the compound of Example 198 and a solution of ammonia in dioxane and proceeding in analogy to Procedure AA, adding however ammonia (4.0 eq.) and HATU (0.5 eq.) again after 20 h, the title compound was obtained, after purification by prep-HPLC (acidic conditions) and treatment with HCl, as an amorphous solid (48% yield).
MS (ESI, m/z): 350.19 [M+H⁺].

### Example 255: 2-{3-[3-(3-ethyl-ureido)-isoquinolin-8-ylamino]-phenyl}-acetamide hydrochloride:

Starting from the compound of Example 209 and a solution of ammonia in dioxane and proceeding in analogy to Procedure AA, adding however ammonia (4.0 eq.) and HATU (0.5 eq.) again after 20 h, the title compound was obtained, after purification by prep-HPLC (acidic conditions) and treatment with HCl, as an amorphous solid (40% yield).
MS (ESI, m/z): 364.21 [M+H⁺].

### Example 256: 1-ethyl-3-[8-(6-methyl-pyridin-3-yloxymethyl)-isoquinolin-3-yl]-urea hydrochloride:

Starting from the compound of Preparation R and 5-hydroxy-2-methylpyridine and proceeding in analogy to Procedure AC, the title compound was obtained, after purification by prep-HPLC (acidic conditions) and treatment with HCl, as an amorphous solid (34% yield).
MS (ESI, m/z): 337.20 [M+H⁺].

### Example 257: 1-ethyl-3-[8-(5-methyl-pyridin-2-yloxymethyl)-isoquinolin-3-yl]-urea hydrochloride:

Starting from the compound of Preparation R and 2-hydroxy-5-methylpyridine and proceeding in analogy to Procedure AC, the title compound was obtained, after purification by prep-HPLC (acidic conditions) and treatment with HCl, as an amorphous solid (55% yield).
MS (ESI, m/z): 337.20 [M+H⁺].

### Example 258: 2-[3-(3-ethyl-ureido)-isoquinolin-8-ylmethoxy]-benzoic acid methyl ester hydrochloride:

Starting from the compound of Preparation R and methyl salicylate and proceeding in analogy to Procedure AC, the title compound was obtained, after purification by prep-HPLC (acidic conditions) and treatment with HCl, as an amorphous solid (50% yield).
MS (ESI, m/z): 380.19 [M+H⁺].

### Example 259: 1-ethyl-3-[8-(2-methyl-pyridin-3-yloxymethyl)-isoquinolin-3-yl]-urea hydrochloride:

Starting from the compound of Preparation R and 3-hydroxy-2-methylpyridine and proceeding in analogy to Procedure AC, the title compound was obtained, after purification by prep-HPLC (acidic conditions) and treatment with HCl, as an amorphous solid (60% yield).
¹H NMR (d6-DMSO) δ: 9.32 (br. s, 1H); 9.29 (s, 1H); 8.41-8.33 (m, 2H); 8.08 (s, 1H); 7.93-7.80 (m, 2H); 7.68-7.56 (m, 2H); 7.19 (br. s, 1H); 5.86 (s, 2H); 3.21-3.12 (m, 2H); 2.59 (s, 3H); 1.08 (t, J = 7.2 Hz, 3H). HCl and water were not observed.
MS (ESI, m/z): 337.20 [M+H⁺].

### Example 260: 1-[8-(3-amino-phenoxymethyl)-isoquinolin-3-yl]-3-ethyl-urea hydrochloride:

Starting from the compound of Preparation R and 3-aminophenol and proceeding in analogy to Procedure AC, the title compound was obtained, after purification by prep-HPLC (acidic conditions) and treatment with HCl, as an amorphous solid (64% yield).
¹H NMR (d6-DMSO) δ: 9.30 (s, 1H); 9.22 (s, 1H); 8.04 (s, 1H); 7.81-7.76 (m, 1H); 7.66-7.59 (m, 1H); 7.53 (dd, J = 6.7, 0.4 Hz, 1H); 7.41 (t, J = 8.2 Hz, 1H); 7.15 (dd, J = 8.2, 2.1 Hz, 1H); 7.03 (t, J = 2.1 Hz, 1H); 6.97-6.92 (m, 1H); 5.63 (s, 2H); 3.16 (d, J = 7.2 Hz, 2H); 1.08 (t, J = 7.2 Hz, 3H). Three NH, HCl and water were not observed.
MS (ESI, m/z): 337.21 [M+H⁺].

### Example 261: 1-ethyl-3-[8-(pyridin-2-yloxymethyl)-isoquinolin-3-yl]-urea hydrochloride:

Starting from the compound of Preparation R and 2-hydroxypyridine and proceeding in analogy to Procedure AC, the title compound was obtained, after purification by prep-HPLC (acidic conditions) and treatment with HCl, as an amorphous solid (46% yield).
MS (ESI, m/z): 323.19 [M+H⁺].

### Example 262: 1-ethyl-3-[8-(pyridin-4-yloxymethyl)-isoquinolin-3-yl]-urea hydrochloride:

Starting from the compound of Preparation R and 4-hydroxypyridine and proceeding in analogy to Procedure AC, the title compound was obtained, after purification by prep-HPLC (acidic conditions) and treatment with HCl, as an amorphous solid (55% yield).
MS (ESI, m/z): 323.18 [M+H⁺].

### Example 263: 1-ethyl-3-[8-(pyridin-2-ylaminomethyl)-isoquinolin-3-yl]-urea hydrochloride:

Starting from the compound of Preparation R and 2-aminopyridine and proceeding in analogy to Procedure AM, adding however DIPEA (2.2 eq.) and heating at 100°C, the title compound was obtained, after purification by prep-HPLC (acidic conditions) and treatment with HCl, as an amorphous solid (12% yield).
MS (ESI, m/z): 322.20 [M+H⁺].

### Example 264: 5-[3-(3-ethyl-ureido)-isoquinolin-8-ylamino]-2-methyl-benzoic acid methyl ester:

Starting from the compound of Preparation K and methyl 5-amino-2-methylbenzoate and proceeding in analogy to Procedure AB, however using BrettPhos as catalyst instead, the title compound was obtained, after purification by CC (DCM/MeOH +1% NH₄OH 100:0 to 95:5) followed by prep-HPLC (acidic conditions), as an amorphous solid (2% yield).
MS (ESI, m/z): 379.13 [M+H⁺].

### Example 265: 5-[3-(3-ethyl-ureido)-isoquinolin-8-ylamino]-2-methyl-benzoic acid:

Starting from the compound of Preparation K and methyl 5-amino-2-methylbenzoate and proceeding in analogy to Procedure AB, however using BrettPhos as catalyst instead, the title compound was obtained, after purification by CC (DCM/MeOH +1% NH₄OH 100:0 to 90:10) followed by prep-HPLC (acidic conditions), as an amorphous solid (3% yield).
¹H NMR (d6-DMSO) δ: 12.73 (s, 1H); 9.26 (s, 1H); 9.01 (s, 1H); 8.56 (s, 1H); 7.90 (s, 1H); 7.64 (d, J = 2.3 Hz, 1H); 7.43 (t, J = 7.9 Hz, 1H); 7.27-7.16 (m, 3H); 7.12-7.05 (m, 1H); 7.03 (d, J = 7.5 Hz, 1H); 3.22-3.11 (m, 2H); 2.44 (s, 3H); 1.08 (t, J = 7.2 Hz, 3H).
MS (ESI, m/z): 365.14 [M+H⁺].

### Example 266: 4-[3-(3-ethyl-ureido)-isoquinolin-8-ylamino]-2-methyl-benzoic acid methyl ester:

Starting from the compound of Preparation K and methyl 4-amino-2-methylbenzoate and proceeding in analogy to Procedure AB, however using BrettPhos as catalyst instead, the title compound was obtained, after purification by CC (DCM/MeOH +1% NH₄OH 100:0 to 90:10) followed by prep-HPLC (acidic conditions), as an amorphous solid (24% yield).
¹H NMR (d6-DMSO) δ: 9.17 (s, 1H); 9.05 (s, 1H); 8.89 (s, 1H); 7.97 (s, 1H); 7.77 (d, J= 8.3 Hz, 1H); 7.58-7.51 (m, 1H); 7.45-7.39 (m, 1H); 7.26 (d, J= 7.2 Hz, 1 H); 7.03 (t, J= 5.3 Hz, 1H); 6.94-6.88 (m, 2H); 3.74 (s, 3H); 3.22-3.10 (m, 2H); 2.46 (s, 3H); 1.07 (t, J = 7.2 Hz, 3H).
MS (ESI, m/z): 379.14 [M+H⁺].

### Example 267: 4-[3-(3-ethyl-ureido)-isoquinolin-8-ylamino]-2-methyl-benzoic acid:

1*N* NaOH (4 eq.) was added to a suspension of the compound of Example 266 (0.1 mmol; 1.0 eq.) in dry dioxane (1.0 mL), in a glass vial, under inert atmosphere (N₂), at rt. The reaction mixture was stirred for 1 h at 100°C, cooled down to rt and concentrated under reduced pressure. The residue was taken in water and acidified to pH 4.5 with 1*N* HCl. A precipitate appeared and was filtered. The title compound was obtained, after trituration of the solid in Et₂O, as an orange solid (74% yield).
MS (ESI, m/z): 365.14 [M+H⁺].

### Example 268: 1-[5,8-bis-(pyridin-3-ylamino)-isoquinolin-3-yl]-3-ethyl-urea:

Starting from the compound of Preparation S and 3-aminopyridine and proceeding in analogy to Procedure AB, the title compound was obtained, after purification by CC (DCM/MeOH +1% NH₄OH 100:0 to 96:4) followed by prep-HPLC (basic conditions), as an amorphous solid (3% yield).
MS (ESI, m/z): 400.06 [M+H⁺].

### Example 269: 1-ethyl-3-(8-methyl-5-methylaminomethyl-isoquinolin-3-yl)-urea:

### 269.1. 1-ethyl-3-(8-methyl-5-vinyl-isoquinolin-3 yl)-urea:

Starting from the compound of Example 1 (1.0 g) and vinylboronic anhydride pyridine complex (390 mg) and proceeding in analogy to Procedure B, the title compound was obtained, after purification by CC (Hept/EA 100:0 to 40:60), as a yellow solid (793 mg; 96% yield).
¹H NMR (d6-DMSO) δ: 9.14 (d, J= 0.6 Hz, 1H); 9.06 (s, 1H); 8.26 (s, 1H); 7.69 (d, J = 7.3 Hz, 1H); 7.28-7.16 (m, 2H); 7.13 (t, J = 5.6 Hz, 1H); 5.82 (dd, J = 17.4, 1.4 Hz, 1H); 5.46 (dd, J= 11.0, 1.4 Hz, 1H); 3.22-3.12 (m, 2H); 2.67 (s, 3H); 1.08 (t, J= 7.2 Hz, 3H).
MS (ESI, m/z): 256.2 [M+H⁺].

### 269.2. 1-ethyl-3-(5-formyl-8-methyl-isoquinolin-3-yl)-urea:

Starting from intermediate 269.1 (581 mg) and proceeding in analogy to Procedure AD, the title compound was obtained, without additional purification, as a yellow solid (391 mg; 67% yield).
¹H NMR (d6-DMSO) δ: 10.19 (s, 1H); 9.26 (d, J = 0.8 Hz, 1H); 9.24-9.21 (m, 2H); 8.16 (d, J = 7.3 Hz, 1H); 7.42 (dd, J = 7.2, 0. Hz, 1H); 7.23 (t, J = 5.4 Hz, 1H); 3.24-3.15 (m, 2H); 2.77 (s, 3H); 1.09 (t, J = 7.2 Hz, 3H).
MS (ESI, m/z): 258.3 [M+H⁺].

### 269.3. 1-ethyl-3-(8-methyl-5-methylaminomethyl-isoquinolin-3-yl)-urea:

To a solution of intermediate 269.2 (50 mg) in 1:1 DCE/MeOH (2 mL) were added 2.0*M* methylamine in THF (0.1 mL, 1.0 eq.) and 3A molecular sieves at rt. The reaction mixture was stirred at rt overnight, cooled to 0°C and treated with NaBH₄. The reaction mixture was stirred at rt for 1 h and diluted with 9:1 DCM/MeOH and sat. aq. NaHCO₃ solution. The layers are separated and the aq. layer is extracted with 9:1 DCM/MeOH (3x). The combined org. layers are washed with brine, dried over MgSO₄, filtered and concentrated under reduced pressure. The title compound was obtained, after purification by prep-HPLC (basic conditions), as an amorphous solid (2.3 mg; 5% yield).
¹H NMR (d6-DMSO) δ: 9.16 (s, 1H); 9.08 (s, 1H); 8.13 (s, 1H); 7.59 (d, J = 7.2 Hz, 1H); 7.26-7.18 (m, 2H); 6.15 (br. s, 1H); 4.19 (dd, J = 14.1,3.3 Hz, 1H); 3.79 (dd, J = 14.1, 9.1 Hz, 1H); 3.24-3.12 (m, 2H); 2.68 (s, 3H); 2.17 (d, J = 5.3 Hz, 3H); 1.09 (t, J = 7.2 Hz, 3H).
MS (ESI, m/z): 273.22 [M+H⁺].

### Example 270: 1-ethyl-3-{5-[(methylamino)methyl]-8-(pyridin-3-ylamino)-isoquinolin-3-yl}-urea:

### 270.1. 1-{8-chloro-5-[(methylamino)methyl]-isoquinolin-3-yl}-3-ethyl-urea:

Starting from the compound of Preparation W and 2*M* methylamine in THF and proceeding in analogy to Procedure AN, the title compound was obtained, after purification by CC (DCM/MeOH +1% NH₄OH 100:0 to 95:5), as a beige solid (47% yield).
¹H NMR (d6-DMSO) δ: 9.22 (s, 1H); 9.18 (s, 1H); 8.29 (s, 1H); 7.61-7.55 (m, 1H); 7.48-7.43 (m, 1H); 7.04 (t, J = 5.5 Hz, 1H); 3.92 (s, 2H); 3.23-3.11 (m, 2H); 2.34 (s, 3H); 2.11 (br. s, 1H); 1.08 (t, J = 7.2 Hz, 3H).
MS (ESI, m/z): 293.05 [M+H⁺].

### 270.2. 1-ethyl-3-{5-[(methylamino)methyl]-8-(pyridin-3-ylamino)-isoquinolin-3-yl}-urea:

Starting from intermediate 270.1 and 3-aminopyridine and proceeding in analogy to Procedure AB, the title compound was obtained, after purification by CC (DCM/MeOH +1% NH₄OH 100:0 to 93:7), as a yellow solid (52% yield).
¹H NMR (d6-DMSO) δ: 9.24 (s, 1H); 9.05 (s, 1H); 8.58 (s, 1H); 8.40 (d, J = 2.7 Hz, 1H); 8.15 (s, 1H); 8.06 (dd, J = 4.6, 1.2 Hz, 1H); 7.47 (d, J = 7.8 Hz, 1 H); 7.46-7.40 (m, 1H); 7.23 (dd, J = 8.3, 4.6 Hz, 1H); 7.18 (t, J = 5.5 Hz, 1H); 7.07 (d, J = 7.7 Hz, 1H); 3.87 (s, 2H); 3.23-3.12 (m, 2H); 2.35 (s, 3H); 2.06 (br. s, 1H); 1.08 (t, J = 7.2 Hz, 3H).
MS (ESI, m/z): 351.16 [M+H⁺].

### Example 271: 1-ethyl-3-{8-[(6-methoxy-pyridin)-2-ylamino]-5-[(methylamino)methyl]-isoquinolin-3-yl}-urea:

Starting from intermediate 270.1 and 2-amino-6-methoxypyridine and proceeding in analogy to Procedure AB, however using BrettPhos as catalyst instead, the title compound was obtained, after purification by prep-HPLC (basic conditions), as a yellow solid (16% yield).
MS (ESI, m/z): 381.05 [M+H⁺].

### Example 272: 3-{[3-(3-ethyl-ureido)-5-[(methylamino)methyl]-isoquinolin-8-yl]amino}-benzoic acid:

Starting from intermediate 270.1 and *tert*-butyl 3-aminobenzoate and proceeding in analogy to Procedure AB, however using BrettPhos as catalyst instead, the title compound was obtained, after purification by prep-HPLC (acidic conditions), as a yellow solid (9% yield).
MS (ESI, m/z): 394.14 [M+H⁺].

### Example 273: 1-{5-[(dimethylamino)methyl]-8-[(6-methoxy-pyridin-2-yl)amino]-isoquinolin-3-yl}-3-ethyl-urea

### 273.1. 1-{8-chloro-5-[(dimethylamino)methyl]-isoquinolin-3-yl}-3-ethyl-urea:

Starting from the compound of Preparation W and 2*M* methylamine in THF and proceeding in analogy to Procedure AN, the title compound was obtained, after purification by CC (DCM/MeOH +1% NH₄OH 100:0 to 95:5), as a white solid (76% yield).
MS (ESI, m/z): 307.06 [M+H⁺].

### 273.2. 1-{5-[(dimethylamino)methyl]-8-[(6-methoxy-pyridin-2-yl)amino}-isoquinolin-3-yl}-3-ethyl-urea:

Starting from intermediate 273.1 and 2-amino-6-methoxypyridine and proceeding in analogy to Procedure AB, however using BrettPhos as catalyst instead, the title compound was obtained, after purification by prep-HPLC (basic conditions), as a yellow solid (31% yield).
MS (ESI, m/z): 394.96 [M+H⁺].

### Example 274: 1-ethyl-3-[5-morpholin-4-yl-8-(pyridin-3-ylamino)-isoquinolin-3-yl]-urea:

### 274.1. 1-(8-chloro-5-morpholin-4-yl-isoquinolin-3-yl)-3-ethyl-urea:

Starting from the compound of Preparation G and morpholine (1.25 eq.) and proceeding in analogy to Procedure R, the title compound was obtained, after purification by CC (DCM/MeOH 100:0 to 97:3), as a yellow solid (45% yield).
¹H NMR (d6-DMSO) δ: 9.24 (s, 1H); 9.20 (s, 1H); 8.31 (s, 1H); 7.44 (d, J = 8.0 Hz, 1H); 7.20 (t, J = 5.3 Hz, 1H); 7.16 (d, J = 8.0 Hz, 1H); 3.90-3.84 (m, 4H); 3.25-3.16 (m, 2H); 3.05-2.98 (m, 4H); 1.11 (t, J = 7.2 Hz, 3H).
MS (ESI, m/z): 335.15 [M+H⁺].

### 274.2. 1-ethyl-3-[5-morpholin-4-yl-8-(pyridin-3-ylamino)-isoquinolin-3-yl]-urea:

Starting from intermediate 274.1 and 3-aminopyridine and proceeding in analogy to Procedure AB, however using BrettPhos as catalyst instead, the title compound was obtained, after purification by CC (DCM/MeOH 100:0 to 95:5), as a yellow solid (31% yield).
¹H NMR (d6-DMSO) δ: 9.16 (s, 1H); 9.10 (s, 1H); 8.41 (s, 1H); 8.32 (d, J = 2.6 Hz, 1H); 8.19 (s, 1H); 8.00 (d, J = 4.6 Hz, 1H); 7.31 (d, J = 5.7 Hz, 2H); 7.18 (dd, J = 8.2, 4.6 Hz, 1H); 7.11 (d, J = 10.3 Hz, 2H); 3.87-3.80 (m, 4H); 3.24-3.12 (m, 2H); 2.99-2.91 (m, 4H); 1.08 (t, J = 7.2 Hz, 3H).
MS (ESI, m/z): 393.05 [M+H⁺].

### Example 275: 1-ethyl-3-{8-[(6-methoxy-pyridin-2-yl)amino]-5-(morpholin-4-ylmethyl)-isoquinolin-3-yl}-urea:

### 275.1. 1-[8-chloro-5-(morpholin-4-ylmethyl)-isoquinolin-3-yl]-3-ethyl-urea:

Starting from the compound of Preparation W and morpholine and proceeding in analogy to Procedure AN, the title compound was obtained, after purification by CC (DCM/MeOH 100:0 to 97:3), as a beige solid (56% yield).
MS (ESI, m/z): 349.07 [M+H⁺].

### 275.2. 1-ethyl-3-{8-[(6-methoxy-pyridin-2-yl)amino]-5-(morpholin-4-ylmethyl)-isoquinolin-3-yl}-urea:

Starting from intermediate 275.1 and 2-amino-6-methoxypyridine and proceeding in analogy to Procedure AB, however using BrettPhos as catalyst instead, the title compound was obtained, after purification by prep-HPLC (basic conditions), as a yellow solid (31% yield).
MS (ESI, m/z): 437.10 [M+H⁺].

### Example 276: 1-ethyl-3-[8-(6-methoxy-pyridin-2-ylamino)-5-(4-methyl-piperazin-1-ylmethyl)-isoquinolin-3-yl]-urea:

### 276.1. 1-[8-chloro-5-(4-methyl-piperazin-1-ylmethyl)-isoquinolin-3-yl]-3-ethyl-urea:

Starting from the compound of Preparation W and 1-methylpiperazine and proceeding in analogy to Procedure AN, the title compound was obtained, after purification by CC (DCM/MeOH +1% NH₄OH 100:0 to 96:4), as a yellow solid (93% yield).
¹H NMR (d6-DMSO) δ: 9.22 (s, 2H); 8.34 (d, J = 0.6 Hz, 1H); 7.55-7.50 (m, 1H); 7.47-7.42 (m, 1H); 7.17 (t, J = 5.5 Hz, 1H); 3.70 (s, 2H); 3.23-3.12 (m, 2H); 2.45 (br. s, 4H); 2.29 (br. s, 4H); 2.12 (s, 3H); 1.09 (t, J = 7.2 Hz, 3H).
MS (ESI, m/z): 361.88 [M+H⁺].

### 276.2. 1-ethyl-3-[8-(6-methoxy-pyridin-2-ylamino)-5-(4-methyl-piperazin-1-ylmethyl)-isoquinolin-3-yl]-urea:

Starting from intermediate 276.1 and 2-amino-6-methoxypyridine and proceeding in analogy to Procedure AB, however using BrettPhos as catalyst instead, the title compound was obtained, after purification by prep-HPLC (basic conditions), as a yellow solid (42% yield).
¹H NMR (d6-DMSO) δ: 9.26 (s, 1H); 9.07 (s, 2H); 8.15 (s, 1H); 7.78 (d, J = 7.8 Hz, 1H); 7.49 (t, J = 7.9 Hz, 1H); 7.46 (d, J = 7.9 Hz, 1H); 7.40 (t, J = 5.1 Hz, 1H); 6.54 (d, J = 7.8 Hz, 1H); 6.18 (d, J = 7.9 Hz, 1H); 3.73 (s, 3H); 3.65 (s, 2H); 3.24-3.12 (m, 2H); 2.45 (br. s, 4H); 2.29 (br. s, 4H); 2.12 (s, 3H); 1.09 (t, J = 7.2 Hz, 3H).
MS (ESI, m/z): 450.17 [M+H⁺].

### Example 277: 1-ethyl-3-[5-(4-methyl-piperazin-1-yl)-8-(pyridin-3-ylamino)-isoquinolin-3-yl]-urea:

Starting from the compound of Preparation U and 1-methylpiperazine and proceeding in analogy to Procedure AB, however using BrettPhos as catalyst instead and adding all reagents again after 23 h, the title compound was obtained, after purification by prep-HPLC (basic conditions), as a yellow solid (15% yield).
MS (ESI, m/z): 406.12 [M+H⁺].

### Example 278: 1-ethyl-3-(8-methyl-5-pyrimidin-4-yl-isoquinolin-3-yl)-urea:

Starting from the compound of Preparation X (1.0 eq.) and 4-bromo-pyrimidine hydrobromide (2.0 eq.) and proceeding in analogy to Procedure C, the title compound was obtained, after purification by CC (DCM/MeOH 100:0 to 96:4), as a yellow solid (40% yield).
¹H NMR (d6-DMSO) δ: 9.33 (d, J = 1.3 Hz, 1H); 9.25 (d, J = 0.8 Hz, 1H); 9.06 (s, 1H); 8.93 (d, J = 5.2 Hz, 1H); 8.37 (d, J = 0.7 Hz, 1H); 7.79 (dd, J = 5.2, 1.4 Hz, 1H); 7.76 (d, J = 7.3 Hz, 1H); 7.34 (dd, J = 7.3, 0.9 Hz, 1H); 7.16 (t, J = 5.4 Hz, 1H); 3.18-3.07 (m, 2H); 2.75 (d, J = 0.4 Hz, 3H); 1.05 (t, J = 7.2 Hz, 3H).
MS (ESI, m/z): 308.24 [M+H⁺].

### Example 279: 1-ethyl-3-(8-methyl-5-pyridazin-4-yl-isoquinolin-3-yl)-urea:

Starting from the compound of Preparation X (1.0 eq.) and 4-bromo-pyridazine hydrobromide (2.0 eq.) and proceeding in analogy to Procedure C, the title compound was obtained, after purification by CC (DCM/MeOH 100:0 to 96:4) followed by prep-HPLC (acidic conditions), as a yellow solid (32% yield).
¹H NMR (d6-DMSO) δ: 9.38 (d, J = 0.9 Hz, 1H); 9.36 (s, 1H); 9.26 (d, J = 0.5 Hz, 1H); 9.09 (s, 1H); 8.03 (s, 1H); 7.85-7.81 (m, 1H); 7.61 (d, J = 7.2 Hz, 1H); 7.34 (dd, J = 7.2, 0.8 Hz, 1H); 7.08 (t, J = 5.8 Hz, 1H); 3.17-3.06 (m, 2H); 2.75 (s, 3H); 1.04 (t, J = 7.2 Hz, 3H).
MS (ESI, m/z): 308.27 [M+H⁺].

### Example 280: 1-ethyl-3-[5-(1H-imidazol-4-yl)-8-methyl-isoquinolin-3-yl]-urea hydrochloride:

### 280.1. 4-bromo-imidazole-1-carboxylic acid tert-butyl ester:

To a solution of 4-bromo-1*H*-imidazole (100 mg) in anhydrous THF (3.5 mL) was added di-*tert*-butyl dicarbonate (149 mg) and DMAP (125 mg). The reaction mixture was stirred at rt for 30 min and partitioned between 9:1 DCM/MeOH and a sat. aq. NaHCO₃ solution. The layers were separated and the aq. layer was extracted with 9:1 DCM/MeOH (3x). The combined org. layers were washed with water and brine, dried over MgSO₄, filtered and concentrated under reduced pressure. The title compound was obtained, after purification by CC (DCM/MeOH 100:0 to 98:2), as a white solid (143 mg; 85% yield).
¹H NMR (d6-DMSO) δ: 8.18 (d, J = 1.0 Hz, 1H); 7.70 (d, J = 1.0 Hz, 1H); 1.54 (s, 9H).

### 280.2. 1-ethyl-3-[5-(1H-imidazol-4-yl)-8-methyl-isoquinolin-3-yl]-urea hydrochloride:

Starting from the compound of Preparation X and intermediate 280.1 and proceeding in analogy to Procedure AP, the title compound was obtained, after purification by prep-HPLC (acidic conditions) and treatment with HCl, as an amorphous solid (26% yield).
MS (ESI, m/z): 296.19 [M+H⁺].

### Example 281: 1-ethyl-3-[8-methyl-5-(1H-pyrazol-4-yl)-isoquinolin-3-yl]-urea hydrochloride:

Starting from the compound of Preparation X and 4-bromo-pyrazole-1-carboxylic acid *tert*-butyl ester and proceeding in analogy to Procedure AP, the title compound was obtained, after purification by prep-HPLC (acidic conditions) and treatment with HCl, as an amorphous solid (31% yield).
MS (ESI, m/z): 296.19 [M+H⁺].

### Example 282: 1-ethyl-3-(8-methyl-5-pyridazin-3-yl-isoquinolin-3-yl)-urea hydrochloride:

Starting from the compound of Preparation X and 3-bromopyridazine and proceeding in analogy to Procedure AP, the title compound was obtained, after purification by prep-HPLC (acidic conditions) and treatment with HCl, as an amorphous solid (17% yield).
MS (ESI, m/z): 308.21 [M+H⁺].

### Example 283: 1-ethyl-3-[8-methyl-5-(1-methyl-1H-imidazol-4-yl)-isoquinolin-3-yl]-urea hydrochloride:

Starting from the compound of Preparation X and 4-bromo-1-methyl-1*H-*imidazole and proceeding in analogy to Procedure AP, the title compound was obtained, after purification by prep-HPLC (acidic conditions) and treatment with HCl, as an amorphous solid (13% yield).
MS (ESI, m/z): 310.23 [M+H⁺].

### Example 284: 1-ethyl-3-[8-methyl-5-(1-methyl-1H-pyrazol-4-yl)-isoquinolin-3-yl]-urea hydrochloride:

Starting from the compound of Preparation X and 4-bromo-1-methyl-1*H-*pyrazole and proceeding in analogy to Procedure AP, the title compound was obtained, after purification by prep-HPLC (acidic conditions) and treatment with HCl, as an amorphous solid (25% yield).
MS (ESI, m/z): 310.22 [M+H⁺].

### Example 285: 1-ethyl-3-(8-methyl-5-thiazol-4-yl-isoquinolin-3-yl)-urea hydrochloride:

Starting from the compound of Preparation X and 4-bromothiazole and proceeding in analogy to Procedure AP, the title compound was obtained, after purification by prep-HPLC (acidic conditions) and treatment with HCl, as an amorphous solid (41% yield).
MS (ESI, m/z): 313.14 [M+H⁺].

### Example 286: 1-(8-chloro-5-pyrimidin-4-yl-isoquinolin-3-yl)-3-ethyl-urea:

Starting from the compound of Preparation Y (1.0 eq.) and 4-bromo-pyrimidine hydrobromide (1.5 eq.) and proceeding in analogy to Procedure C, the title compound was obtained, after purification by prep-HPLC (acidic conditions), as a yellow solid (5% yield).
MS (ESI, m/z): 328.11 [M+H⁺].

### Example 287: 1-ethyl-3-[8-(6-methyl-pyridin-3-ylamino)-5-pyrimidin-4-yl-isoquinolin-3-yl]-urea:

Starting from the compound of Example 286 and 5-aminopicoline and proceeding in analogy to Procedure AB, using however BrettPhos as catalyst instead, the title compound was obtained, after purification by prep-HPLC (basic conditions), as a yellow solid (40% yield).
MS (ESI, m/z): 400.24 [M+H⁺].

### Example 288: 1-[8-chloro-5-(1H-imidazol-4-yl)-isoquinolin-3-yl]-3-ethyl-urea:

### 288.1. 4-[8-chloro-3-(3-ethyl-ureido)-isoquinolin-5-yl]-imidazole-1-carboxylic acid tert-butyl ester:

Starting from the compound of Preparation Y (1.0 eq.) and intermediate 280.1 (1.5 eq.) and proceeding in analogy to Procedure C, using however bis(di-*tert*-butyl(4-dimethylaminophenyl)phosphine)dichloropalladium(II) (0.05 eq.) and toluene as catalyst and solvent instead and heating at 105°C, a mixture containing the title compound was obtained, after purification by CC (DCM/MeOH +1% NH₄OH 100:0 to 90:10), as a yellow solid.
MS (ESI, m/z): 416.14 [M+H⁺].

### 288.2. 1-[8-chloro-5-(1H-imidazol-4-yl)-isoquinolin-3-yl]-3-ethyl-urea:

Intermediate 288.1 was dissolved in TFA and stirred at rt for 1 h. The reaction mixture was concentrated under reduced pressure and the title compound was obtained, after purification by prep-HPLC (acidic conditions) followed by CC (DCM/MeOH +1% NH₄OH 100:0 to 92:8), as a yellow solid (10% yield).
MS (ESI, m/z): 316.17 [M+H⁺].

### Example 289: 1-(8-chloro-5-pyrimidin-4-yl-isoquinolin-3-yl)-3-ethyl-urea:

Starting from the compound of Preparation Y (1.0 eq.) and 4-bromo-pyrazole-1-carboxylic acid *tert*-butyl ester (1.5 eq.) and proceeding in analogy to Procedure C, using however *bis*(di-*tert-*butyl(4-dimethylaminophenyl)phosphine)dichloropalladium(II) (0.05 eq.) as catalyst and toluene as solvent instead and heating at 105°C, the title compound was obtained, after purification by prep-HPLC (acidic conditions), as a yellow solid (13% yield).
MS (ESI, m/z): 316.19 [M+H⁺].

### Example 290: 1-ethyl-3-[8-(6-methoxy-pyridin-2-ylamino)-5-thiazol-4-yl-isoquinolin-3-yl]-urea:

### 290.1. 1-(8-chloro-5-thiazol-4-yl-isoquinolin-3-yl)-3-ethyl-urea:

Starting from the compound of Preparation Y (1.0 eq.) and 4-bromothiazole (1.5 eq.) and proceeding in analogy to Procedure C, the title compound was obtained, after filtration of the reaction mixture, as a yellow solid (75% yield).
¹H NMR (d6-DMSO) δ: 9.33 (d, J = 1.9 Hz, 1H); 9.31 (d, J = 0.6 Hz, 1H); 9.19 (s, 1H); 8.56 (d, J = 0.5 Hz, 1H); 8.01 (d, J = 1.9 Hz, 1H); 7.82 (d, J = 7.7 Hz, 1H); 7.60 (d, J = 7.7 Hz, 1H); 6.99 (t, J = 5.8 Hz, 1H); 3.19-3.08 (m, 2H); 1.06 (t, J = 7.2 Hz, 3H).
MS (ESI, m/z): 333.02 [M+H⁺].

### 290.2. 1-ethyl-3-[8-(6-methoxy-pyridin-2-ylamino)-5-thiazol-4-yl-isoquinolin-3 yl]-urea:

Starting from intermediate 290.1 and 2-amino-6-methoxypyridine and proceeding in analogy to Procedure AB, however using BrettPhos as catalyst instead, the title compound was obtained, after purification by prep-HPLC (basic conditions), as a yellow solid (8% yield).
¹H NMR (d6-DMSO) δ: 9.37 (s, 1H); 9.30 (s, 1H); 9.28 (d, J = 1.9 Hz, 1H); 9.03 (s, 1H); 8.42 (s, 1H); 7.99 (d, J = 8.1 Hz, 1H); 7.87 (d, J = 1.9 Hz, 1H); 7.82 (d, J = 8.1 Hz, 1H); 7.55 (t, J = 7.9 Hz, 1H); 7.15 (t, J = 5.8 Hz, 1H); 6.65 (d, J = 7.9 Hz, 1H); 6.24 (d, J = 7.9 Hz, 1H); 3.77 (s, 3H); 3.20-3.08 (m, 2H); 1.06 (t, J = 7.2 Hz, 3H).
MS (ESI, m/z): 421.08 [M+H⁺].

### Example 291: 1-ethyl-3-[8-(2-methoxy-pyridin-3-ylamino)-isoquinolin-3-yl]-urea hydrochloride:

Starting from the compound of Preparation K and 2-methoxypyridin-3-amine and proceeding in analogy to Procedure U, the title compound was obtained, after purification by prep-HPLC (acidic conditions) and treatment with HCl, as an amorphous solid (71% yield).
MS (ESI, m/z): 338.19 [M+H⁺].

### Example 292: 1-ethyl-3-[8-(4-hydroxymethyl-pyrazol-1-ylmethyl)-isoquinolin-3-yl]-urea:

### 292.1. (1H-pyrazol-4-yl)-methanol:

To a suspension of LiAlH₄ (5.3 g) in dry THF (100 mL) in a round-bottomed flask, under inert atmosphere (N₂), was added portionwise ethyl pyrazole-4-carboxylate (10.0 g) at rt. The reaction mixture was stirred at rt overnight and then poured into a sat. aq. Rochelle salt solution. The mixture was stirred at rt for 3 h and diluted with EA. The layers were separated and the aq. layer was extracted with EA (3x). The combined org. layers were dried over MgSO₄, filtered and concentrated under reduced pressure. The title compound was obtained, after drying, as a white solid (5.2 g; 76% yield).
¹H NMR (d6-DMSO) δ: 12.60 (s, 1H); 7.50 (s, 2H); 4.77 (br. s, 1H); 4.38 (br. s, 2H).

### 292.2. 1-ethyl-3-[8-(4-hydroxymethyl-pyrazol-1-ylmethyl)-isoquinolin-3-yl]-urea:

Starting from the compound of Preparation R and intermediate 292.1 and proceeding in analogy to Procedure AC, the title compound was obtained, after purification by prep-HPLC (acidic conditions), as an amorphous solid (56% yield).
¹H NMR (d6-DMSO) δ: 9.32 (s, 1H); 9.02 (s, 1H); 8.01 (s, 1H); 7.73-7.68 (m, 1H); 7.68 (d, J = 0.5 Hz, 1H); 7.55 (dd, J = 8.4, 7.0 Hz, 1H); 7.37 (s, 1H); 7.10 (dd, J = 7.0, 0.7 Hz, 1H); 7.06 (t, J = 5.8 Hz, 1H); 5.80 (s, 2H); 4.76 (t, J = 5.5 Hz, 1H); 4.31 (d, J = 5.5 Hz, 2H); 3.22-3.11 (m, 2H); 1.08 (t, J = 7.2 Hz, 3H).
MS (ESI, m/z): 325.77 [M+H⁺].

### Example 293: 1-ethyl-3-[8-(5-hydroxymethyl-imidazol-1-ylmethyl)-isoquinolin-3-yl]-urea and 1-ethyl-3-[8-(4-hydroxymethyl-imidazol-1-ylmethyl)-isoquinolin-3-yl]-urea:

Starting from the compound of Preparation R and 4(5)-(hydroxymethyl)imidazole and proceeding in analogy to Procedure AC, the title compounds were obtained as a 7:3 mixture, after purification by prep-HPLC (acidic conditions), as an amorphous solid (67% yield).
MS (ESI, m/z): 325.77 [M+H⁺].

### Example 294: 1-[3-(3-ethyl-ureido)-isoquinolin-8-ylmethyl]-1H-imidazole-2-carboxylic acid:

Starting from the compound of Preparation R and ethyl imidazole-2-carboxylate and proceeding in analogy to Procedure AQ, the title compound was obtained, after purification by prep-HPLC (basic conditions), as an amorphous solid (22% yield).
MS (ESI, m/z): 340.10 [M+H⁺].

### Example 295: 1-ethyl-3-(8-pyrazol-1-ylmethyl-isoquinolin-3-yl)-urea:

Starting from the compound of Preparation R and pyrazole and proceeding in analogy to Procedure AQ, the title compound was obtained, after purification by prep-HPLC (basic conditions), as an amorphous solid (23% yield).
¹H NMR (d6-DMSO) δ: 9.32 (s, 1H); 9.07 (s, 1H); 8.00 (s, 1H); 7.85 (s, 1H); 7.74-7.69 (m, 1H); 7.57 (t, J = 7.5 Hz, 1H); 7.48 (s, 1H); 7.15 (br. s, 1H); 7.06 (d, J = 6.9 Hz, 1H); 6.29 (s, 1H); 5.87 (s, 2H); 3.22-3.13 (m, 2H); 1.09 (t, J = 7.1 Hz, 3H). MS (ESI, m/z): 296.11 [M+H⁺].

### Example 296: 1-[8-(2,4-dimethyl-imidazol-1-ylmethyl)-isoquinolin-3-yl]-3-ethyl-urea and 1-[8-(2,5-dimethyl-imidazol-1-ylmethyl)-isoquinolin-3-yl]-3-ethyl-urea:

Starting from the compound of Preparation R and 2,4-dimethylimidazole and proceeding in analogy to Procedure AQ, the title compounds were obtained as a 8:2 mixture, after purification by prep-HPLC (basic conditions), as an amorphous solid (23% yield).
MS (ESI, m/z): 324.16 [M+H⁺].

### Example 297: 1-ethyl-3-[8-(2-methyl-imidazol-1-ylmethyl)-isoquinolin-3-yl]-urea:

Starting from the compound of Preparation R and 2-methylimidazole and proceeding in analogy to Procedure AQ, the title compound was obtained, after purification by prep-HPLC (basic conditions), as an amorphous solid (57% yield).
MS (ESI, m/z): 309.99 [M+H⁺].

### Example 298: 1-ethyl-3-(8-[1,2,3]triazol-1-ylmethyl-isoquinolin-3-yl)-urea:

Starting from the compound of Preparation R and 1*H*-1,2,3-triazole and proceeding in analogy to Procedure AQ, the title compound was obtained, after purification by prep-HPLC (basic conditions), as an amorphous solid (16% yield).
MS (ESI, m/z): 297.10 [M+H⁺].

### Example 299: 1-ethyl-3-(8-imidazol-1-ylmethyl-isoquinolin-3-yl)-urea:

Starting from the compound of Preparation R and imidazole and proceeding in analogy to Procedure AQ, the title compound was obtained, after purification by prep-HPLC (basic conditions), as an amorphous solid (7% yield).
MS (ESI, m/z): 296.12 [M+H⁺].

### Example 300: 1-ethyl-3-[8-(4-methyl-pyrazol-1-ylmethyl)-isoquinolin-3-yl]-urea:

Starting from the compound of Preparation R and 4-methylpyrazole and proceeding in analogy to Procedure AQ, the title compound was obtained, after purification by prep-HPLC (basic conditions), as an amorphous solid (31% yield).
MS (ESI, m/z): 310.15 [M+H⁺].

### Example 301: 1-ethyl-3-{8-[(5-methyl-isoxazol-3-ylamino)-methyl]-isoquinolin-3-yl}-urea hydrochloride:

Starting from the compound of Preparation R and 3-amino-5-methylisoxazole (3.0 eq.) and proceeding in analogy to Procedure AM, however heating at 100°C, the title compound was obtained, after purification by prep-HPLC (acidic conditions) and treatment with HCl, as an amorphous solid (13% yield).
MS (ESI, m/z): 325.97 [M+H⁺].

### Example 302: 1-ethyl-3-{8-[(6-methoxy-pyridin-3-ylamino)-methyl]-isoquinolin-3-yl}-urea hydrochloride:

Starting from the compound of Preparation R and 5-amino-2-methoxypyridine (3.0 eq.) and proceeding in analogy to Procedure AM, however heating at 100°C, the title compound was obtained, after purification by prep-HPLC (acidic conditions) and treatment with HCl, as an amorphous solid (27% yield).
MS (ESI, m/z): 352.23 [M+H⁺].

### Example 303: 1-ethyl-3-{8-[(2-methyl-2H-pyrazol-3-ylamino)-methyl]-isoquinolin-3-yl}-urea hydrochloride:

Starting from the compound of Preparation R and 2-methyl-2*H*-pyrazol-3-ylamine (3.0 eq.) and proceeding in analogy to Procedure AM, however heating at 100°C, the title compound was obtained, after purification by prep-HPLC (acidic conditions) and treatment with HCl, as an amorphous solid (19% yield).
MS (ESI, m/z): 325.16 [M+H⁺].

### Example 304: 1-ethyl-3-[8-(pyrimidin-2-ylaminomethyl)-isoquinolin-3-yl]-urea:

Starting from the compound of Preparation Z and 2-bromopyrimidine and proceeding in analogy to Procedure R, the title compound was obtained, after purification by CC (DCM/MeOH +1% NH₄OH 100:0 to 98:2) followed by prep-HPLC (basic conditions), as a white solid (28% yield).
MS (ESI, m/z): 323.14 [M+H⁺].

### Pharmacological properties of the invention compounds

### In vitro assays

### Experimental methods:

Minimal inhibitory concentrations (MICs; mg/l) were determined in cation-adjusted Mueller-Hinton Broth (supplemented with 3% (v/v) lysed horse blood for testing *Streptococcus pneumoniae*) by a microdilution method following the description given in "Methods for Dilution Antimicrobial Susceptibility Tests for Bacteria that Grow Aerobically", Approved standard, 7th ed., Clinical and Laboratory Standards Institute (CLSI) Document M7-A7, Wayne, PA, USA, 2006.

### Results:

All Example compounds were tested against several Gram positive and Gram negative bacteria.

Typical antibacterial test results are given in the table hereafter (MIC in mg/l).

| **Example No.** | **MIC for *S. pneumoniae ATCC 49619*** | **Example No.** | **MIC for *S. pneumoniae ATCC 49619*** | **Example No.** | **MIC for *S. pneumoniae ATCC 49619*** |
|---|---|---|---|---|---|
| 1 | 1 | 2 | 2 | 3 | 1 |
| 4 | 8 | 5 | 1 | 6 | 0.5 |
| 7 | 4 | 8 | 4 | 9 | 2 |
| 10 | 2 | 11 | 2 | 12 | 8 |
| 13 | 2 | 14 | 4 | 15 | 0.5 |
| 16 | 2 | 17 | 1 | 18 | 0.25 |
| 19 | 1 | 20 | 0.5 | 21 | 1 |
| 22 | 0.5 | 23 | 8 | 24 | 0.25 |
| 25 | 0.5 | 26 | 0.5 | 27 | 2 |
| 28 | 2 | 29 | 0.5 | 30 | 1 |
| 31 | 1 | 32 | 16 | 33 | 1 |
| 34 | 2 | 35 | 0.5 | 36 | 0.125 |
| 37 | 1 | 38 | 1 | 39 | 1 |
| 40 | 2 | 41 | 2 | 42 | 2 |
| 43 | 8 | 44 | 4 | 45 | 2 |
| 46 | 1 | 47 | 2 | 48 | 1 |
| 49 | 2 | 50 | 8 | 51 | 1 |
| 52 | 2 | 53 | 8 | 54 | 16 |
| 55 | 8 | 56 | 8 | 57 | 0.5 |
| 58 | 8 | 59 | 4 | 60 | 4 |
| 61 | 1 | 62 | 0.25 | 63 | 2 |
| 64 | 1 | 65 | 2 | 66 | 4 |
| 67 | 16 | 68 | 4 | 69 | 16 |
| 70 | 16 | 71 | 16 | 72 | 2 |
| 73 | 8 | 74 | 2 | 75 | 4 |
| 76 | 1 | 77 | 1 | 78 | 2 |
| 79 | 1 | 80 | 16 | 81 | 0.5 |
| 82 | 8 | 83 | 1 | 84 | 0.5 |
| 85 | 16 | 86 | 16 | 87 | 8 |
| 88 | 2 | 89 | 0.5 | 90 | 2 |
| 91 | 2 | 92 | 0.25 | 93 | 2 |
| 94 | 0.25 | 95 | 16 | 96 | 2 |
| 97 | 2 | 98 | 4 | 99 | 2 |
| 100 | 2 | 101 | 1 | 102 | 2 |
| 103 | 0.5 | 104 | 8 | 105 | 8 |
| 106 | 0.125 | 107 | 1 | 108 | 0.5 |
| 109 | 2 | 110 | 1 | 111 | 1 |
| 112 | 0.5 | 113 | 1 | 114 | 0.5 |
| 115 | 4 | 116 | 0.125 | 117 | 0.25 |
| 118 | 0.06 | 119 | 0.25 | 120 | 0.125 |
| 121 | 0.25 | 122 | 8 | 123 | 4 |
| 124 | 4 | 125 | 4 | 126 | 8 |
| 127 | 16 | 128 | 8 | 129 | 16 |
| 130 | 8 | 131 | 4 | 132 | 8 |
| 133 | 16 | 134 | 16 | 135 | 16 |
| 136 | 16 | 137 | 8 | 138 | 1 |
| 139 | 4 | 140 | 2 | 141 | 0.5 |
| 142 | 0.25 | 143 | 0.125 | 144 | 0.5 |
| 145 | 1 | 146 | 4 | 147 | 4 |
| 148 | 2 | 149 | 2 | 150 | 4 |
| 151 | 4 | 152 | 4 | 153 | 8 |
| 154 | 8 | 155 | 2 | 156 | 2 |
| 157 | 2 | 158 | 2 | 159 | 2 |
| 160 | 2 | 161 | 2 | 162 | 2 |
| 163 | 2 | 164 | 2 | 165 | 4 |
| 166 | 8 | 167 | 8 | 168 | 0.5 |
| 169 | 1 | 170 | 2 | 171 | 2 |
| 172 | 2 | 173 | 2 | 174 | 2 |
| 175 | 16 | 176 | 2 | 177 | 2 |
| 178 | 2 | 179 | 4 | 180 | 4 |
| 181 | 8 | 182 | 2 | 183 | 4 |
| 184 | 4 | 185 | 4 | 186 | 8 |
| 187 | 8 | 188 | 8 | 189 | 8 |
| 190 | 1 | 191 | 8 | 192 | 2 |
| 193 | 1 | 194 | 4 | 195 | 4 |
| 196 | 8 | 197 | 8 | 198 | 1 |
| 199 | 1 | 200 | 1 | 201 | 2 |
| 202 | 1 | 203 | 2 | 204 | 2 |
| 205 | 4 | 206 | 4 | 207 | 2 |
| 208 | 8 | 209 | 4 | 210 | 8 |
| 211 | 4 | 212 | 8 | 213 | 4 |
| 214 | 16 | 215 | 16 | 216 | 8 |
| 217 | 0.5 | 218 | 1 | 219 | 0.25 |
| 220 | 16 | 221 | 0.5 | 222 | 1 |
| 223 | 0.5 | 224 | 0.5 | 225 | 0.125 |
| 226 | 1 | 227 | 0.125 | 228 | 2 |
| 229 | 0.25 | 230 | 0.5 | 231 | 0.5 |
| 232 | 2 | 233 | 0.5 | 234 | 0.25 |
| 235 | 0.25 | 236 | 0.5 | 237 | 0.25 |
| 238 | 0.5 | 239 | 0.5 | 240 | 2 |
| 241 | ≤ 0.031 | 242 | 0.5 | 243 | 1 |
| 244 | 2 | 245 | 0.5 | 246 | 0.5 |
| 247 | 0.25 | 248 | 0.5 | 249 | 0.5 |
| 250 | 1 | 251 | 1 | 252 | 2 |
| 253 | 4 | 254 | 1 | 255 | 4 |
| 256 | 8 | 257 | 8 | 258 | 8 |
| 259 | 4 | 260 | 8 | 261 | 8 |
| 262 | 8 | 263 | 4 | 264 | 8 |
| 265 | 1 | 266 | 4 | 267 | 2 |
| 268 | 4 | 269 | 8 | 270 | 4 |
| 271 | 4 | 272 | 8 | 273 | 4 |
| 274 | 2 | 275 | 4 | 276 | 4 |
| 277 | 2 | 278 | 2 | 279 | 1 |
| 280 | 1 | 281 | 0.5 | 282 | 8 |
| 283 | 8 | 284 | 4 | 285 | 2 |
| 286 | 8 | 287 | 0.25 | 288 | 2 |
| 289 | 2 | 290 | 0.5 | 291 | 4 |
| 292 | 8 | 293 | 4 | 294 | 4 |
| 295 | 8 | 296 | 8 | 297 | 4 |
| 298 | 4 | 299 | 4 | 300 | 4 |
| 301 | 4 | 302 | 8 | 303 | 4 |
| 304 | 4 | | | | |

## Claims

1. A compound of formula I 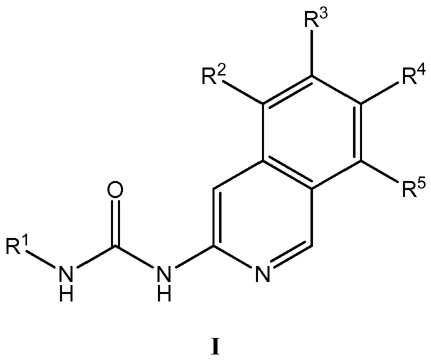 wherein
R¹ represents (C₁-C₃)alkyl, (C₂-C₃)haloalkyl or cyclopropyl;
R⁴ represents H;
R³ represents H, R⁵ represents H and R² represents phenyl or 4-carboxyphenyl; or
R² represents H, R⁵ represents H and R³ represents benzoylamino; or
R² represents H, R³ represents H and R⁵ represents -NH-CO-R⁶, -CH₂-O-R ⁷, -NH-R⁸, -CH₂-NH-R⁹, -CH₂-R¹⁰, (pyridin-3-ylmethyl)amino, pyridin-3-yloxy or 4-carboxyphenyl; or
R³ represents H, R⁵ represents methyl and R² represents halogen, (C₁-C₃)alkylamino, (C₁-C₃)alkylaminomethyl, 2-(aminocarbonyl)-ethyl, pyrimidin-4-yl, pyridazin-3-yl, pyridazin-4-yl, 1*H*-imidazol-4-yl, 1-methyl-1*H*-imidazol-4-yl, 1*H*-pyrazol-4-yl, 1-methyl-1*H*-pyrazol-4-yl, imidazo[1,2-*a*]pyridin-6-yl, 4-methyl-thiophen-3-yl, thiazol-4-yl, isoquinolin-5-yl or 1*H*-indol-4-yl or a group having the formula (A1), (A2) or (A3) shown hereafter 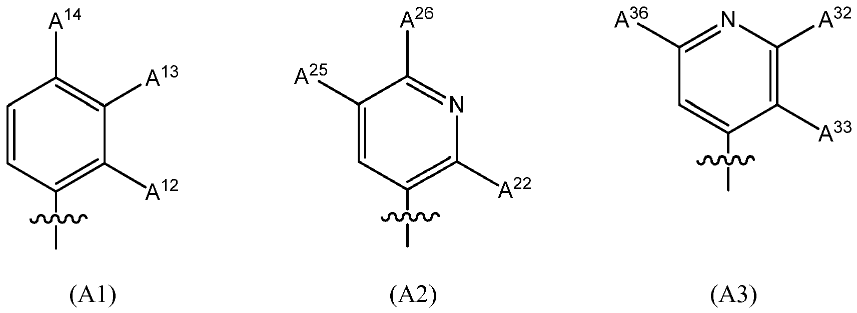
wherein
each of A¹³ and A¹⁴ represents H and A¹² represents H or OH, or
each of A¹² and A¹⁴ represents H and A¹³ represents OH, acetylamino, aminomethyl, sulfamoyl or hydroxymethyl, or
each of A¹² and A¹³ represents H and A¹⁴ represents OH, methylaminocarbonyl, dimethylaminocarbonyl, methoxycarbonylamino, sulfamoyl or hydroxymethyl;
each of A²⁵ and A²⁶ represents H and A²² represents H or halogen, or
each of A²² and A²⁶ represents H and A²⁵ represents methyl or halogen, or
each of A²² and A²⁵ represents H and A²⁶ represents hydroxymethyl;
each of A³³ and A³⁶ represents H and A³² represents H, halogen, amino, methyl or methoxy, or
A³³ represents H and each of A³² and A³⁶ represents methyl; or
R² represents H, R⁵ represents methyl and R³ represents amino, vinyl, (C₁-C₄)alkylamino, cyclopropylmethylamino, (2-(benzyloxy)ethyl)amino, methoxymethylcarbonylamino, (thiazol-5-yl)carbonylamino, pyridin-3-ylcarbonylamino, pyridin-4-ylcarbonylamino, naphthalen-2-yl, furan-2-yl, furan-3-yl, 1*H*-pyrazol-4-yl, thiophen-3-yl, 4-methyl-thiophen-3-yl, quinolin-3-yl, isoquinolin-4-yl, isoquinolin-5-yl, imidazo[1,2-*a*]pyridin-6-yl, 1*H*-indol-4-yl or a group having the formula (B1), (B2), (B3), (B4), (B5), (B6), (B7), (B8), (B9), (B10), (B11) or (B12) shown hereafter 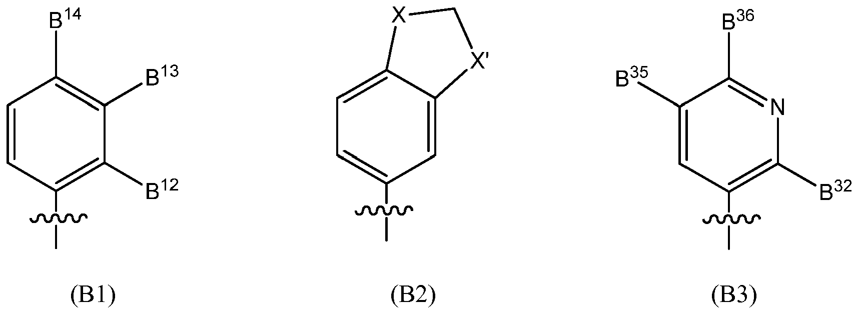 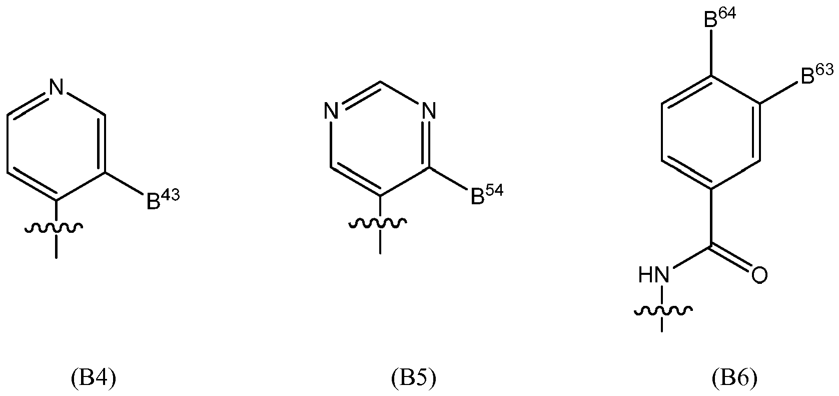 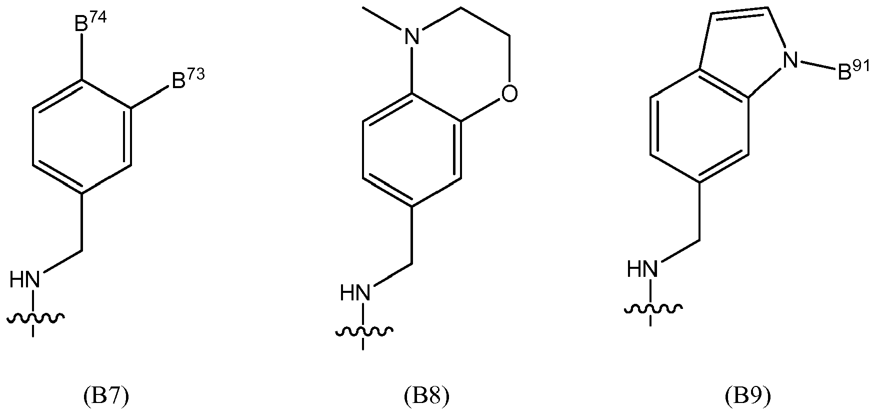 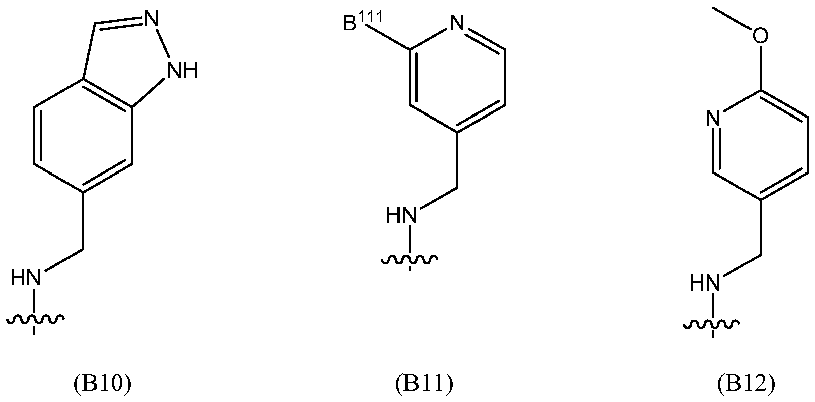
wherein
each of B¹³ and B¹⁴ represents H and B¹² represents OH, methyl, acetylamino, (C₁-C₂)alkoxycarbonyl or dimethylaminocarbonyl, or
each of B¹² and B¹⁴ represents H and B¹³ represents OH, halogen, acetyl, acetylamino, acetylaminomethyl, aminocarbonyl, sulfamoyl, (C₁-C₂)alkoxy, (C₁-C₂)alkoxycarbonyl, cyano, amino-(C₁-C₂)alkyl or hydroxy-(C₁-C₂)alkyl, or
each of B¹² and B¹³ represents H and B¹⁴ represents acetyl, acetylamino, acetylaminomethyl, aminocarbonyl, dimethylaminocarbonyl, sulfamoyl, (C₁-C₂)alkylsulfonyl, cyanomethyl or hydroxymethyl;
X and X' each represent -O- or one of X and X' represents -O- and the other represents -CH₂-;
each of B³⁵ and B³⁶ represents H and B³² represents H or halogen, or
each of B³² and B³⁶ represents H and B³⁵ represents halogen, methyl, methylsulfanyl, methanesulfonyl or methoxycarbonyl, or
each of B³² and B³⁵ represents H and B³⁶ represents halogen, methoxy or hydroxymethyl; B⁴³ represents H or halogen;
B⁵⁴ represents H or (C₁-C₃)alkyl;
B⁶⁴ represents H and B⁶³ represents H, methoxycarbonyl or dimethylamino, or
B⁶³ represents H and B⁶⁴ represents methoxy, methoxycarbonyl, dimethylamino or methanesulfonyl;
B⁷⁴ represents H and B⁷³ represents H, methoxy, methoxycarbonyl, carboxy, 2-hydroxyethoxy, 1*H*-1,2,4-triazol-1-yl or 1*H*-1,2,4-triazol-1-ylmethyl, or
B⁷³ represents H and B⁷⁴ represents carboxy, acetylamino, ((aminocarbonyl)methyl)oxy, *N*-methylsulfamoyl or 3-methyl-1*H*-1,2,4-triazol-1-yl;
B⁹¹ represents H or methyl;
B¹¹¹ represents H or morpholino; or
R² represents H, R⁵ represents pyridin-3-ylamino and R³ represents propylamino, 3-acetylamino-phenyl, benzylamino, 5-methylpyridin-3-yl or 3-(*N*,*N*-dimethylamino)phenylcarbonylamino; or
R² represents fluorine, R³ represents H and R⁵ represents bromine, benzyl, benzoylamino, 4-carboxyphenyl, benzylamino, pyridin-3-yl or (pyridin-3-ylmethyl)amino; or
R² represents bromine, R³ represents H and R⁵ represents methoxy; or
R² represents fluorine, R⁵ represents methyl and R³ represents quinolin-3-yl, benzylamino, a phenyl group optionally substituted once by acetylamino or sulfamoyl, pyridin-2-yl, or a pyridin-3-yl group optionally substituted once by methyl; or
R² represents fluorine, R³ represents pyridin-3-yl and R⁵ represents pyridin-3-yl or 4-carboxyphenyl; or
R² represents fluorine, R³ represents benzylamino and R⁵ represents chlorine or 2-methoxy-pyrimidin-5-yl; or
R² represents fluorine, R³ represents quinolin-3-yl and R⁵ represents quinolin-3-yl; or
R² represents fluorine, R³ represents 3-(acetylamino)phenyl and R⁵ represents pyridin-3-yl, pyridin-3-ylamino or 2-methoxypyrimidin-5-yl; or
R² represents fluorine, R³ represents 4-sulfamoyl-phenyl and R⁵ represents chlorine; or
R² represents fluorine, R⁵ represents pyridin-3-ylamino and R³ represents benzylamino or 5-methylpyridin-3-yl; or
R³ represents H, R² represents (C₁-C₃)alkylaminomethyl, *N*,*N*-dimethylaminomethyl, pyridin-3-yl, pyridin-4-yl, 2-methylpyridin-4-yl, 2-methoxypyridin-4-yl, 2-aminopyridin-4-yl, 2,6-dimethylpyridin-4-yl, pyrimidin-4-yl, 1*H*-imidazol-4-yl, 1*H*-pyrazol-4-yl, thiazol-4-yl, pyridin-3-ylamino, 3-(aminomethyl)phenyl, 4-hydroxyphenyl, 4-(hydroxymethyl)phenyl, 4-sulfamoyl-phenyl, morpholino, morpholinomethyl, 4-methylpiperazin-1-yl or 4-methyl-piperazin-1-ylmethyl and R⁵ represents chlorine, vinyl, methoxy, (3-carboxyphenyl)amino, (3-(methoxycarbonyl)phenyl)amino, (3-(dimethylcarbamoyl)phenyl)amino, (3-(methylcarbamoyl)phenyl)amino, pyridin-4-yl, (6-methyl-pyridin-2-yl)amino, (6-methoxy-pyridin-2-yl)amino, pyridin-3-ylamino, (6-methyl-pyridin-3-yl)amino or pyridin-3-yloxy;
R⁶ represents phenyl, 1*H*-pyrrol-2-yl or 1*H*-1,2,3-triazol-5-yl;
R⁷ represents pyridin-2-yl, 5-methyl-pyridin-2-yl, 2-methyl-pyridin-3-yl, 6-methyl-pyridin-3-yl, pyridin-4-yl, 3-methoxycarbonyl-pyridin-4-yl, 2-(methoxycarbonyl)phenyl, 3-hydroxy-phenyl or 3-amino-phenyl;
R⁸ represents pyridin-2-yl, 6-methoxy-pyridin-2-yl, 5-methoxy-pyridin-2-yl, 4-methoxy-pyridin-2-yl, 3-methyl-pyridin-2-yl, 4-methyl-pyridin-2-yl, 5-methyl-pyridin-2-yl, 6-methyl-pyridin-2-yl, 5-aminocarbonyl-pyridin-2-yl, 4-fluoro-pyridin-2-yl, 5-methoxycarbonyl-pyridin-2-yl, 6-methoxycarbonyl-pyridin-2-yl, pyridin-3-yl, 5-fluoro-pyridin-3-yl, 2-methoxypyridin-3-yl, 5-methoxy-pyridin-3-yl, 6-methoxy-pyridin-3-yl, 6-acetylamino-pyridin-3-yl, 6-methyl-pyridin-3-yl, pyridin-4-yl, 2-methoxy-pyridin-4-yl, 3-fluoro-pyridin-4-yl, pyrazin-2-yl, pyrimidin-2-yl, pyrimidin-4-yl, pyrimidin-5-yl, phenyl, 3-hydroxyphenyl, 3-methoxyphenyl, 3-carboxyphenyl, 3-carbamoylphenyl, 3-acetylamino-phenyl, 3-sulfamoylphenyl, 3-methoxycarbonyl-phenyl, 4-methyl-3-methoxycarbonyl-phenyl, 4-methyl-3-carboxyphenyl, 3-(carboxymethyl)phenyl, 3-(methylcarbamoyl)phenyl, 3-(dimethylcarbamoyl)phenyl, 3-(benzylcarbamoyl)phenyl, 3-(2-amino-2-oxoethyl)phenyl, 3-(2-(dimethylamino)-2-oxoethyl)phenyl, 3-(2-(benzylamino)-2-oxoethyl)phenyl, 4-methoxycarbonyl-phenyl, 3-methyl-4-methoxycarbonyl-phenyl, 4-carboxyphenyl, 3-methyl-4-carboxyphenyl, 3-carbamoyl-4-methoxyphenyl, 4-methoxy-3-(methylcarbamoyl)phenyl, 3-(dimethylcarbamoyl)-4-methoxyphenyl, 4-hydroxyphenyl, 4-(carboxymethyl)phenyl, 4-cyanophenyl, 4-acetylamino-phenyl, 4-carbamoylphenyl, 4-(methylcarbamoyl)phenyl, 4-(dimethylcarbamoyl)phenyl, 4-(2-amino-2-oxoethyl)phenyl, 4-(2-(dimethylamino)-2-oxoethyl)phenyl, 4-(2-(methylamino)-2-oxoethyl)phenyl, 4-(2-(benzylamino)-2-oxoethyl)phenyl, 5-methyl-1,3,4-oxadiazol-2-yl or imidazo[1,2-*a*]pyridin-7-yl;
R⁹ represents pyridin-2-yl, pyridin-3-yl, 6-methoxypyridin-3-yl, pyrimidin-2-yl, 1,3-dimethyl-1*H*-pyrazol-5-yl, 1-methyl-1*H*-pyrazol-5-yl, 5-methylisoxazol-3-yl or 3-methylisothiazol-5-yl;
R¹⁰ represents 1*H*-imidazol-1-yl, 4-(hydroxymethyl)-1*H*-imidazol-1-yl, 5-(hydroxymethyl)-1*H* imidazol-1-yl, 2-carboxy-1*H*-imidazol-1-yl, 2,4-dimethyl-1*H*-imidazol-1-yl, 2,5-dimethyl-1*H*-imidazol-1-yl, 2-methyl-1*H*-imidazol-1-yl, 1*H*-1,2,3-triazol-1-yl, 1*H*-pyrazol-1-yl, 4-methyl-1*H*-pyrazol-1-yl or 4-(hydroxymethyl)-1*H*-pyrazol-1-yl;
or a salt of such a compound.

2. A compound of formula I according to claim 1, which is also a compound of formula I_{P2} 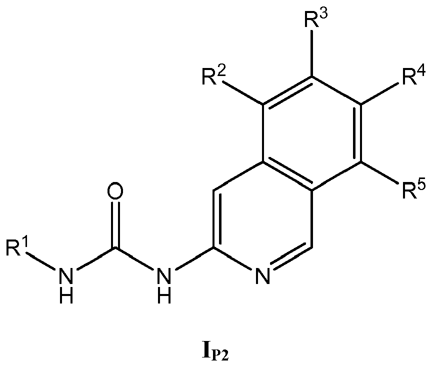 wherein
R¹ represents (C₁-C₃)alkyl, (C₂-C₃)haloalkyl or cyclopropyl;
R⁴ represents H;
R³ represents H, R⁵ represents H and R² represents phenyl or 4-carboxyphenyl; or
R² represents H, R⁵ represents H and R³ represents benzoylamino; or
R² represents H, R³ represents H and R⁵ represents -NH-CO-R⁶, -CH₂-O-R⁷, -NH-R⁸, -CH₂-NH-R⁹, (pyridin-3-ylmethyl)amino, pyridin-3-yloxy or 4-carboxyphenyl; or
R³ represents H, R⁵ represents methyl and R² represents halogen, (C₁-C₃)alkylamino, 2-(aminocarbonyl)-ethyl, imidazo[1,2-*a*]pyridin-6-yl, 4-methyl-thiophen-3-yl, isoquinolin-5-yl or 1*H*-indol-4-yl or a group having the formula (A1), (A2) or (A3) shown hereafter 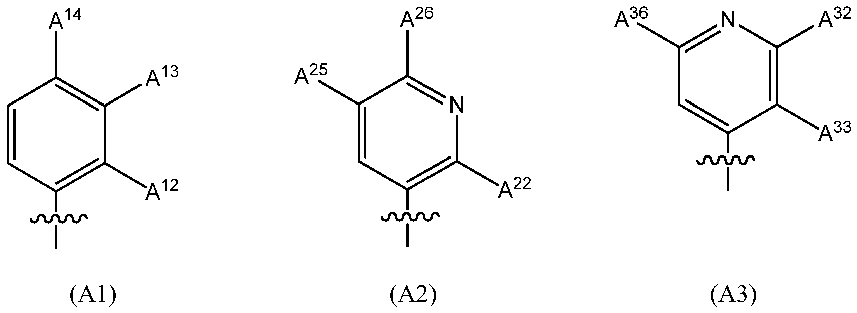
wherein
each of A¹³ and A¹⁴ represents H and A¹² represents H or OH, or
each of A¹² and A¹⁴ represents H and A¹³ represents OH, acetylamino, aminomethyl, sulfamoyl or hydroxymethyl, or
each of A¹² and A¹³ represents H and A¹⁴ represents OH, methylaminocarbonyl, dimethylaminocarbonyl, methoxycarbonylamino, sulfamoyl or hydroxymethyl;
each of A²⁵ and A²⁶ represents H and A²² represents H or halogen, or
each of A²² and A²⁶ represents H and A²⁵ represents methyl or halogen, or
each of A²² and A²⁵ represents H and A²⁶ represents hydroxymethyl;
each of A³³ and A³⁶ represents H and A³² represents H, halogen, amino, methyl or methoxy, or
A³³ represents H and each of A³² and A³⁶ represents methyl; or
R² represents H, R⁵ represents methyl and R³ represents amino, vinyl, (C₁-C₄)alkylamino, cyclopropylmethylamino, (2-(benzyloxy)ethyl)amino, methoxymethylcarbonylamino, (thiazol-5-yl)carbonylamino, pyridin-3-ylcarbonylamino, pyridin-4-ylcarbonylamino, naphthalen-2-yl, furan-2-yl, furan-3-yl, 1*H*-pyrazol-4-yl, thiophen-3-yl, 4-methyl-thiophen-3-yl, quinolin-3-yl, isoquinolin-4-yl, isoquinolin-5-yl, imidazo[1,2-*a*]pyridin-6-yl, 1*H*-indol-4-yl or a group having the formula (B1), (B2), (B3), (B4), (B5), (B6), (B7), (B8), (B9), (B10), (B11) or (B12) shown hereafter 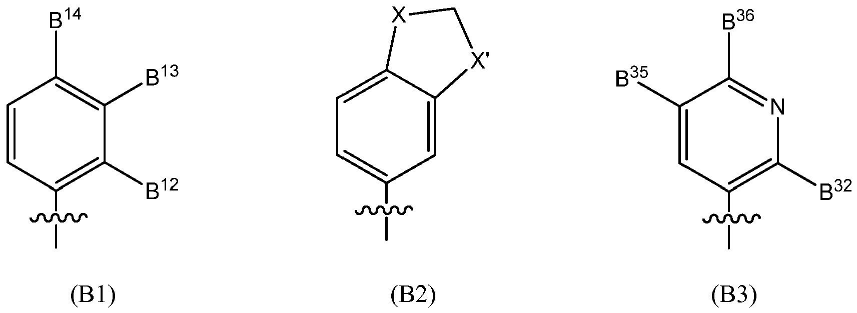 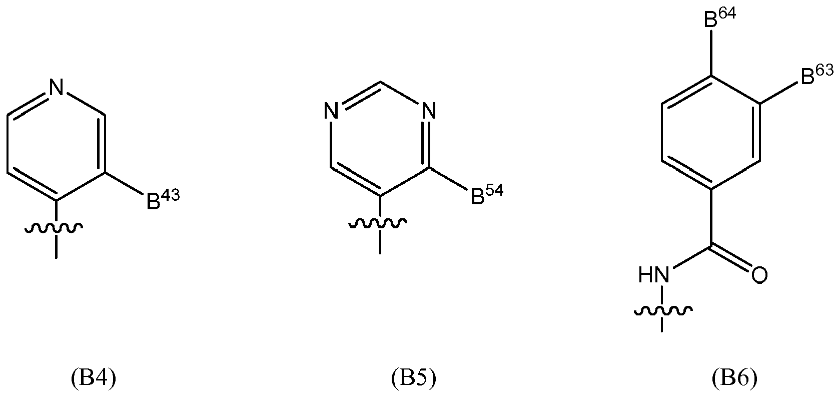 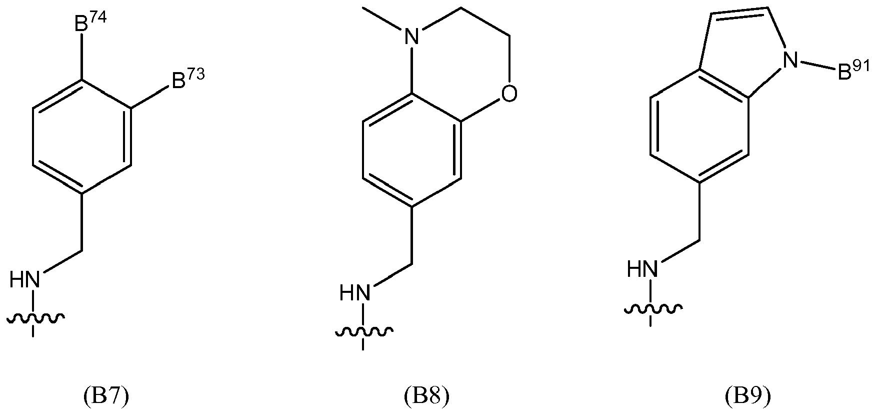 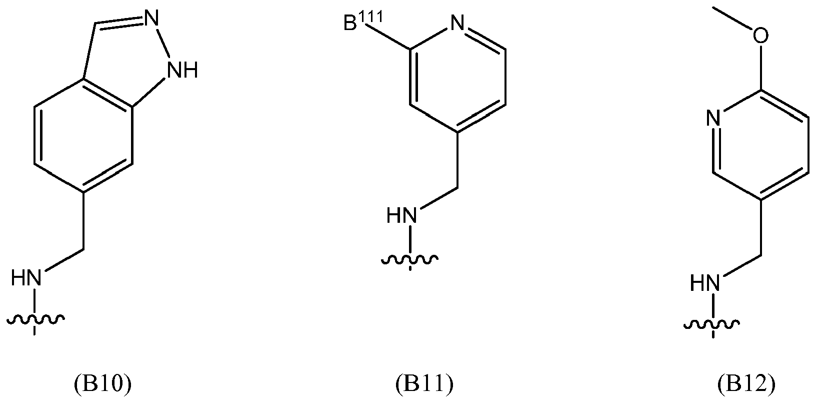
wherein
each of B¹³ and B¹⁴ represents H and B¹² represents OH, methyl, acetylamino, (C₁-C₂)alkoxycarbonyl or dimethylaminocarbonyl, or
each of B¹² and B¹⁴ represents H and B¹³ represents OH, halogen, acetyl, acetylamino, acetylaminomethyl, aminocarbonyl, sulfamoyl, (C₁-C₂)alkoxy, (C₁-C₂)alkoxycarbonyl, cyano, amino-(C₁-C₂)alkyl or hydroxy-(C₁-C₂)alkyl, or
each of B¹² and B¹³ represents H and B¹⁴ represents acetyl, acetylamino, acetylaminomethyl, aminocarbonyl, dimethylaminocarbonyl, sulfamoyl, (C₁-C₂)alkylsulfonyl, cyanomethyl or hydroxymethyl;
X and X' each represent -O- or one of X and X' represents -O- and the other represents -CH₂-;
each of B³⁵ and B³⁶ represents H and B³² represents H or halogen, or
each of B³² and B³⁶ represents H and B³⁵ represents halogen, methyl, methylsulfanyl, methanesulfonyl or methoxycarbonyl, or
each of B³² and B³⁵ represents H and B³⁶ represents halogen, methoxy or hydroxymethyl; B⁴³ represents H or halogen;
B⁵⁴ represents H or (C₁-C₃)alkyl;
B⁶⁴ represents H and B⁶³ represents H, methoxycarbonyl or dimethylamino, or
B⁶³ represents H and B⁶⁴ represents methoxy, methoxycarbonyl, dimethylamino or methanesulfonyl;
B⁷⁴ represents H and B⁷³ represents H, methoxy, methoxycarbonyl, carboxy, 2-hydroxyethoxy, 1*H*-1,2,4-triazol-1-yl or 1*H*-1,2,4-triazol-1-ylmethyl, or
B⁷³ represents H and B⁷⁴ represents carboxy, acetylamino, ((aminocarbonyl)methyl)oxy, *N*-methylsulfamoyl or 3-methyl-1*H*-1,2,4-triazol-1-yl;
B⁹¹ represents H or methyl;
B¹¹¹ represents H or morpholino; or
R² represents H, R⁵ represents pyridin-3-ylamino and R³ represents propylamino, 3-acetylamino-phenyl, benzylamino, 5-methylpyridin-3-yl or 3-(*N*,*N*-dimethylamino)phenylcarbonylamino; or
R² represents fluorine, R³ represents H and R⁵ represents bromine, benzyl, benzoylamino, 4-carboxyphenyl, benzylamino, pyridin-3-yl or (pyridin-3-ylmethyl)amino; or
R² represents bromine, R³ represents H and R⁵ represents methoxy; or
R² represents fluorine, R⁵ represents methyl and R³ represents quinolin-3-yl, benzylamino, a phenyl group optionally substituted once by acetylamino or sulfamoyl, pyridin-2-yl, or a pyridin-3-yl group optionally substituted once by methyl; or
R² represents fluorine, R³ represents pyridin-3-yl and R⁵ represents pyridin-3-yl or 4-carboxyphenyl; or
R² represents fluorine, R³ represents benzylamino and R⁵ represents chlorine or 2-methoxy-pyrimidin-5-yl; or
R² represents fluorine, R³ represents quinolin-3-yl and R⁵ represents quinolin-3-yl; or
R² represents fluorine, R³ represents 3-(acetylamino)phenyl and R⁵ represents pyridin-3-yl, pyridin-3-ylamino or 2-methoxypyrimidin-5-yl; or
R² represents fluorine, R³ represents 4-sulfamoyl-phenyl and R⁵ represents chlorine; or
R² represents fluorine, R⁵ represents pyridin-3-ylamino and R³ represents benzylamino or 5-methylpyridin-3-yl; or
R³ represents H, R² represents pyridin-3-yl, pyridin-4-yl, 2-methylpyridin-4-yl, 2-methoxypyridin-4-yl, 2-aminopyridin-4-yl, 2,6-dimethylpyridin-4-yl, 3-(aminomethyl)phenyl, 4-hydroxyphenyl, 4-(hydroxymethyl)phenyl, 4-sulfamoyl-phenyl and R⁵ represents vinyl, methoxy, (3-carboxyphenyl)amino, (3-(methoxycarbonyl)phenyl)amino, (3-(dimethylcarbamoyl)phenyl)amino, (3-(methylcarbamoyl)phenyl)amino, pyridin-4-yl, (6-methyl-pyridin-2-yl)amino, (6-methoxy-pyridin-2-yl)amino, pyridin-3-ylamino, (6-methyl-pyridin-3-yl)amino or pyridin-3-yloxy;
R⁶ represents phenyl, 1*H*-pyrrol-2-yl or 1*H*-1,2,3-triazol-5-yl;
R⁷ represents pyridin-2-yl, 5-methyl-pyridin-2-yl, 2-methyl-pyridin-3-yl, 6-methyl-pyridin-3-yl, pyridin-4-yl, 3-methoxycarbonyl-pyridin-4-yl, 2-(methoxycarbonyl)phenyl, 3-hydroxy-phenyl or 3-amino-phenyl;
R⁸ represents pyridin-2-yl, 6-methoxy-pyridin-2-yl, 5-methoxy-pyridin-2-yl, 4-methoxy-pyridin-2-yl, 3-methyl-pyridin-2-yl, 4-methyl-pyridin-2-yl, 5-methyl-pyridin-2-yl, 6-methyl-pyridin-2-yl, 5-aminocarbonyl-pyridin-2-yl, 4-fluoro-pyridin-2-yl, 5-methoxycarbonyl-pyridin-2-yl, 6-methoxycarbonyl-pyridin-2-yl, pyridin-3-yl, 5-fluoro-pyridin-3-yl, 5-methoxy-pyridin-3-yl, 6-methoxy-pyridin-3-yl, 6-acetylamino-pyridin-3-yl, 6-methyl-pyridin-3-yl, pyridin-4-yl, 2-methoxy-pyridin-4-yl, 3-fluoro-pyridin-4-yl, pyrazin-2-yl, pyrimidin-2-yl, pyrimidin-4-yl, pyrimidin-5-yl, phenyl, 3-hydroxyphenyl, 3-methoxyphenyl, 3-carboxyphenyl, 3-carbamoylphenyl, 3-acetylamino-phenyl, 3-sulfamoylphenyl, 3-methoxycarbonyl-phenyl, 3-(carboxymethyl)phenyl, 3-(methylcarbamoyl)phenyl, 3-(dimethylcarbamoyl)phenyl, 3-(benzylcarbamoyl)phenyl, 3-(2-amino-2-oxoethyl)phenyl, 3-(2-(dimethylamino)-2-oxoethyl)phenyl, 3-(2-(benzylamino)-2-oxoethyl)phenyl, 4-methoxycarbonyl-phenyl, 4-carboxyphenyl, 3-carbamoyl-4-methoxyphenyl, 4-methoxy-3-(methylcarbamoyl)phenyl, 3-(dimethylcarbamoyl)-4-methoxyphenyl, 4-hydroxyphenyl, 4-(carboxymethyl)phenyl, 4-cyanophenyl, 4-acetylamino-phenyl, 4-carbamoylphenyl, 4-(methylcarbamoyl)phenyl, 4-(dimethylcarbamoyl)phenyl, 4-(2-amino-2-oxoethyl)phenyl, 4-(2-(dimethylamino)-2-oxoethyl)phenyl, 4-(2-(methylamino)-2-oxoethyl)phenyl, 4-(2-(benzylamino)-2-oxoethyl)phenyl, 5-methyl-1,3,4-oxadiazol-2-yl or imidazo[1,2-*a*]pyridin-7-yl;
R⁹ represents pyridin-2-yl, pyridin-3-yl, 1,3-dimethyl-1*H*-pyrazol-5-yl or 3-methylisothiazol-5-yl;
or a salt of such a compound.

3. A compound of formula I according to claim 1, which is also a compound of formula I_{P1} 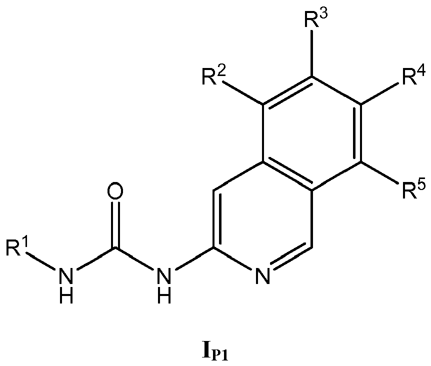 wherein
R¹ represents (C₁-C₃)alkyl, (C₂-C₃)haloalkyl or cyclopropyl;
R⁴ represents H;
R³ represents H, R⁵ represents H and R² represents phenyl or 4-carboxyphenyl; or
R² represents H, R⁵ represents H and R³ represents benzoylamino; or
R² represents H, R³ represents H and R⁵ represents benzoylamino, pyridin-3-ylmethylamino, pyridin-2-ylamino, pyridin-3-ylamino, pyridin-3-yloxy or 4-carboxyphenyl; or
R³ represents H, R⁵ represents methyl and R² represents halogen, (C₁-C₃)alkylamino, 2-(aminocarbonyl)-ethyl, imidazo[1,2-*a*]pyridin-6-yl, 4-methyl-thiophen-3-yl, isoquinolin-5-yl or 1*H*-indol-4-yl or a group having the formula (A1), (A2) or (A3) shown hereafter 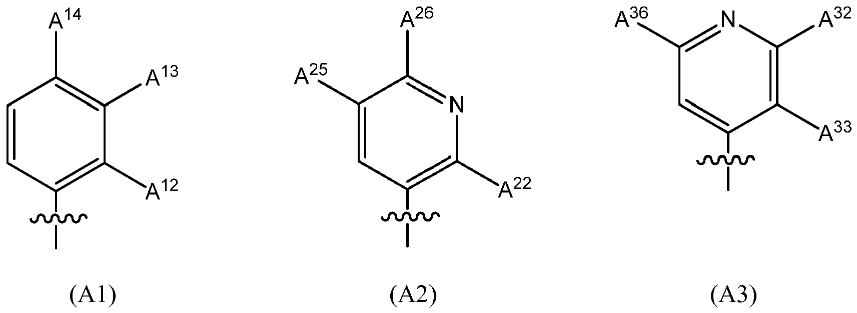
wherein
each of A¹³ and A¹⁴ represents H and A¹² represents OH, or
each of A¹² and A¹⁴ represents H and A¹³ represents OH, acetylamino, aminomethyl, aminosulfonyl or hydroxymethyl, or
each of A¹² and A¹³ represents H and A¹⁴ represents OH, methylaminocarbonyl, dimethylaminocarbonyl, methoxycarbonylamino, aminosulfonyl or hydroxymethyl;
each of A²⁵ and A²⁶ represents H and A²² represents H or halogen, or
each of A²² and A²⁶ represents H and A²⁵ represents methyl or halogen, or
each of A²² and A²⁵ represents H and A²⁶ represents hydroxymethyl;
each of A³³ and A³⁶ represents H and A³² represents H, halogen, amino, methyl or methoxy, or
A³³ represents H and each of A³² and A³⁶ represents methyl; or
R² represents H, R⁵ represents methyl and R³ represents amino, (C₁-C₄)alkylamino, cyclopropylmethylamino, 2-benzyloxy-ethylamino, (thiazol-5-yl)carbonylamino, pyridin-3-ylcarbonylamino, pyridin-4-ylcarbonylamino, pyridin-4-ylmethylamino, naphthalen-2-yl, furan-2-yl, furan-3-yl, 1*H*-pyrazol-4-yl, thiophen-3-yl, 4-methyl-thiophen-3-yl, quinolin-3-yl, isoquinolin-4-yl, isoquinolin-5-yl, imidazo[1,2-*a*]pyridin-6-yl, 1*H*-indol-4-yl or a group having the formula (B1), (B2), (B3), (B4), (B5), (B6), (B7), (B8) or (B9) shown hereafter 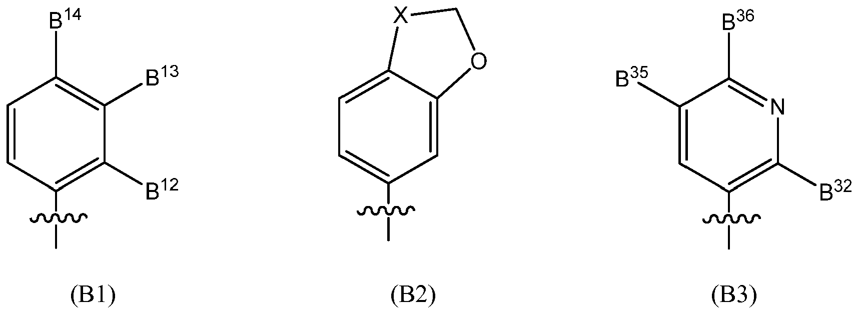 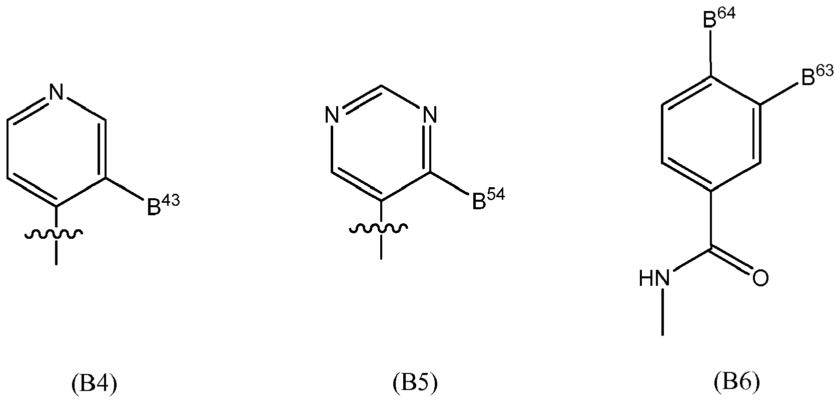 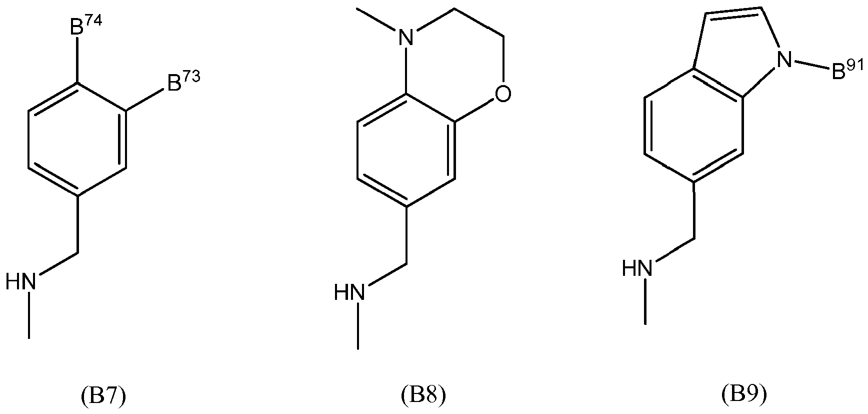
wherein
each of B¹³ and B¹⁴ represents H and B¹² represents OH, methyl, acetylamino, ethoxycarbonyl or dimethylaminocarbonyl, or
each of B¹² and B¹⁴ represents H and B¹³ represents OH, halogen, acetyl, acetylamino, acetylaminomethyl, aminocarbonyl, aminosulfonyl, (C₁-C₂)alkoxy, (C₁-C₂)alkoxycarbonyl, cyano, amino-(C₁-C₂)alkyl or hydroxy-(C₁-C₂)alkyl, or
each of B¹² and B¹³ represents H and B¹⁴ represents acetyl, acetylamino, acetylaminomethyl, aminocarbonyl, dimethylaminocarbonyl, aminosulfonyl, (C₁-C₂)alkylsulfonyl, cyanomethyl or hydroxymethyl;
X represents -O- or -CH₂-;
each of B³⁵ and B³⁶ represents H and B³² represents H or halogen, or
each of B³² and B³⁶ represents H and B³⁵ represents halogen, methyl, methylsulfanyl, methanesulfonyl or methoxycarbonyl, or
each of B³² and B³⁵ represents H and B³⁶ represents halogen, methoxy or hydroxymethyl; B⁴³ represents H or halogen;
B⁵⁴ represents H or (C₁-C₃)alkyl;
B⁶⁴ represents H and B⁶³ represents H or dimethylamino, or
B⁶³ represents H and B⁶⁴ represents methoxy, dimethylamino or methylsulfonyl;
B⁷⁴ represents H and B⁷³ represents H, methoxycarbonyl, carboxy or 2-hydroxyethoxy, or
B⁷³ represents H and B⁷⁴ represents carboxy, acetylamino or 3-methyl-[1,2,4]triazol-1-yl;
B⁹¹ represents H or methyl;
R² represents fluorine, R³ represents H and R⁵ represents bromine, benzyl, benzoylamino, 4-carboxyphenyl, benzylamino or (pyridin-3-yl)methylamino; or
R² represents bromine, R³ represents H and R⁵ represents methyl or methoxy; or
R² represents fluorine, R⁵ represents methyl and R³ represents H, quinolin-3-yl, benzylamino, a phenyl group optionally substituted once by acetylamino or aminosulfonyl, pyridin-2-yl, or a pyridin-3-yl group optionally substituted once by methyl; or
R² represents fluorine, R³ represents pyridin-3-yl and R⁵ represents pyridin-3-yl or 4-carboxyphenyl; or
R² represents fluorine, R³ represents benzylamino and R⁵ represents chlorine or 2-methoxy-pyrimidin-5-yl; or
R² represents fluorine, R³ represents quinolin-3-yl and R⁵ represents quinolin-3-yl; or
R² represents fluorine, R³ represents 3-acetylaminophenyl and R⁵ represents pyridin-3-yl or 2-methoxypyrimidin-5-yl; or
R² represents fluorine, R³ represents 4-aminosulfonylphenyl and R⁵ represents chlorine; or R² represents pyridin-4-yl, R³ represents H and R⁵ represents vinyl, methoxy or pyridin-4-yl;
or a salt of such a compound.

4. A compound of formula I according to one of claims 1 to 3, wherein R¹ represents (C₁-C₃)alkyl;
or a salt of such a compound.

5. A compound of formula I according to claim 4, wherein R¹ represents ethyl;
or a salt of such a compound.

6. A compound of formula I according to one of claims 1 to 5, wherein R² represents H;
or a salt of such a compound.

7. A compound of formula I according to claim 6, wherein R⁵ represents methyl;
or a salt of such a compound.

8. A compound of formula I according to claim 6, wherein:
- R³ represents a group (B1) wherein either each of B¹² and B¹⁴ represents H and B¹³ represents OH, halogen, acetyl, acetylamino, acetylaminomethyl, aminocarbonyl, dimethylaminocarbonyl, aminosulfonyl, (C₁-C₂)alkylsulfonyl, (C₁-C₂)alkoxy, (C₁-C₂)alkoxycarbonyl, cyano, amino-(C₁-C₂)alkyl or hydroxy-(C₁-C₂)alkyl, or each of B¹² and B¹³ represents H and B¹⁴ represents acetyl, acetylamino, acetylaminomethyl, aminocarbonyl, dimethylaminocarbonyl, aminosulfonyl, (C₁-C₂)alkylsulfonyl, cyanomethyl or hydroxymethyl; or
- R³ represents a group (B2) wherein X represents CH₂; or
- R³ represents a group (B3); or
- R³ represents a group (B4); or
- R³ represents a group (B6); or
- R³ represents quinolin-3-yl, imidazo[1,2-*a*]pyridin-6-yl, (thiazol-5-yl)carbonylamino, pyridin-3-ylcarbonylamino or pyridin-4-ylcarbonylamino;
or a salt of such a compound.

9. A compound of formula I according to one of claims 1 to 5, wherein R³ represents H;
or a salt of such a compound.

10. A compound of formula I according to claim 9, wherein R⁵ represents methyl;
or a salt of such a compound.

11. A compound of formula I according to claim 10, wherein:
- R² represents halogen; or
- R² represents a group (A1); or
- R² represents a group (A3);
or a salt of such a compound.

12. A compound of formula I according to claim 1, which is selected from the following:
- 1-(5-bromo-8-methyl-isoquinolin-3-yl)-3-ethyl-urea;
- 1-ethyl-3-[5-(4-hydroxy-phenyl)-8-methyl-isoquinolin-3-yl]-urea;
- 1-ethyl-3-[5-(2-hydroxy-phenyl)-8-methyl-isoquinolin-3-yl]-urea;
- *N*-{3-[3-(3-ethyl-ureido)-8-methyl-isoquinolin-5-yl]-phenyl}-acetamide;
- 4-[3-(3-ethyl-ureido)-8-methyl-isoquinolin-5-yl]-*N*,*N*-dimethyl-benzamide;
- 4-[3-(3-ethyl-ureido)-8-methyl-isoquinolin-5-yl]-*N*-methyl-benzamide;
- 1-ethyl-3-(5-imidazo[1,2-*a*]pyridin-6-yl-8-methyl-isoquinolin-3-yl)-urea;
- 1-ethyl-3-[5-(6-hydroxymethyl-pyridin-3-yl)-8-methyl-isoquinolin-3-yl]-urea;
- 1-ethyl-3-[5-(5-fluoro-pyridin-3-yl)-8-methyl-isoquinolin-3-yl]-urea;
- 1-ethyl-3-[8-methyl-5-(5-methyl-pyridin-3-yl)-isoquinolin-3-yl]-urea;
- 1-[5-((3-(aminomethyl)-phenyl)-8-methyl-isoquinolin-3-yl]-3-ethyl-urea;
- 1-ethyl-3-[5-(3-hydroxymethyl-phenyl)-8-methyl-isoquinolin-3-yl]-urea;
- 3-[3-(3-ethyl-ureido)-8-methyl-isoquinolin-5-yl]-benzenesulfonamide;
- 1-ethyl-3-(6-furan-3-yl-8-methyl-isoquinolin-3-yl)-urea;
- 1-ethyl-3-[6-(4-hydroxymethyl-phenyl)-8-methyl-isoquinolin-3-yl]-urea;
- 1-ethyl-3-(8-methyl-6-pyridin-3-yl-isoquinolin-3-yl)-urea;
- *N*-{3-[3-(3-ethyl-ureido)-8-methyl-isoquinolin-6-yl]-benzyl}-acetamide;
- *N*-{4-[3-(3-ethyl-ureido)-8-methyl-isoquinolin-6-yl]-phenyl}-acetamide;
- 1-ethyl-3-[6-(3-hydroxymethyl-phenyl)-8-methyl-isoquinolin-3-yl]-urea;
- 1-ethyl-3-[6-(3-hydroxy-phenyl)-8-methyl-isoquinolin-3-yl]-urea;
- 1-ethyl-3-(8-methyl-6-pyridin-4-yl-isoquinolin-3-yl)-urea;
- 3-[3-(3-ethyl-ureido)-8-methyl-isoquinolin-6-yl]-benzenesulfonamide;
- 2-[3-(3-ethyl-ureido)-8-methyl-isoquinolin-6-yl]-*N*,*N*-dimethyl-benzamide;
- 4-[3-(3-ethyl-ureido)-8-methyl-isoquinolin-6-yl]-*N*,*N*-dimethyl-benzamide;
- 1-ethyl-3-(6-imidazo[1,2-*a*]pyridin-6-yl-8-methyl-isoquinolin-3-yl)-urea;
- 1-[6-(3-(aminomethyl)-phenyl)-8-methyl-isoquinolin-3-yl]-3-ethyl-urea;
- 1-[5-(2-chloro-pyridin-4-yl)-8-methyl-isoquinolin-3-yl]-3-ethyl-urea;
- 1-ethyl-3-[8-methyl-6-(1*H*-pyrazol-4-yl)-isoquinolin-3-yl]-urea;
- 1-[6-(3-acetyl-phenyl)-8-methyl-isoquinolin-3-yl]-3-ethyl-urea;
- 4-[3-(3-ethyl-ureido)-8-methyl-isoquinolin-6-yl]-benzamide;
- 3-[3-(3-ethyl-ureido)-8-methyl-isoquinolin-6-yl]-benzamide;
- 1-(8-bromo-5-fluoro-isoquinolin-3-yl)-3-ethyl-urea;
- 4-[3-(3-ethyl-ureido)-5-fluoro-6-pyridin-3-yl-isoquinolin-8-yl]-benzoic acid;
- 1-ethyl-3-[5-(4-hydroxymethyl-phenyl)-8-methyl-isoquinolin-3-yl]-urea;
- 4-[3-(3-ethyl-ureido)-8-methyl-isoquinolin-5-yl]-benzenesulfonamide;
- 1-ethyl-3-(8-methyl-6-quinolin-3-yl-isoquinolin-3-yl)-urea;
- *N*-{3-[3-(3-ethyl-ureido)-8-methyl-isoquinolin-6-yl]-phenyl}-acetamide;
- 4-[3-(3-ethyl-ureido)-8-methyl-isoquinolin-6-yl]-benzenesulfonamide;
- 1-ethyl-3-[8-methyl-6-(5-methyl-pyridin-3-yl)-isoquinolin-3-yl]-urea;
- 1-ethyl-3-[8-methyl-5-(2-methyl-pyridin-4-yl)-isoquinolin-3-yl]-urea;
- 1-ethyl-3-[5-(2-fluoro-pyridin-4-yl)-8-methyl-isoquinolin-3-yl]-urea;
- 1-ethyl-3-[5-(2-methoxy-pyridin-4-yl)-8-methyl-isoquinolin-3-yl]-urea;
- 1-ethyl-3-(5-phenyl-isoquinolin-3-yl)-urea;
- 1-[5-(2,6-dimethyl-pyridin-4-yl)-8-methyl-isoquinolin-3-yl]-3-ethyl-urea;
- 1-[5-(2-amino-pyridin-4-yl)-8-methyl-isoquinolin-3-yl]-3-ethyl-urea;
- 1-(8-benzyl-5-fluoro-isoquinolin-3-yl)-3-ethyl-urea;
- 1-ethyl-3-(5-fluoro-8-methyl-6-pyridin-3-yl-isoquinolin-3-yl)-urea;
- 1-ethyl-3-(5-fluoro-8-methyl-6-pyridin-2-yl-isoquinolin-3-yl)-urea;
- 1-(6-benzylamino-8-chloro-5-fluoro-isoquinolin-3-yl)-3-ethyl-urea;
- 1-ethyl-3-(8-methyl-5-pyridin-3-yl-isoquinolin-3-yl)-urea;
- *N*-[3-(3-ethyl-ureido)-5-fluoro-isoquinolin-8-yl]-benzamide;
- *N*-[3-(3-ethyl-ureido)-isoquinolin-6-yl]-benzamide;
- 1-ethyl-3-(5-fluoro-6,8-di-pyridin-3-yl-isoquinolin-3-yl)-urea;
- 1-(6-amino-8-methyl-isoquinolin-3-yl)-3-ethyl-urea;
- 1-ethyl-3-{5-fluoro-8-[(pyridin-3-ylmethyl)-amino]-isoquinolin-3-yl}-urea;
- 1-(8-benzylamino-5-fluoro-isoquinolin-3-yl)-3-ethyl-urea;
- 1-(6-benzylamino-5-fluoro-8-methyl-isoquinolin-3-yl)-3-ethyl-urea;
- 1-(6-benzylamino-8-methyl-isoquinolin-3-yl)-3-ethyl-urea;
- 3-[3-(3-ethyl-ureido)-8-methyl-isoquinolin-5-yl]-propionamide;
- 1-ethyl-3-(5-fluoro-8-methyl-isoquinolin-3-yl)-urea;
- 1-ethyl-3-(8-methyl-5-pyridin-4-yl-isoquinolin-3-yl)-urea;
- thiazole-5-carboxylic acid [3-(3-ethyl-ureido)-8-methyl-isoquinolin-6-yl]-amide;
- 1-ethyl-3-(5-ethylamino-8-methyl-isoquinolin-3-yl)-urea;
- 1-ethyl-3-[8-methyl-5-(4-methyl-thiophen-3-yl)-isoquinolin-3-yl]-urea;
- {4-[3-(3-ethyl-ureido)-8-methyl-isoquinolin-5-yl]-phenyl}-carbamic acid methyl ester;
- 1-ethyl-3-[5-(2-fluoro-pyridin-3-yl)-8-methyl-isoquinolin-3-yl]-urea;
- 1-ethyl-3-(8-methyl-[5,5']biisoquinolinyl-3-yl)-urea;
- 1-ethyl-3-[5-(3-hydroxy-phenyl)-8-methyl-isoquinolin-3-yl]-urea;
- 1-ethyl-3-[5-(1*H*-indol-4-yl)-8-methyl-isoquinolin-3-yl]-urea;
- *N*-[3-(3-ethyl-ureido)-isoquinolin-8-yl]-benzamide;
- 1-ethyl-3- {8-[(pyridin-3-ylmethyl)-amino]-isoquinolin-3-yl}-urea;
- 1-(5,8-di-pyridin-4-yl-isoquinolin-3-yl)-3-ethyl-urea;
- 1-(5-bromo-8-methoxy-isoquinolin-3-yl)-3-ethyl-urea;
- 4-[3-(3-ethyl-ureido)-5-fluoro-isoquinolin-8-yl]-benzoic acid;
- 1-ethyl-3-(8-methyl-6-pyrimidin-5-yl-isoquinolin-3-yl)-urea;
- 1-ethyl-3-[6-(3-methoxy-phenyl)-8-methyl-isoquinolin-3-yl]-urea;
- 1-(6-(benzo[1,3]dioxol-5-yl)-8-methyl-isoquinolin-3-yl)-3-ethyl-urea;
- 1-ethyl-3-(6-furan-2-yl-8-methyl-isoquinolin-3-yl)-urea;
- 1-ethyl-3-(8-methyl-6-naphthalen-2-yl-isoquinolin-3-yl)-urea;
- 1-ethyl-3-[8-methyl-6-(4-methyl-thiophen-3-yl)-isoquinolin-3-yl]-urea;
- *N*-{4-[3-(3-ethyl-ureido)-8-methyl-isoquinolin-6-yl]-benzyl}-acetamide;
- 1-[6-(2-chloro-pyridin-3-yl)-8-methyl-isoquinolin-3-yl]-3-ethyl-urea;
- 1-[6-(2,3-dihydro-benzofuran-5-yl)-8-methyl-isoquinolin-3-yl]-3-ethyl-urea;
- 3-[3-(3-ethyl-ureido)-8-methyl-isoquinolin-6-yl]-benzoic acid ethyl ester;
- 2-[3-(3-ethyl-ureido)-8-methyl-isoquinolin-6-yl]-benzoic acid ethyl ester;
- *N*-{2-[3-(3-ethyl-ureido)-8-methyl-isoquinolin-6-yl]-phenyl}-acetamide;
- 5-[3-(3-ethyl-ureido)-8-methyl-isoquinolin-6-yl]-nicotinic acid methyl ester;
- 1-ethyl-3-[6-(3-fluoro-phenyl)-8-methyl-isoquinolin-3-yl]-urea;
- 1-ethyl-3-[6-(6-methoxy-pyridin-3-yl)-8-methyl-isoquinolin-3-yl]-urea;
- 1-ethyl-3-[6-(1*H*-indol-4-yl)-8-methyl-isoquinolin-3-yl]-urea;
- 1-ethyl-3-(8'-methyl-[4,6']biisoquinolinyl-3'-yl)-urea;
- 1-[6-(4-(cyanomethyl)-phenyl)-8-methyl-isoquinolin-3-yl]-3-ethyl-urea;
- 1-ethyl-3-(8-methyl-6-thiophen-3-yl-isoquinolin-3-yl)-urea;
- 1-ethyl-3-[6-(4-methanesulfonyl-phenyl)-8-methyl-isoquinolin-3-yl]-urea;
- 1-ethyl-3-[6-(4-isopropyl-pyrimidin-5-yl)-8-methyl-isoquinolin-3-yl]-urea;
- 1-ethyl-3-[8-methyl-6-(5-methylsulfanyl-pyridin-3-yl)-isoquinolin-3-yl]-urea;
- 1-ethyl-3-[6-(3-fluoro-pyridin-4-yl)-8-methyl-isoquinolin-3-yl]-urea;
- 1-[6-(3-chloro-pyridin-4-yl)-8-methyl-isoquinolin-3-yl]-3-ethyl-urea;
- 1-ethyl-3-[6-(6-fluoro-pyridin-3-yl)-8-methyl-isoquinolin-3-yl]-urea;
- 1-ethyl-3-[6-(2-fluoro-pyridin-3-yl)-8-methyl-isoquinolin-3-yl]-urea;
- 1-ethyl-3-[6-(6-hydroxymethyl-pyridin-3-yl)-8-methyl-isoquinolin-3-yl]-urea;
- 1-ethyl-3-[6-(5-fluoro-pyridin-3-yl)-8-methyl-isoquinolin-3-yl]-urea;
- 1-ethyl-3-[6-(5-methanesulfonyl-pyridin-3-yl)-8-methyl-isoquinolin-3-yl]-urea;
- 1-ethyl-3-(8'-methyl-[5,6']biisoquinolinyl-3'-yl)-urea;
- 1-ethyl-3-(8-methyl-6-o-tolyl-isoquinolin-3-yl)-urea;
- *N*-[3-(3-ethyl-ureido)-8-methyl-isoquinolin-6-yl]-benzamide;
- 1-[6-(3-cyano-phenyl)-8-methyl-isoquinolin-3-yl]-3-ethyl-urea;
- 1-[6-(4-acetyl-phenyl)-8-methyl-isoquinolin-3-yl]-3-ethyl-urea;
- 1-ethyl-3-[6-(2-hydroxy-phenyl)-8-methyl-isoquinolin-3-yl]-urea;
- 1-[6-benzylamino-5-fluoro-8-(2-methoxy-pyrimidin-5-yl)-isoquinolin-3-yl]-3-ethyl-urea;
- 4-[8-chloro-3-(3-ethyl-ureido)-5-fluoro-isoquinolin-6-yl]-benzenesulfonamide;
- 1-ethyl-3-(5-fluoro-6,8-di-quinolin-3-yl-isoquinolin-3-yl)-urea;
- *N*-{3-[3-(3-ethyl-ureido)-5-fluoro-8-pyridin-3-yl-isoquinolin-6-yl]-phenyl}-acetamide;
- *N*-{3-[3-(3-ethyl-ureido)-5-fluoro-8-(2-methoxy-pyrimidin-5-yl)-isoquinolin-6-yl]-phenyl} -acetamide;
- 1-ethyl-3-(8-methyl-6-propylamino-isoquinolin-3-yl)-urea;
- *N*-[3-(3-ethyl-ureido)-8-methyl-isoquinolin-6-yl]-isonicotinamide;
- *N*-[3-(3-ethyl-ureido)-8-methyl-isoquinolin-6-yl]-4-methanesulfonyl-benzamide;
- 3-dimethylamino-*N*-[3-(3-ethyl-ureido)-8-methyl-isoquinolin-6-yl]-benzamide;
- *N*-[3-(3-ethyl-ureido)-8-methyl-isoquinolin-6-yl]-nicotinamide;
- *N*-[3-(3-ethyl-ureido)-8-methyl-isoquinolin-6-yl]-4-methoxy-benzamide;
- 4-dimethylamino-*N*-[3-(3-ethyl-ureido)-8-methyl-isoquinolin-6-yl]-benzamide;
- 1-ethyl-3- {8-methyl-6-[(pyridin-4-ylmethyl)-amino]-isoquinolin-3-yl}-urea;
- 4-[3-(3-ethyl-ureido)-isoquinolin-8-yl]-benzoic acid;
- 4-[3-(3-ethyl-ureido)-isoquinolin-5-yl]-benzoic acid;
- 1-ethyl-3-(8-methoxy-5-pyridin-4-yl-isoquinolin-3-yl)-urea;
- 3-{[3-(3-ethyl-ureido)-8-methyl-isoquinolin-6-ylamino]-methyl}-benzoic acid methyl ester;
- 4-{[3-(3-ethyl-ureido)-8-methyl-isoquinolin-6-ylamino]-methyl}-benzoic acid;
- 3-{[3-(3-ethyl-ureido)-8-methyl-isoquinolin-6-ylamino]-methyl}-benzoic acid;
- 1-ethyl-3-{6-[3-(2-hydroxy-ethoxy)-benzylamino]-8-methyl-isoquinolin-3-yl}-urea;
- 1-ethyl-3-{8-methyl-6-[(4-methyl-3,4-dihydro-2*H*-benzo[1,4]oxazin-7-ylmethyl)-amino]-isoquinolin-3-yl}-urea;
- 1-ethyl-3-{8-methyl-6-[4-(3-methyl-[1,2,4]triazol-1-yl)-benzylamino]-isoquinolin-3-yl}-urea;
- 1-[6-(2-benzyloxy-ethylamino)-8-methyl-isoquinolin-3-yl]-3-ethyl-urea;
- 1-{6-[(cyclopropylmethyl)-amino]-8-methyl-isoquinolin-3-yl}-3-ethyl-urea;
- 1-ethyl-3-{6-[(1*H*-indol-6-ylmethyl)-amino]-8-methyl-isoquinolin-3-yl}-urea;
- 1-ethyl-3-{8-methyl-6-[((1-methyl-1*H*-indol-6-yl)methyl)-amino]-isoquinolin-3-yl}-urea;
- 1-ethyl-3-(6-isobutylamino-8-methyl-isoquinolin-3-yl)-urea;
- *N*-(4-{[3-(3-ethyl-ureido)-8-methyl-isoquinolin-6-ylamino]-methyl}-phenyl)-acetamide;
- 1-(2-fluoro-ethyl)-3-(8-methyl-5-pyridin-4-yl-isoquinolin-3-yl)-urea;
- 1-cyclopropyl-3-(8-methyl-5-pyridin-4-yl-isoquinolin-3-yl)-urea;
- 1-ethyl-3-[5-(pyridin-4-yl)-8-vinylisoquinolin-3-yl]-urea;
- 1-ethyl-3-[5-fluoro-8-methyl-6-(5-methylpyridin-3-yl)isoquinolin-3-yl]-urea;
- *N*-{3-[3-(3-ethyl-ureido)-5-fluoro-8-methyl-isoquinolin-6-yl]-phenyl}-acetamide;
- 1-ethyl-3-[5-fluoro-8-methyl-6-(quinolin-3-yl)-isoquinolin-3-yl]-urea;
- 4-[3-(3-ethyl-ureido)-5-fluoro-8-methyl-isoquinolin-6-yl]-benzenesulfonamide;
- 1-ethyl-3-[8-(pyridin-3-ylamino)-isoquinolin-3-yl]-urea;
- 1-ethyl-3-[8-(pyridin-2-ylamino)-isoquinolin-3-yl]-urea;
- 1-ethyl-3-[8-(pyridin-3-yloxy)-isoquinolin-3-yl]-urea;
- 1-ethyl-3-[8-(pyrazin-2-ylamino)-isoquinolin-3-yl]-urea;
- 1-ethyl-3-[8-(pyrimidin-5-ylamino)-isoquinolin-3-yl]-urea;
- 1-ethyl-3-[8-(imidazo[1,2-a]pyridin-7-ylamino)-isoquinolin-3-yl]-urea;
- 1-ethyl-3-[8-(pyrimidin-4-ylamino)-isoquinolin-3-yl]-urea;
- 1-ethyl-3-[8-(pyrimidin-2-ylamino)-isoquinolin-3-yl]-urea;
- 1-ethyl-3-[8-(pyridin-4-ylamino)-isoquinolin-3-yl]-urea;
- 1-ethyl-3-[8-(5-methyl-[1,3,4]oxadiazol-2-ylamino)-isoquinolin-3-yl]-urea;
- 1-ethyl-3-[8-(6-methyl-pyridin-2-ylamino)-isoquinolin-3-yl]-urea;
- 6-[3-(3-ethyl-ureido)-isoquinolin-8-ylamino]-nicotinamide;
- 1-ethyl-3-[8-(3-fluoro-pyridin-4-ylamino)-isoquinolin-3-yl]-urea;
- 1-ethyl-3-[8-(4-fluoro-pyridin-2-ylamino)-isoquinolin-3-yl]-urea;
- 1-ethyl-3-[8-(5-fluoro-pyridin-3-ylamino)-isoquinolin-3-yl]-urea;
- 1-ethyl-3-[8-(4-methoxy-pyridin-2-ylamino)-isoquinolin-3-yl]-urea;
- *N*-{5-[3-(3-ethyl-ureido)-isoquinolin-8-ylamino]-pyridin-2-yl}-acetamide;
- 1-ethyl-3-[8-(4-methyl-pyridin-2-ylamino)-isoquinolin-3-yl]-urea;
- 1-ethyl-3-[8-(5-methyl-pyridin-2-ylamino)-isoquinolin-3-yl]-urea;
- 1-ethyl-3-[8-(6-methyl-pyridin-3-ylamino)-isoquinolin-3-yl]-urea;
- 6-[3-(3-ethyl-ureido)-isoquinolin-8-ylamino]-nicotinic acid methyl ester;
- 6-[3-(3-ethyl-ureido)-isoquinolin-8-ylamino]-pyridine-2-carboxylic acid methyl ester;
- 1-ethyl-3-[8-(3-methyl-pyridin-2-ylamino)-isoquinolin-3-yl]-urea;
- 1-ethyl-3-[8-(3-methoxy-phenylamino)-isoquinolin-3-yl]-urea;
- 1-ethyl-3-(8-phenylamino-isoquinolin-3-yl)-urea;
- 1-ethyl-3-[8-(3-hydroxy-phenylamino)-isoquinolin-3-yl]-urea;
- 3-[3-(3-ethyl-ureido)-isoquinolin-8-ylamino]-benzenesulfonamide;
- *N*-{3-[3-(3-ethyl-ureido)-isoquinolin-8-ylamino]-phenyl}-acetamide;
- *N*-{4-[3-(3-ethyl-ureido)-isoquinolin-8-ylamino]-phenyl}-acetamide;
- 1-[8-(4-cyano-phenylamino)-isoquinolin-3-yl]-3-ethyl-urea;
- 1-ethyl-3-[8-(4-hydroxy-phenylamino)-isoquinolin-3-yl]-urea;
- 3-[3-(3-ethyl-ureido)-isoquinolin-8-ylamino]-benzoic acid methyl ester;
- 1-ethyl-3-[8-(5-methoxy-pyridin-3-ylamino)-isoquinolin-3-yl]-urea;
- 1-ethyl-3-[8-(6-methoxy-pyridin-2-ylamino)-isoquinolin-3-yl]-urea;
- 4-[3-(3-ethyl-ureido)-isoquinolin-8-ylamino]-benzoic acid methyl ester;
- 1-ethyl-3-[8-(2-methoxy-pyridin-4-ylamino)-isoquinolin-3-yl]-urea;
- 1-ethyl-3-[8-(5-methoxy-pyridin-2-ylamino)-isoquinolin-3-yl]-urea;
- 1*H*-pyrrole-2-carboxylic acid [3-(3-ethyl-ureido)-isoquinolin-8-yl]-amide;
- 3*H*-[1,2,3]triazole-4-carboxylic acid [3-(3-ethyl-ureido)-isoquinolin-8-yl]-amide;
- 1-ethyl-3-{6-[(1*H*-indazol-6-ylmethyl)-amino]-8-methyl-isoquinolin-3-yl}-urea;
- 1-ethyl-3-{8-methyl-6-[(2-morpholin-4-yl-pyridin-4-ylmethyl)-amino]-isoquinolin-3-yl}-urea;
- 2-(4-{[3-(3-ethyl-ureido)-8-methyl-isoquinolin-6-ylamino]-methyl}-phenoxy)-acetamide;
- 1-ethyl-3-[8-methyl-6-(3-[1,2,4]triazol-1-yl-benzylamino)-isoquinolin-3-yl]-urea;
- 1-{6-[(3-((1*H*-1,2,4-triazol-1-yl)methyl)benzyl)amino]-8-methylisoquinolin-3-yl}-3-ethylurea;
- 4-{[3-(3-ethyl-ureido)-8-methyl-isoquinolin-6-ylamino]-methyl}-*N*-methyl-benzenesulfonamide;
- 1-ethyl-3-[8-(pyridin-3-ylaminomethyl)-isoquinolin-3-yl]-urea;
- 1-{8-[(2,5-dimethyl-2*H*-pyrazol-3-ylamino)-methyl]-isoquinolin-3-yl}-3-ethyl-urea;
- 1-ethyl-3-{8-[(3-methyl-isothiazol-5-ylamino)-methyl]-isoquinolin-3-yl}-urea;
- *N*-[3-(3-ethyl-ureido)-8-methyl-isoquinolin-6-yl]-terephthalamic acid methyl ester;
- *N*-[3-(3-ethyl-ureido)-8-methyl-isoquinolin-6-yl]-isophthalamic acid methyl ester;
- *N*-[3-(3-ethyl-ureido)-8-methyl-isoquinolin-6-yl]-2-methoxy-acetamide;
- 1-ethyl-3-{6-[(6-methoxy-pyridin-3-ylmethyl)-amino]-8-methyl-isoquinolin-3-yl}-urea;
- 1-ethyl-3-[6-(3-methoxy-benzylamino)-8-methyl-isoquinolin-3-yl]-urea;
- 3-[3-(3-ethyl-ureido)-isoquinolin-8-ylamino]-benzoic acid;
- 3-[3-(3-ethyl-ureido)-isoquinolin-8-ylamino]-*N*,*N*-dimethyl-benzamide;
- 3-[3-(3-ethyl-ureido)-isoquinolin-8-ylamino]-*N*-methyl-benzamide;
- *N*-benzyl-3-[3-(3-ethyl-ureido)-isoquinolin-8-ylamino]-benzamide;
- 4-[3-(3-ethyl-ureido)-isoquinolin-8-ylamino]-benzoic acid;
- 4-[3-(3-ethyl-ureido)-isoquinolin-8-ylamino]-*N*,*N*-dimethyl-benzamide;
- 4-[3-(3-ethyl-ureido)-isoquinolin-8-ylamino]-*N*-methyl-benzamide;
- {4-[3-(3-ethyl-ureido)-isoquinolin-8-ylamino]-phenyl}-acetic acid;
- 2-{4-[3-(3-ethyl-ureido)-isoquinolin-8-ylamino]-phenyl}-*N*,*N*-dimethyl-acetamide;
- 2-{4-[3-(3-ethyl-ureido)-isoquinolin-8-ylamino]-phenyl}-*N*-methyl-acetamide;
- *N*-benzyl-2-{4-[3-(3-ethyl-ureido)-isoquinolin-8-ylamino]-phenyl}-acetamide;
- {3-[3-(3-ethyl-ureido)-isoquinolin-8-ylamino]-phenyl}-acetic acid;
- *N*-benzyl-2-{3-[3-(3-ethyl-ureido)-isoquinolin-8-ylamino]-phenyl}-acetamide;
- 2-{3-[3-(3-ethyl-ureido)-isoquinolin-8-ylamino]-phenyl}-*N*,*N*-dimethyl-acetamide;
- 4-[3-(3-ethyl-ureido)-isoquinolin-8-ylmethoxy]-nicotinic acid methyl ester;
- 1-ethyl-3-[8-(3-hydroxy-phenoxymethyl)-isoquinolin-3-yl]-urea;
- 1-ethyl-3-[8-(6-methoxy-pyridin-3-ylamino)-isoquinolin-3-yl]-urea;
- 1-ethyl-3-(5-fluoro-8-pyridin-3-yl-isoquinolin-3-yl)-urea;
- 1-ethyl-3-(8-methyl-6-vinyl-isoquinolin-3-yl)-urea;
- 1-ethyl-3-[8-(6-methoxy-pyridin-2-ylamino)-5-pyridin-4-yl-isoquinolin-3-yl]-urea;
- 1-ethyl-3-[8-(6-methyl-pyridin-3-ylamino)-5-pyridin-4-yl-isoquinolin-3-yl]-urea;
- 1-ethyl-3-[8-(6-methyl-pyridin-2-ylamino)-5-pyridin-4-yl-isoquinolin-3-yl]-urea;
- 3-[3-(3-ethyl-ureido)-5-pyridin-4-yl-isoquinolin-8-ylamino]-benzoic acid methyl ester;
- 1-ethyl-3-[5-(4-hydroxymethyl-phenyl)-8-(pyridin-3-yloxy)-isoquinolin-3-yl]-urea;
- 1-ethyl-3-[5-pyridin-4-yl-8-(pyridin-3-yloxy)-isoquinolin-3-yl]-urea;
- 1-ethyl-3-[5-pyridin-3-yl-8-(pyridin-3-yloxy)-isoquinolin-3-yl]-urea;
- 1-ethyl-3-[5-pyridin-3-yl-8-(pyridin-3-ylamino)-isoquinolin-3-yl]-urea;
- 1-ethyl-3-[5-(2-methoxy-pyridin-4-yl)-8-(pyridin-3-ylamino)-isoquinolin-3-yl]-urea;
- 1-ethyl-3-[5-(4-hydroxymethyl-phenyl)-8-(pyridin-3-ylamino)-isoquinolin-3-yl]-urea;
- 1-ethyl-3-[5-pyridin-4-yl-8-(pyridin-3-ylamino)-isoquinolin-3-yl]-urea;
- 1-ethyl-3-[5-(4-hydroxy-phenyl)-8-(pyridin-3-ylamino)-isoquinolin-3-yl]-urea;
- 1-ethyl-3-[5-(2-methyl-pyridin-4-yl)-8-(pyridin-3-ylamino)-isoquinolin-3-yl]-urea;
- 4-[3-(3-ethyl-ureido)-8-(pyridin-3-ylamino)-isoquinolin-5-yl]-benzenesulfonamide;
- 1-[5-(2,6-dimethyl-pyridin-4-yl)-8-(pyridin-3-ylamino)-isoquinolin-3-yl]-3-ethyl-urea;
- 1-(5-(3-(aminomethyl)phenyl)-8-(pyridin-3-ylamino)isoquinolin-3-yl)-3-ethylurea;
- 1-[5-(2-amino-pyridin-4-yl)-8-(pyridin-3-ylamino)-isoquinolin-3-yl]-3-ethyl-urea;
- *N*-{3-[3-(3-ethyl-ureido)-5-fluoro-8-(pyridin-3-ylamino)-isoquinolin-6-yl]-phenyl}-acetamide;
- 1-ethyl-3-[5-fluoro-6-(5-methyl-pyridin-3-yl)-8-(pyridin-3-ylamino)-isoquinolin-3-yl]-urea;
- *N*-{3-[3-(3-ethyl-ureido)-8-(pyridin-3-ylamino)-isoquinolin-6-yl]-phenyl}-acetamide;
- 1-ethyl-3-[6-(5-methyl-pyridin-3-yl)-8-(pyridin-3-ylamino)-isoquinolin-3-yl]-urea;
- 1-ethyl-3-[6-propylamino-8-(pyridin-3-ylamino)-isoquinolin-3-yl]-urea;
- 1-[6-benzylamino-8-(pyridin-3-ylamino)-isoquinolin-3-yl]-3-ethyl-urea;
- 1-[6-benzylamino-5-fluoro-8-(pyridin-3-ylamino)-isoquinolin-3-yl]-3-ethyl-urea;
- 3-dimethylamino-*N*-[3-(3-ethyl-ureido)-8-(pyridin-3-ylamino)-isoquinolin-6-yl]-benzamide;
- 5-[3-(3-ethyl-ureido)-isoquinolin-8-ylamino]-2-methoxy-benzamide;
- 5-[3-(3-ethyl-ureido)-isoquinolin-8-ylamino]-2-methoxy-*N*-methyl-benzamide;
- 5-[3-(3-ethyl-ureido)-isoquinolin-8-ylamino]-2-methoxy-*N*,*N*-dimethyl-benzamide;
- 1-ethyl-3-[5-(2-methyl-pyridin-4-yl)-8-(6-methyl-pyridin-3-ylamino)-isoquinolin-3-yl]-urea;
- 3-[3-(3-ethyl-ureido)-5-(2-methyl-pyridin-4-yl)-isoquinolin-8-ylamino]-benzoic acid;
- 3-[3-(3-ethyl-ureido)-5-(2-methyl-pyridin-4-yl)-isoquinolin-8-ylamino]-*N*-methyl-benzamide;
- 3-[3-(3-ethyl-ureido)-5-(2-methyl-pyridin-4-yl)-isoquinolin-8-ylamino]-*N*,*N*-dimethyl-benzamide;
- 3-[3-(3-ethyl-ureido)-5-(4-hydroxymethyl-phenyl)-isoquinolin-8-ylamino]-benzoic acid;
- 3-[3-(3-ethyl-ureido)-5-(4-hydroxymethyl-phenyl)-isoquinolin-8-ylamino]-*N*-methyl-benzamide;
- 3-[3-(3-ethyl-ureido)-5-(4-hydroxymethyl-phenyl)-isoquinolin-8-ylamino]-*N*,*N*-dimethyl-benzamide;
- 4-[3-(3-ethyl-ureido)-isoquinolin-8-ylamino]-benzamide;
- 2-{4-[3-(3-ethyl-ureido)-isoquinolin-8-ylamino]-phenyl}-acetamide;
- 3-[3-(3-ethyl-ureido)-isoquinolin-8-ylamino]-benzamide;
- 2-{3-[3-(3-ethyl-ureido)-isoquinolin-8-ylamino]-phenyl}-acetamide;
- 1-ethyl-3-[8-(6-methyl-pyridin-3-yloxymethyl)-isoquinolin-3-yl]-urea;
- 1-ethyl-3-[8-(5-methyl-pyridin-2-yloxymethyl)-isoquinolin-3-yl]-urea;
- 2-[3-(3-ethyl-ureido)-isoquinolin-8-ylmethoxy]-benzoic acid methyl ester;
- 1-ethyl-3-[8-(2-methyl-pyridin-3-yloxymethyl)-isoquinolin-3-yl]-urea;
- 1-[8-(3-amino-phenoxymethyl)-isoquinolin-3-yl]-3-ethyl-urea;
- 1-ethyl-3-[8-(pyridin-2-yloxymethyl)-isoquinolin-3-yl]-urea;
- 1-ethyl-3-[8-(pyridin-4-yloxymethyl)-isoquinolin-3-yl]-urea;
- 1-ethyl-3-[8-(pyridin-2-ylaminomethyl)-isoquinolin-3-yl]-urea;
- 5-[3-(3-ethyl-ureido)-isoquinolin-8-ylamino]-2-methyl-benzoic acid methyl ester;
- 5-[3-(3-ethyl-ureido)-isoquinolin-8-ylamino]-2-methyl-benzoic acid;
- 4-[3-(3-ethyl-ureido)-isoquinolin-8-ylamino]-2-methyl-benzoic acid methyl ester;
- 4-[3-(3-ethyl-ureido)-isoquinolin-8-ylamino]-2-methyl-benzoic acid;
- 1-[5,8-*bis*-(pyridin-3-ylamino)-isoquinolin-3-yl]-3-ethyl-urea;
- 1-ethyl-3-(8-methyl-5-methylaminomethyl-isoquinolin-3-yl)-urea;
- 1-ethyl-3- {5-[(methylamino)methyl]-8-(pyridin-3-ylamino)-isoquinolin-3-yl}-urea;
- 1-ethyl-3- {8-[(6-methoxy-pyridin)-2-ylamino]-5-[(methylamino)methyl]-isoquinolin-3-yl}-urea;
- 3-{[3-(3-ethyl-ureido)-5-[(methylamino)methyl]-isoquinolin-8-yl]amino}-benzoic acid;
- 1-{5-[(dimethylamino)methyl]-8-[(6-methoxy-pyridin-2-yl)amino]-isoquinolin-3-yl}-3-ethyl-urea;
- 1-ethyl-3-[5-morpholin-4-yl-8-(pyridin-3-ylamino)-isoquinolin-3-yl]-urea;
- 1-ethyl-3- {8-[(6-methoxy-pyridin-2-yl)amino]-5-(morpholin-4-ylmethyl)-isoquinolin-3-yl}-urea;
- 1-ethyl-3-[8-(6-methoxy-pyridin-2-ylamino)-5-(4-methyl-piperazin-1-ylmethyl)-isoquinolin-3-yl]-urea;
- 1-ethyl-3-[5-(4-methyl-piperazin-1-yl)-8-(pyridin-3-ylamino)-isoquinolin-3-yl]-urea;
- 1-ethyl-3-(8-methyl-5-pyrimidin-4-yl-isoquinolin-3-yl)-urea;
- 1-ethyl-3-(8-methyl-5-pyridazin-4-yl-isoquinolin-3-yl)-urea;
- 1-ethyl-3-[5-(1*H*-imidazol-4-yl)-8-methyl-isoquinolin-3-yl]-urea;
- 1-ethyl-3-[8-methyl-5-(1*H*-pyrazol-4-yl)-isoquinolin-3-yl]-urea;
- 1-ethyl-3-(8-methyl-5-pyridazin-3-yl-isoquinolin-3-yl)-urea;
- 1-ethyl-3-[8-methyl-5-(1-methyl-1*H*-imidazol-4-yl)-isoquinolin-3-yl]-urea;
- 1-ethyl-3-[8-methyl-5-(1-methyl-1*H*-pyrazol-4-yl)-isoquinolin-3-yl]-urea;
- 1-ethyl-3-(8-methyl-5-thiazol-4-yl-isoquinolin-3-yl)-urea;
- 1-(8-chloro-5-pyrimidin-4-yl-isoquinolin-3-yl)-3-ethyl-urea;
- 1-ethyl-3-[8-(6-methyl-pyridin-3-ylamino)-5-pyrimidin-4-yl-isoquinolin-3-yl]-urea;
- 1-[8-chloro-5-(1*H*-imidazol-4-yl)-isoquinolin-3-yl]-3-ethyl-urea;
- 1-(8-chloro-5-pyrimidin-4-yl-isoquinolin-3-yl)-3-ethyl-urea;
- 1-ethyl-3-[8-(6-methoxy-pyridin-2-ylamino)-5-thiazol-4-yl-isoquinolin-3-yl]-urea;
- 1-ethyl-3-[8-(2-methoxy-pyridin-3-ylamino)-isoquinolin-3-yl]-urea;
- 1-ethyl-3-[8-(4-hydroxymethyl-pyrazol-1-ylmethyl)-isoquinolin-3-yl]-urea;
- 1-ethyl-3-[8-(5-hydroxymethyl-imidazol-1-ylmethyl)-isoquinolin-3-yl]-urea;
- 1-ethyl-3-[8-(4-hydroxymethyl-imidazol-1-ylmethyl)-isoquinolin-3-yl]-urea;
- 1-[3-(3-ethyl-ureido)-isoquinolin-8-ylmethyl]-1*H*-imidazole-2-carboxylic acid;
- 1-ethyl-3-(8-pyrazol-1-ylmethyl-isoquinolin-3-yl)-urea;
- 1-[8-(2,4-dimethyl-imidazol-1-ylmethyl)-isoquinolin-3-yl]-3-ethyl-urea;
- 1-[8-(2,5-dimethyl-imidazol-1-ylmethyl)-isoquinolin-3-yl]-3-ethyl-urea;
- 1-ethyl-3-[8-(2-methyl-imidazol-1-ylmethyl)-isoquinolin-3-yl]-urea;
- 1-ethyl-3-(8-[1,2,3]triazol-1-ylmethyl-isoquinolin-3-yl)-urea;
- 1-ethyl-3-(8-imidazol-1-ylmethyl-isoquinolin-3-yl)-urea;
- 1-ethyl-3-[8-(4-methyl-pyrazol-1-ylmethyl)-isoquinolin-3-yl]-urea;
- 1-ethyl-3-{8-[(5-methyl-isoxazol-3-ylamino)-methyl]-isoquinolin-3-yl}-urea;
- 1-ethyl-3-{8-[(6-methoxy-pyridin-3-ylamino)-methyl]-isoquinolin-3-yl}-urea;
- 1-ethyl-3-{8-[(2-methyl-2*H*-pyrazol-3-ylamino)-methyl]-isoquinolin-3-yl}-urea;
- 1-ethyl-3-[8-(pyrimidin-2-ylaminomethyl)-isoquinolin-3-yl]-urea;
or a salt of such a compound.

13. A compound of formula I as defined in one of claims 1 to 12, or a pharmaceutically acceptable salt thereof, for use as a medicament.

14. A pharmaceutical composition containing, as active principle, a compound of formula I as defined in one of claims 1 to 12, or a pharmaceutically acceptable salt thereof, and at least one therapeutically inert excipient.

15. A compound of formula I as defined in one of claims 1 to 12, or a pharmaceutically acceptable salt thereof, for use in the prevention or treatment of a bacterial infection.

## Patentansprüche

1. Verbindung von Formel I 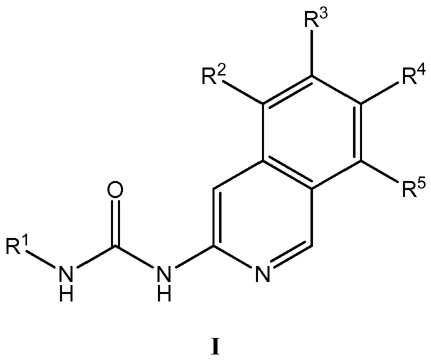 wobei
R¹ (C₁-C₃)Alkyl, (C₂-C₃)Haloalkyl oder Cyclopropyl repräsentiert;
R⁴ H repräsentiert;
R³ H repräsentiert, R⁵ H repräsentiert und R² Phenyl oder 4-Carboxyphenyl repräsentiert; oder
R² H repräsentiert, R⁵ H repräsentiert und R³ Benzoylamino repräsentiert; oder
R² H repräsentiert, R³ H repräsentiert und R⁵ -NH-CO-R⁶, -CH₂-O-R⁷, -NH-R⁸, -CH₂-NH-R⁹, -CH₂-R¹⁰, (Pyridin-3-ylmethyl)amino, Pyridin-3-yloxy oder 4-Carboxyphenyl repräsentiert; oder
R³ H repräsentiert, R⁵ Methyl repräsentiert und R² Folgendes repräsentiert: Halogen, (C₁-C₃)Alkylamino, (C₁-C₃)Alkylaminomethyl, 2-(Aminocarbonyl)-ethyl, Pyrimidin-4-yl, Pyridazin-3-yl, Pyridazin-4-yl, 1*H*-Imidazol-4-yl, 1-Methyl-1*H*-imidazol-4-yl, 1*H*-Pyrazol-4-yl, 1-Methyl-1*H*-pyrazol-4-yl, Imidazo[1,2-*a*]pyridin-6-yl, 4-Methyl-thiophen-3-yl, Thiazol-4-yl, Isochinolin-5-yl oder 1*H*-Indol-4-yl oder eine Gruppe der Formel (A1), (A2) oder (A3), wie nachfolgend gezeigt 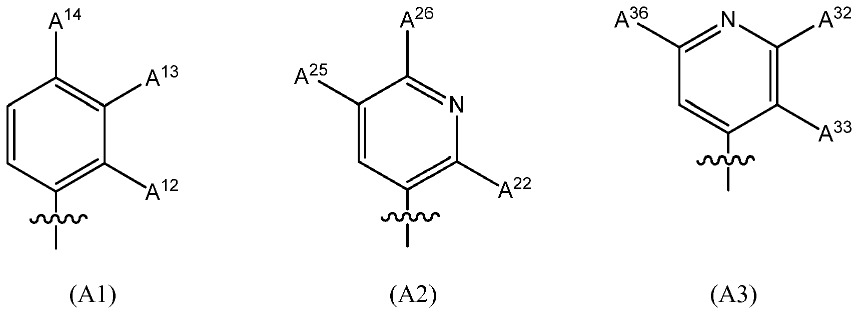
wobei
A¹³ und A¹⁴ jeweils H repräsentieren und A¹² H oder OH repräsentiert, oder
A¹² und A¹⁴ jeweils H repräsentieren und A¹³ OH, Acetylamino, Aminomethyl, Sulfamoyl oder Hydroxymethyl repräsentiert, oder
A¹² und A¹³ jeweils H repräsentieren und A¹⁴ OH, Methylaminocarbonyl, Dimethylaminocarbonyl, Methoxycarbonylamino, Sulfamoyl oder Hydroxymethyl repräsentiert;
A²⁵ und A²⁶ jeweils H repräsentieren und A²² H oder Halogen repräsentiert, oder
A²² und A²⁶ jeweils H repräsentieren und A²⁵ Methyl oder Halogen repräsentiert, oder
A²² und A²⁵ jeweils H repräsentieren und A²⁶ Hydroxymethyl repräsentiert;
A³³ und A³⁶ jeweils H repräsentieren und A³² H, Halogen, Amino, Methyl oder Methoxy repräsentiert, oder
A³³ H repräsentiert und A³² und A³⁶ jeweils Methyl repräsentieren; oder
R² H repräsentiert, R⁵ Methyl repräsentiert und R³ Folgendes repräsentiert: Amino, Vinyl, (C₁-C₄)Alkylamino, Cyclopropylmethylamino, (2-(Benzyloxy)ethyl)amino, Methoxymethylcarbonylamino, (Thiazol-5-yl)carbonylamino, Pyridin-3-ylcarbonylamino, Pyridin-4-ylcarbonylamino, Naphthalen-2-yl, Furan-2-yl, Furan-3-yl, 1*H*-Pyrazol-4-yl, Thiophen-3-yl, 4-Methyl-thiophen-3-yl, Chinolin-3-yl, Isochinolin-4-yl, Isochinolin-5-yl, Imidazo[1,2-*a*]pyridin-6-yl, 1*H*-Indol-4-yl oder eine Gruppe der Formel (B1), (B2), (B3), (B4), (B5), (B6), (B7), (B8), (B9), (B10), (B11) oder (B12), wie nachfolgend gezeigt 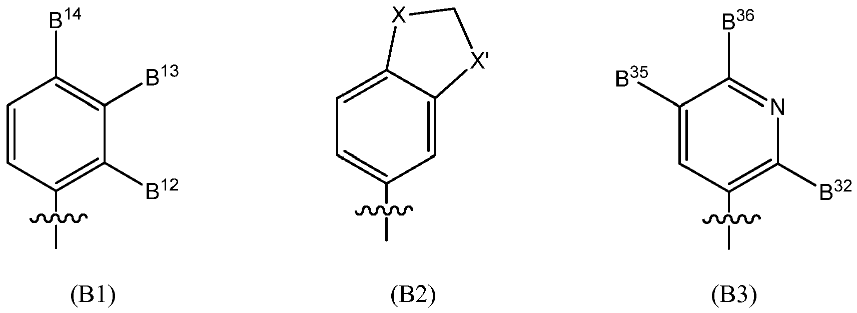 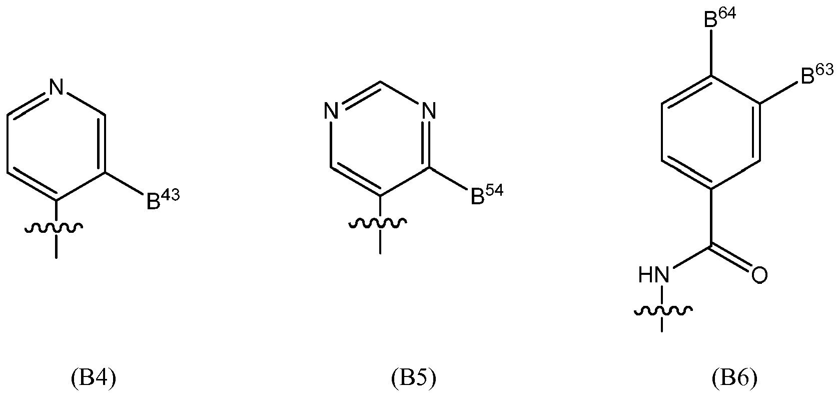 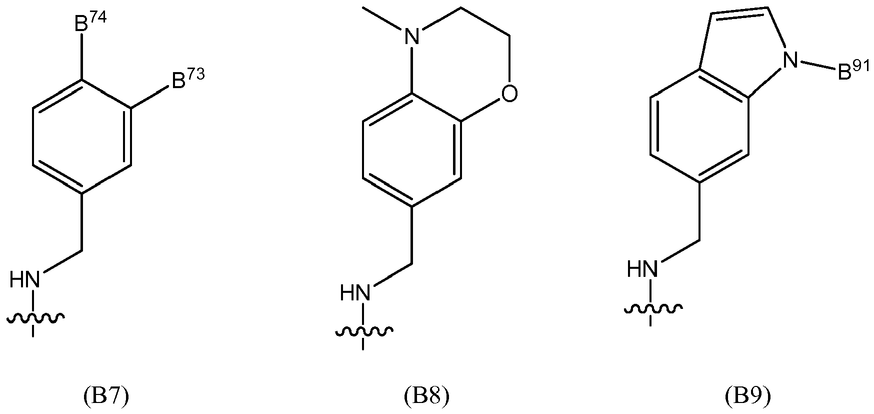 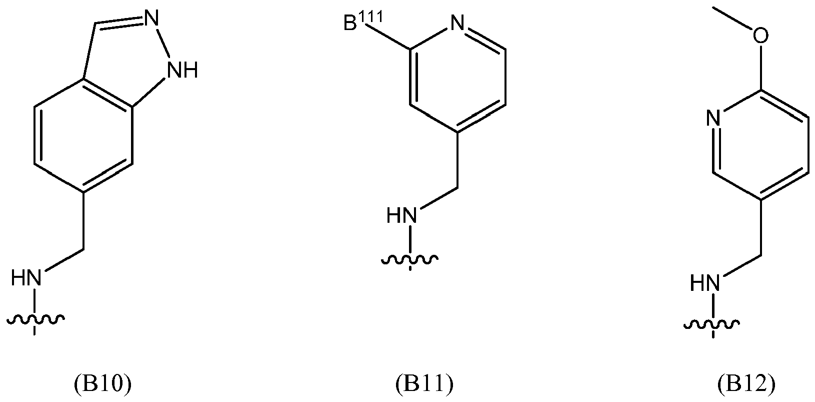 wobei
B¹³ und B¹⁴ jeweils H repräsentieren und B¹² OH, Methyl, Acetylamino, (C₁-C₂)Alkoxycarbonyl oder Dimethylaminocarbonyl repräsentiert, oder
B¹² und B¹⁴ jeweils H repräsentieren und B¹³ OH, Halogen, Acetyl, Acetylamino, Acetylaminomethyl, Aminocarbonyl, Sulfamoyl, (C₁-C₂)Alkoxy, (C₁-C₂)Alkoxycarbonyl, Cyano, Amino-(C₁-C₂)alkyl oder Hydroxy-(C₁-C₂)alkyl repräsentiert, oder
B¹² und B¹³ jeweils H repräsentieren und B¹⁴ Acetyl, Acetylamino, Acetylaminomethyl, Aminocarbonyl, Dimethylaminocarbonyl, Sulfamoyl, (C₁-C₂)Alkylsulfonyl, Cyanomethyl oder Hydroxymethyl repräsentiert;
X und X' jeweils -O- repräsentieren oder eines aus X und X' -O- repräsentiert und das andere -CH₂- repräsentiert;
B3⁵ und B³⁶ jeweils H repräsentieren und B³² H oder Halogen repräsentiert, oder
B³² und B³⁶ jeweils H repräsentieren und B³⁵ Halogen, Methyl, Methylsulfanyl, Methansulfonyl oder Methoxycarbonyl repräsentiert, oder
B³² und B³⁵ jeweils H repräsentieren und B³⁶ Halogen, Methoxy oder Hydroxymethyl repräsentiert;
B⁴³ H oder Halogen repräsentiert;
B⁵⁴ H oder (C₁-C₃)Alkyl repräsentiert;
B⁶⁴ H repräsentiert und B⁶³ H, Methoxycarbonyl oder Dimethylamino repräsentiert, oder
B⁶³ H repräsentiert und B⁶⁴ Methoxy, Methoxycarbonyl, Dimethylamino oder Methansulfonyl repräsentiert;
B⁷⁴ H repräsentiert und B⁷³ H, Methoxy, Methoxycarbonyl, Carboxy, 2-Hydroxyethoxy, 1*H*-1,2,4-Triazol-1-yl oder 1*H*-1,2,4-Triazol-1-ylmethyl repräsentiert, oder
B⁷³ H repräsentiert und B⁷⁴ Carboxy, Acetylamino, ((Aminocarbonyl)methyl)oxy, *N*-Methylsulfamoyl oder 3-Methyl-1*H*-1,2,4-triazol-1-yl repräsentiert;
B⁹¹ H oder Methyl repräsentiert;
B¹¹¹ H oder Morpholino repräsentiert; oder
R² H repräsentiert, R⁵ Pyridin-3-ylamino repräsentiert und R³ Propylamino, 3-Acetylamino-phenyl, Benzylamino, 5-Methylpyridin-3-yl oder 3-(*N*,*N*-Dimethylamino)phenylcarbonylamino repräsentiert; oder
R² Fluor repräsentiert, R³ H repräsentiert und R⁵ Brom, Benzyl, Benzoylamino, 4-Carboxyphenyl, Benzylamino, Pyridin-3-yl oder (Pyridin-3-ylmethyl)amino repräsentiert; oder
R² Brom repräsentiert, R³ H repräsentiert und R⁵ Methoxy repräsentiert; oder
R² Fluor repräsentiert, R⁵ Methyl repräsentiert und R³ Chinolin-3-yl, Benzylamino, eine Phenylgruppe, die optional einmal substituiert ist durch Acetylamino oder Sulfamoyl, Pyridin-2-yl, oder eine Pyridin-3-yl-Gruppe repräsentiert, die optional einmal durch Methyl substituiert ist; oder
R² Fluor repräsentiert, R³ Pyridin-3-yl repräsentiert und R⁵ Pyridin-3-yl oder 4-Carboxyphenyl repräsentiert; oder
R² Fluor repräsentiert, R³ Benzylamino repräsentiert und R⁵ Chlor oder 2-Methoxy-pyrimidin-5-yl repräsentiert; oder
R² Fluor repräsentiert, R³ Chinolin-3-yl repräsentiert und R⁵ Chinolin-3-yl repräsentiert; oder
R² Fluor repräsentiert, R³ 3-(Acetylamino)phenyl repräsentiert und R⁵ Pyridin-3-yl, Pyridin-3-ylamino oder 2-Methoxypyrimidin-5-yl repräsentiert; oder
R² Fluor repräsentiert, R³ 4-Sulfamoyl-phenyl repräsentiert und R⁵ Chlor repräsentiert; oder
R² Fluor repräsentiert, R⁵ Pyridin-3-ylamino repräsentiert und R³ Benzylamino oder 5-Methylpyridin-3-yl repräsentiert; oder
R³ H repräsentiert, R² Folgendes repräsentiert: (C₁-C₃)Alkylaminomethyl, *N*,*N*-Dimethylaminomethyl, Pyridin-3-yl, Pyridin-4-yl, 2-Methylpyridin-4-yl, 2-Methoxypyridin-4-yl, 2-Aminopyridin-4-yl, 2,6-Dimethylpyridin-4-yl, Pyrimidin-4-yl, 1*H*-Imidazol-4-yl, 1*H*-Pyrazol-4-yl, Thiazol-4-yl, Pyridin-3-ylamino, 3-(Aminomethyl)phenyl, 4-Hydroxyphenyl, 4-(Hydroxymethyl)phenyl, 4-Sulfamoyl-phenyl, Morpholino, Morpholinomethyl, 4-Methylpiperazin-1-yl oder 4-Methyl-piperazin-1-ylmethyl und R⁵ Folgendes repräsentiert: Chlor, Vinyl, Methoxy, (3-Carboxyphenyl)amino, (3-(Methoxycarbonyl)phenyl)amino, (3-(Dimethylcarbamoyl)phenyl)amino, (3-(Methylcarbamoyl)phenyl)amino, Pyridin-4-yl, (6-Methyl-pyridin-2-yl)amino, (6-Methoxy-pyridin-2-yl)amino, Pyridin-3-ylamino, (6-Methyl-pyridin-3-yl)amino oder Pyridin-3-yloxy;
R⁶ Phenyl, 1*H*-Pyrrol-2-yl oder 1*H*-1,2,3-Triazol-5-yl repräsentiert;
R⁷ Pyridin-2-yl, 5-Methyl-pyridin-2-yl, 2-Methyl-pyridin-3-yl, 6-Methyl-pyridin-3-yl, Pyridin-4-yl, 3-Methoxycarbonyl-pyridin-4-yl, 2-(Methoxycarbonyl)phenyl, 3-Hydroxyphenyl oder 3-Amino-phenyl repräsentiert;
R⁸ Folgendes repräsentiert: Pyridin-2-yl, 6-Methoxy-pyridin-2-yl, 5-Methoxy-pyridin-2-yl, 4-Methoxy-pyridin-2-yl, 3-Methyl-pyridin-2-yl, 4-Methyl-pyridin-2-yl, 5-Methyl-pyridin-2-yl, 6-Methyl-pyridin-2-yl, 5-Aminocarbonyl-pyridin-2-yl, 4-Fluor-pyridin-2-yl, 5-Methoxycarbonyl-pyridin-2-yl, 6-Methoxycarbonyl-pyridin-2-yl, Pyridin-3-yl, 5-Fluorpyridin-3-yl, 2-Methoxypyridin-3-yl, 5-Methoxy-pyridin-3-yl, 6-Methoxy-pyridin-3-yl, 6-Acetylamino-pyridin-3-yl, 6-Methyl-pyridin-3-yl, Pyridin-4-yl, 2-Methoxy-pyridin-4-yl, 3-Fluor-pyridin-4-yl, Pyrazin-2-yl, Pyrimidin-2-yl, Pyrimidin-4-yl, Pyrimidin-5-yl, Phenyl, 3-Hydroxyphenyl, 3-Methoxyphenyl, 3-Carboxyphenyl, 3-Carbamoylphenyl, 3-Acetylamino-phenyl, 3-Sulfamoylphenyl, 3-Methoxycarbonyl-phenyl, 4-Methyl-3-Methoxycarbonyl-phenyl, 4-Methyl-3-carboxyphenyl, 3-(Carboxymethyl)phenyl, 3-(Methylcarbamoyl)phenyl, 3-(Dimethylcarbamoyl)phenyl, 3-(Benzylcarbamoyl)phenyl, 3-(2-Amino-2-oxoethyl)phenyl, 3-(2-(Dimethylamino)-2-oxoethyl)phenyl, 3-(2-(Benzylamino)-2-oxoethyl)phenyl, 4-Methoxycarbonyl-phenyl, 3-Methyl-4-methoxycarbonyl-phenyl, 4-Carboxyphenyl, 3-Methyl-4-carboxyphenyl, 3-Carbamoyl-4-methoxyphenyl, 4-Methoxy-3-(methylcarbamoyl)phenyl, 3-(Dimethylcarbamoyl)-4-methoxyphenyl, 4-Hydroxyphenyl, 4-(Carboxymethyl)phenyl, 4-Cyanophenyl, 4-Acetylamino-phenyl, 4-Carbamoylphenyl, 4-(Methylcarbamoyl)phenyl, 4-(Dimethylcarbamoyl)phenyl, 4-(2-Amino-2-oxoethyl)phenyl, 4-(2-(Dimethylamino)-2-oxoethyl)phenyl, 4-(2-(Methylamino)-2-oxoethyl)phenyl, 4-(2-(Benzylamino)-2-oxoethyl)phenyl, 5-Methyl-1,3,4-oxadiazol-2-yl oder Imidazo[1,2-*a*]pyridin-7-yl;
R⁹ Pyridin-2-yl, Pyridin-3-yl, 6-Methoxypyridin-3-yl, Pyrimidin-2-yl, 1,3-Dimethyl-1*H*-pyrazol-5-yl, 1-Methyl-1*H*-pyrazol-5-yl, 5-Methylisoxazol-3-yl oder 3-Methylisothiazol-5-yl repräsentiert;
R¹⁰ 1*H*-Imidazol-1-yl, 4-(Hydroxymethyl)-1*H*-imidazol-1-yl, 5-(Hydroxymethyl)-1*H*-imidazol-1-yl, 2-Carboxy-1*H*-imidazol-1-yl, 2,4-Dimethyl-1*H*-imidazol-1-yl, 2,5-Dimethyl-1*H*-imidazol-1-yl, 2-Methyl-1*H*-imidazol-1-yl, 1*H*- 1,2,3-Triazol-1-yl, 1*H*-Pyrazol-1-yl, 4-Methyl-1*H*-pyrazol-1-yl oder 4-(Hydroxymethyl)-1*H*-pyrazol-1-yl repräsentiert;
oder ein Salz einer solchen Verbindung.

2. Verbindung von Formel I nach Anspruch 1, die auch eine Verbindung von Formel I_{P2} ist 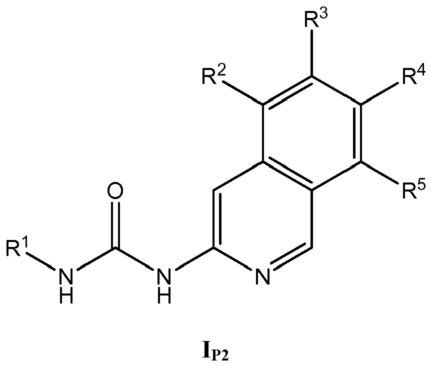 wobei
R¹ (C₁-C₃)Alkyl, (C₂-C₃)Haloalkyl oder Cyclopropyl repräsentiert;
R⁴ H repräsentiert;
R³ H repräsentiert, R⁵ H repräsentiert und R² Phenyl oder 4-Carboxyphenyl repräsentiert; oder
R² H repräsentiert, R⁵ H repräsentiert und R³ Benzoylamino repräsentiert; oder
R² H repräsentiert, R³ H repräsentiert und R⁵ -NH-CO-R⁶, -CH₂-O-R⁷, -NH-R⁸, -CH₂-NH-R⁹, (Pyridin-3-ylmethyl)amino, Pyridin-3-yloxy oder 4-Carboxyphenyl repräsentiert; oder
R³ H repräsentiert, R⁵ Methyl repräsentiert und R² Halogen, (C₁-C₃)Alkylamino, 2-(Aminocarbonyl)-ethyl, Imidazo[1,2-*a*]pyridin-6-yl, 4-Methyl-thiophen-3-yl, Isochinolin-5-yl oder 1*H*-Indol-4-yl oder eine Gruppe der Formel (A1), (A2) oder (A3) repräsentiert, wie nachfolgend gezeigt 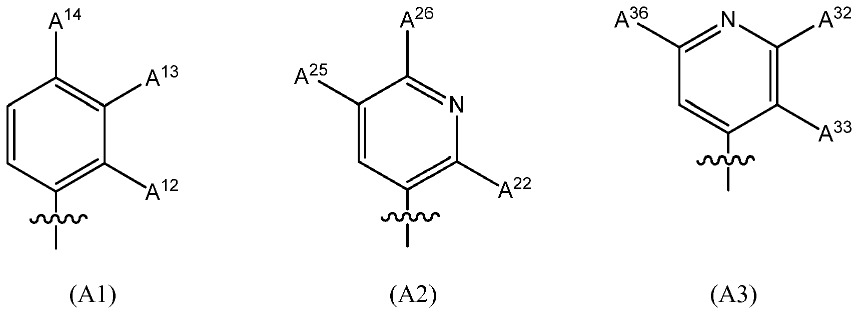
wobei
A¹³ und A¹⁴ jeweils H repräsentieren und A¹² H oder OH repräsentiert; oder
A¹² und A¹⁴ jeweils H repräsentieren und A¹³ OH, Acetylamino, Aminomethyl, Sulfamoyl oder Hydroxymethyl repräsentiert, oder
A¹² und A¹³ jeweils H repräsentieren und A¹⁴ OH, Methylaminocarbonyl, Dimethylaminocarbonyl, Methoxycarbonylamino, Sulfamoyl oder Hydroxymethyl repräsentiert;
A²⁵ und A²⁶ jeweils H repräsentieren und A²² H oder Halogen repräsentiert, oder
A²² und A²⁶ jeweils H repräsentieren und A²⁵ Methyl oder Halogen repräsentiert, oder
A²² und A²⁵ jeweils H repräsentieren und A²⁶ Hydroxymethyl repräsentiert;
A³³ und A³⁶ jeweils H repräsentieren und A³² H, Halogen, Amino, Methyl oder Methoxy repräsentiert, oder
A³³ H repräsentiert und A³² und A³⁶ jeweils Methyl repräsentieren; oder
R² H repräsentiert, R⁵ Methyl repräsentiert und R³ Folgendes repräsentiert: Amino, Vinyl, (C₁-C₄)Alkylamino, Cyclopropylmethylamino, (2-(Benzyloxy)ethyl)amino, Methoxymethylcarbonylamino, (Thiazol-5-yl)carbonylamino, Pyridin-3-ylcarbonylamino, Pyridin-4-ylcarbonylamino, Naphthalen-2-yl, Furan-2-yl, Furan-3-yl, 1*H*-Pyrazol-4-yl, Thiophen-3-yl, 4-Methyl-thiophen-3-yl, Chinolin-3-yl, Isochinolin-4-yl, Isochinolin-5-yl, Imidazo[1,2-*a*]pyridin-6-yl, 1*H*-Indol-4-yl oder eine Gruppe der Formel (B1), (B2), (B3), (B4), (B5), (B6), (B7), (B8), (B9), (B10), (B11) oder (B12), wie nachfolgend gezeigt 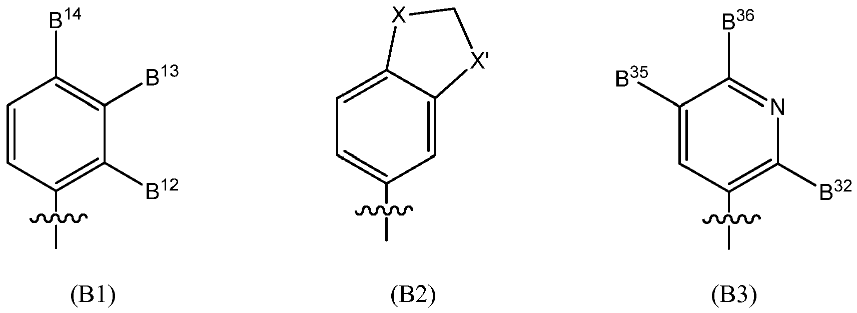 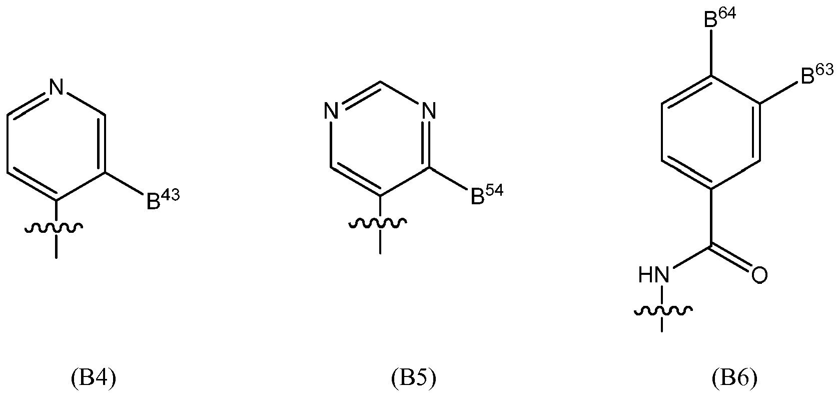 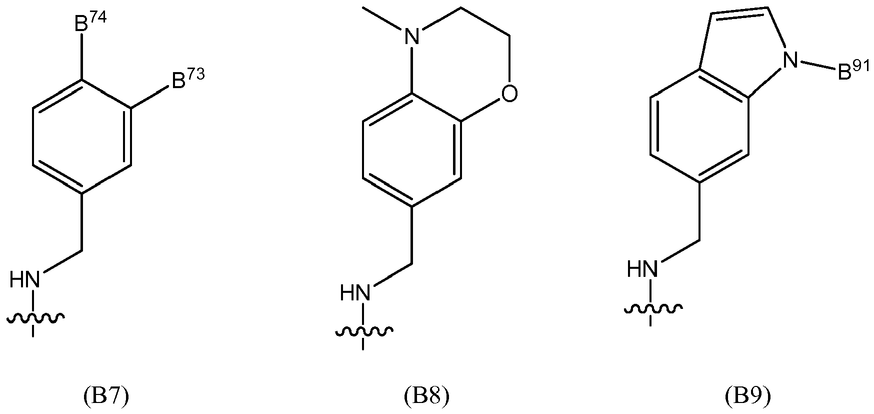 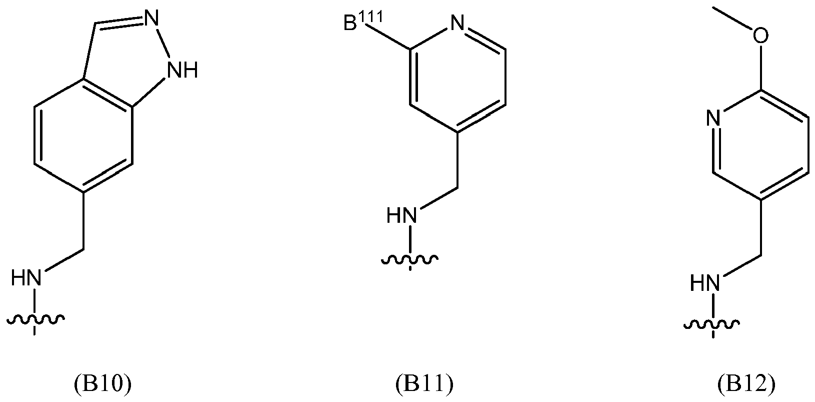 wobei
B¹³ und B¹⁴ jeweils H repräsentieren und B¹² OH, Methyl, Acetylamino, (C₁-C₂)Alkoxycarbonyl oder Dimethylaminocarbonyl repräsentiert, oder
B¹² und B¹⁴ jeweils H repräsentieren und B¹³ OH, Halogen, Acetyl, Acetylamino, Acetylaminomethyl, Aminocarbonyl, Sulfamoyl, (C₁-C₂)Alkoxy, (C₁-C₂)Alkoxycarbonyl, Cyano, Amino-(C₁-C₂)alkyl oder Hydroxy-(C₁-C₂)alkyl repräsentiert, oder
B¹² und B¹³ jeweils H repräsentieren und B¹⁴ Acetyl, Acetylamino, Acetylaminomethyl, Aminocarbonyl, Dimethylaminocarbonyl, Sulfamoyl, (C₁-C₂)Alkylsulfonyl, Cyanomethyl oder Hydroxymethyl repräsentiert;
X und X' jeweils -O- repräsentieren oder eines aus X und X' -O- repräsentiert und das andere -CH₂- repräsentiert;
B3⁵ und B³⁶ jeweils H repräsentieren und B³² H oder Halogen repräsentiert, oder
B³² und B³⁶ jeweils H repräsentieren und B³⁵ Halogen, Methyl, Methylsulfanyl, Methansulfonyl oder Methoxycarbonyl repräsentiert, oder
B³² und B³⁵ jeweils H repräsentieren und B³⁶ Halogen, Methoxy oder Hydroxymethyl repräsentiert;
B⁴³ H oder Halogen repräsentiert;
B⁵⁴ H oder (C₁-C₃)Alkyl repräsentiert;
B⁶⁴ H repräsentiert und B⁶³ H, Methoxycarbonyl oder Dimethylamino repräsentiert, oder
B⁶³ H repräsentiert und B⁶⁴ Methoxy, Methoxycarbonyl, Dimethylamino oder Methansulfonyl repräsentiert;
B⁷⁴ H repräsentiert und B⁷³ H, Methoxy, Methoxycarbonyl, Carboxy, 2-Hydroxyethoxy, 1*H*-1,2,4-Triazol-1-yl oder 1*H*-1,2,4-Triazol-1-ylmethyl repräsentiert, oder
B⁷³ H repräsentiert und B⁷⁴ Carboxy, Acetylamino, ((Aminocarbonyl)methyl)oxy, *N*-Methylsulfamoyl oder 3-Methyl-1*H*-1,2,4-triazol-1-yl repräsentiert;
B⁹¹ H oder Methyl repräsentiert;
B¹¹¹ H oder Morpholino repräsentiert; oder
R² H repräsentiert, R⁵ Pyridin-3-ylamino repräsentiert und R³ Propylamino, 3-Acetylamino-phenyl, Benzylamino, 5-Methylpyridin-3-yl oder 3-(*N*,*N*-Dimethylamino)phenylcarbonylamino repräsentiert; oder
R² Fluor repräsentiert, R³ H repräsentiert und R⁵ Brom, Benzyl, Benzoylamino, 4-Carboxyphenyl, Benzylamino, Pyridin-3-yl oder (Pyridin-3-ylmethyl)amino repräsentiert; oder
R² Brom repräsentiert, R³ H repräsentiert und R⁵ Methoxy repräsentiert; oder
R² Fluor repräsentiert, R⁵ Methyl repräsentiert und R³ Chinolin-3-yl, Benzylamino, eine Phenylgruppe, die optional einmal substituiert ist durch Acetylamino oder Sulfamoyl, Pyridin-2-yl, oder eine Pyridin-3-yl-Gruppe repräsentiert, die optional einmal durch Methyl substituiert ist; oder
R² Fluor repräsentiert, R³ Pyridin-3-yl repräsentiert und R⁵ Pyridin-3-yl oder 4-Carboxyphenyl repräsentiert; oder
R² Fluor repräsentiert, R³ Benzylamino repräsentiert und R⁵ Chlor oder 2-Methoxy-pyrimidin-5-yl repräsentiert; oder
R² Fluor repräsentiert, R³ Chinolin-3-yl repräsentiert und R⁵ Chinolin-3-yl repräsentiert; oder
R² Fluor repräsentiert, R³ 3-(Acetylamino)phenyl repräsentiert und R⁵ Pyridin-3-yl, Pyridin-3-ylamino oder 2-Methoxypyrimidin-5-yl repräsentiert; oder
R² Fluor repräsentiert, R³ 4-Sulfamoyl-phenyl repräsentiert und R⁵ Chlor repräsentiert; oder
R² Fluor repräsentiert, R⁵ Pyridin-3-ylamino repräsentiert und R³ Benzylamino oder 5-Methylpyridin-3-yl repräsentiert; oder
R³ H repräsentiert, R² Pyridin-3-yl, Pyridin-4-yl, 2-Methylpyridin-4-yl, 2-Methoxypyridin-4-yl, 2-Aminopyridin-4-yl, 2,6-Dimethylpyridin-4-yl, 3-(Aminomethyl)phenyl, 4-Hydroxyphenyl, 4-(Hydroxymethyl)phenyl, 4-Sulfamoyl-phenyl repräsentiert und R⁵ Vinyl, Methoxy, (3-Carboxyphenyl)amino, (3-(Methoxycarbonyl)phenyl)amino, (3-(Dimethylcarbamoyl)phenyl)amino, (3-(Methylcarbamoyl)phenyl)amino, Pyridin-4-yl, (6-Methyl-pyridin-2-yl)amino, (6-Methoxy-pyridin-2-yl)amino, Pyridin-3-ylamino, (6-Methyl-pyridin-3-yl)amino oder Pyridin-3-yloxy repräsentiert;
R⁶ Phenyl, 1*H*-Pyrrol-2-yl oder 1*H*-1,2,3-Triazol-5-yl repräsentiert;
R⁷ Pyridin-2-yl, 5-Methyl-pyridin-2-yl, 2-Methyl-pyridin-3-yl, 6-Methyl-pyridin-3-yl, Pyridin-4-yl, 3-Methoxycarbonyl-pyridin-4-yl, 2-(Methoxycarbonyl)phenyl, 3-Hydroxy-phenyl oder 3-Amino-phenyl repräsentiert;
R⁸ Folgendes repräsentiert: Pyridin-2-yl, 6-Methoxy-pyridin-2-yl, 5-Methoxy-pyridin-2-yl, 4-Methoxy-pyridin-2-yl, 3-Methyl-pyridin-2-yl, 4-Methyl-pyridin-2-yl, 5-Methyl-pyridin-2-yl, 6-Methyl-pyridin-2-yl, 5-Aminocarbonyl-pyridin-2-yl, 4-Fluor-pyridin-2-yl, 5-Methoxycarbonyl-pyridin-2-yl, 6-Methoxycarbonyl-pyridin-2-yl, Pyridin-3-yl, 5-Fluor-pyridin-3-yl, 5-Methoxy-pyridin-3-yl, 6-Methoxy-pyridin-3-yl, 6-Acetylamino-pyridin-3-yl, 6-Methyl-pyridin-3-yl, Pyridin-4-yl, 2-Methoxy-pyridin-4-yl, 3-Fluor-pyridin-4-yl, Pyrazin-2-yl, Pyrimidin-2-yl, Pyrimidin-4-yl, Pyrimidin-5-yl, Phenyl, 3-Hydroxyphenyl, 3-Methoxyphenyl, 3-Carboxyphenyl, 3-Carbamoylphenyl, 3-Acetylamino-phenyl, 3-Sulfamoylphenyl, 3-Methoxycarbonyl-phenyl, 3-(Carboxymethyl)phenyl, 3-(Methylcarbamoyl)phenyl, 3-(Dimethylcarbamoyl)phenyl, 3-(Benzylcarbamoyl)phenyl, 3-(2-Amino-2-oxoethyl)phenyl, 3-(2-(Dimethylamino)-2-oxoethyl)phenyl, 3-(2-(Benzylamino)-2-oxoethyl)phenyl, 4-Methoxycarbonyl-phenyl, 4-Carboxyphenyl, 3-Carbamoyl-4-methoxyphenyl, 4-Methoxy-3-(methylcarbamoyl)phenyl, 3-(Dimethylcarbamoyl)-4-methoxyphenyl, 4-Hydroxyphenyl, 4-(Carboxymethyl)phenyl, 4-Cyanophenyl, 4-Acetylamino-phenyl, 4-Carbamoylphenyl, 4-(Methylcarbamoyl)phenyl, 4-(Dimethylcarbamoyl)phenyl, 4-(2-Amino-2-oxoethyl)phenyl, 4-(2-(Dimethylamino)-2-oxoethyl)phenyl, 4-(2-(Methylamino)-2-oxoethyl)phenyl, 4-(2-(Benzylamino)-2-oxoethyl)phenyl, 5-Methyl-1,3,4-oxadiazol-2-yl oder Imidazo[1,2-*a*]pyridin-7-yl;
R⁹ Pyridin-2-yl, Pyridin-3-yl, 1,3-Dimethyl-1*H*-pyrazol-5-yl oder 3-Methylisothiazol-5-yl repräsentiert;
oder ein Salz einer solchen Verbindung.

3. Verbindung von Formel I nach Anspruch 1, die auch eine Verbindung von Formel I_{P1} ist 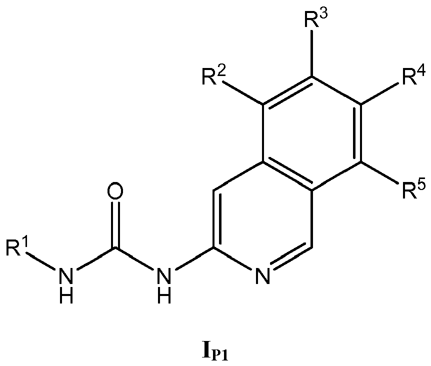 wobei
R¹ (C₁-C₃)Alkyl, (C₂-C₃)Haloalkyl oder Cyclopropyl repräsentiert;
R⁴ H repräsentiert;
R³ H repräsentiert, R⁵ H repräsentiert und R² Phenyl oder 4-Carboxyphenyl repräsentiert; oder
R² H repräsentiert, R⁵ H repräsentiert und R³ Benzoylamino repräsentiert; oder
R² H repräsentiert, R³ H repräsentiert und R⁵ Benzoylamino, Pyridin-3-ylmethylamino, Pyridin-2-ylamino, Pyridin-3-ylamino, Pyridin-3-yloxy oder 4-Carboxyphenyl repräsentiert; oder
R³ H repräsentiert, R⁵ Methyl repräsentiert und R² Halogen, (C₁-C₃)Alkylamino, 2-(Aminocarbonyl)-ethyl, Imidazo[1,2-*a*]pyridin-6-yl, 4-Methyl-thiophen-3-yl, Isochinolin-5-yl oder 1*H*-Indol-4-yl oder eine Gruppe der Formel (A1), (A2) oder (A3) repräsentiert, wie nachfolgend gezeigt 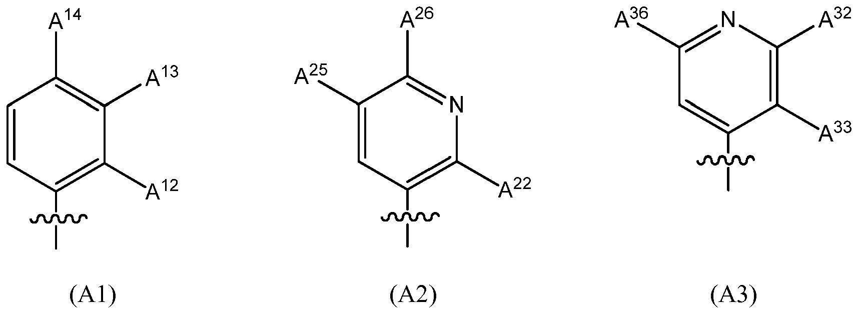
wobei
A¹³ und A¹⁴ jeweils H repräsentieren und A¹² OH repräsentiert; oder
A¹² und A¹⁴ jeweils H repräsentieren und A¹³ OH, Acetylamino, Aminomethyl, Aminosulfonyl oder Hydroxymethyl repräsentiert, oder
A¹² und A¹³ jeweils H repräsentieren und A¹⁴ OH, Methylaminocarbonyl, Dimethylaminocarbonyl, Methoxycarbonylamino, Aminosulfonyl oder Hydroxymethyl repräsentiert;
A²⁵ und A²⁶ jeweils H repräsentieren und A²² H oder Halogen repräsentiert, oder
A²² und A²⁶ jeweils H repräsentieren und A²⁵ Methyl oder Halogen repräsentiert, oder
A²² und A²⁵ jeweils H repräsentieren und A²⁶ Hydroxymethyl repräsentiert;
A³³ und A³⁶ jeweils H repräsentieren und A³² H, Halogen, Amino, Methyl oder Methoxy repräsentiert, oder
A³³ H repräsentiert und A³² und A³⁶ jeweils Methyl repräsentieren; oder
R² H repräsentiert, R⁵ Methyl repräsentiert und R³ Folgendes repräsentiert: Amino, (C₁-C₄)Alkylamino, Cyclopropylmethylamino, 2-Benzyloxy-ethylamino, (Thiazol-5-yl)carbonylamino, Pyridin-3-ylcarbonylamino, Pyridin-4-ylcarbonylamino, Pyridin-4-ylmethylamino, Naphthalen-2-yl, Furan-2-yl, Furan-3-yl, 1*H*-Pyrazol-4-yl, Thiophen-3-yl, 4-Methyl-thiophen-3-yl, Chinolin-3-yl, Isochinolin-4-yl, Isochinolin-5-yl, Imidazo[1,2-*a*]pyridin-6-yl, 1*H*-Indol-4-yl oder eine Gruppe der Formel (B1), (B2), (B3), (B4), (B5), (B6), (B7), (B8) oder (B9), wie nachfolgend gezeigt 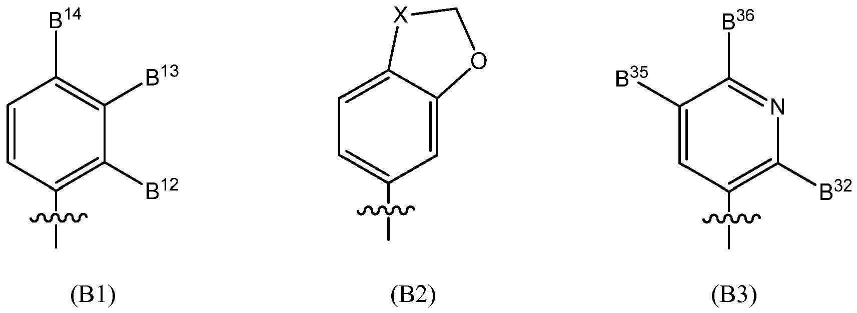 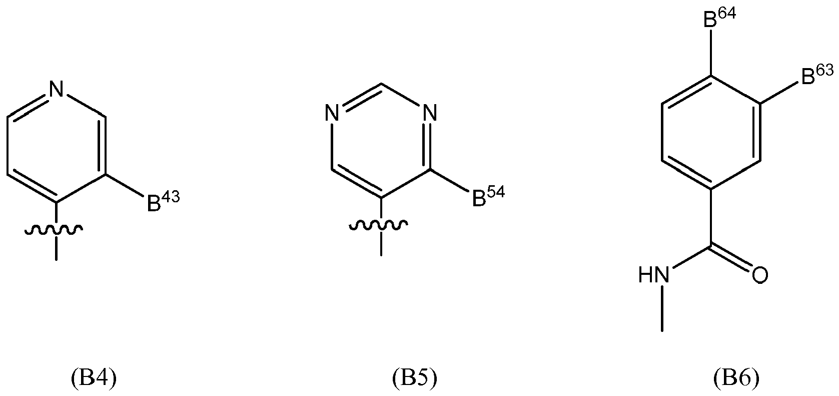 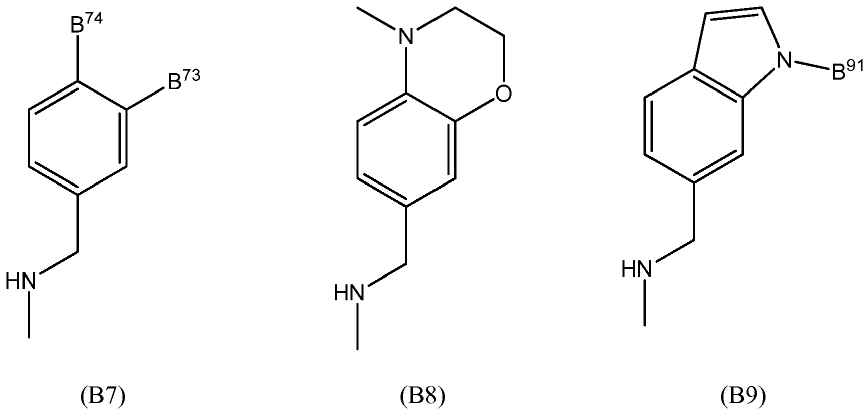
wobei
B¹³ und B¹⁴ jeweils H repräsentieren und B¹² OH, Methyl, Acetylamino, Ethoxycarbonyl oder Dimethylaminocarbonyl repräsentiert, oder
B¹² und B¹⁴ jeweils H repräsentieren und B¹³ OH, Halogen, Acetyl, Acetylamino, Acetylaminomethyl, Aminocarbonyl, Aminosulfonyl, (C₁-C₂)Alkoxy, (C₁-C₂)Alkoxycarbonyl, Cyano, Amino-(C₁-C₂)alkyl oder Hydroxy-(C₁-C₂)alkyl repräsentiert, oder
B¹² und B¹³ jeweils H repräsentieren und B¹⁴ Acetyl, Acetylamino, Acetylaminomethyl, Aminocarbonyl, Dimethylaminocarbonyl, Aminosulfonyl, (C₁-C₂)Alkylsulfonyl, Cyanomethyl oder Hydroxymethyl repräsentiert;
X -O- oder -CH₂- repräsentiert;
B3⁵ und B³⁶ jeweils H repräsentieren und B³² H oder Halogen repräsentiert, oder
B³² und B³⁶ jeweils H repräsentieren und B³⁵ Halogen, Methyl, Methylsulfanyl, Methansulfonyl oder Methoxycarbonyl repräsentiert, oder
B³² und B³⁵ jeweils H repräsentieren und B³⁶ Halogen, Methoxy oder Hydroxymethyl repräsentiert;
B⁴³ H oder Halogen repräsentiert;
B⁵⁴ H oder (C₁-C₃)Alkyl repräsentiert;
B⁶⁴ H repräsentiert und B⁶³ H oder Dimethylamino repräsentiert, oder
B⁶³ H repräsentiert und B⁶⁴ Methoxy, Dimethylamino oder Methylsulfonyl repräsentiert;
B⁷⁴ H repräsentiert und B⁷³ H, Methoxycarbonyl, Carboxy oder 2-Hydroxyethoxy repräsentiert, oder
B⁷³ H repräsentiert und B⁷⁴ Carboxy, Acetylamino oder 3-Methyl-[1,2,4]triazol-1-yl repräsentiert;
B⁹¹ H oder Methyl repräsentiert;
R² Fluor repräsentiert, R³ H repräsentiert und R⁵ Brom, Benzyl, Benzoylamino, 4-Carboxyphenyl, Benzylamino oder (Pyridin-3-yl)methylamino repräsentiert; oder
R² Brom repräsentiert, R³ H repräsentiert und R⁵ Methyl oder Methoxy repräsentiert; oder
R² Fluor repräsentiert, R⁵ Methyl repräsentiert und R³ H, Chinolin-3-yl, Benzylamino, eine Phenylgruppe, die optional einmal substituiert ist durch Acetylamino oder Aminosulfonyl, Pyridin-2-yl, oder eine Pyridin-3-yl-Gruppe repräsentiert, die optional einmal durch Methyl substituiert ist; oder
R² Fluor repräsentiert, R³ Pyridin-3-yl repräsentiert und R⁵ Pyridin-3-yl oder 4-Carboxyphenyl repräsentiert; oder
R² Fluor repräsentiert, R³ Benzylamino repräsentiert und R⁵ Chlor oder 2-Methoxy-pyrimidin-5-yl repräsentiert; oder
R² Fluor repräsentiert, R³ Chinolin-3-yl repräsentiert und R⁵ Chinolin-3-yl repräsentiert; oder
R² Fluor repräsentiert, R³ 3-Acetylaminophenyl repräsentiert und R⁵ Pyridin-3-yl oder 2-Methoxypyrimidin-5-yl repräsentiert; oder
R² Fluor repräsentiert, R³ 4-Aminosulfonylphenyl repräsentiert und R⁵ Chlor repräsentiert; oder
R² Pyridin-4-yl repräsentiert, R³ H repräsentiert und R⁵ Vinyl, Methoxy oder Pyridin-4-yl repräsentiert;
oder ein Salz einer solchen Verbindung.

4. Verbindung von Formel I nach einem der Ansprüche 1 bis 3, wobei R¹ (C₁-C₃)Alkyl repräsentiert;
oder ein Salz einer solchen Verbindung.

5. Verbindung von Formel I nach Anspruch 4, wobei R¹ Ethyl repräsentiert;
oder ein Salz einer solchen Verbindung.

6. Verbindung von Formel I nach einem der Ansprüche 1 bis 5, wobei R² H repräsentiert; oder ein Salz einer solchen Verbindung.

7. Verbindung von Formel I nach Anspruch 6, wobei R⁵ Methyl repräsentiert;
oder ein Salz einer solchen Verbindung.

8. Verbindung von Formel I nach Anspruch 6, wobei:
- R³ eine Gruppe (B1) repräsentiert, wobei entweder B¹² und B¹⁴ jeweils H repräsentieren und B¹³ OH, Halogen, Acetyl, Acetylamino, Acetylaminomethyl, Aminocarbonyl, Dimethylaminocarbonyl, Aminosulfonyl, (C₁-C₂)Alkylsulfonyl, (C₁-C₂)Alkoxy, (C₁-C₂)Alkoxycarbonyl, Cyano, Amino-(C₁-C₂)alkyl oder Hydroxy-(C₁-C₂)alkyl repräsentiert, oder B¹² und B¹³ jeweils H repräsentieren und B¹⁴ Acetyl, Acetylamino, Acetylaminomethyl, Aminocarbonyl, Dimethylaminocarbonyl, Aminosulfonyl, (C₁-C₂)Alkylsulfonyl, Cyanomethyl oder Hydroxymethyl repräsentiert; oder
- R³ eine Gruppe (B2) repräsentiert, wobei X CH₂ repräsentiert; oder
- R³ eine Gruppe (B3) repräsentiert; oder
- R³ eine Gruppe (B4) repräsentiert; oder
- R³ eine Gruppe (B6) repräsentiert; oder
- R³ Chinolin-3-yl, Imidazo[1,2-*a*]pyridin-6-yl, (Thiazol-5-yl)carbonylamino, Pyridin-3-ylcarbonylamino oder Pyridin-4-ylcarbonylamino repräsentiert;
oder ein Salz einer solchen Verbindung.

9. Verbindung von Formel I nach einem der Ansprüche 1 bis 5, wobei R³ H repräsentiert;
oder ein Salz einer solchen Verbindung.

10. Verbindung von Formel I nach Anspruch 9, wobei R⁵ Methyl repräsentiert;
oder ein Salz einer solchen Verbindung.

11. Verbindung von Formel I nach Anspruch 10, wobei:
- R² Halogen repräsentiert; oder
- R² eine Gruppe (A1) repräsentiert; oder
- R² eine Gruppe (A3) repräsentiert;
oder ein Salz einer solchen Verbindung.

12. Verbindung von Formel I nach Anspruch 1, die ausgewählt ist aus den Folgenden:
- 1-(5-Brom-8-methyl-isochinolin-3-yl)-3-ethyl-harnstoff;
- 1-Ethyl-3-[5-(4-hydroxy-phenyl)-8-methyl-isochinolin-3-yl]-harnstoff;
- 1-Ethyl-3-[5-(2-hydroxy-phenyl)-8-methyl-isochinolin-3-yl]-harnstoff;
- *N*- {3-[3-(3-Ethyl-ureido)-8-methyl-isochinolin-5-yl]-phenyl}-acetamid;
- 4-[3-(3-Ethyl-ureido)-8-methyl-isochinolin-5-yl]-*N*,*N-*dimethyl-benzamid;
- 4-[3-(3-Ethyl-ureido)-8-methyl-isochinolin-5-yl]-*N*-methyl-benzamid;
- 1-Ethyl-3-(5-imidazo[1,2-*a*]pyridin-6-yl-8-methyl-isochinolin-3-yl)-harnstoff;
- 1-Ethyl-3-[5-(6-hydroxymethyl-pyridin-3-yl)-8-methyl-isochinolin-3-yl]-harnstoff;
- 1-Ethyl-3-[5-(5-fluor-pyridin-3-yl)-8-methyl-isochinolin-3-yl]-harnstoff;
- 1-Ethyl-3-[8-methyl-5-(5-methyl-pyridin-3-yl)-isochinolin-3-yl]-harnstoff;
- 1-[5-((3-(Aminomethyl)-phenyl)-8-methyl-isochinolin-3-yl]-3-ethyl-harnstoff;
- 1-Ethyl-3-[5-(3-hydroxymethyl-phenyl)-8-methyl-isochinolin-3-yl]-harnstoff;
- 3-[3-(3-Ethyl-ureido)-8-methyl-isochinolin-5-yl]-benzolsulfonamid;
- 1-Ethyl-3-(6-furan-3-yl-8-methyl-isochinolin-3-yl)-harnstoff;
- 1-Ethyl-3-[6-(4-hydroxymethyl-phenyl)-8-methyl-isochinolin-3-yl]-harnstoff;
- 1-Ethyl-3-(8-methyl-6-pyridin-3-yl-isochinolin-3-yl)-harnstoff;
- *N*-{3-[3-(3-Ethyl-ureido)-8-methyl-isochinolin-6-yl]-benzyl}-acetamid;
- *N*-{4-[3-(3-Ethyl-ureido)-8-methyl-isochinolin-6-yl]-phenyl}-acetamid;
- 1-Ethyl-3-[6-(3-hydroxymethyl-phenyl)-8-methyl-isochinolin-3-yl]-harnstoff;
- 1-Ethyl-3-[6-(3-hydroxy-phenyl)-8-methyl-isochinolin-3-yl]-harnstoff;
- 1-Ethyl-3-(8-methyl-6-pyridin-4-yl-isochinolin-3-yl)-harnstoff;
- 3-[3-(3-Ethyl-ureido)-8-methyl-isochinolin-6-yl]-benzolsulfonamid;
- 2-[3-(3-Ethyl-ureido)-8-methyl-isochinolin-6-yl]-*N*,*N*-dimethyl-benzamid;
- 4-[3-(3-Ethyl-ureido)-8-methyl-isochinolin-6-yl]-*N*,*N*-dimethyl-benzamid;
- 1-Ethyl-3-(6-imidazo[1,2-*a*]pyridin-6-yl-8-methyl-isochinolin-3-yl)-harnstoff;
- 1-[6-(3-(Aminomethyl)-phenyl)-8-methyl-isochinolin-3-yl]-3-ethyl-harnstoff;
- 1-[5-(2-Chlor-pyridin-4-yl)-8-methyl-isochinolin-3-yl]-3-ethyl-harnstoff;
- 1-Ethyl-3-[8-methyl-6-(1*H*-pyrazol-4-yl)-isochinolin-3-yl]-harnstoff;
- 1-[6-(3-Acetyl-phenyl)-8-methyl-isochinolin-3-yl]-3-ethyl-harnstoff;
- 4-[3-(3-Ethyl-ureido)-8-methyl-isochinolin-6-yl]-benzamid;
- 3-[3-(3-Ethyl-ureido)-8-methyl-isochinolin-6-yl]-benzamid;
- 1-(8-Brom-5-fluor-isochinolin-3-yl)-3-ethyl-harnstoff;
- 4-[3-(3-Ethyl-ureido)-5-fluor-6-pyridin-3-yl-isochinolin-8-yl]-benzoesäure;
- 1-Ethyl-3-[5-(4-hydroxymethyl-phenyl)-8-methyl-isochinolin-3-yl]-harnstoff;
- 4-[3-(3-Ethyl-ureido)-8-methyl-isochinolin-5-yl]-benzolsulfonamid;
- 1-Ethyl-3-(8-methyl-6-chinolin-3-yl-isochinolin-3-yl)-harnstoff;
- *N*-{3-[3-(3-Ethyl-ureido)-8-methyl-isochinolin-6-yl]-phenyl}-acetamid;
- 4-[3-(3-Ethyl-ureido)-8-methyl-isochinolin-6-yl]-benzolsulfonamid;
- 1-Ethyl-3-[8-methyl-6-(5-methyl-pyridin-3-yl)-isochinolin-3-yl]-harnstoff;
- 1-Ethyl-3-[8-methyl-5-(2-methyl-pyridin-4-yl)-isochinolin-3-yl]-harnstoff;
- 1-Ethyl-3-[5-(2-fluor-pyridin-4-yl)-8-methyl-isochinolin-3-yl]-harnstoff;
- 1-Ethyl-3-[5-(2-methoxy-pyridin-4-yl)-8-methyl-isochinolin-3-yl]-harnstoff;
- 1-Ethyl-3-(5-phenyl-isochinolin-3-yl)-harnstoff;
- 1-[5-(2,6-Dimethyl-pyridin-4-yl)-8-methyl-isochinolin-3-yl]-3-ethyl-harnstoff;
- 1-[5-(2-Amino-pyridin-4-yl)-8-methyl-isochinolin-3-yl]-3-ethyl-harnstoff;
- 1-(8-Benzyl-5-fluor-isochinolin-3-yl)-3-ethyl-harnstoff;
- 1-Ethyl-3-(5-fluor-8-methyl-6-pyridin-3-yl-isochinolin-3-yl)-harnstoff;
- 1-Ethyl-3-(5-fluor-8-methyl-6-pyridin-2-yl-isochinolin-3-yl)-harnstoff;
- 1-(6-Benzylamino-8-chlor-5-fluor-isochinolin-3-yl)-3-ethyl-harnstoff;
- 1-Ethyl-3-(8-methyl-5-pyridin-3-yl-isochinolin-3-yl)-harnstoff;
- *N*-[3-(3-Ethyl-ureido)-5-fluor-isochinolin-8-yl]-benzamid;
- *N*-[3-(3-Ethyl-ureido)-isochinolin-6-yl]-benzamid;
- 1-Ethyl-3-(5-fluor-6,8-di-pyridin-3-yl-isochinolin-3-yl)-harnstoff;
- 1-(6-Amino-8-methyl-isochinolin-3-yl)-3-ethyl-harnstoff;
- 1-Ethyl-3-{5-fluor-8-[(pyridin-3-ylmethyl)-amino]-isochinolin-3-yl}-harnstoff;
- 1-(8-Benzylamino-5-fluor-isochinolin-3-yl)-3-ethyl-harnstoff;
- 1-(6-Benzylamino-5-fluor-8-methyl-isochinolin-3-yl)-3-ethyl-harnstoff;
- 1-(6-Benzylamino-8-methyl-isochinolin-3-yl)-3-ethyl-harnstoff;
- 3-[3-(3-Ethyl-ureido)-8-methyl-isochinolin-5-yl]-propionamid;
- 1-Ethyl-3-(5-fluor-8-methyl-isochinolin-3-yl)-harnstoff;
- 1-Ethyl-3-(8-methyl-5-pyridin-4-yl-isochinolin-3-yl)-harnstoff;
- Thiazol-5-carbonsäure [3-(3-ethyl-ureido)-8-methyl-isochinolin-6-yl]-amid;
- 1-Ethyl-3-(5-ethylamino-8-methyl-isochinolin-3-yl)-harnstoff;
- 1-Ethyl-3-[8-methyl-5-(4-methyl-thiophen-3-yl)-isochinolin-3-yl]-harnstoff;
- {4-[3-(3-Ethyl-ureido)-8-methyl-isochinolin-5-yl]-phenyl}-carbaminsäuremethylester;
- 1-Ethyl-3-[5-(2-fluor-pyridin-3-yl)-8-methyl-isochinolin-3-yl]-harnstoff;
- 1-Ethyl-3-(8-methyl-[5,5']büsochinolinyl-3-yl)-harnstoff;
- 1-Ethyl-3-[5-(3-hydroxy-phenyl)-8-methyl-isochinolin-3-yl]-harnstoff;
- 1-Ethyl-3-[5-(1*H*-indol-4-yl)-8-methyl-isochinolin-3-yl]-harnstoff;
- *N*-[3-(3-Ethyl-ureido)-isochinolin-8-yl]-benzamid;
- 1-Ethyl-3-{8-[(pyridin-3-ylmethyl)-amino]-isochinolin-3-yl}-harnstoff;
- 1-(5,8-Di-pyridin-4-yl-isochinolin-3-yl)-3-ethyl-harnstoff;
- 1-(5-Brom-8-methoxy-isochinolin-3-yl)-3-ethyl-harnstoff;
- 4-[3-(3-Ethyl-ureido)-5-fluor-isochinolin-8-yl]-benzoesäure;
- 1-Ethyl-3-(8-methyl-6-pyrimidin-5-yl-isochinolin-3-yl)-harnstoff;
- 1-Ethyl-3-[6-(3-methoxy-phenyl)-8-methyl-isochinolin-3-yl]-harnstoff;
- 1-(6-(Benzo[1,3]dioxol-5-yl)-8-methyl-isochinolin-3-yl)-3-ethyl-harnstoff;
- 1-Ethyl-3-(6-furan-2-yl-8-methyl-isochinolin-3-yl)-harnstoff;
- 1-Ethyl-3-(8-methyl-6-naphthalen-2-yl-isochinolin-3-yl)-harnstoff;
- 1-Ethyl-3-[8-methyl-6-(4-methyl-thiophen-3-yl)-isochinolin-3-yl]-harnstoff;
- *N*-{4-[3-(3-Ethyl-ureido)-8-methyl-isochinolin-6-yl]-benzyl}-acetamid;
- 1-[6-(2-Chlor-pyridin-3-yl)-8-methyl-isochinolin-3-yl]-3-ethyl-harnstoff;
- 1-[6-(2,3-Dihydro-benzofuran-5-yl)-8-methyl-isochinolin-3-yl]-3-ethyl-harnstoff;
- 3-[3-(3-Ethyl-ureido)-8-methyl-isochinolin-6-yl]-benzoesäureethylester;
- 2-[3-(3-Ethyl-ureido)-8-methyl-isochinolin-6-yl]-benzoesäureethylester;
- *N*-{2-[3-(3-Ethyl-ureido)-8-methyl-isochinolin-6-yl]-phenyl}-acetamid;
- 5-[3-(3-Ethyl-ureido)-8-methyl-isochinolin-6-yl]-nicotinsäuremethylester;
- 1-Ethyl-3-[6-(3-fluor-phenyl)-8-methyl-isochinolin-3-yl]-harnstoff;
- 1-Ethyl-3-[6-(6-methoxy-pyridin-3-yl)-8-methyl-isochinolin-3-yl]-harnstoff;
- 1-Ethyl-3-[6-(1*H*-indol-4-yl)-8-methyl-isochinolin-3-yl]-harnstoff;
- 1-Ethyl-3-(8'-methyl-[4,6']biisochinolinyl-3'-yl)-harnstoff;
- 1-[6-(4-(Cyanomethyl)-phenyl)-8-methyl-isochinolin-3-yl]-3-ethyl-harnstoff;
- 1-Ethyl-3-(8-methyl-6-thiophen-3-yl-isochinolin-3-yl)-harnstoff;
- 1-Ethyl-3-[6-(4-methansulfonyl-phenyl)-8-methyl-isochinolin-3-yl]-harnstoff;
- 1-Ethyl-3-[6-(4-isopropyl-pyrimidin-5-yl)-8-methyl-isochinolin-3-yl]-harnstoff;
- 1-Ethyl-3-[8-methyl-6-(5-methylsulfanyl-pyridin-3-yl)-isochinolin-3-yl]-harnstoff;
- 1-Ethyl-3-[6-(3-fluor-pyridin-4-yl)-8-methyl-isochinolin-3-yl]-harnstoff;
- 1-[6-(3-Chlor-pyridin-4-yl)-8-methyl-isochinolin-3-yl]-3-ethyl-harnstoff;
- 1-Ethyl-3-[6-(6-fluor-pyridin-3-yl)-8-methyl-isochinolin-3-yl]-harnstoff;
- 1-Ethyl-3-[6-(2-fluor-pyridin-3-yl)-8-methyl-isochinolin-3-yl]-harnstoff;
- 1-Ethyl-3-[6-(6-hyäroxymethyl-pyridin-3-yl)-8-methyl-isochinolin-3-yl]-harnstoff;
- 1-Ethyl-3-[6-(5-fluor-pyridin-3-yl)-8-methyl-isochinolin-3-yl]-harnstoff;
- 1-Ethyl-3-[6-(5-methansulfonyl-pyridin-3-yl)-8-methyl-isochinolin-3-yl]-harnstoff;
- 1-Ethyl-3-(8'-methyl-[5,6']büsochinolinyl-3'-yl)-harnstoff;
- 1-Ethyl-3-(8-methyl-6-o-tolyl-isochinolin-3-yl)-harnstoff;
- *N*-[3-(3-Ethyl-ureido)-8-methyl-isochinolin-6-yl]-benzamid;
- 1-[6-(3-Cyano-phenyl)-8-methyl-isochinolin-3-yl]-3-ethyl-harnstoff;
- 1-[6-(4-Acetyl-phenyl)-8-methyl-isochinolin-3-yl]-3-ethyl-harnstoff;
- 1-Ethyl-3-[6-(2-hydroxy-phenyl)-8-methyl-isochinolin-3-yl]-harnstoff;
- 1-[6-Benzylamino-5-fluor-8-(2-methoxy-pyrimidin-5-yl)-isochinolin-3-yl]-3-ethyl-harnstoff;
- 4-[8-Chlor-3-(3-ethyl-ureido)-5-fluor-isochinolin-6-yl]-benzolsulfonamid;
- 1-Ethyl-3-(5-fluor-6,8-di-chinolin-3-yl-isochinolin-3-yl)-harnstoff;
- *N*-{3-[3-(3-Ethyl-ureido)-5-fluor-8-pyridin-3-yl-isochinolin-6-yl]-phenyl}-acetamid;
- *N*-{3-[3-(3-Ethyl-ureido)-5-fluor-8-(2-methoxy-pyrimidin-5-yl)-isochinolin-6-yl]-phenyl} -acetamid;
- 1-Ethyl-3-(8-methyl-6-propylamino-isochinolin-3-yl)-harnstoff;
- *N*-[3-(3-Ethyl-ureido)-8-methyl-isochinolin-6-yl]-isonicotinamid;
- *N*-[3-(3-Ethyl-ureido)-8-methyl-isochinolin-6-yl]-4-methansulfonyl-benzamid;
- 3-Dimethylamino-N [3-(3-ethyl-ureido)-8-methyl-isochinolin-6-yl]-benzamid;
- *N*-[3-(3-Ethyl-ureido)-8-methyl-isochinolin-6-yl]-nicotinamid;
- *N*-[3-(3-Ethyl-ureido)-8-methyl-isochinolin-6-yl]-4-methoxy-benzamid;
- 4-Dimethylamino-*N*-[3-(3-ethyl-ureido)-8-methyl-isochinolin-6-yl]-benzamid;
- 1-Ethyl-3- {8-methyl-6-[(pyridin-4-ylmethyl)-amino]-isochinolin-3-yl}-harnstoff;
- 4-[3-(3-Ethyl-ureido)-isochinolin-8-yl]-benzoesäure;
- 4-[3-(3-Ethyl-ureido)-isochinolin-5-yl]-benzoesäure;
- 1-Ethyl-3-(8-methoxy-5-pyridin-4-yl-isochinolin-3-yl)-harnstoff;
- 3-{[3-(3-Ethyl-ureido)-8-methyl-isochinolin-6-ylamino]-methyl}-benzoesäuremethylester;
- 4-{[3-(3-Ethyl-ureido)-8-methyl-isochinolin-6-ylamino]-methyl}-benzoesäure;
- 3-{[3-(3-Ethyl-ureido)-8-methyl-isochinolin-6-ylamino]-methyl}-benzoesäure;
- 1-Ethyl-3-{6-[3-(2-hydroxy-ethoxy)-benzylamino]-8-methyl-isochinolin-3-yl}-harnstoff;
- 1-Ethyl-3-{8-methyl-6-[(4-methyl-3,4-dihydro-2*H*-benzo[1,4]oxazin-7-ylmethyl)-amino]-isochinolin-3-yl}-harnstoff;
- 1-Ethyl-3-{8-methyl-6-[4-(3-methyl-[1,2,4]triazol-1-yl)-benzylamino]-isochinolin-3-yl}-harnstoff;
- 1-[6-(2-Benzyloxy-ethylamino)-8-methyl-isochinolin-3-yl]-3-ethyl-harnstoff;
- 1-{6-[(Cyclopropylmethyl)-amino]-8-methyl-isochinolin-3-yl}-3-ethyl-harnstoff;
- 1-Ethyl-3-{6-[(1*H*-indol-6-ylmethyl)-amino]-8-methyl-isochinolin-3-yl}-harnstoff;
- 1-Ethyl-3-{8-methyl-6-[((1-methyl-1*H*-indol-6-yl)methyl)-amino]-isochinolin-3-yl}-harnstoff;
- 1-Ethyl-3-(6-isobutylamino-8-methyl-isochinolin-3-yl)-harnstoff;
- *N-*(4-{[3-(3-Ethyl-ureido)-8-methyl-isochinolin-6-ylamino]-methyl}-phenyl)-acetamid;
- 1-(2-Fluor-ethyl)-3-(8-methyl-5-pyridin-4-yl-isochinolin-3-yl)-harnstoff;
- 1-Cyclopropyl-3-(8-methyl-5-pyridin-4-yl-isochinolin-3-yl)-harnstoff;
- 1-Ethyl-3-[5-(pyridin-4-yl)-8-vinylisochinolin-3-yl]-harnstoff;
- 1-Ethyl-3-[5-fluor-8-methyl-6-(5-methylpyridin-3-yl)isochinolin-3-yl]-harnstoff;
- *N*-{3-[3-(3-Ethyl-ureido)-5-fluor-8-methyl-isochinolin-6-yl]-phenyl}-acetamid;
- 1-Ethyl-3-[5-fluor-8-methyl-6-(chinolin-3-yl)-isochinolin-3-yl]-harnstoff;
- 4-[3-(3-Ethyl-ureido)-5-fluor-8-methyl-isochinolin-6-yl]-benzolsulfonamid;
- 1-Ethyl-3-[8-(pyridin-3-ylamino)-isochinolin-3-yl]-harnstoff;
- 1-Ethyl-3-[8-(pyridin-2-ylamino)-isochinolin-3-yl]-harnstoff;
- 1-Ethyl-3-[8-(pyridin-3-yloxy)-isochinolin-3-yl]-harnstoff;
- 1-Ethyl-3-[8-(pyrazin-2-ylamino)-isochinolin-3-yl]-harnstoff;
- 1-Ethyl-3-[8-(pyrimidin-5-ylamino)-isochinolin-3-yl]-harnstoff;
- 1-Ethyl-3-[8-(imidazo[1,2-a]pyridin-7-ylamino)-isochinolin-3-yl]-harnstoff;
- 1-Ethyl-3-[8-(pyrimidin-4-ylamino)-isochinolin-3-yl]-harnstoff;
- 1-Ethyl-3-[8-(pyrimidin-2-ylamino)-isochinolin-3-yl]-harnstoff;
- 1-Ethyl-3-[8-(pyridin-4-ylamino)-isochinolin-3-yl]-harnstoff;
- 1-Ethyl-3-[8-(5-methyl-[1,3,4]oxadiazol-2-ylamino)-isochinolin-3-yl]-harnstoff;
- 1-Ethyl-3-[8-(6-methyl-pyridin-2-ylamino)-isochinolin-3-yl]-harnstoff;
- 6-[3-(3-Ethyl-ureido)-isochinolin-8-ylamino]-nicotinamid;
- 1-Ethyl-3-[8-(3-fluor-pyridin-4-ylamino)-isochinolin-3-yl]-harnstoff;
- 1-Ethyl-3-[8-(4-fluor-pyridin-2-ylamino)-isochinolin-3-yl]-harnstoff;
- 1-Ethyl-3-[8-(5-fluor-pyridin-3-ylamino)-isochinolin-3-yl]-harnstoff;
- 1-Ethyl-3-[8-(4-methoxy-pyridin-2-ylamino)-isochinolin-3-yl]-harnstoff;
- *N*-{5-[3-(3-Ethyl-ureido)-isochinolin-8-ylamino]-pyridin-2-yl}-acetamid;
- 1-Ethyl-3-[8-(4-methyl-pyridin-2-ylamino)-isochinolin-3-yl]-harnstoff;
- 1-Ethyl-3-[8-(5-methyl-pyridin-2-ylamino)-isochinolin-3-yl]-harnstoff;
- 1-Ethyl-3-[8-(6-methyl-pyridin-3-ylamino)-isochinolin-3-yl]-harnstoff;
- 6-[3-(3-Ethyl-ureido)-isochinolin-8-ylamino]-nicotinsäuremethylester;
- 6-[3-(3-Ethyl-ureido)-isochinolin-8-ylamino]-pyridin-2-carbonsäuremethylester;
- 1-Ethyl-3-[8-(3-methyl-pyridin-2-ylamino)-isochinolin-3-yl]-harnstoff;
- 1-Ethyl-3-[8-(3-methoxy-phenylamino)-isochinolin-3-yl]-harnstoff;
- 1-Ethyl-3-(8-phenylamino-isochinolin-3-yl)-harnstoff;
- 1-Ethyl-3-[8-(3-hydroxy-phenylamino)-isochinolin-3-yl]-harnstoff;
- 3-[3-(3-Ethyl-ureido)-isochinolin-8-ylamino]-benzolsulfonamid;
- *N*-{3-[3-(3-Ethyl-ureido)-isochinolin-8-ylamino]-phenyl}-acetamid;
- *N*-{4-[3-(3-Ethyl-ureido)-isochinolin-8-ylamino]-phenyl}-acetamid;
- 1-[8-(4-Cyano-phenylamino)-isochinolin-3-yl]-3-ethyl-harnstoff;
- 1-Ethyl-3-[8-(4-hydroxy-phenylamino)-isochinolin-3-yl]-harnstoff;
- 3-[3-(3-Ethyl-ureido)-isochinolin-8-ylamino]-benzoesäuremethylester;
- 1-Ethyl-3-[8-(5-methoxy-pyridin-3-ylamino)-isochinolin-3-yl]-harnstoff;
- 1-Ethyl-3-[8-(6-methoxy-pyridin-2-ylamino)-isochinolin-3-yl]-harnstoff;
- 4-[3-(3-Ethyl-ureido)-isochinolin-8-ylamino]-benzoesäuremethylester;
- 1-Ethyl-3-[8-(2-methoxy-pyridin-4-ylamino)-isochinolin-3-yl]-harnstoff;
- 1-Ethyl-3-[8-(5-methoxy-pyridin-2-ylamino)-isochinolin-3-yl]-harnstoff;
- 1*H*-Pyrrol-2-carbonsäure [3-(3-ethyl-ureido)-isochinolin-8-yl]-amid;
- 3*H*-[1,2,3]Triazol-4-carbonsäure [3-(3-ethyl-ureido)-isochinolin-8-yl]-amid;
- 1-Ethyl-3-{6-[(1*H*-indazol-6-ylmethyl)-amino]-8-methyl-isochinolin-3-yl}-harnstoff;
- 1-Ethyl-3-{8-methyl-6-[(2-morpholin-4-yl-pyridin-4-ylmethyl)-amino]-isochinolin-3-yl}-harnstoff;
- 2-(4-{[3-(3-Ethyl-ureido)-8-methyl-isochinolin-6-ylamino]-methyl}-phenoxy)-acetamid;
- 1-Ethyl-3-[8-methyl-6-(3-[1,2,4]triazol-1-yl-benzylamino)-isochinolin-3-yl]-harnstoff;
- 1-{6-[(3-((1*H*-1,2,4-Triazol-1-yl)methyl)benzyl)amino]-8-methylisochinolin-3-yl}-3-ethylharnstoff;
- 4-{[3-(3-Ethyl-ureido)-8-methyl-isochinolin-6-ylamino]-methyl}-*N*-methyl-benzolsulfonamid;
- 1-Ethyl-3-[8-(pyridin-3-ylaminomethyl)-isochinolin-3-yl]-harnstoff;
- 1-{8-[(2,5-Dimethyl-2*H*-pyrazol-3-ylamino)-methyl]-isochinolin-3-yl}-3-ethyl-harnstoff;
- 1-Ethyl-3-{8-[(3-methyl-isothiazol-5-ylamino)-methyl]-isochinolin-3yl}-harnstoff;
- *N*-[3-(3-Ethyl-ureido)-8-methyl-isochinolin-6-yl]-terephthalamsäuremethylester;
- *N*-[3-(3-Ethyl-ureido)-8-methyl-isochinolin-6-yl]-isophthalamsäuremethylester;
- *N*-[3-(3-Ethyl-ureido)-8-methyl-isochinolin-6-yl]-2-methoxy-acetamid,
- 1-Ethyl-3-{6-[(6-methoxy-pyridin-3-ylmethyl)-amino]-8-methyl-isochinolin-3-yl}-harnstoff;
- 1-Ethyl-3-[6-(3-methoxy-benzylamino)-8-methyl-isochinolin-3-yl]-harnstoff;
- 3-[3-(3-Ethyl-ureido)-isochinolin-8-ylamino]-benzoesäure;
- 3-[3-(3-Ethyl-ureido)-isochinolin-8-ylamino]-*N*,*N*-dimethyl-benzamid;
- 3-[3-(3-Ethyl-ureido)-isochinolin-8-ylamino]-*N*-methyl-benzamid;
- *N*-Benzyl-3-[3-(3-ethyl-ureido)-isochinolin-8-ylamino]-benzamid;
- 4-[3-(3-Ethyl-ureido)-isochinolin-8-ylamino]-benzoesäure;
- 4-[3-(3-Ethyl-ureido)-isochinolin-8-ylamino]-*N*,*N*-dimethyl-benzamid;
- 4-[3-(3-Ethyl-ureido)-isochinolin-8-ylamino]-*N*-methyl-benzamid;
- {4-[3-(3-Ethyl-ureido)-isochinolin-8-ylamino]-phenyl}-essigsäure;
- 2-{4-[3-(3-Ethyl-ureido)-isochinolin-8-ylamino]-phenyl}-*N*,*N*-dimethyl-acetamid;
- 2-{4-[3-(3-Ethyl-ureido)-isochinolin-8-ylamino]-phenyl}-*N*-methyl-acetamid;
- *N*-Benzyl-2-{4-[3-(3-ethyl-ureido)-isochinolin-8-ylamino]-phenyl}-acetamid;
- {3-[3-(3-Ethyl-ureido)-isochinolin-8-ylamino]-phenyl}-essigsäure;
- *N*-Benzyl-2-{3-[3-(3-ethyl-ureido)-isochinolin-8-ylamino]-phenyl}-acetamid;
- 2-{3-[3-(3-Ethyl-ureido)-isochinolin-8-ylamino]-phenyl}-*N*,*N*-dimethyl-acetamid;
- 4-[3-(3-Ethyl-ureido)-isochinolin-8-ylmethoxy]-nicotinsäuremethylester;
- 1-Ethyl-3-[8-(3-hydroxy-phenoxymethyl)-isochinolin-3-yl]-harnstoff;
- 1-Ethyl-3-[8-(6-methoxy-pyridin-3-ylamino)-isochinolin-3-yl]-harnstoff;
- 1-Ethyl-3-(5-fluor-8-pyridin-3-yl-isochinolin-3-yl)-harnstoff;
- 1-Ethyl-3-(8-methyl-6-vinyl-isochinolin-3-yl)-harnstoff;
- 1-Ethyl-3-[8-(6-methoxy-pyridin-2-ylamino)-5-pyridin-4-yl-isochinolin-3-yl]-harnstoff;
- 1-Ethyl-3-[8-(6-methyl-pyridin-3-ylamino)-5-pyridin-4-yl-isochinolin-3-yl]-harnstoff;
- 1-Ethyl-3-[8-(6-methyl-pyridin-2-ylamino)-5-pyridin-4-yl-isochinolin-3-yl]-harnstoff;
- 3-[3-(3-Ethyl-ureido)-5-pyridin-4-yl-isochinolin-8-ylamino]-benzoesäuremethylester;
- 1-Ethyl-3-[5-(4-hydroxymethyl-phenyl)-8-(pyridin-3-yloxy)-isochinolin-3-yl]-harnstoff;
- 1-Ethyl-3-[5-pyridin-4-yl-8-(pyridin-3-yloxy)-isochinolin-3-yl]-harnstoff;
- 1-Ethyl-3-[5-pyridin-3-yl-8-(pyridin-3-yloxy)-isochinolin-3-yl]-harnstoff;
- 1-Ethyl-3-[5-pyridin-3-yl-8-(pyridin-3-ylamino)-isochinolin-3-yl]-harnstoff;
- 1-Ethyl-3-[5-(2-methoxy-pyridin-4-yl)-8-(pyridin-3-ylamino)-isochinolin-3-yl]-harnstoff;
- 1-Ethyl-3-[5-(4-hyäroxymethyl-phenyl)-8-(pyridin-3-ylamino)-isochinolin-3-yl]-harnstoff;
- 1-Ethyl-3-[5-pyridin-4-yl-8-(pyridin-3-ylamino)-isochinolin-3-yl]-harnstoff;
- 1-Ethyl-3-[5-(4-hydroxy-phenyl)-8-(pyridin-3-ylamino)-isochinolin-3-yl]-harnstoff;
- 1-Ethyl-3-[5-(2-methyl-pyridin-4-yl)-8-(pyridin-3-ylamino)-isochinolin-3-yl]-harnstoff;
- 4-[3-(3-Ethyl-ureido)-8-(pyridin-3-ylamino)-isochinolin-5-yl]-benzolsulfonamid;
- 1-[5-(2,6-Dimethyl-pyridin-4-yl)-8-(pyridin-3-ylamino)-isochinolin-3-yl]-3-ethyl-harnstoff;
- 1-(5-(3-(Aminomethyl)phenyl)-8-(pyridin-3-ylamino)isochinolin-3-yl)-3-ethylharnstoff;
- 1-[5-(2-Amino-pyridin-4-yl)-8-(pyridin-3-ylamino)-isochinolin-3-yl]-3-ethyl-harnstoff;
- *N*-{3-[3-(3-Ethyl-ureido)-5-fluor-8-(pyridin-3-ylamino)-isochinolin-6-yl]-phenyl}-acetamid;
- 1-Ethyl-3-[5-fluor-6-(5-methyl-pyridin-3-yl)-8-(pyridin-3-ylamino)-isochinolin-3-yl]-harnstoff;
- *N*-{3-[3-(3-Ethyl-ureido)-8-(pyridin-3-ylamino)-isochinolin-6-yl]-phenyl}-acetamid;
- 1-Ethyl-3-[6-(5-methyl-pyridin-3-yl)-8-(pyridin-3-ylamino)-isochinolin-3-yl]-harnstoff;
- 1-Ethyl-3-[6-propylamino-8-(pyridin-3-ylamino)-isochinolin-3-yl]-harnstoff;
- 1-[6-Benzylamino-8-(pyridin-3-ylamino)-isochinolin-3-yl]-3-ethyl-harnstoff;
- 1-[6-Benzylamino-5-fluor-8-(pyridin-3-ylamino)-isochinolin-3-yl]-3-ethyl-harnstoff;
- 3-Dimethylamino-*N*-[3-(3-ethyl-ureido)-8-(pyridin-3-ylamino)-isochinolin-6-yl]-benzamid;
- 5-[3-(3-Ethyl-ureido)-isochinolin-8-ylamino]-2-methoxy-benzamid;
- 5-[3-(3-Ethyl-ureido)-isochinolin-8-ylamino]-2-methoxy-*N*-methyl-benzamid;
- 5-[3-(3-Ethyl-ureido)-isochinolin-8-ylamino]-2-methoxy-*N*,*N*-dimethyl-benzamid;
- 1-Ethyl-3-[5-(2-methyl-pyridin-4-yl)-8-(6-methyl-pyridin-3-ylamino)-isochinolin-3-yl]-harnstoff;
- 3-[3-(3-Ethyl-ureido)-5-(2-methyl-pyridin-4-yl)-isochinolin-8-ylamino]-benzoesäure;
- 3-[3-(3-Ethyl-ureido)-5-(2-methyl-pyridin-4-yl)-isochinolin-8-ylamino]-*N*-methyl-benzamid;
- 3-[3-(3-Ethyl-ureido)-5-(2-methyl-pyridin-4-yl)-isochinolin-8-ylamino]-*N*,*N*-dimethyl-benzamid;
- 3-[3-(3-Ethyl-ureido)-5-(4-hydroxymethyl-phenyl)-isochinolin-8-ylamino]-benzoesäure;
- 3-[3-(3-Ethyl-ureido)-5-(4-hydroxymethyl-phenyl)-isochinolin-8-ylamino]-*N*-methyl-benzamid;
- 3-[3-(3-Ethyl-ureido)-5-(4-hydroxymethyl-phenyl)-isochinolin-8-ylamino]-*N*,*N-*dimethyl-benzamid;
- 4-[3-(3-Ethyl-ureido)-isochinolin-8-ylamino]-benzamid;
- 2-{4-[3-(3-Ethyl-ureido)-isochinolin-8-ylamino]-phenyl}-acetamid;
- 3-[3-(3-Ethyl-ureido)-isochinolin-8-ylamino]-benzamid;
- 2-{3-[3-(3-Ethyl-ureido)-isochinolin-8-ylamino]-phenyl}-acetamid;
- 1-Ethyl-3-[8-(6-methyl-pyridin-3-yloxymethyl)-isochinolin-3-yl]-harnstoff;
- 1-Ethyl-3-[8-(5-methyl-pyridin-2-yloxymethyl)-isochinolin-3-yl]-harnstoff;
- 2-[3-(3-Ethyl-ureido)-isochinolin-8-ylmethoxy]-benzoesäuremethylester;
- 1-Ethyl-3-[8-(2-methyl-pyridin-3-yloxymethyl)-isochinolin-3-yl]-harnstoff;
- 1-[8-(3-Amino-phenoxymethyl)-isochinolin-3-yl]-3-ethyl-harnstoff;
- 1-Ethyl-3-[8-(pyridin-2-yloxymethyl)-isochinolin-3-yl]-harnstoff;
- 1-Ethyl-3-[8-(pyridin-4-yloxymethyl)-isochinolin-3-yl]-harnstoff;
- 1-Ethyl-3-[8-(pyridin-2-ylaminomethyl)-isochinolin-3-yl]-harnstoff;
- 5-[3-(3-Ethyl-ureido)-isochinolin-8-ylamino]-2-methyl-benzoesäuremethylester;
- 5-[3-(3-Ethyl-ureido)-isochinolin-8-ylamino]-2-methyl-benzoesäure;
- 4-[3-(3-Ethyl-ureido)-isochinolin-8-ylamino]-2-methyl-benzoesäuremethylester;
- 4-[3-(3-Ethyl-ureido)-isochinolin-8-ylamino]-2-methyl-benzoesäure;
- 1-[5,8-*Bis*-(pyridin-3-ylamino)-isochinolin-3-yl]-3-ethyl-harnstoff;
- 1-Ethyl-3-(8-methyl-5-methylaminomethyl-isochinolin-3-yl)-harnstoff;
- 1-Ethyl-3- {5-[(methylamino)methyl]-8-(pyridin-3-ylamino)-isochinolin-3-yl}-harnstoff;
- 1-Ethyl-3- {8-[(6-methoxy-pyridin)-2-ylamino]-5-[(methylamino)methyl]-isochinolin-3-yl}-harnstoff;
- 3-{[3-(3-Ethyl-ureido)-5-[(methylamino)methyl]-isochinolin-8-yl]amino}-benzoesäure;
- 1- {5-[(Dimethylamino)methyl]-8-[(6-methoxy-pyridin-2-yl)amino]-isochinolin-3-yl}-3-ethyl-harnstoff;
- 1-Ethyl-3-[5-morpholin-4-yl-8-(pyridin-3-ylamino)-isochinolin-3-yl]-harnstoff;
- 1-Ethyl-3- {8-[(6-methoxy-pyridin-2-yl)amino]-5-(morpholin-4-ylmethyl)-isochinolin-3-yl}-harnstoff;
- 1-Ethyl-3-[8-(6-methoxy-pyridin-2-ylamino)-5-(4-methyl-piperazin-1-ylmethyl)-isochinolin-3-yl]-harnstoff;
- 1-Ethyl-3-[5-(4-methyl-piperazin-1-yl)-8-(pyridin-3-ylamino)-isochinolin-3-yl]-harnstoff;
- 1-Ethyl-3-(8-methyl-5-pyrimidin-4-yl-isochinolin-3-yl)-harnstoff;
- 1-Ethyl-3-(8-methyl-5-pyridazin-4-yl-isochinolin-3-yl)-harnstoff;
- 1-Ethyl-3-[5-(1*H*-imidazol-4-yl)-8-methyl-isochinolin-3-yl]-harnstoff;
- 1-Ethyl-3-[8-methyl-5-(1*H*-pyrazol-4-yl)-isochinolin-3-yl]-harnstoff;
- 1-Ethyl-3-(8-methyl-5-pyridazin-3-yl-isochinolin-3-yl)-harnstoff;
- 1-Ethyl-3-[8-methyl-5-(1-methyl-1*H*-imidazol-4-yl)-isochinolin-3-yl]-harnstoff;
- 1-Ethyl-3-[8-methyl-5-(1-methyl-1*H*-pyrazol-4-yl)-isochinolin-3-yl]-harnstoff;
- 1-Ethyl-3-(8-methyl-5-thiazol-4-yl-isochinolin-3-yl)-harnstoff;
- 1-(8-Chlor-5-pyrimidin-4-yl-isochinolin-3-yl)-3-ethyl-harnstoff;
- 1-Ethyl-3-[8-(6-methyl-pyridin-3-ylamino)-5-pyrimidin-4-yl-isochinolin-3-yl]-harnstoff;
- 1-[8-Chlor-5-(1*H*-imidazol-4-yl)-isochinolin-3-yl]-3-ethyl-harnstoff;
- 1-(8-Chlor-5-pyrimidin-4-yl-isochinolin-3-yl)-3-ethyl-harnstoff;
- 1-Ethyl-3-[8-(6-methoxy-pyridin-2-ylamino)-5-thiazol-4-yl-isochinolin-3-yl]-harnstoff;
- 1-Ethyl-3-[8-(2-methoxy-pyridin-3-ylamino)-isochinolin-3-yl]-harnstoff;
- 1-Ethyl-3-[8-(4-hydroxymethyl-pyrazol-1-ylmethyl)-isochinolin-3-yl]-harnstoff;
- 1-Ethyl-3-[8-(5-hydroxymethyl-imidazol-1-ylmethyl)-isochinolin-3-yl]-harnstoff;
- 1-Ethyl-3-[8-(4-hydroxymethyl-imidazol-1-ylmethyl)-isochinolin-3-yl]-harnstoff;
- 1-[3-(3-Ethyl-ureido)-isochinolin-8-ylmethyl]-1*H*-imidazol-2-carbonsäure;
- 1-Ethyl-3-(8-pyrazol-1-ylmethyl-isochinolin-3-yl)-harnstoff;
- 1-[8-(2,4-Dimethyl-imidazol-1-ylmethyl)-isochinolin-3-yl]-3-ethyl-harnstoff;
- 1-[8-(2,5-Dimethyl-imidazol-1-ylmethyl)-isochinolin-3-yl]-3-ethyl-harnstoff;
- 1-Ethyl-3-[8-(2-methyl-imidazol-1-ylmethyl)-isochinolin-3-yl]-harnstoff;
- 1-Ethyl-3-(8-[1,2,3]triazol-1-ylmethyl-isochinolin-3-yl)-harnstoff;
- 1-Ethyl-3-(8-imidazol-1-ylmethyl-isochinolin-3-yl)-harnstoff;
- 1-Ethyl-3-[8-(4-methyl-pyrazol-1-ylmethyl)-isochinolin-3-yl]-harnstoff;
- 1-Ethyl-3-{8-[(5-methyl-isoxazol-3-ylamino)-methyl]-isochinolin-3-yl}-harnstoff;
- 1-Ethyl-3-{8-[(6-methoxy-pyridin-3-ylamino)-methyl]-isochinolin-3-yl}-harnstoff;
- 1-Ethyl-3-{8-[(2-methyl-2H-pyrazol-3-ylamino)-methyl]-isochinolin-3-yl}-harnstoff;
- 1-Ethyl-3-[8-(pyrimidin-2-ylaminomethyl)-isochinolin-3-yl]-harnstoff;
oder ein Salz einer solchen Verbindung.

13. Verbindung von Formel I nach einem der Ansprüche 1 bis 12 oder ein pharmazeutisch akzeptables Salz davon zur Verwendung als Medikament.

14. Pharmazeutische Zusammensetzung, die als Wirkstoff eine Verbindung von Formel I nach einem der Ansprüche 1 bis 12 oder ein pharmazeutisch akzeptables Salz davon und wenigstens einen therapeutisch inerten Exzipienten enthält.

15. Verbindung von Formel I nach einem der Ansprüche 1 bis 12 oder ein pharmazeutisch akzeptables Salz davon zur Verwendung bei der Verhütung oder Behandlung einer bakteriellen Infektion.

## Revendications

1. Composé de formule I 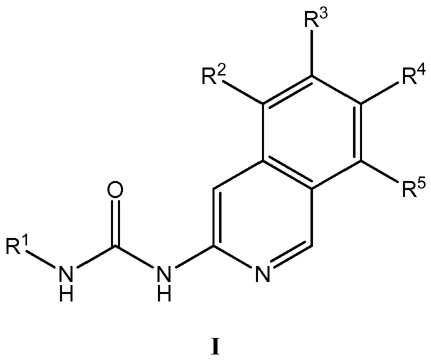 où
R¹ représente un (C₁-C₃)alkyle, un (C₂-C₃)haloalkyle ou un cyclopropyle ;
R⁴ représente un H ;
R³ représente un H, R⁵ représente un H et R² représente un phényle ou un 4-carboxyphényle ; ou
R² représente un H, R⁵ représente un H et R³ représente un benzoylamino ; ou
R² représente un H, R³ représente un H et R⁵ représente un -NH-CO-R⁶, -CH₂-O-R⁷, -NH-R⁸, -CH₂-NH-R⁹, -CH₂-R¹⁰, (pyridin-3-ylméthyl)amino, pyridin-3-yloxy ou 4-carboxyphényle ; ou
R³ représente un H, R⁵ représente un méthyle et R² représente un halogène, (C₁-C₃)alkylamino, (C₁-C₃)alkylaminométhyle, 2-(aminocarbonyl)-éthyle, pyrimidin-4-yle, pyridazin-3-yle, pyridazin-4-yle, 1*H*-imidazol-4-yle, 1-méthyl-1*H*-imidazol-4-yle, 1*H*-pyrazol-4-yle, 1-méthyl-1*H*-pyrazol-4-yle, imidazo[1,2-*a*]pyridin-6-yle, 4-méthyl-thiophén-3-yle, thiazol-4-yle, isoquinoléin-5-yle ou 1*H*-indol-4-yle ou un groupement de formule (A1), (A2) ou (A3) illustré ci-après 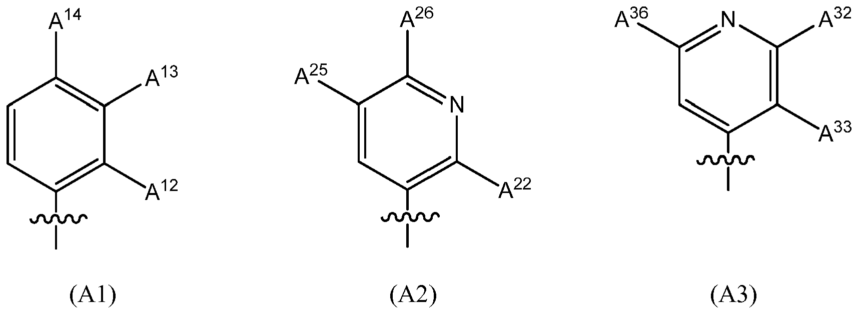
où
chacun des radicaux A¹³ et A¹⁴ représente un H et A¹² représente un H ou un OH, ou chacun des radicaux A¹² et A¹⁴ représente un H et A¹³ représente un OH, acétylamino, aminométhyle, sulfamoyle ou hydroxyméthyle, ou
chacun des radicaux A¹² et A¹³ représente un H et A¹⁴ représente un OH, méthylaminocarbonyle, diméthylaminocarbonyle, méthoxycarbonylamino, sulfamoyle ou hydroxyméthyle ;
chacun des radicaux A²⁵ et A²⁶ représente un H et A²² représente un H ou un halogène, ou
chacun des radicaux A²² et A²⁶ représente un H et A²⁵ représente un méthyle ou un halogène, ou
chacun des radicaux A²² et A²⁵ représente un H et A²⁶ représente un hydroxyméthyle ;
chacun des radicaux A³³ et A³⁶ représente un H et A³² représente un H, halogène, amino, méthyle ou méthoxy, ou
A³³ représente un H et chacun des radicaux A³² et A³⁶ représente un méthyle ; ou
R² représente un H, R⁵ représente un méthyle et R³ représente un amino, vinyle, (C₁-C₄)alkylamino, cyclopropylméthylamino, (2-(benzyloxy)éthyl)amino, méthoxyméthylcarbonylamino, (thiazol-5-yl)carbonylamino, pyridin-3-ylcarbonylamino, pyridin-4-ylcarbonylamino, naphtalén-2-yle, furan-2-yle, furan-3-yle, 1*H*-pyrazol-4-yle, thiophén-3-yle, 4-méthyl-thiophén-3-yle, quinoléin-3-yle, isoquinoléin-4-yle, isoquinoléin-5-yle, imidazo[1,2-*a*]pyridin-6-yle, 1*H*-indol-4-yle ou un groupement de formule (B1), (B2), (B3), (B4), (B5), (B6), (B7), (B8), (B9), (B10), (B11) ou (B12) illustré ci-après 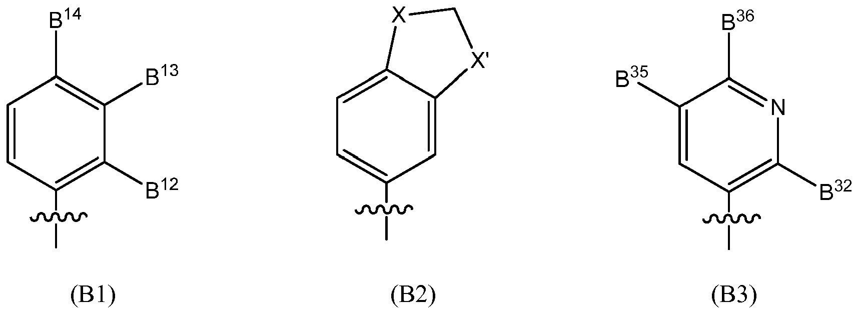 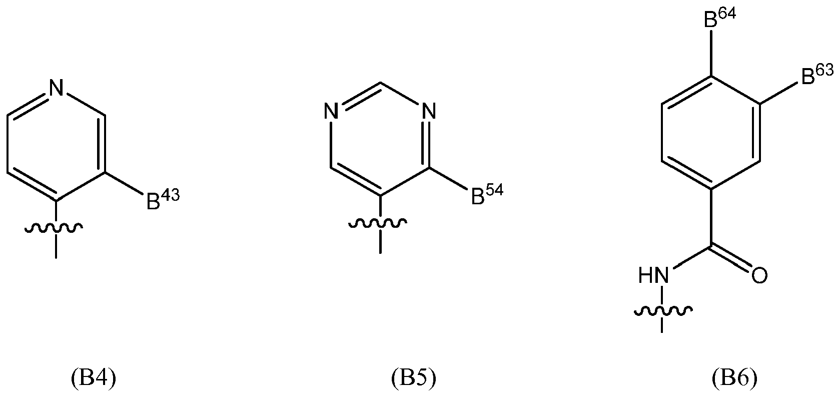 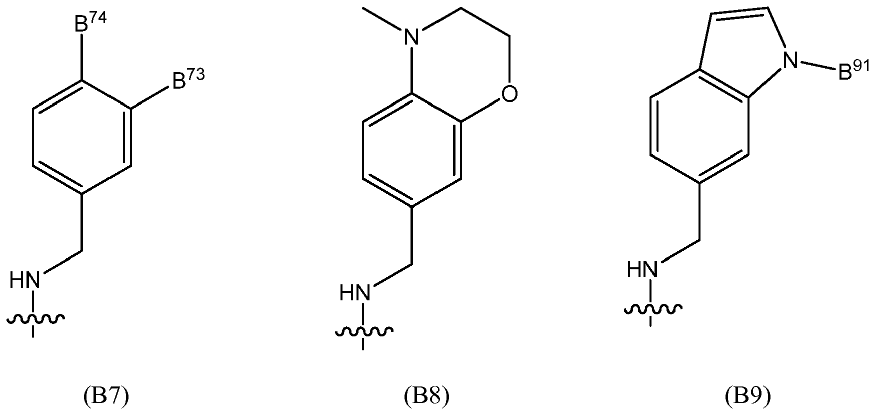 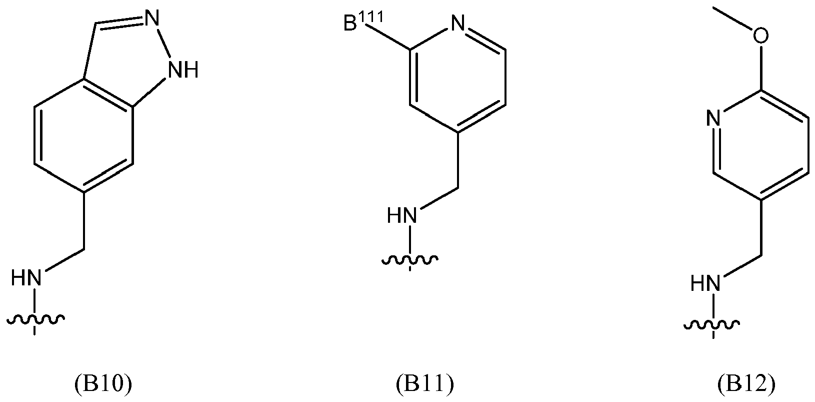
où
chacun des radicaux B¹³ et B¹⁴ représente un H et B¹² représente un OH, méthyle, acétylamino, (C₁-C₂)alkoxycarbonyle ou diméthylaminocarbonyle, ou
chacun des radicaux B¹² et B¹⁴ représente un H et B¹³ représente un OH, halogène, acétyle, acétylamino, acétylaminométhyle, aminocarbonyle, sulfamoyle, (C₁-C₂)alkoxy, (C₁-C₂)alkoxycarbonyle, cyano, amino-(C₁-C₂)alkyle ou hydroxy-(C₁-C₂)alkyle, ou
chacun des radicaux B¹² et B¹³ représente un H et B¹⁴ représente un acétyle, acétylamino, acétylaminométhyle, aminocarbonyle, diméthylaminocarbonyle, sulfamoyle, (C₁-C₂)alkylsulfonyle, cyanométhyle ou hydroxyméthyle ;
X et X' représentent chacun -O- ou un des radicaux X et X' représente -O- et l'autre représente -CH₂- ;
chacun des radicaux B³⁵ et B³⁶ représente un H et B³² représente un H ou un halogène, ou
chacun des radicaux B³² et B³⁶ représente un H et B³⁵ représente un halogène, méthyle, méthylsulfanyle, méthanesulfonyle ou méthoxycarbonyle, ou
chacun des radicaux B³² et B³⁵ représente un H et B³⁶ représente un halogène, un méthoxy ou un hydroxyméthyle ;
B⁴³ représente un H ou un halogène ;
B⁵⁴ représente un H ou un (C₁-C₃)alkyle ;
B⁶⁴ représente un H et B⁶³ représente un H, un méthoxycarbonyle ou un diméthylamino, ou
B⁶³ représente un H et B⁶⁴ représente un méthoxy, méthoxycarbonyle, diméthylamino ou méthanesulfonyle ;
B⁷⁴ représente un H et B⁷³ représente un H, méthoxy, méthoxycarbonyle, carboxy, 2-hydroxyéthoxy, 1*H*-1,2,4-triazol-1-yle ou 1*H*-1,2,4-triazol-1-ylméthyle, ou
B⁷³ représente un H et B⁷⁴ représente un carboxy, acétylamino, ((aminocarbonyl)méthyl)oxy, N-méthylsulfamoyle ou 3-méthyl-1*H*-1,2,4-triazol-1-yle ;
B⁹¹ représente un H ou un méthyle ;
B¹¹¹ représente un H ou un morpholino ; ou
R² représente un H, R⁵ représente un pyridin-3-ylamino et R³ représente un propylamino, 3-acétylamino-phényle, benzylamino, 5-méthylpyridin-3-yle ou 3-(*N,N-*diméthylamino)phénylcarbonylamino ; ou
R² représente un fluor, R³ représente un H et R⁵ représente un brome, benzyle, benzoylamino, 4-carboxyphényle, benzylamino, pyridin-3-yle ou (pyridin-3-ylméthyl)amino ; ou
R² représente un brome, R³ représente un H et R⁵ représente un méthoxy ; ou
R² représente un fluor, R⁵ représente un méthyle et R³ représente un quinoléin-3-yle, un benzylamino, un groupement phényle éventuellement substitué une fois par un acétylamino ou un sulfamoyle, un pyridin-2-yle ou un groupement pyridin-3-yle éventuellement substitué une fois par un méthyle ; ou
R² représente un fluor, R³ représente un pyridin-3-yle et R⁵ représente un pyridin-3-yle ou un 4-carboxyphényle ; ou
R² représente un fluor, R³ représente un benzylamino et R⁵ représente un chlore ou un 2-méthoxy-pyrimidin-5-yle ; ou
R² représente un fluor, R³ représente un quinoléin-3-yle et R⁵ représente un quinoléin-3-yle ; ou
R² représente un fluor, R³ représente un 3-(acétylamino)phényle et R⁵ représente un pyridin-3-yle, un pyridin-3-ylamino ou un 2-méthoxypyrimidin-5-yle ; ou
R² représente un fluor, R³ représente un 4-sulfamoyl-phényle et R⁵ représente un chlore ; ou
R² représente un fluor, R⁵ représente un pyridin-3-ylamino et R³ représente un benzylamino ou un 5-méthylpyridin-3-yle ; ou
R³ représente un H, R² représente un (C₁-C₃)alkylaminométhyle, *N,N*-diméthylaminométhyle, pyridin-3-yle, pyridin-4-yle, 2-méthylpyridin-4-yle, 2-méthoxypyridin-4-yle, 2-aminopyridin-4-yle, 2,6-diméthylpyridin-4-yle, pyrimidin-4-yle, 1*H*-imidazol-4-yle, 1*H*-pyrazol-4-yle, thiazol-4-yle, pyridin-3-ylamino, 3-(aminométhyl)phényle, 4-hydroxyphényle, 4-(hydroxyméthyl)phényle, 4-sulfamoyl-phényle, morpholino, morpholinométhyle, 4-méthylpipérazin-1-yle ou 4-méthyl-pipérazin-1-ylméthyle et R⁵ représente un chlore, vinyle, méthoxy, (3-carboxyphényl)amino, (3-(méthoxycarbonyl)phényl)amino, (3-(diméthylcarbamoyl)phényl)amino, (3-(méthylcarbamoyl)phényl)amino, pyridin-4-yle, (6-méthyl-pyridin-2-yl)amino, (6-méthoxy-pyridin-2-yl)amino, pyridin-3-ylamino, (6-méthyl-pyridin-3-yl)amino ou pyridin-3-yloxy ;
R⁶ représente un phényle, un 1*H*-pyrrol-2-yle ou un 1*H*-1,2,3-triazol-5-yle ;
R⁷ représente un pyridin-2-yle, 5-méthyl-pyridin-2-yle, 2-méthyl-pyridin-3-yle, 6-méthyl-pyridin-3-yle, pyridin-4-yle, 3-méthoxycarbonyl-pyridin-4-yle, 2-(méthoxycarbonyl)phényle, 3-hydroxy-phényle ou 3-amino-phényle ;
R⁸ représente un pyridin-2-yle, 6-méthoxy-pyridin-2-yle, 5-méthoxy-pyridin-2-yle, 4-méthoxy-pyridin-2-yle, 3-méthyl-pyridin-2-yle, 4-méthyl-pyridin-2-yle, 5-méthyl-pyridin-2-yle, 6-méthyl-pyridin-2-yle, 5-aminocarbonyl-pyridin-2-yle, 4-fluoro-pyridin-2-yle, 5-méthoxycarbonyl-pyridin-2-yle, 6-méthoxycarbonyl-pyridin-2-yle, pyridin-3-yle, 5-fluoro-pyridin-3-yle, 2-méthoxypyridin-3-yle, 5-méthoxypyridin-3-yle, 6-méthoxypyridin-3-yle, 6-acétylamino-pyridin-3-yle, 6-méthyl-pyridin-3-yle, pyridin-4-yle, 2-méthoxypyridin-4-yle, 3-fluoro-pyridin-4-yle, pyrazin-2-yle, pyrimidin-2-yle, pyrimidin-4-yle, pyrimidin-5-yle, phényle, 3-hydroxyphényle, 3-méthoxyphényle, 3-carboxyphényle, 3-carbamoylphényle, 3-acétylamino-phényle, 3-sulfamoylphényle, 3-méthoxycarbonyl-phényle, 4-méthyl-3-méthoxycarbonyl-phényle, 4-méthyl-3-carboxyphényle, 3-(carboxyméthyl)phényle, 3-(méthylcarbamoyl)phényle, 3-(diméthylcarbamoyl)phényle, 3-(benzylcarbamoyl)phényle, 3-(2-amino-2-oxoéthyl)phényle, 3-(2-(diméthylamino)-2-oxoéthyl)phényle, 3-(2-(benzylamino)-2-oxoéthyl)phényle, 4-méthoxycarbonyl-phényle, 3-méthyl-4-méthoxycarbonyl-phényle, 4-carboxyphényle, 3-méthyl-4-carboxyphényle, 3-carbamoyl-4-méthoxyphényle, 4-méthoxy-3-(méthylcarbamoyl)phényle, 3-(diméthylcarbamoyl)-4-méthoxyphényle, 4-hydroxyphényle, 4-(carboxyméthyl)phényle, 4-cyanophényle, 4-acétylamino-phényle, 4-carbamoylphényle, 4-(méthylcarbamoyl)phényle, 4-(diméthylcarbamoyl)phényle, 4-(2-amino-2-oxoéthyl)phényle, 4-(2-(diméthylamino)-2-oxoéthyl)phényle, 4-(2-(méthylamino)-2-oxoéthyl)phényle, 4-(2-(benzylamino)-2-oxoéthyl)phényle, 5-méthyl-1,3,4-oxadiazol-2-yle ou imidazo[1,2-*a*]pyridin-7-yle ;
R⁹ représente un pyridin-2-yle, pyridin-3-yle, 6-méthoxypyridin-3-yle, pyrimidin-2-yle, 1,3-diméthyl-1*H*-pyrazol-5-yle, 1-méthyl-1*H*-pyrazol-5-yle, 5-méthylisoxazol-3-yle ou 3-méthylisothiazol-5-yle ;
R¹⁰ représente un 1*H*-imidazol-1-yle, 4-(hydroxyméthyl)-1*H*-imidazol-1-yle, 5-(hydroxyméthyl)-1*H*-imidazol-1-yle, 2-carboxy-1*H*-imidazol-1-yle, 2,4-diméthyl-1*H*-imidazol-1-yle, 2,5-diméthyl-1*H* imidazol-1-yle, 2-méthyl-1*H* imidazol-1-yle, 1*H*-1,2,3-triazol-1-yle, 1*H*-pyrazol-1-yle, 4-méthyl-1*H*-pyrazol-1-yle ou 4-(hydroxyméthyl)-1*H*-pyrazol-1-yle ;
ou sel de celui-ci.

2. Composé de formule I selon la revendication 1, qui est également un composé de formule I_{P2} 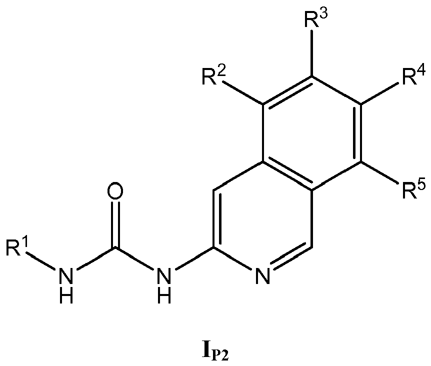 où
R¹ représente un (C₁-C₃)alkyle, un (C₂-C₃)haloalkyle ou un cyclopropyle ;
R⁴ représente un H ;
R³ représente un H, R⁵ représente un H et R² représente un phényle ou un 4-carboxyphényle ; ou
R² représente un H, R⁵ représente un H et R³ représente un benzoylamino ; ou
R² représente un H, R³ représente un H et R⁵ représente un -NH-CO-R⁶, -CH₂-O-R⁷, -NH-R⁸, -CH₂-NH-R⁹, (pyridin-3-ylméthyl)amino, pyridin-3-yloxy ou 4-carboxyphényle ; ou
R³ représente un H, R⁵ représente un méthyle et R² représente un halogène, (C₁-C₃)alkylamino, 2-(aminocarbonyl)-éthyle, imidazo[1,2-*a*]pyridin-6-yle, 4-méthyl-thiophén-3-yle, isoquinoléin-5-yle ou 1*H*-indol-4-yle ou un groupement de formule (A1), (A2) ou (A3) illustré ci-après 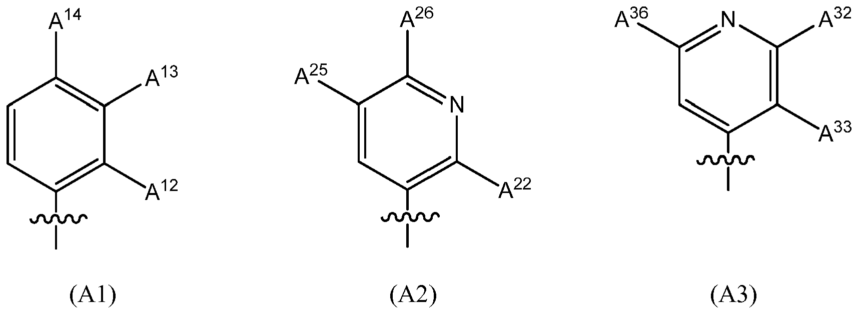
où
chacun des radicaux A¹³ et A¹⁴ représente un H et A¹² représente un H ou un OH, ou chacun des radicaux A¹² et A¹⁴ représente un H et A¹³ représente un OH, acétylamino, aminométhyle, sulfamoyle ou hydroxyméthyle, ou
chacun des radicaux A¹² et A¹³ représente un H et A¹⁴ représente un OH, méthylaminocarbonyle, diméthylaminocarbonyle, méthoxycarbonylamino, sulfamoyle ou hydroxyméthyle ;
chacun des radicaux A²⁵ et A²⁶ représente un H et A²² représente un H ou un halogène, ou
chacun des radicaux A²² et A²⁶ représente un H et A²⁵ représente un méthyle ou un halogène, ou
chacun des radicaux A²² et A²⁵ représente un H et A²⁶ représente un hydroxyméthyle ;
chacun des radicaux A³³ et A³⁶ représente un H et A³² représente un H, halogène, amino, méthyle ou méthoxy, ou
A³³ représente un H et chacun des radicaux A³² et A³⁶ représente un méthyle ; ou
R² représente un H, R⁵ représente un méthyle et R³ représente un amino, vinyle, (C₁-C₄)alkylamino, cyclopropylméthylamino, (2-(benzyloxy)éthyl)amino, méthoxyméthylcarbonylamino, (thiazol-5-yl)carbonylamino, pyridin-3-ylcarbonylamino, pyridin-4-ylcarbonylamino, naphtalén-2-yle, furan-2-yle, furan-3-yle, 1*H*-pyrazol-4-yle, thiophén-3-yle, 4-méthyl-thiophén-3-yle, quinoléin-3-yle, isoquinoléin-4-yle, isoquinoléin-5-yle, imidazo[1,2-*a*]pyridin-6-yle, 1*H*-indol-4-yle ou un groupement de formule (B1), (B2), (B3), (B4), (B5), (B6), (B7), (B8), (B9), (B10), (B11) ou (B12) illustré ci-après 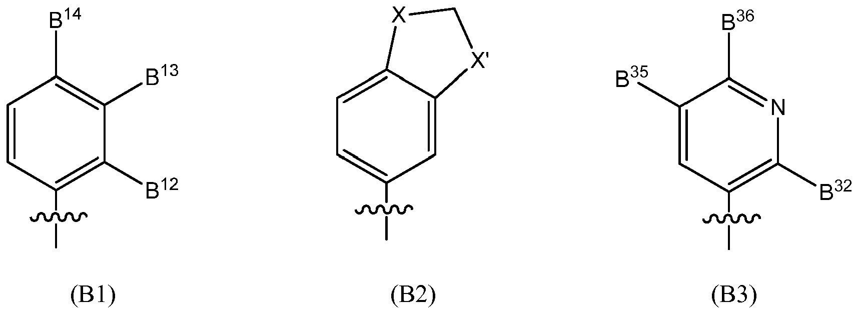 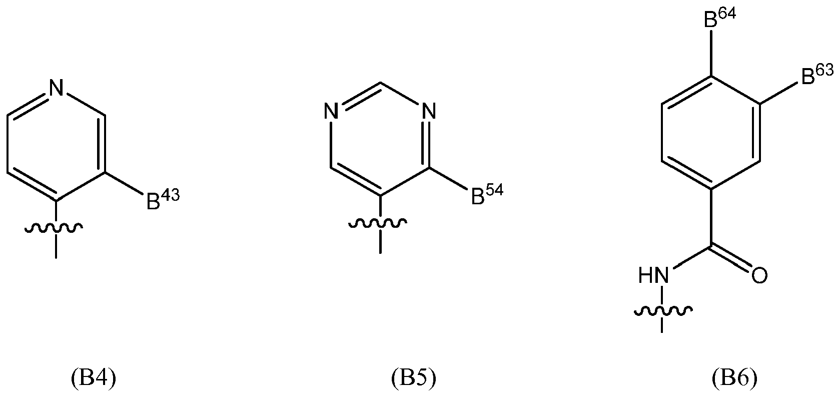 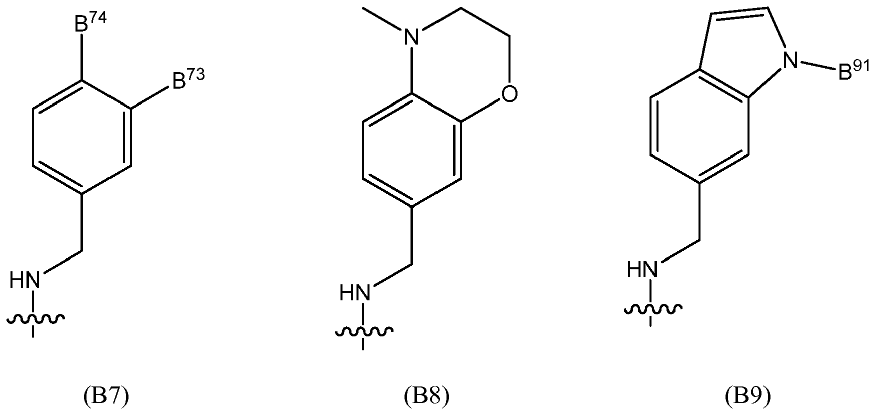 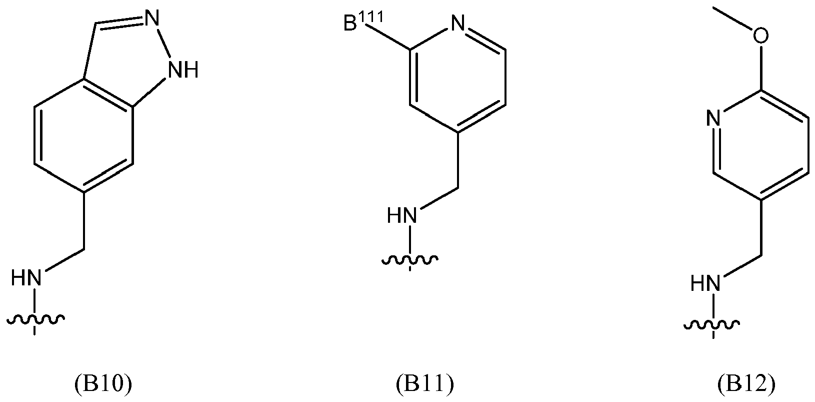
où
chacun des radicaux B¹³ et B¹⁴ représente un H et B¹² représente un OH, méthyle, acétylamino, (C₁-C₂)alkoxycarbonyle ou diméthylaminocarbonyle, ou
chacun des radicaux B¹² et B¹⁴ représente un H et B¹³ représente un OH, halogène, acétyle, acétylamino, acétylaminométhyle, aminocarbonyle, sulfamoyle, (C₁-C₂)alkoxy, (C₁-C₂)alkoxycarbonyle, cyano, amino-(C₁-C₂)alkyle ou hydroxy-(C₁-C₂)alkyle, ou
chacun des radicaux B¹² et B¹³ représente un H et B¹⁴ représente un acétyle, acétylamino, acétylaminométhyle, aminocarbonyle, diméthylaminocarbonyle, sulfamoyle, (C₁-C₂)alkylsulfonyle, cyanométhyle ou hydroxyméthyle ;
X et X' représentent chacun -O- ou un des radicaux X et X' représente -O- et l'autre représente -CH₂- ;
chacun des radicaux B³⁵ et B³⁶ représente un H et B³² représente un H ou un halogène, ou
chacun des radicaux B³² et B³⁶ représente un H et B³⁵ représente un halogène, méthyle, méthylsulfanyle, méthanesulfonyle ou méthoxycarbonyle, ou
chacun des radicaux B³² et B³⁵ représente un H et B³⁶ représente un halogène, un méthoxy ou un hydroxyméthyle ;
B⁴³ représente un H ou un halogène ;
B⁵⁴ représente un H ou un (C₁-C₃)alkyle ;
B⁶⁴ représente un H et B⁶³ représente un H, un méthoxycarbonyle ou un diméthylamino, ou B⁶³ représente un H et B⁶⁴ représente un méthoxy, méthoxycarbonyle, diméthylamino ou méthanesulfonyle ;
B⁷⁴ représente un H et B⁷³ représente un H, méthoxy, méthoxycarbonyle, carboxy, 2-hydroxyéthoxy, 1*H*-1,2,4-triazol-1-yle ou 1*H*-1,2,4-triazol-1-ylméthyle, ou
B⁷³ représente un H et B⁷⁴ représente un carboxy, acétylamino, ((aminocarbonyl)méthyl)oxy, *N*-méthylsulfamoyle ou 3-méthyl-1*H*-1,2,4-triazol-1-yle ;
B⁹¹ représente un H ou un méthyle ;
B¹¹¹ représente un H ou un morpholino ; ou
R² représente un H, R⁵ représente un pyridin-3-ylamino et R³ représente un propylamino, 3-acétylamino-phényle, benzylamino, 5-méthylpyridin-3-yle ou 3-(*N,N-*diméthylamino)phénylcarbonylamino ; ou
R² représente un fluor, R³ représente un H et R⁵ représente un brome, benzyle, benzoylamino, 4-carboxyphényle, benzylamino, pyridin-3-yle ou (pyridin-3-ylméthyl)amino ; ou
R² représente un brome, R³ représente un H et R⁵ représente un méthoxy ; ou
R² représente un fluor, R⁵ représente un méthyle et R³ représente un quinoléin-3-yle, un benzylamino, un groupement phényle éventuellement substitué une fois par un acétylamino ou un sulfamoyle, un pyridin-2-yle ou un groupement pyridin-3-yle éventuellement substitué une fois par un méthyle ; ou
R² représente un fluor, R³ représente un pyridin-3-yle et R⁵ représente un pyridin-3-yle ou un 4-carboxyphényle ; ou
R² représente un fluor, R³ représente un benzylamino et R⁵ représente un chlore ou un 2-méthoxy-pyrimidin-5-yle ; ou
R² représente un fluor, R³ représente un quinoléin-3-yle et R⁵ représente un quinoléin-3-yle ; ou
R² représente un fluor, R³ représente un 3-(acétylamino)phényle et R⁵ représente un pyridin-3-yle, un pyridin-3-ylamino ou un 2-méthoxypyrimidin-5-yle ; ou
R² représente un fluor, R³ représente un 4-sulfamoyl-phényle et R⁵ représente un chlore ; ou
R² représente un fluor, R⁵ représente un pyridin-3-ylamino et R³ représente un benzylamino ou un 5-méthylpyridin-3-yle ; ou
R³ représente un H, R² représente un pyridin-3-yle, pyridin-4-yle, 2-méthylpyridin-4-yle, 2-méthoxypyridin-4-yle, 2-aminopyridin-4-yle, 2,6-diméthylpyridin-4-yle, 3-(aminométhyl)phényle, 4-hydroxyphényle, 4-(hydroxyméthyl)phényle ou 4-sulfamoyl-phényle et R⁵ représente un vinyle, méthoxy, (3-carboxyphényl)amino, (3-(méthoxycarbonyl)phényl)amino, (3-(diméthylcarbamoyl)phényl)amino, (3-(méthylcarbamoyl)phényl)amino, pyridin-4-yle, (6-méthyl-pyridin-2-yl)amino, (6-méthoxy-pyridin-2-yl)amino, pyridin-3-ylamino, (6-méthyl-pyridin-3-yl)amino ou pyridin-3-yloxy ;
R⁶ représente un phényle, un 1*H*-pyrrol-2-yle ou un 1*H*-1,2,3-triazol-5-yle ;
R⁷ représente un pyridin-2-yle, 5-méthyl-pyridin-2-yle, 2-méthyl-pyridin-3-yle, 6-méthyl-pyridin-3-yle, pyridin-4-yle, 3-méthoxycarbonyl-pyridin-4-yle, 2-(méthoxycarbonyl)phényle, 3-hydroxy-phényle ou 3-amino-phényle ;
R⁸ représente un pyridin-2-yle, 6-méthoxy-pyridin-2-yle, 5-méthoxy-pyridin-2-yle, 4-méthoxy-pyridin-2-yle, 3-méthyl-pyridin-2-yle, 4-méthyl-pyridin-2-yle, 5-méthyl-pyridin-2-yle, 6-méthyl-pyridin-2-yle, 5-aminocarbonyl-pyridin-2-yle, 4-fluoro-pyridin-2-yle, 5-méthoxycarbonyl-pyridin-2-yle, 6-méthoxycarbonyl-pyridin-2-yle, pyridin-3-yle, 5-fluoro-pyridin-3-yle, 5-méthoxy-pyridin-3-yle, 6-méthoxy-pyridin-3-yle, 6-acétylamino-pyridin-3-yle, 6-méthyl-pyridin-3-yle, pyridin-4-yle, 2-méthoxy-pyridin-4-yle, 3-fluoro-pyridin-4-yle, pyrazin-2-yle, pyrimidin-2-yle, pyrimidin-4-yle, pyrimidin-5-yle, phényle, 3-hydroxyphényle, 3-méthoxyphényle, 3-carboxyphényle, 3-carbamoylphényle, 3-acétylamino-phényle, 3-sulfamoylphényle, 3-méthoxycarbonyl-phényle, 3-(carboxyméthyl)phényle, 3-(méthylcarbamoyl)phényle, 3-(diméthylcarbamoyl)phényle, 3-(benzylcarbamoyl)phényle, 3-(2-amino-2-oxoéthyl)phényle, 3-(2-(diméthylamino)-2-oxoéthyl)phényle, 3-(2-(benzylamino)-2-oxoéthyl)phényle, 4-méthoxycarbonyl-phényle, 4-carboxyphényle, 3-carbamoyl-4-méthoxyphényle, 4-méthoxy-3-(méthylcarbamoyl)phényle, 3-(diméthylcarbamoyl)-4-méthoxyphényle, 4-hydroxyphényle, 4-(carboxyméthyl)phényle, 4-cyanophényle, 4-acétylamino-phényle, 4-carbamoylphényle, 4-(méthylcarbamoyl)phényle, 4-(diméthylcarbamoyl)phényle, 4-(2-amino-2-oxoéthyl)phényle, 4-(2-(diméthylamino)-2-oxoéthyl)phényle, 4-(2-(méthylamino)-2-oxoéthyl)phényle, 4-(2-(benzylamino)-2-oxoéthyl)phényle, 5-méthyl-1,3,4-oxadiazol-2-yle ou imidazo[1,2-*a*]pyridin-7-yle ;
R⁹ représente un pyridin-2-yle, pyridin-3-yle, 1,3-diméthyl-1*H*-pyrazol-5-yle ou 3-méthylisothiazol-5-yle ;
ou sel de celui-ci.

3. Composé de formule I selon la revendication 1, qui est également un composé de formule I_{P1} 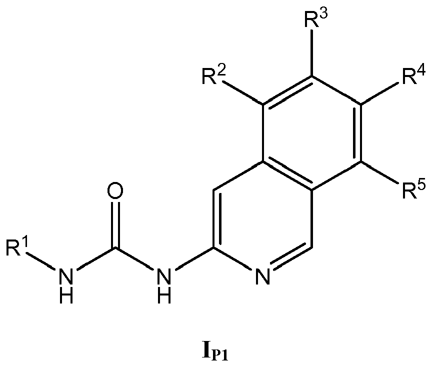 où
R¹ représente un (C₁-C₃)alkyle, un (C₂-C₃)haloalkyle ou un cyclopropyle ;
R⁴ représente un H ;
R³ représente un H, R⁵ représente un H et R² représente un phényle ou un 4-carboxyphényle ; ou
R² représente un H, R⁵ représente un H et R³ représente un benzoylamino ; ou
R² représente un H, R³ représente un H et R⁵ représente un benzoylamino, pyridin-3-ylméthylamino, pyridin-2-ylamino, pyridin-3-ylamino, pyridin-3-yloxy ou 4-carboxyphényle ; ou
R³ représente un H, R⁵ représente un méthyle et R² représente un halogène, (C₁-C₃)alkylamino, 2-(aminocarbonyl)-éthyle, imidazo[1,2-*a*]pyridin-6-yle, 4-méthyl-thiophén-3-yle, isoquinoléin-5-yle ou 1*H*-indol-4-yle ou un groupement de formule (A1), (A2) ou (A3) illustré ci-après 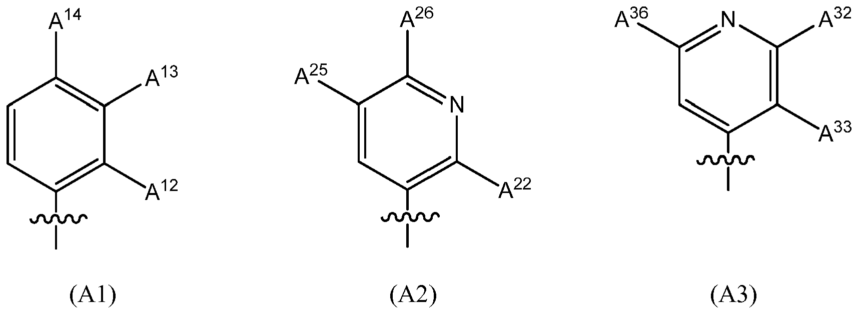
où
chacun des radicaux A¹³ et A¹⁴ représente un H et A¹² représente un OH, ou
chacun des radicaux A¹² et A¹⁴ représente un H et A¹³ représente un OH, acétylamino, aminométhyle, aminosulfonyle ou hydroxyméthyle, ou
chacun des radicaux A¹² et A¹³ représente un H et A¹⁴ représente un OH, méthylaminocarbonyle, diméthylaminocarbonyle, méthoxycarbonylamino, aminosulfonyle ou hydroxyméthyle ;
chacun des radicaux A²⁵ et A²⁶ représente un H et A²² représente un H ou un halogène, ou
chacun des radicaux A²² et A²⁶ représente un H et A²⁵ représente un méthyle ou un halogène, ou
chacun des radicaux A²² et A²⁵ représente un H et A²⁶ représente un hydroxyméthyle ;
chacun des radicaux A³³ et A³⁶ représente un H et A³² représente un H, halogène, amino, méthyle ou méthoxy, ou
A³³ représente un H et chacun des radicaux A³² et A³⁶ représente un méthyle ; ou
R² représente un H, R⁵ représente un méthyle et R³ représente un amino, (C₁-C₄)alkylamino, cyclopropylméthylamino, 2-benzyloxy-éthylamino, (thiazol-5-yl)carbonylamino, pyridin-3-ylcarbonylamino, pyridin-4-ylcarbonylamino, pyridin-4-ylméthylamino, naphtalén-2-yle, furan-2-yle, furan-3-yle, 1*H*-pyrazol-4-yle, thiophén-3-yle, 4-méthyl-thiophén-3-yle, quinoléin-3-yle, isoquinoléin-4-yle, isoquinoléin-5-yle, imidazo[1,2-*a*]pyridin-6-yle, 1*H*-indol-4-yle ou un groupement de formule (B1), (B2), (B3), (B4), (B5), (B6), (B7), (B8) ou (B9) illustré ci-après 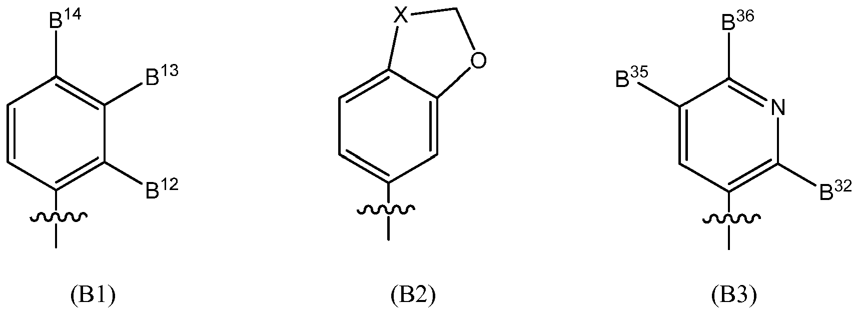 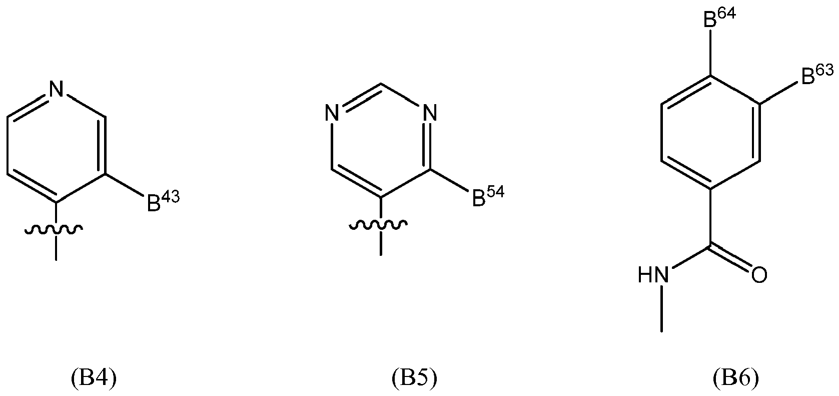 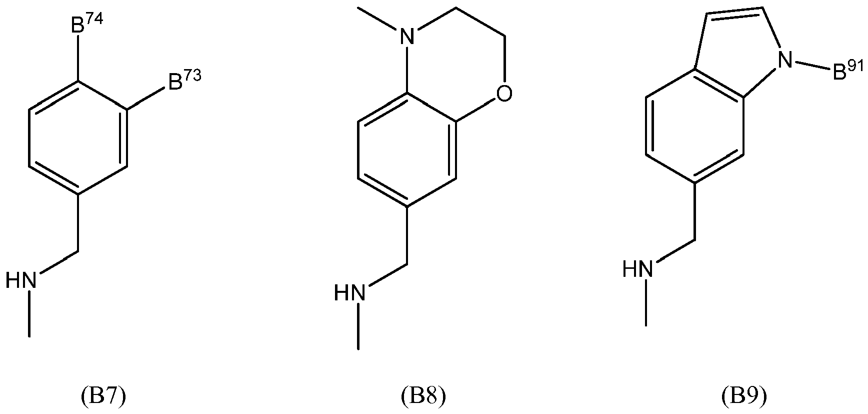
où
chacun des radicaux B¹³ et B¹⁴ représente un H et B¹² représente un OH, méthyle, acétylamino, éthoxycarbonyle ou diméthylaminocarbonyle, ou
chacun des radicaux B¹² et B¹⁴ représente un H et B¹³ représente un OH, halogène, acétyle, acétylamino, acétylaminométhyle, aminocarbonyle, aminosulfonyle, (C₁-C₂)alkoxy, (C₁-C₂)alkoxycarbonyle, cyano, amino-(C₁-C₂)alkyle ou hydroxy-(C₁-C₂)alkyle, ou chacun des radicaux B¹² et B¹³ représente un H et B¹⁴ représente un acétyle, acétylamino, acétylaminométhyle, aminocarbonyle, diméthylaminocarbonyle, aminosulfonyle, (C₁-C₂)alkylsulfonyle, cyanométhyle ou hydroxyméthyle ;
X représente -O- ou -CH₂- ;
chacun des radicaux B³⁵ et B³⁶ représente un H et B³² représente un H ou un halogène, ou
chacun des radicaux B³² et B³⁶ représente un H et B³⁵ représente un halogène, méthyle, méthylsulfanyle, méthanesulfonyle ou méthoxycarbonyle, ou
chacun des radicaux B³² et B³⁵ représente un H et B³⁶ représente un halogène, un méthoxy ou un hydroxyméthyle ;
B⁴³ représente un H ou un halogène ;
B⁵⁴ représente un H ou un (C₁-C₃)alkyle ;
B⁶⁴ représente un H et B⁶³ représente un H ou un diméthylamino, ou
B⁶³ représente un H et B⁶⁴ représente un méthoxy, un diméthylamino ou un méthylsulfonyle ;
B⁷⁴ représente un H et B⁷³ représente un H, méthoxycarbonyle, un carboxy ou un 2-hydroxyéthoxy, ou
B⁷³ représente un H et B⁷⁴ représente un carboxy, un acétylamino ou un 3-méthyl-[1,2,4]triazol-1-yle ;
B⁹¹ représente un H ou un méthyle ;
R² représente un fluor, R³ représente un H et R⁵ représente un brome, benzyle, benzoylamino, 4-carboxyphényle, benzylamino ou (pyridin-3-yl)méthylamino ; ou
R² représente un brome, R³ représente un H et R⁵ représente un méthyle ou un méthoxy ; ou
R² représente un fluor, R⁵ représente un méthyle et R³ représente un H, un quinoléin-3-yle, un benzylamino, un groupement phényle éventuellement substitué une fois par un acétylamino ou un aminosulfonyle, un pyridin-2-yle ou un groupement pyridin-3-yle éventuellement substitué une fois par un méthyle ; ou
R² représente un fluor, R³ représente un pyridin-3-yle et R⁵ représente un pyridin-3-yle ou un 4-carboxyphényle ; ou
R² représente un fluor, R³ représente un benzylamino et R⁵ représente un chlore ou un 2-méthoxy-pyrimidin-5-yle ; ou
R² représente un fluor, R³ représente un quinoléin-3-yle et R⁵ représente un quinoléin-3-yle ; ou
R² représente un fluor, R³ représente un 3-acétylaminophényle et R⁵ représente un pyridin-3-yle ou un 2-méthoxypyrimidin-5-yle ; ou
R² représente un fluor, R³ représente un 4-aminosulfonylphényle et R⁵ représente un chlore ; ou
R² représente un pyridin-4-yle, R³ représente un H et R⁵ représente un vinyle, un méthoxy ou un pyridin-4-yle ;
ou sel de celui-ci.

4. Composé de formule I selon l'une des revendications 1 à 3, où R¹ représente un (C₁-C₃)alkyle ;
ou sel de celui-ci.

5. Composé de formule I selon la revendication 4, où R¹ représente un éthyle ;
ou sel de celui-ci.

6. Composé de formule I selon l'une des revendications 1 à 5, où R² représente un H ;
ou sel de celui-ci.

7. Composé de formule I selon la revendication 6, où R⁵ représente un méthyle ;
ou sel de celui-ci.

8. Composé de formule I selon la revendication 6, où :
- R³ représente un groupement (B1) dans lequel soit chacun des radicaux B¹² et B¹⁴ représente un H et B¹³ représente un OH, halogène, acétyle, acétylamino, acétylaminométhyle, aminocarbonyle, diméthylaminocarbonyle, aminosulfonyle, (C₁-C₂)alkylsulfonyle, (C₁-C₂)alkoxy, (C₁-C₂)alkoxycarbonyle, cyano, amino-(C₁-C₂)alkyle ou hydroxy-(C₁-C₂)alkyle, soit chacun des radicaux B¹² et B¹³ représente un H et B¹⁴ représente un acétyle, acétylamino, acétylaminométhyle, aminocarbonyle, diméthylaminocarbonyle, aminosulfonyle, (C₁-C₂)alkylsulfonyle, cyanométhyle ou hydroxyméthyle ; ou
- R³ représente un groupement (B2) dans lequel X représente CH₂ ; ou
- R³ représente un groupement (B3) ; ou
- R³ représente un groupement (B4) ; ou
- R³ représente un groupement (B6) ; ou
- R³ représente un quinoléin-3-yle, imidazo[1,2-a]pyridin-6-yle, (thiazol-5-yl)carbonylamino, pyridin-3-ylcarbonylamino ou pyridin-4-ylcarbonylamino ;
ou sel de celui-ci.

9. Composé de formule I selon l'une des revendications 1 à 5, où R³ représente un H ;
ou sel de celui-ci.

10. Composé de formule I selon la revendication 9, où R⁵ représente un méthyle ;
ou sel de celui-ci.

11. Composé de formule I selon la revendication 10, où :
- R² représente un halogène ; ou
- R² représente un groupement (A1) ; ou
- R² représente un groupement (A3) ;
ou sel de celui-ci.

12. Composé de formule I selon la revendication 1, qui est sélectionné parmi les suivants :
- 1-(5-bromo-8-méthyl-isoquinoléin-3-yl)-3-éthyl-urée ;
- 1-éthyl-3-[5-(4-hydroxy-phényl)-8-méthyl-isoquinoléin-3-yl]-urée ;
- 1-éthyl-3-[5-(2-hydroxy-phényl)-8-méthyl-isoquinoléin-3-yl]-urée ;
- *N*-{3-[3-(3-éthyl-uréido)-8-méthyl-isoquinoléin-5-yl]-phényl}-acétamide ;
- 4-[3-(3-éthyl-uréido)-8-méthyl-isoquinoléin-5-yl]-*N,N*-diméthyl-benzamide ;
- 4-[3-(3-éthyl-uréido)-8-méthyl-isoquinoléin-5-yl]-*N*-méthyl-benzamide ;
- 1-éthyl-3-(5-imidazo[1,2-*a*]pyridin-6-yl-8-méthyl-isoquinoléin-3-yl)-urée ;
- 1-éthyl-3-[5-(6-hydroxyméthyl-pyridin-3-yl)-8-méthyl-isoquinoléin-3-yl]-urée ;
- 1-éthyl-3-[5-(5-fluoro-pyridin-3-yl)-8-méthyl-isoquinoléin-3-yl]-urée ;
- 1-éthyl-3-[8-méthyl-5-(5-méthyl-pyridin-3-yl)-isoquinoléin-3-yl]-urée ;
- 1-[5-((3-(aminométhyl)-phényl)-8-méthyl-isoquinoléin-3-yl]-3-éthyl-urée ;
- 1-éthyl-3-[5-(3-hydroxyméthyl-phényl)-8-méthyl-isoquinoléin-3-yl]-urée ;
- 3-[3-(3-éthyl-uréido)-8-méthyl-isoquinoléin-5-yl]-benzènesulfonamide ;
- 1-éthyl-3-(6-furan-3-yl-8-méthyl-isoquinoléin-3-yl)-urée ;
- 1-éthyl-3-[6-(4-hydroxyméthyl-phényl)-8-méthyl-isoquinoléin-3-yl]-urée ;
- 1-éthyl-3-(8-méthyl-6-pyridin-3-yl-isoquinoléin-3-yl)-urée ;
- *N*-{3-[3-(3-éthyl-uréido)-8-méthyl-isoquinoléin-6-yl]-benzyl}-acétamide ;
- *N*-{4-[3-(3-éthyl-uréido)-8-méthyl-isoquinoléin-6-yl]-phényl}-acétamide ;
- 1-éthyl-3-[6-(3-hydroxyméthyl-phényl)-8-méthyl-isoquinoléin-3-yl]-urée ;
- 1-éthyl-3-[6-(3-hydroxy-phényl)-8-méthyl-isoquinoléin-3-yl]-urée ;
- 1-éthyl-3-(8-méthyl-6-pyridin-4-yl-isoquinoléin-3-yl)-urée ;
- 3-[3-(3-éthyl-uréido)-8-méthyl-isoquinoléin-6-yl]-benzènesulfonamide ;
- 2-[3-(3-éthyl-uréido)-8-méthyl-isoquinoléin-6-yl]-*N,N*-diméthyl-benzamide ;
- 4-[3-(3-éthyl-uréido)-8-méthyl-isoquinoléin-6-yl]-*N,N*-diméthyl-benzamide ;
- 1-éthyl-3-(6-imidazo[1,2-*a*]pyridin-6-yl-8-méthyl-isoquinoléin-3-yl)-urée ;
- 1-[6-(3-(aminométhyl)-phényl)-8-méthyl-isoquinoléin-3-yl]-3-éthyl-urée ;
- 1-[5-(2-chloro-pyridin-4-yl)-8-méthyl-isoquinoléin-3-yl]-3-éthyl-urée ;
- 1-éthyl-3-[8-méthyl-6-(1*H*-pyrazol-4-yl)-isoquinoléin-3-yl]-urée ;
- 1-[6-(3-acétyl-phényl)-8-méthyl-isoquinoléin-3-yl]-3-éthyl-urée ;
- 4-[3-(3-éthyl-uréido)-8-méthyl-isoquinoléin-6-yl]-benzamide ;
- 3-[3-(3-éthyl-uréido)-8-méthyl-isoquinoléin-6-yl]-benzamide ;
- 1-(8-bromo-5-fluoro-isoquinoléin-3-yl)-3-éthyl-urée ;
- acide 4-[3-(3-éthyl-uréido)-5-fluoro-6-pyridin-3-yl-isoquinoléin-8-yl]-benzoïque ;
- 1-éthyl-3-[5-(4-hydroxyméthyl-phényl)-8-méthyl-isoquinoléin-3-yl]-urée ;
- 4-[3-(3-éthyl-uréido)-8-méthyl-isoquinoléin-5-yl]-benzènesulfonamide ;
- 1-éthyl-3-(8-méthyl-6-quinoléin-3-yl-isoquinoléin-3-yl)-urée ;
- *N*-{3-[3-(3-éthyl-uréido)-8-méthyl-isoquinoléin-6-yl]-phényl}-acétamide ;
- 4-[3-(3-éthyl-uréido)-8-méthyl-isoquinoléin-6-yl]-benzènesulfonamide ;
- 1-éthyl-3-[8-méthyl-6-(5-méthyl-pyridin-3-yl)-isoquinoléin-3-yl]-urée ;
- 1-éthyl-3-[8-méthyl-5-(2-méthyl-pyridin-4-yl)-isoquinoléin-3-yl]-urée ;
- 1-éthyl-3-[5-(2-fluoro-pyridin-4-yl)-8-méthyl-isoquinoléin-3-yl]-urée ;
- 1-éthyl-3-[5-(2-méthoxy-pyridin-4-yl)-8-méthyl-isoquinoléin-3-yl]-urée ;
- 1-éthyl-3-(5-phényl-isoquinoléin-3-yl)-urée ;
- 1-[5-(2,6-diméthyl-pyridin-4-yl)-8-méthyl-isoquinoléin-3-yl]-3-éthyl-urée ;
- 1-[5-(2-amino-pyridin-4-yl)-8-méthyl-isoquinoléin-3-yl]-3-éthyl-urée ;
- 1-(8-benzyl-5-fluoro-isoquinoléin-3-yl)-3-éthyl-urée ;
- 1-éthyl-3-(5-fluoro-8-méthyl-6-pyridin-3-yl-isoquinoléin-3-yl)-urée ;
- 1-éthyl-3-(5-fluoro-8-méthyl-6-pyridin-2-yl-isoquinoléin-3-yl)-urée ;
- 1-(6-benzylamino-8-chloro-5-fluoro-isoquinoléin-3-yl)-3-éthyl-urée ;
- 1-éthyl-3-(8-méthyl-5-pyridin-3-yl-isoquinoléin-3-yl)-urée ;
- *N*-[3-(3-éthyl-uréido)-5-fluoro-isoquinoléin-8-yl]-benzamide ,
- N-[3-(3-éthyl-uréido)-isoquinoléin-6-yl]-benzamide ,
- 1-éthyl-3-(5-fluoro-6,8-dipyridin-3-yl-isoquinoléin-3-yl)-urée ;
- 1-(6-amino-8-méthyl-isoquinoléin-3-yl)-3-éthyl-urée ;
- 1-éthyl-3-{5-fluoro-8-[(pyridin-3-ylméthyl)-amino]-isoquinoléin-3-yl}-urée;
- 1-(8-benzylamino-5-fluoro-isoquinoléin-3-yl)-3-éthyl-urée ;
- 1-(6-benzylamino-5-fluoro-8-méthyl-isoquinoléin-3-yl)-3-éthyl-urée ;
- 1-(6-benzylamino-8-méthyl-isoquinoléin-3-yl)-3-éthyl-urée ;
- 3-[3-(3-éthyl-uréido)-8-méthyl-isoquinoléin-5-yl]-propionamide ;
- 1-éthyl-3-(5-fluoro-8-méthyl-isoquinoléin-3-yl)-urée ;
- 1-éthyl-3-(8-méthyl-5-pyridin-4-yl-isoquinoléin-3-yl)-urée ;
- [3-(3-éthyl-uréido)-8-méthyl-isoquinoléin-6-yl]-amide de l'acide thiazol-5-carboxylique ;
- 1-éthyl-3-(5-éthylamino-8-méthyl-isoquinoléin-3-yl)-urée ;
- 1-éthyl-3-[8-méthyl-5-(4-méthyl-thiophén-3-yl)-isoquinoléin-3-yl]-urée ;
- ester méthylique de l'acide {4-[3-(3-éthyl-uréido)-8-méthyl-isoquinoléin-5-yl]-phényl}-carbamique ;
- 1-éthyl-3-[5-(2-fluoro-pyridin-3-yl)-8-méthyl-isoquinoléin-3-yl]-urée ;
- 1-éthyl-3-(8-méthyl-[5,5']biisoquinoléinyl-3-yl)-urée ;
- 1-éthyl-3-[5-(3-hydroxy-phényl)-8-méthyl-isoquinoléin-3-yl]-urée ;
- 1-éthyl-3-[5-(1*H*-indol-4-yl)-8-méthyl-isoquinoléin-3-yl]-urée ;
- *N*-[3-(3-éthyl-uréido)-isoquinoléin-8-yl]-benzamide ;
- 1-éthyl-3-{8-[(pyridin-3-ylméthyl)-amino]-isoquinoléin-3-yl}-urée;
- 1-(5,8-dipyridin-4-yl-isoquinoléin-3-yl)-3-éthyl-urée ;
- 1-(5-bromo-8-méthoxy-isoquinoléin-3-yl)-3-éthyl-urée ;
- acide 4-[3-(3-éthyl-uréido)-5-fluoro-isoquinoléin-8-yl]-benzoïque ;
- 1-éthyl-3-(8-méthyl-6-pyrimidin-5-yl-isoquinoléin-3-yl)-urée ;
- 1-éthyl-3-[6-(3-méthoxy-phényl)-8-méthyl-isoquinoléin-3-yl]-urée ;
- 1-(6-(benzo[1,3]dioxol-5-yl)-8-méthyl-isoquinoléin-3-yl)-3-éthyl-urée ;
- 1-éthyl-3-(6-furan-2-yl-8-méthyl-isoquinoléin-3-yl)-urée ;
- 1-éthyl-3-(8-méthyl-6-naphtalén-2-yl-isoquinoléin-3-yl)-urée ;
- 1-éthyl-3-[8-méthyl-6-(4-méthyl-thiophén-3-yl)-isoquinoléin-3-yl]-urée ;
- *N*-{4-[3-(3-éthyl-uréido)-8-méthyl-isoquinoléin-6-yl]-benzyl}-acétamide ;
- 1-[6-(2-chloro-pyridin-3-yl)-8-méthyl-isoquinoléin-3-yl]-3-éthyl-urée ;
- 1-[6-(2,3-dihydro-benzofuran-5-yl)-8-méthyl-isoquinoléin-3-yl]-3-éthyl-urée ;
- ester éthylique de l'acide 3-[3-(3-éthyl-uréido)-8-méthyl-isoquinoléin-6-yl]-benzoïque ;
- ester éthylique de l'acide 2-[3-(3-éthyl-uréido)-8-méthyl-isoquinoléin-6-yl]-benzoïque ;
- *N*-{2-[3-(3-éthyl-uréido)-8-méthyl-isoquinoléin-6-yl]-phényl}-acétamide ;
- ester méthylique de l'acide 5-[3-(3-éthyl-uréido)-8-méthyl-isoquinoléin-6-yl]-nicotinique ;
- 1-éthyl-3-[6-(3-fluoro-phényl)-8-méthyl-isoquinoléin-3-yl]-urée ;
- 1-éthyl-3-[6-(6-méthoxy-pyridin-3-yl)-8-méthyl-isoquinoléin-3-yl]-urée ;
- 1-éthyl-3-[6-(1*H*-indol-4-yl)-8-méthyl-isoquinoléin-3-yl]-urée ;
- 1-éthyl-3-(8'-méthyl-[4,6']biisoquinoléinyl-3'-yl)-urée ;
- 1-[6-(4-(cyanométhyl)-phényl)-8-méthyl-isoquinoléin-3-yl]-3-éthyl-urée ;
- 1-éthyl-3-(8-méthyl-6-thiophén-3-yl-isoquinoléin-3-yl)-urée ;
- 1-éthyl-3-[6-(4-méthanesulfonyl-phényl)-8-méthyl-isoquinoléin-3-yl]-urée ;
- 1-éthyl-3-[6-(4-isopropyl-pyrimidin-5-yl)-8-méthyl-isoquinoléin-3-yl]-urée ;
- 1-éthyl-3-[8-méthyl-6-(5-méthylsulfanyl-pyridin-3-yl)-isoquinoléin-3-yl]-urée ;
- 1-éthyl-3-[6-(3-fluoro-pyridin-4-yl)-8-méthyl-isoquinoléin-3-yl]-urée ;
- 1-[6-(3-chloro-pyridin-4-yl)-8-méthyl-isoquinoléin-3-yl]-3-éthyl-urée ;
- 1-éthyl-3-[6-(6-fluoro-pyridin-3-yl)-8-méthyl-isoquinoléin-3-yl]-urée ;
- 1-éthyl-3-[6-(2-fluoro-pyridin-3-yl)-8-méthyl-isoquinoléin-3-yl]-urée ;
- 1-éthyl-3-[6-(6-hydroxyméthyl-pyridin-3-yl)-8-méthyl-isoquinoléin-3-yl]-urée ;
- 1-éthyl-3-[6-(5-fluoro-pyridin-3-yl)-8-méthyl-isoquinoléin-3-yl]-urée ;
- 1-éthyl-3-[6-(5-méthanesulfonyl-pyridin-3-yl)-8-méthyl-isoquinoléin-3-yl]-urée ;
- 1-éthyl-3-(8'-méthyl-[5,6']biisoquinoléinyl-3'-yl)-urée ;
- 1-éthyl-3-(8-méthyl-6-0-tolyl-isoquinoléin-3-yl)-urée ;
- *N*-[3-(3-éthyl-uréido)-8-méthyl-isoquinoléin-6-yl]-benzamide ;
- 1-[6-(3-cyano-phényl)-8-méthyl-isoquinoléin-3-yl]-3-éthyl-urée ;
- 1-[6-(4-acétyl-phényl)-8-méthyl-isoquinoléin-3-yl]-3-éthyl-urée ;
- 1-éthyl-3-[6-(2-hydroxy-phényl)-8-méthyl-isoquinoléin-3-yl]-urée ;
- 1-[6-benzylamino-5-fluoro-8-(2-méthoxy-pyrimidin-5-yl)-isoquinoléin-3-yl]-3-éthyl-urée ;
- 4-[8-chloro-3-(3-éthyl-uréido)-5-fluoro-isoquinoléin-6-yl]-benzènesulfonamide ;
- 1-éthyl-3-(5-fluoro-6,8-di-quinoléin-3-yl-isoquinoléin-3-yl)-urée ;
- *N*-{3-[3-(3-éthyl-uréido)-5-fluoro-8-pyridin-3-yl-isoquinoléin-6-yl]-phényl}-acétamide ;
- *N*-{3-[3-(3-éthyl-uréido)-5-fluoro-8-(2-méthoxy-pyrimidin-5-yl)-isoquinoléin-6-yl]-phényl}-acétamide ;
- 1-éthyl-3-(8-méthyl-6-propylamino-isoquinoléin-3-yl)-urée ;
- *N*-[3-(3-éthyl-uréido)-8-méthyl-isoquinoléin-6-yl]-isonicotinamide ;
- *N*-[3-(3-éthyl-uréido)-8-méthyl-isoquinoléin-6-yl]-4-méthanesulfonyl-benzamide ;
- 3-diméthylamino-*N*-[3-(3-éthyl-uréido)-8-méthyl-isoquinoléin-6-yl]-benzamide ;
- *N*-[3-(3-éthyl-uréido)-8-méthyl-isoquinoléin-6-yl]-nicotinamide ;
- *N*-[3-(3-éthyl-uréido)-8-méthyl-isoquinoléin-6-yl]-4-méthoxy-benzamide ;
- 4-diméthylamino-*N*-[3-(3-éthyl-uréido)-8-méthyl-isoquinoléin-6-yl]-benzamide ;
- 1-éthyl-3-{8-méthyl-6-[(pyridin-4-ylméthyl)-amino]-isoquinoléin-3-yl}-urée;
- acide 4-[3-(3-éthyl-uréido)-isoquinoléin-8-yl]-benzoïque ;
- acide 4-[3-(3-éthyl-uréido)-isoquinoléin-5-yl]-benzoïque ;
- 1-éthyl-3-(8-méthoxy-5-pyridin-4-yl-isoquinoléin-3-yl)-urée ;
- ester méthylique de l'acide 3-{[3-(3-éthyl-uréido)-8-méthyl-isoquinoléin-6-ylamino]-méthyl}-benzoïque ;
- acide 4-{[3-(3-éthyl-uréido)-8-méthyl-isoquinoléin-6-ylamino]-méthyl}-benzoïque ;
- acide 3-{[3-(3-éthyl-uréido)-8-méthyl-isoquinoléin-6-ylamino]-méthyl}-benzoïque ;
- 1-éthyl-3-{6-[3-(2-hydroxy-éthoxy)-benzylamino]-8-méthyl-isoquinoléin-3-yl}-urée;
- 1-éthyl-3-{8-méthyl-6-[(4-méthyl-3,4-dihydro-2*H*-benzo[1,4]oxazin-7-ylméthyl)-amino]-isoquinoléin-3-yl}-urée ;
- 1-éthyl-3-{8-méthyl-6-[4-(3-méthyl-[1,2,4]triazol-1-yl)-benzylamino]-isoquinoléin-3-yl}-urée ;
- 1-[6-(2-benzyloxy-éthylamino)-8-méthyl-isoquinoléin-3-yl]-3-éthyl-urée ;
- 1-{6-[(cyclopropylméthyl)-amino]-8-méthyl-isoquinoléin-3-yl}-3-éthyl-urée ;
- 1-éthyl-3-{6-[(1*H*-indol-6-ylméthyl)-amino]-8-méthyl-isoquinoléin-3-yl}-urée;
- 1-éthyl-3-{8-méthyl-6-[((1-méthyl-1*H*-indol-6-yl)méthyl)-amino]-isoquinoléin-3-yl}-urée ;
- 1-éthyl-3-(6-isobutylamino-8-méthyl-isoquinoléin-3-yl)-urée ;
- *N*-(4-{[3-(3-éthyl-uréido)-8-méthyl-isoquinoléin-6-ylamino]-méthyl}-phényl)-acétamide ;
- 1-(2-fluoro-éthyl)-3-(8-méthyl-5-pyridin-4-yl-isoquinoléin-3-yl)-urée ;
- 1-cyclopropyl-3-(8-méthyl-5-pyridin-4-yl-isoquinoléin-3-yl)-urée ;
- 1-éthyl-3-[5-(pyridin-4-yl)-8-vinylisoquinoléin-3-yl]-urée ;
- 1-éthyl-3-[5-fluoro-8-méthyl-6-(5-méthylpyridin-3-yl)isoquinoléin-3-yl]-urée ;
- *N*-{3-[3-(3-éthyl-uréido)-5-fluoro-8-méthyl-isoquinoléin-6-yl]-phényl}-acétamide ;
- 1-éthyl-3-[5-fluoro-8-méthyl-6-(quinoléin-3-yl)-isoquinoléin-3-yl]-urée ;
- 4-[3-(3-éthyl-uréido)-5-fluoro-8-méthyl-isoquinoléin-6-yl]-benzènesulfonamide ;
- 1-éthyl-3-[8-(pyridin-3-ylamino)-isoquinoléin-3-yl]-urée ;
- 1-éthyl-3-[8-(pyridin-2-ylamino)-isoquinoléin-3-yl]-urée ;
- 1-éthyl-3-[8-(pyridin-3-yloxy)-isoquinoléin-3-yl]-urée ;
- 1-éthyl-3-[8-(pyrazin-2-ylamino)-isoquinoléin-3-yl]-urée ;
- 1-éthyl-3-[8-(pyrimidin-5-ylamino)-isoquinoléin-3-yl]-urée ;
- 1-éthyl-3-[8-(imidazo[1,2-a]pyridin-7-ylamino)-isoquinoléin-3-yl]-urée ;
- 1-éthyl-3-[8-(pyrimidin-4-ylamino)-isoquinoléin-3-yl]-urée ;
- 1-éthyl-3-[8-(pyrimidin-2-ylamino)-isoquinoléin-3-yl]-urée ;
- 1-éthyl-3-[8-(pyridin-4-ylamino)-isoquinoléin-3-yl]-urée ;
- 1-éthyl-3-[8-(5-méthyl-[1,3,4]oxadiazol-2-ylamino)-isoquinoléin-3-yl]-urée ;
- 1-éthyl-3-[8-(6-méthyl-pyridin-2-ylamino)-isoquinoléin-3-yl]-urée ;
- 6-[3-(3-éthyl-uréido)-isoquinoléin-8-ylamino]-nicotinamide ;
- 1-éthyl-3-[8-(3-fluoro-pyridin-4-ylamino)-isoquinoléin-3-yl]-urée ;
- 1-éthyl-3-[8-(4-fluoro-pyridin-2-ylamino)-isoquinoléin-3-yl]-urée ;
- 1-éthyl-3-[8-(5-fluoro-pyridin-3-ylamino)-isoquinoléin-3-yl]-urée ;
- 1-éthyl-3-[8-(4-méthoxy-pyridin-2-ylamino)-isoquinoléin-3-yl]-urée ;
- *N*-{5-[3-(3-éthyl-uréido)-isoquinoléin-8-ylamino]-pyridin-2-yl}-acétamide ;
- 1-éthyl-3-[8-(4-méthyl-pyridin-2-ylamino)-isoquinoléin-3-yl]-urée ;
- 1-éthyl-3-[8-(5-méthyl-pyridin-2-ylamino)-isoquinoléin-3-yl]-urée ;
- 1-éthyl-3-[8-(6-méthyl-pyridin-3-ylamino)-isoquinoléin-3-yl]-urée ;
- ester méthylique de l'acide 6-[3-(3-éthyl-uréido)-isoquinoléin-8-ylamino]-nicotinique ;
- ester méthylique de l'acide 6-[3-(3-éthyl-uréido)-isoquinoléin-8-ylamino]-pyridin-2-carboxylique ;
- 1-éthyl-3-[8-(3-méthyl-pyridin-2-ylamino)-isoquinoléin-3-yl]-urée ;
- 1-éthyl-3-[8-(3-méthoxy-phénylamino)-isoquinoléin-3-yl]-urée ;
- 1-éthyl-3-(8-phénylamino-isoquinoléin-3-yl)-urée ;
- 1-éthyl-3-[8-(3-hydroxy-phénylamino)-isoquinoléin-3-yl]-urée ;
- 3-[3-(3-éthyl-uréido)-isoquinoléin-8-ylamino]-benzènesulfonamide ;
- *N*-{3-[3-(3-éthyl-uréido)-isoquinoléin-8-ylamino]-phényl}-acétamide ;
- *N*-{4-[3-(3-éthyl-uréido)-isoquinoléin-8-ylamino]-phényl}-acétamide ;
- 1-[8-(4-cyano-phénylamino)-isoquinoléin-3-yl]-3-éthyl-urée ;
- 1-éthyl-3-[8-(4-hydroxy-phénylamino)-isoquinoléin-3-yl]-urée ;
- ester méthylique de l'acide 3-[3-(3-éthyl-uréido)-isoquinoléin-8-ylamino]-benzoïque ;
- 1-éthyl-3-[8-(5-méthoxy-pyridin-3-ylamino)-isoquinoléin-3-yl]-urée ;
- 1-éthyl-3-[8-(6-méthoxy-pyridin-2-ylamino)-isoquinoléin-3-yl]-urée ;
- ester méthylique de l'acide 4-[3-(3-éthyl-uréido)-isoquinoléin-8-ylamino]-benzoïque ;
- 1-éthyl-3-[8-(2-méthoxy-pyridin-4-ylamino)-isoquinoléin-3-yl]-urée ;
- 1-éthyl-3-[8-(5-méthoxy-pyridin-2-ylamino)-isoquinoléin-3-yl]-urée ;
- [3-(3-éthyl-uréido)-isoquinoléin-8-yl]-amide de l'acide 1*H*-pyrrol-2-carboxylique ;
- [3-(3-éthyl-uréido)-isoquinoléin-8-yl]-amide de l'acide 3*H*-[1,2,3]triazol-4-carboxylique ;
- 1-éthyl-3-{6-[(1*H*-indazol-6-ylméthyl)-amino]-8-méthyl-isoquinoléin-3-yl}-urée ;
- 1-éthyl-3-{8-méthyl-6-[(2-morpholin-4-yl-pyridin-4-ylméthyl)-amino]-isoquinoléin-3-yl}-urée ;
- 2-(4-{[3-(3-éthyl-uréido)-8-méthyl-isoquinoléin-6-ylamino]-méthyl}-phénoxy)-acétamide ;
- 1-éthyl-3-[8-méthyl-6-(3-[1,2,4]triazol-1-yl-benzylamino)-isoquinoléin-3-yl]-urée ;
- 1-{6-[(3-((1*H*-1,2,4-triazol-1-yl)méthyl)benzyl)amino]-8-méthylisoquinoléin-3-yl}-3-éthylurée ;
- 4-{[3-(3-éthyl-uréido)-8-méthyl-isoquinoléin-6-ylamino]-méthyl}-*N*-méthyl-benzènesulfonamide ;
- 1-éthyl-3-[8-(pyridin-3-ylaminométhyl)-isoquinoléin-3-yl]-urée ;
- 1-{8-[(2,5-diméthyl-2*H*-pyrazol-3-ylamino)-méthyl]-isoquinoléin-3-yl}-3-éthyl-urée ;
- 1-éthyl-3-{8-[(3-méthyl-isothiazol-5-ylamino)-méthyl]-isoquinoléin-3yl}-urée ;
- ester méthylique de l'acide *N*-[3-(3-éthyl-uréido)-8-méthyl-isoquinoléin-6-yl]-téréphtalamique ;
- ester méthylique de l'acide *N*-[3-(3-éthyl-uréido)-8-méthyl-isoquinoléin-6-yl]-isophtalamique ;
- *N*-[3-(3-éthyl-uréido)-8-méthyl-isoquinoléin-6-yl]-2-méthoxy-acétamide ;
- 1-éthyl-3-{6-[(6-méthoxy-pyridin-3-ylméthyl)-amino]-8-méthyl-isoquinoléin-3-yl}-urée;
- 1-éthyl-3-[6-(3-méthoxy-benzylamino)-8-méthyl-isoquinoléin-3-yl]-urée ;
- acide 3-[3-(3-éthyl-uréido)-isoquinoléin-8-ylamino]-benzoïque ;
- 3-[3-(3-éthyl-uréido)-isoquinoléin-8-ylamino]-*N,N*-diméthyl-benzamide ;
- 3-[3-(3-éthyl-uréido)-isoquinoléin-8-ylamino]-*N*-méthyl-benzamide ;
- *N*-benzyl-3-[3-(3-éthyl-uréido)-isoquinoléin-8-ylamino]-benzamide ;
- acide 4-[3-(3-éthyl-uréido)-isoquinoléin-8-ylamino]-benzoïque ;
- 4-[3-(3-éthyl-uréido)-isoquinoléin-8-ylamino]-*N,N*-diméthyl-benzamide ;
- 4-[3-(3-éthyl-uréido)-isoquinoléin-8-ylamino]-*N*-méthyl-benzamide ;
- acide {4-[3-(3-éthyl-uréido)-isoquinoléin-8-ylamino]-phényl}-acétique;
- 2-{4-[3-(3-éthyl-uréido)-isoquinoléin-8-ylamino]-phényl}-*N,N*-diméthyl-acétamide ;
- 2-{4-[3-(3-éthyl-uréido)-isoquinoléin-8-ylamino]-phényl}-*N*-méthyl-acétamide ;
- *N*-benzyl-2-{4-[3-(3-éthyl-uréido)-isoquinoléin-8-ylamino]-phényl}-acétamide ;
- acide {3-[3-(3-éthyl-uréido)-isoquinoléin-8-ylamino]-phényl}-acétique;
- *N*-benzyl-2-{3-[3-(3-éthyl-uréido)-isoquinoléin-8-ylamino]-phényl}-acétamide ;
- 2-{3-[3-(3-éthyl-uréido)-isoquinoléin-8-ylamino]-phényl}-*N,N*-diméthyl-acétamide ;
- ester méthylique de l'acide 4-[3-(3-éthyl-uréido)-isoquinoléin-8-ylméthoxy]-nicotinique ;
- 1-éthyl-3-[8-(3-hydroxy-phénoxyméthyl)-isoquinoléin-3-yl]-urée ;
- 1-éthyl-3-[8-(6-méthoxy-pyridin-3-ylamino)-isoquinoléin-3-yl]-urée ;
- 1-éthyl-3-(5-fluoro-8-pyridin-3-yl-isoquinoléin-3-yl)-urée ;
- 1-éthyl-3-(8-méthyl-6-vinyl-isoquinoléin-3-yl)-urée ;
- 1-éthyl-3-[8-(6-méthoxy-pyridin-2-ylamino)-5-pyridin-4-yl-isoquinoléin-3-yl]-urée ;
- 1-éthyl-3-[8-(6-méthyl-pyridin-3-ylamino)-5-pyridin-4-yl-isoquinoléin-3-yl]-urée ;
- 1-éthyl-3-[8-(6-méthyl-pyridin-2-ylamino)-5-pyridin-4-yl-isoquinoléin-3-yl]-urée ;
- ester méthylique de l'acide 3-[3-(3-éthyl-uréido)-5-pyridin-4-yl-isoquinoléin-8-ylamino]-benzoïque ;
- 1-éthyl-3-[5-(4-hydroxyméthyl-phényl)-8-(pyridin-3-yloxy)-isoquinoléin-3-yl]-urée ;
- 1-éthyl-3-[5-pyridin-4-yl-8-(pyridin-3-yloxy)-isoquinoléin-3-yl]-urée ;
- 1-éthyl-3-[5-pyridin-3-yl-8-(pyridin-3-yloxy)-isoquinoléin-3-yl]-urée ;
- 1-éthyl-3-[5-pyridin-3-yl-8-(pyridin-3-ylamino)-isoquinoléin-3-yl]-urée ;
- 1-éthyl-3-[5-(2-méthoxy-pyridin-4-yl)-8-(pyridin-3-ylamino)-isoquinoléin-3-yl]-urée ;
- 1-éthyl-3-[5-(4-hydroxyméthyl-phényl)-8-(pyridin-3-ylamino)-isoquinoléin-3-yl]-urée ;
- 1-éthyl-3-[5-pyridin-4-yl-8-(pyridin-3-ylamino)-isoquinoléin-3-yl]-urée ;
- 1-éthyl-3-[5-(4-hydroxy-phényl)-8-(pyridin-3-ylamino)-isoquinoléin-3-yl]-urée ;
- 1-éthyl-3-[5-(2-méthyl-pyridin-4-yl)-8-(pyridin-3-ylamino)-isoquinoléin-3-yl]-urée ;
- 4-[3-(3-éthyl-uréido)-8-(pyridin-3-ylamino)-isoquinoléin-5-yl]-benzènesulfonamide ;
- 1-[5-(2,6-diméthyl-pyridin-4-yl)-8-(pyridin-3-ylamino)-isoquinoléin-3-yl]-3-éthyl-urée ;
- 1-(5-(3-(aminométhyl)phényl)-8-(pyridin-3-ylamino)isoquinoléin-3-yl)-3-éthylurée ;
- 1-[5-(2-amino-pyridin-4-yl)-8-(pyridin-3-ylamino)-isoquinoléin-3-yl]-3-éthyl-urée ;
- *N*-{3-[3-(3-éthyl-uréido)-5-fluoro-8-(pyridin-3-ylamino)-isoquinoléin-6-yl]-phényl}-acétamide ;
- 1-éthyl-3-[5-fluoro-6-(5-méthyl-pyridin-3-yl)-8-(pyridin-3-ylamino)-isoquinoléin-3-yl]-urée ;
- *N*-{3-[3-(3-éthyl-uréido)-8-(pyridin-3-ylamino)-isoquinoléin-6-yl]-phényl}-acétamide ;
- 1-éthyl-3-[6-(5-méthyl-pyridin-3-yl)-8-(pyridin-3-ylamino)-isoquinoléin-3-yl]-urée ;
- 1-éthyl-3-[6-propylamino-8-(pyridin-3-ylamino)-isoquinoléin-3-yl]-urée ;
- 1-[6-benzylamino-8-(pyridin-3-ylamino)-isoquinoléin-3-yl]-3-éthyl-urée ;
- 1-[6-benzylamino-5-fluoro-8-(pyridin-3-ylamino)-isoquinoléin-3-yl]-3-éthyl-urée ;
- 3-diméthylamino-*N-*[3-(3-éthyl-uréido)-8-(pyridin-3-ylamino)-isoquinoléin-6-yl]-benzamide ;
- 5-[3-(3-éthyl-uréido)-isoquinoléin-8-ylamino]-2-méthoxy-benzamide ;
- 5-[3-(3-éthyl-uréido)-isoquinoléin-8-ylamino]-2-méthoxy-*N*-méthyl-benzamide ;
- 5-[3-(3-éthyl-uréido)-isoquinoléin-8-ylamino]-2-méthoxy-*N,N*-diméthyl-benzamide ;
- 1-éthyl-3-[5-(2-méthyl-pyridin-4-yl)-8-(6-méthyl-pyridin-3-ylamino)-isoquinoléin-3-yl]-urée ;
- acide 3-[3-(3-éthyl-uréido)-5-(2-méthyl-pyridin-4-yl)-isoquinoléin-8-ylamino]-benzoïque ;
- 3-[3-(3-éthyl-uréido)-5-(2-méthyl-pyridin-4-yl)-isoquinoléin-8-ylamino]-*N*-méthyl-benzamide ;
- 3-[3-(3-éthyl-uréido)-5-(2-méthyl-pyridin-4-yl)-isoquinoléin-8-ylamino]-*N,N*-diméthyl-benzamide ;
- acide 3-[3-(3-éthyl-uréido)-5-(4-hydroxyméthyl-phényl)-isoquinoléin-8-ylamino]-benzoïque ;
- 3-[3-(3-éthyl-uréido)-5-(4-hydroxyméthyl-phényl)-isoquinoléin-8-ylamino]-N-méthyl-benzamide ;
- 3-[3-(3-éthyl-uréido)-5-(4-hydroxyméthyl-phényl)-isoquinoléin-8-ylamino]-*N,N*-diméthyl-benzamide ;
- 4-[3-(3-éthyl-uréido)-isoquinoléin-8-ylamino]-benzamide ;
- 2-{4-[3-(3-éthyl-uréido)-isoquinoléin-8-ylamino]-phényl}-acétamide ;
- 3-[3-(3-éthyl-uréido)-isoquinoléin-8-ylamino]-benzamide ;
- 2-{3-[3-(3-éthyl-uréido)-isoquinoléin-8-ylamino]-phényl}-acétamide ;
- 1-éthyl-3-[8-(6-méthyl-pyridin-3-yloxyméthyl)-isoquinoléin-3-yl]-urée ;
- 1-éthyl-3-[8-(5-méthyl-pyridin-2-yloxyméthyl)-isoquinoléin-3-yl]-urée ;
- ester méthylique de l'acide 2-[3-(3-éthyl-uréido)-isoquinoléin-8-ylméthoxy]-benzoïque ;
- 1-éthyl-3-[8-(2-méthyl-pyridin-3-yloxyméthyl)-isoquinoléin-3-yl]-urée ;
- 1-[8-(3-amino-phénoxyméthyl)-isoquinoléin-3-yl]-3-éthyl-urée ;
- 1-éthyl-3-[8-(pyridin-2-yloxyméthyl)-isoquinoléin-3-yl]-urée ;
- 1-éthyl-3-[8-(pyridin-4-yloxyméthyl)-isoquinoléin-3-yl]-urée ;
- 1-éthyl-3-[8-(pyridin-2-ylaminométhyl)-isoquinoléin-3-yl]-urée ;
- ester méthylique de l'acide 5-[3-(3-éthyl-uréido)-isoquinoléin-8-ylamino]-2-méthyl-benzoïque ;
- acide 5-[3-(3-éthyl-uréido)-isoquinoléin-8-ylamino]-2-méthyl-benzoïque ;
- ester méthylique de l'acide 4-[3-(3-éthyl-uréido)-isoquinoléin-8-ylamino]-2-méthyl-benzoïque ;
- acide 4-[3-(3-éthyl-uréido)-isoquinoléin-8-ylamino]-2-méthyl-benzoïque ;
- 1-[5,8-*bis*-(pyridin-3-ylamino)-isoquinoléin-3-yl]-3-éthyl-urée ;
- 1-éthyl-3-(8-méthyl-5-méthylaminométhyl-isoquinoléin-3-yl)-urée ;
- 1-éthyl-3-{5-[(méthylamino)méthyl]-8-(pyridin-3-ylamino)-isoquinoléin-3-yl}-urée ;
- 1-éthyl-3-{8-[(6-méthoxy-pyridin)-2-ylamino]-5-[(méthylamino)méthyl]-isoquinoléin-3-yl}-urée ;
- acide 3-{[3-(3-éthyl-uréido)-5-[(méthylamino)méthyl]-isoquinoléin-8-yl]amino}-benzoïque ;
- 1-{5-[(diméthylamino)méthyl]-8-[(6-méthoxy-pyridin-2-yl)amino]-isoquinoléin-3-yl}-3-éthyl-urée ;
- 1-éthyl-3-[5-morpholin-4-yl-8-(pyridin-3-ylamino)-isoquinoléin-3-yl]-urée ;
- 1-éthyl-3-{8-[(6-méthoxy-pyridin-2-yl)amino]-5-(morpholin-4-ylméthyl)-isoquinoléin-3-yl}-urée ;
- 1-éthyl-3-[8-(6-méthoxy-pyridin-2-ylamino)-5-(4-méthyl-pipérazin-1-ylméthyl)-isoquinoléin-3-yl]-urée ;
- 1-éthyl-3-[5-(4-méthyl-pipérazin-1-yl)-8-(pyridin-3-ylamino)-isoquinoléin-3-yl]-urée ;
- 1-éthyl-3-(8-méthyl-5-pyrimidin-4-yl-isoquinoléin-3-yl)-urée ;
- 1-éthyl-3-(8-méthyl-5-pyridazin-4-yl-isoquinoléin-3-yl)-urée ;
- 1-éthyl-3-[5-(1*H*-imidazol-4-yl)-8-méthyl-isoquinoléin-3-yl]-urée ;
- 1-éthyl-3-[8-méthyl-5-(1*H*-pyrazol-4-yl)-isoquinoléin-3-yl]-urée ;
- 1-éthyl-3-(8-méthyl-5-pyridazin-3-yl-isoquinoléin-3-yl)-urée ;
- 1-éthyl-3-[8-méthyl-5-(1-méthyl-1*H*-imidazol-4-yl)-isoquinoléin-3-yl]-urée ;
- 1-éthyl-3-[8-méthyl-5-(1-méthyl-1*H*-pyrazol-4-yl)-isoquinoléin-3-yl]-urée ;
- 1-éthyl-3-(8-méthyl-5-thiazol-4-yl-isoquinoléin-3-yl)-urée ;
- 1-(8-chloro-5-pyrimidin-4-yl-isoquinoléin-3-yl)-3-éthyl-urée ;
- 1-éthyl-3-[8-(6-méthyl-pyridin-3-ylamino)-5-pyrimidin-4-yl-isoquinoléin-3-yl]-urée ;
- 1-[8-chloro-5-(1*H*-imidazol-4-yl)-isoquinoléin-3-yl]-3-éthyl-urée ;
- 1-(8-chloro-5-pyrimidin-4-yl-isoquinoléin-3-yl)-3-éthyl-urée ;
- 1-éthyl-3-[8-(6-méthoxy-pyridin-2-ylamino)-5-thiazol-4-yl-isoquinoléin-3-yl]-urée ;
- 1-éthyl-3-[8-(2-méthoxy-pyridin-3-ylamino)-isoquinoléin-3-yl]-urée ;
- 1-éthyl-3-[8-(4-hydroxyméthyl-pyrazol-1-ylméthyl)-isoquinoléin-3-yl]-urée ;
- 1-éthyl-3-[8-(5-hydroxyméthyl-imidazol-1-ylméthyl)-isoquinoléin-3-yl]-urée ;
- 1-éthyl-3-[8-(4-hydroxyméthyl-imidazol-1-ylméthyl)-isoquinoléin-3-yl]-urée ;
- acide 1-[3-(3-éthyl-uréido)-isoquinoléin-8-ylméthyl]-1*H*-imidazol-2-carboxylique ;
- 1-éthyl-3-(8-pyrazol-1-ylméthyl-isoquinoléin-3-yl)-urée ;
- 1-[8-(2,4-diméthyl-imidazol-1-ylméthyl)-isoquinoléin-3-yl]-3-éthyl-urée ;
- 1-[8-(2,5-diméthyl-imidazol-1-ylméthyl)-isoquinoléin-3-yl]-3-éthyl-urée ;
- 1-éthyl-3-[8-(2-méthyl-imidazol-1-ylméthyl)-isoquinoléin-3-yl]-urée ;
- 1-éthyl-3-(8-[1,2,3]triazol-1-ylméthyl-isoquinoléin-3-yl)-urée ;
- 1-éthyl-3-(8-imidazol-1-ylméthyl-isoquinoléin-3-yl)-urée ;
- 1-éthyl-3-[8-(4-méthyl-pyrazol-1-ylméthyl)-isoquinoléin-3-yl]-urée ;
- 1-éthyl-3-{8-[(5-méthyl-isoxazol-3-ylamino)-méthyl]-isoquinoléin-3-yl}-urée;
- 1-éthyl-3-{8-[(6-méthoxy-pyridin-3-ylamino)-méthyl]-isoquinoléin-3-yl}-urée;
- 1-éthyl-3-{8-[(2-méthyl-2H-pyrazol-3-ylamino)-méthyl]-isoquinoléin-3-yl}-urée ;
- 1-éthyl-3-[8-(pyrimidin-2-ylaminométhyl)-isoquinoléin-3-yl]-urée ;
ou sel de celui-ci.

13. Composé de formule I selon l'une des revendications 1 à 12, ou sel pharmaceutiquement acceptable de celui-ci, pour une utilisation en tant que médicament.

14. Composition pharmaceutique contenant, comme principe actif, un composé de formule I selon l'une des revendications 1 à 12 ou un sel pharmaceutiquement acceptable de celui-ci et au moins un excipient thérapeutiquement inerte.

15. Composé de formule I selon l'une des revendications 1 à 12, ou sel pharmaceutiquement acceptable de celui-ci, pour une utilisation dans la prévention ou le traitement d'une infection bactérienne.
